(19) Europäisches Patentamt
European Patent Office
Office européen des brevets

(11) EP 4 640 276 A2

(12) EUROPEAN PATENT APPLICATION

(43) Date of publication:
**29.10.2025 Bulletin 2025/44**

(21) Application number: **25186580.4**

(22) Date of filing: **22.05.2020**

(51) International Patent Classification (IPC):
***A61P 25/02*** *(2006.01)*

(52) Cooperative Patent Classification (CPC):
**A61K 31/506; A61K 45/06; A61P 25/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.05.2019 US 201962851897 P**

(62) Document number(s) of the earlier application(s) in accordance with Art. 76 EPC:
**20810334.1 / 3 972 593**

(71) Applicant: **Board Of Regents, The University Of Texas System**
**Austin, TX 78701 (US)**

(72) Inventors:
- **PRICE, Theodore, J.**
  **Dallas, 75254 (US)**
- **SHIERS, Stephanie, I.**
  **Plano, 75074 (US)**

(74) Representative: **Müller-Boré & Partner Patentanwälte PartG mbB**
**Friedenheimer Brücke 21**
**80639 München (DE)**

Remarks:
•The complete document including Reference Table(s) and the Sequence Listing(s) can be downloaded from the EPO website
•This application was filed on 01-07-2025 as a divisional application to the application mentioned under INID code 62.
•Claims filed after the date of receipt of the application (Rule 68(4) EPC).

# (54) INHIBITOR OF MNK FOR THE TREATMENT OF NEUROPATHIC PAIN

(57) The disclosure provides methods and compositions for inhibiting pain and comorbid cognitive defecits by administering a MNK inhibitor to a subject in need thereof. Also provided are methods of using the inhibitor in combination with other pain therapies.

EP 4 640 276 A2

**Description**

## FEDERAL FUNDING STATEMENT

This invention was made with government support under R01 NS065926 awarded by the National Institutes of Health. The government has certain rights in the invention.

## PRIORITY CLAIM

This application claims benefit of priority to U.S. Provisional Application Serial No. 62/851,897, filed May 23, 2019, the entire contents of which are hereby incorporated by reference.

## BACKGROUND

### 1. Field

This disclosure relates to the fields of medicine and cell biology. More specifically, it relates to the use of the MNK inhibitor eFT508 to treat pain, either as a stand-alone therapeutic option, or in combination with other pain therapies.

### 2. Related Art

A devastating health problem in the United States is the inadequate treatment of pain. One third of all Americans suffer from some form of chronic pain, and a third of these have pain, which is resistant to current medical therapy. The economic impact of pain is equally large at approximately $100 billion annually. Opioid/narcotic analgesics, typified by morphine, are the most effective treatments for acute and chronic severe pain. However, their clinical utility is often hampered by the development of analgesic tolerance which requires escalating doses to achieve equivalent pain relief. This complex pathophysiological cycle represents a critical barrier to the quality of life of these patients due to the resulting drug-induced sedation, reduced physical activity, constipation, respiratory depression, high potential for addiction, and other side-effects. Accordingly, there is major interest in new approaches to treat chronic pain without engendering tolerance or unacceptable side-effects.

There are many different causes of neuropathic pain, including trauma to peripheral nerves, metabolic disease, and toxicities produced by drug treatment, all of which are growing indications. For example, as advances in cancer treatment efficacy have transformed treatment of this diverse disease, incidence and duration of chemotherapy-induced peripheral neuropathy (CIPN) have increased. CIPN is the primary dose-limiting side effect of cancer treatment, and no drugs are approved to treat this form of neuropathic pain. Peripheral nerve injury (PNI) is another form of neuropathic pain, often accompanied by severe disruptions in executive functions that depend on an intact medial prefrontal cortex (mPFC). As with CIPN, existing therapeutics are often ineffective in treating such pain and, moreover, even effective pain relief with existing analgesics usually does not improve or may even worsen pain-related cognitive dysfunction.

## SUMMARY

Thus, in accordance with the present disclosure, there is provided a method of treating, inhibiting, preventing or reversing neuropathic pain in a subject comprising administering to said subject an amount of eFT508 sufficient to treat, inhibit, prevent or reverse said neuropathic pain. Also provided is a method of treating, inhibiting, preventing or reversing a cognitive deficit comorbid with neuropathic pain in a subject comprising administering to said subject an amount of eFT508 sufficient to treat, inhibit, prevent or reverse said cognitive deficit. The subject may be a human or a non-human mammal. The neuropathic pain may be chronic neuropathic pain or acute neuropathic pain.

eFT508 may be administered with a second anti-pain agent, such as where eFT508 and said second anti-pain agent are co-formulated, or where eFT508 and said second anti-pain agent are not co-formulated. eFT508 and said second anti-pain agent may be delivered at distinct times. eFT508 may be delivered before said second anti-pain agent, may be delivered after said second anti-pain agent, and/or eFT508 and said second anti-pain agent may be delivered in alternating administrations. The second anti-pain agent may be an opioid or a gabapentanoid.

eFT508 may be delivered over a period of one week, two weeks, three weeks, four weeks, one month, two months, three months, four months, five months, six months, seven months, eight months, nine months, ten months, eleven months, one year, two years or three years. eFT508 may be delivered by continuous infusion, such as by an implanted pump. The neuropathic pain may be the result of an injury, such as a peripheral nerve injury, or such as a penetration wound, a burn, frostbite or a fracture. The neuropathic pain may be the result of a disease, such as diabetes, cancer, spinal nerve disease, multiple sclerosis, arthritis, an autoimmune disease, or an infection, such as viral infection,

fungal infection or bacterial infection. The neuropathic pain may be the result of chemotherapy or the result of surgery.

It is contemplated that any method or composition described herein can be implemented with respect to any other method or composition described herein. The use of the word "a" or "an" when used in conjunction with the term "comprising" in the claims and/or the specification may mean "one," but it is also consistent with the meaning of "one or more," "at least one," and "one or more than one." The word "about" means plus or minus 5% of the stated number.

Other objects, features and advantages of the present disclosure will become apparent from the following detailed description. It should be understood, however, that the detailed description and the specific examples, while indicating specific embodiments of the disclosure, are given by way of illustration only, since various changes and modifications within the spirit and scope of the disclosure will become apparent to those skilled in the art from this detailed description.

## BRIEF DESCRIPTION OF THE FIGURES

The following drawings form part of the present specification and are included to further demonstrate certain aspects of the present disclosure. The disclosure may be better understood by reference to one or more of these drawings in combination with the detailed description.

**FIGS. 1A-C present results related to expression of fused EGFP-L10a protein in Nav1.8-positive nociceptors.** (FIG. 1A) Schematic representation of TRAP showing isolation of translating ribosomes (IP) from Input using anti-eGFP-coated beads. (FIG. 1B) Figure shows a representative set of microscope images of L4-DRG sections from Nav1.8$^{cre}$ / TRAP$^{floxed}$ mice with immunostaining for CGRP, IB4, NF200, and TRPV1. Scale bars, 100 $\mu$m. (FIG. 1C) A total of 9528, 8258 and 8526 neurons were counted for CGRP, NF200 and Prph staining, n = 3 mice/group, and overlap is shown (FIG. 1C).

**FIGS. 2A-C present results related to the effects of paclitaxel treatment on excitability in cervical, thoracic and lumbar mouse DRG neurons.** (FIG. 2A) DRG neurons from mice treated with paclitaxel show hyper-amplitude of cell membrane oscillation. Left column shows representative recordings of the spontaneous activity (SA) observed in vehicle-treated mice DRG neurons from cervical (top trace), thoracic (middle trace) and lumbar (bottom trace) level. Right column shows representative recordings of the SA observed in DRG neurons from paclitaxel-treated mice at different vertebral levels. Neurophysiological activity was recorded from 247 small diameter (<22 $\mu$m) neurons at day 10 after paclitaxel treatment including 129 cells from vehicle-treated control mice (49, 43 and 37 cells for cervical, thoracic and lumbar respectively) and 118 cells from paclitaxel-treated mice (41, 37 and 40 cells for cervical, thoracic and lumbar respectively). (FIGS. 2B-C) Representative action potential responses evoked at 1X, 5X, 8X rheobase in DRG neurons from vehicle and paclitaxel-treated mice. DRG neurons from paclitaxel-treated animals showed that the mean number of action potentials evoked at 3X to 8X rheobase was significantly higher. *p <0.05, **p < 0.01, and ***p < 0.001.

**FIGS. 3A-D present data on principal component analysis (PCA) that reveals specific transcriptomic and translatomic signatures of CIPN.** (FIG. 3A) Linear correlation plot shows high correlation coefficient between biological replicates for the TRAP and Input fractions. (FIG. 3B) Absolute variances calculated for the first 5$^{th}$ principal components. (FIG. 3C) Principal component analysis shows that the first principal component (PC1) differentiates between Input (Transcriptome) and TRAP (Translatome). (FIG. 3D) Heatmap showing the PCA distances between each biological replicate. Related to FIGS. 1A-C.

**FIGS. 4A-F present data that TRAP-seq identifies differentially translated mRNAs in Nav1.8-positive nociceptors.** (FIG. 4A) DRG-TRAP-seq is enriched in neuronal markers such as *Calca* (CGRP) or *Camk2a* while glial (*Aldh1l1, Gfap*), endothelial (*Cldn5, Fit1*) and microglial (*Cxcr3, Aif1*) markers are depleted. (FIG. 4B) The probability density function of the upper decile (ud)TPM distribution for the Input and TRAP fraction where the inflexion point was used to set the expression threshold for further analysis. (FIG. 4C) The threshold effect size is set using the cumulative distribution of the log2 fold change for the Input and TRAP fractions showing a significant shift towards high fold change in the TRAP fraction (Kolmogorov-Smirnov, ****p<0.0001). Related to FIGS. 1A-C. (FIG. 4D) Volcano plot reveals 4 genes up-regulated and 1down-regulated in the transcriptome while more widespread changes are observed in the translatome (TRAP). (FIG. 4E) Heatmap shows the fold change of differentially expressed genes in each biological replicate of vehicle- and paclitaxel-treated animals (TRAP and Input fractions). (FIG. 4F) EnrichR analysis of the up-regulated genes in paclitaxel TRAP-seq samples

**FIGS. 5A-B present data that translation efficiencies on Scn10a-enriched genes show reliable changes in expression level for GPCR and ion channels.** (FIG. 5A) The list of genes in the TRAP-seq dataset is first filtered based on expression level (see FIGS. 4A-F) and the remaining 8270 genes were used for further analysis. Separately, the inventors used available RNA sequencing dataset from magnetic sorted cell to generate a list of 881 genes expressed in Scn10a-positive neurons as well as an interactome dataset built in the laboratory that contains a total of 5347 genes. A final list of 221 of Scn10a-enriched genes was used to calculate the respective translation efficiencies

(TE) in vehicle- or paclitaxel conditions. The Interactome analysis shows higher translation efficiencies for genes encoding ion channels and GPCR. (FIG. 5B) Gene-by-gene analysis reveals that multiple channels have high TE after CIPN such as *P2rx2* (Multiple T-tests with correction for multiple comparisons: *p = 0.039 and q < 0.1) *or TRPV1* (Multiple T-tests with correction for multiple comparisons: *p = 0.0059 and q < 0.1) while *Mrgrpd* is the only GPCR showing a significant increases in TE after CIPN (Multiple T-tests with correction for multiple comparisons: *p = 0.0049 and q < 0.1)

**FIG. 6 present data that TOP-and G-quartet like elements are enriched in the up-regulated genes identified with TRAP-seq.** The motif analysis was performed on the list of up-regulated genes identified with TRAP-seq after CIPN. The inventors show that 3 motifs are significantly enriched in the 5'UTR among them a TOP-like element which consists in a "C" or "T" followed by a stretch of 5 to 15 pyrimidines. Also, the analysis clearly reveals an enrichment in G-quartet like motif as well as in an AG-rich motif.

**FIGS. 7A-E present data that enhanced eIF4E signaling in nociceptors is responsible for evoked and spontaneous pain in paclitaxel-induced neuropathic pain.** (FIG. 7A) Volcano plot showing an increase in *mTOR* mRNA translation in the DRGs after CIPN. (FIG. 7B) Immunoblotting shows an up-regulation of p-eIF4E, p-4EBP1,and mTOR after paclitaxel treatment (p-eiF4E: Veh = 100 $\pm$ 5.39, Pac = 158 $\pm$ 10.57, **p = 0.0026, n = 4-5; p-4EBP1: Veh = 100 $\pm$ 9.8, Pac = 152 $\pm$ 11.27, *p = 0.012, n = 4-5; p-mTOR: Veh = 100 $\pm$ 10.8, Pac = 131 $\pm$ 20.27, p = 0.23, n = 4-5; mTOR: Veh = 100 $\pm$ 24.54, Pac = 195 $\pm$ 25.27, *p = 0.025, n = 4-5). (FIG. 7C) Intrathecal injection of the mTOR kinase inhibitor AZD8055 relieves mechanical allodynia compared to vehicle-treated animals after the first injection (two-way ANOVA: $F_{(7,88)}$ = 3.93; post-hoc Bonferroni: at 1hr: ****p<0.0001, 3hrs: *p = 0.043, 5hrs: **p = 0.0059) but no significant effects were observed after the second injection. (FIG. 7D) $eIF4E^{S209A}$ mice show a deficit in development of paclitaxel-induced mechanical allodynia compared to WT animals. Two-way ANOVA: $F_{(4,44)}$ = 3.14, p = 0.023 with a post-hoc test: at day 7: *p = 0.045; at day 9: ***p = 0.0003; at day 11: **p = 0.0081 $eIF4E^{S209A}$ vs WT, n = 7. (FIG. 7E) $eIF4E^{S209A}$ mice also exhibit a deficit in paclitaxel-induced thermal hyperalgesia compared to WT animals at 49°C (one-way ANOVA: F = 8.39, p = 0.005; post-hoc Bonferroni; Veh WT vs Pac WT:** p = 0.005, Veh WT vs Pac $eIF4E^{S209A}$: *p = 0.071, Veh WT vs Veh $eIF4E^{S209A}$: *p = 0.054), at 50 °C (one-way ANOVA: F =6.23, p = 0.0032; post-hoc Bonferroni; Veh WT vs Pac WT: **p = 0.0044, Veh WT vs Pac $eIF4E^{S209A}$: p > 0.99, Veh WT vs Veh $eIF4E^{S209A}$: p > 0.99, Pac WT vs Pac $eIF4E^{S209A}$: *p = 0.024) and 52 °C (one-way ANOVA: F =7.78, p = 0.0010; post-hoc Bonferroni; Veh WT vs Pac WT: **p = 0.0021, Veh WT vs Pac $eIF4E^{S209A}$: p > 0.99, Veh WT vs Veh $eIF4E^{S209A}$: p > 0.99, Pac WT vs Pac $eIF4E^{S209A}$: *p = 0.045). (FIG. 7F) WT paclitaxel-treated mice show signs of ongoing pain in the CPP paradigm compared with WT vehicle (One-way ANOVA; F= 4.36, p = 0.012, post-hoc Bonferroni Weh WT vs Pac WT: p =0.048) and $eIF4E^{S209A}$ mice treated with paclitaxel, wich did not show signs of spontaneous pain (One-way ANOVE: F = 4.36, *p = 0.012, post-hoc Bonferroni Pac WT vs Pac $eIF4E^{S209A}$: *p = 0.019).

**FIGS. 8A-F present data that the specific MNK inhibitor, eFT508, reverses CIPN.** (FIG. 8A) eFT508 significantly reduced the level of eIF4E phosphorylation at 0.1 and 1 $\mu$M after 1 hr (One-way ANOVA: *F* = 205.2, *p* < 0.0001; post-hoc Tukey: ****$p$ < 0.0001), without affecting other signaling pathways in cultured mouse DRG neurons. (FIG. 8B) Acute treatment with eFT08 relieves mechanical allodynia compared to vehicle after the 1st (Two-way ANOVA: $F_{(7.63)}$ = 14.53, post-hoc Bonferroni at 1 and 3 hrs (eFT508 vs vehicle: ****$p$ < 0.0001) and the 2nd injection (Two-way ANOVA: $F_{(7.63)}$ = 14.53, post-hoc Bonferroni at 1 hr (eFT508 vs vehicle: ** $p$ < 0.01) and 3 hrs (eFT508 vs vehicle: ****$p$ < 0.0001). Also, eFT508 transiently relieves mechanical allodynia on day 1 (Two-way ANOVA: $F_{(7.63)}$ = 14.53, post-hoc Bonferroni eFT508-BL vs eFT508-1hr: ***$p$ < 0.001 and eFT508-BL vs eFT508-3hrs: **$p$ = 0.0012) and day 2 (Two-way ANOVA: $F_{(7.63)}$ = 14.53, post-hoc Bonferroni eFT508-BL vs eFT508-1hr: *$p$ = 0.036 and eFT508-BL vs eFT508-3hrs: **$p$ = 0.0021). (FIG. 8C) Acute treatment with eFT508 did not significantly attenuate thermal hyperalgesia at 3 hrs (Two-way ANOVA: $F_{(1,38)}$ = 8.46, post-hoc Sidak: $p$ = 0.14) but a significant effect was observed at 24 hrs (Two-way ANOVA: $F_{(1,38)}$ = 8.46, post-hoc Sidak: *$p$ < 0.05). (FIG. 8D) $eIF4E^{S209A}$mice showed a deficit in the development of CIPN compared to WT mice (Two-way ANOVA: $F_{(2,117)}$ = 15,82, $p$ < 0.0001 with post-hoc test at day 3: WT vs $eIF4E^{S209A}$: *$p$ = 0.019, WT vs $eIF4E^{S209A}$(eFT508): **$p$ = 0.0065; at day 5 WT vs $eIF4E^{S209A}$: **$p$ = 0.0018, WT vs $eIF4E^{S209A}$(eFT508): **$p$ = 0.003; at day 10 WT vs $eIF4E^{S209A}$: **$p$ = 0.0020, WT vs $eIF4E^{S209A}$(eFT508): *$p$ = 0.021; at day 11 WT vs $eIF4E^{S209A}$: *$p$ = 0.0182, WTvs $eIF4E^{S209A}$(eFT508): *$p$0.028; at day 12WTvs $eIF4E^{S209A}$: *$p$ = 0.022,WTvs $eIF4E^{S209A}$ (eFT508): *$p$ = 0.044. Chronic treatment with 1 mg/kg eFT508 reverses established CIPN without inducing tolerance in WT mice (Two-way ANOVA: F(2,117) 15,82: $p$ < 0.0001 with post-hoc test day 10: (BL vs 2h, **$p$ = 0.0023), day 11: (BL vs 2h, *$p$ = 0.045) and day 12: (BL vs 2h, *$p$ = 0.0497) but was completely without effect in eIF4ES209A mice. (FIG. 8E-F) p-eIF4E is highly expressed in Nav1.8-positive DRG neurons and approximatively 80% of the eGFPe signal co-localizes with p-eIF4E. Scale bars, 100 $\mu$m.

**FIGS. 9A-G present data increased translation of RagA-Ragulator-mTORC1 complex mRNAs in CIPN.** (FIG. 9A) Volcano plot shows an increase in translation efficiency for mRNAs encoding proteins associated with the Rag-Ragulator-mTORC1 complex. (FIG. 9B) Distribution of the normalized reads on the RagA gene (Rraga) showing a prominent peak in the TRAP fraction after CIPN compared to Veh while no changes are observed in the Input fraction. (FIG. 9C) Normalized TPM for the Rraga mRNA shows no differences in the input (RagA: Veh = 1.65 $\pm$ 0.71, Pac: 2.04

$\pm$ 0.17 p = 0.62) while a significant increase is observes in the TRAP fraction (RagA: Veh = 1.004 $\pm$ 0.26, Pac: 3.6 $\pm$ 0.51 p = 0.0043). (FIG. 9D) Heatmap showing increased or decreased translation of the mRNAs encoding proteins of the Ragulator-RagA-mTORC1 complex after CIPN. (FIG. 9E) Immunoblot showing an increase in RagA protein levels in DRGs after CIPN compared to Veh (RagA: Veh = 100 $\pm$ 10.33, Pac = 181 $\pm$ 15.35, **p = 0.0043, n = 4-5). (FIG. 9F) Immunoblot showing a decrease in RagA protein levels in the eIF4E$^{S209A}$ mice compared to WT (RagA: Veh = 100 $\pm$ 5.05, Pac = 84.6 $\pm$ 4.32, *p = 0.035, n = 9) while no differences were observed in mRNA levels (RagA : Veh = 1.007 $\pm$ 0.067, Pac = 1.176 $\pm$ 0.01, p = 0.084, n = 4) (FIG. 9G) Immunoblot showing no differences in the DRG level of RagA protein between eIF4E$^{S209A}$ vehicle- and paclitaxel-treated mice (RagA : Veh = 100 $\pm$ 8.567, Pac = 75 $\pm$ 8.46, *p = 0.095, n = 4).

**FIGS. 10A-G present data that the MNK1-eIF4E axis controls the translation of the RagA GTPase in CIPN.** (FIG. 10A) Immunoblot showing a decrease in RagA protein level after treatment with eFT508 at 1 $\mu$M for 1 hr (one-way ANOVA: F= 4.55, p = 0.040, post-hoc Sidak Veh WT vs: 1 $\mu$M *p = 0.042). (FIG. 10B) Immunoblot showing a significant decrease in the level of p-eIF4E and RagA after eFT508 treatment in DRGs *in vivo* (p-eIF4E: Veh = 100 $\pm$ 2.33, eFT508: 10 $\pm$ 2.43, ****p<0.0001, n = 4; p4EBP1: Veh = 100 $\pm$ 16.03, eFT508 = 53.12 $\pm$ 7.03, *p<0.05; RagA: Veh = 100 $\pm$ 11.03, eFT508: 67 $\pm$ 6.02, *p<0. 042, n = 4 (FIG. 10C) Immunoblot showing a significant decrease in the level of p-eIF4E (Veh = 100 $\pm$ 8.63, MNK1$^{-/-}$: 64.98 $\pm$ 3.63, **p<0.0094, n = 4) and RagA (Veh = 100 $\pm$ 2.54, MNK1$^{-/-}$: 67 $\pm$ 5.70, *p<0. 0010, n = 4) in *MMK1*$^{-/-}$ mice compared to WT. (FIG. 10D) Co-expression level of RagA and p-eIF4E in vehicle-, paclitaxel- and eFT508-treated mice shows high correlation between DRG samples. (FIG. 10E) *MNK1*$^{-/-}$ show a deficit in development of paclitaxel-induced mechanical allodynia compared to WT animals (two-way ANOVA: $F_{(5,45)}$ = 13.45, p < 0.0001 with a post-hoc test: at day 3, 5, 7, 9 ****p<0.0001 *MNK1*$^{-/-}$ vs WT, n = 7). (FIG. 10F) eFT508 treatment decreases spontaneous activity found in DRG neurons after paclitaxel treatment in a reversible fashion. (FIG. 10G) Summary diagram showing proposed MNK1-eIF4E regulation of RagA translation and its influence on mTORC1 activity.

**Extended FIGS. 1-1A-D present data showing immunoaffinity purification of the transgene EGFP-L10a capture various translating mRNA in DRG neurons.** (Extended FIG. 1-1A) Co-immunoprecipitation of the fused protein EGFP-L10a in Nav1.8-TRAP and Rosa26$^{fsTRAP}$ mice. (Extended FIGS. 1-1B-C) DRGs were isolated from 4 different animals (2 males and 2 females) and the tissue was homogenized as described in the Methods section. RNA was then extracted from the Input and TRAP fractions and transformed in cDNA as described in Methods section and qRT-PCR were run using different set of gene-specific primers such as TRPV1, BDNF, Actin and aPKC using primers pairs: *TRPV1* forward 5'- CCCGGAAGACAGATAGCCTGA-3' (SEQ ID NO: 1) & *TRPV1* reverse 5'-TTCAATGGCAATGTGTAATGCTG (SEQ ID NO: 2) ; *Actin* forward 5' - GGCTGTATT CCCCTCCATCG (SEQ ID NO: 3) & *Actin* reverse 5'-CCAGTTGGTAACAATGCCATGT- 3' (SEQ ID NO: 4); *BDNF* forward 5'-TCATACTTCGGTTGCATGAAGG-3' (SEQ ID NO: 5) & *BDNF* reverse 5'-AGACCTCTCGA ACCTGCCC-3' (SEQ ID NO: 6) ; *Prkcz* forward 5'-GCGTGGATGCCATGACAAC-3' (SEQ ID NO: 7) & *Prkcz* reverse 5'-AATGATGAGCACTTCGTCCCT -3' (SEQ ID NO: 8). Agarose gels show an absence of DNA contamination in the Input and TRAP fractions (noRT) as well as an absence of PCR product in the Rosa26$^{fsTRAP}$ animals. The 100kb represents the presence of the amplicon $\approx$ 100 bp in the Input and TRAP fraction for different genes tested. (Extended FIG. 1-1D) Fragment analyzer trace shows the RNA quality for the Input and IP fractions.

**Extended FIGS. 7-1A-C present data that increased translational efficiency in TRAPseq reliably predicts increase in protein level in the DRG.** (Extended FIG. 7-1A) Volcano plot showing an increase in *Esyt1* mRNA translation in the DRGs after CIPN. (Extended FIG. 7-1B) Distribution of the normalized reads on the Esyt1 gene showing a prominent peak in TRAP fraction after CIPN compared to Veh. (Extended FIG. 7-1C) Immunoblot showing expression of Esyt1 protein in the DRG after CIPN induction (Esyt1: Veh = 100 $\pm$ 29.96, Pac = 230 $\pm$ 18.30, **p = 0.0060, n = 4-5). n = number of animals per group.

**Extended FIGS. 7-2A-B present data that CIPN is not associated with an increase of Akt or Ribosomal protein S6 phosphorylation.** (Extended FIG. 7-2A) Immunoblots showing expression of S6, Akt and ERK with and without phosphorylation on day 10 after induction of CIPN (Extended FIG. 7-2B) Quantification of the immunoblots in opposing panel (p-S6: Veh = 100 $\pm$ 20.22, Pac = 134 $\pm$ 24.47, p = 0.34, n = 6; S6: Veh = 100 $\pm$ 14.15, Pac = 133 $\pm$ 24.54, p = 0.22, n =6; p-Akt: Veh = 100 $\pm$ 6.08, Pac = 96.86 $\pm$ 6.63, p = 0.73, n = 6; p-ERK1/2: Veh = 100 $\pm$ 13.33, Pac = 107 $\pm$ 14.94, p = 0.72, n =6).

**Extended FIGS. 8-1A-B presents data showing that CIPN is not associated with an increase in overall protein synthesis in the DRG.** (Extended FIGS. 8-1A-B) General translation is not altered after CIPN as assessed by puromycin incorporation in the DRG and is also not influenced by a single dose of eFT508 (10 mg/kg). Finally, there was also no change in general translation in eIF4E$^{S209A}$ mice compared to any of the other groups (one-way ANOVA: F(3,20) = 0.46, p = 0.71, n =6)).

**Extended FIG. 8-2** shows **the absence of p-eIF4E staining in the eIF4E$^{S209A}$ mice.** Immunostaining of GFP, p-eIF4E and eIF4E showing an absence of p-eIF4E staining in the eIF4E$^{S209A}$ mice.

**Extended FIG. 10-1 shows that *MNK1*$^{-/-}$ mice do not show a decrease in ERK and 4EBP1 expression.** mmunoblots showing the expression of 4EBP1 and ERK between WT and *MNK1*$^{-/-}$ ice (4EBP1: WT = 100 ± 12.22, *MNK1*$^{-/-}$ = 126 ± 8.79, p = 0.13, n = 4; ERK: WT = 100 ± 9.71, *MNK1*$^{-/-}$ = 93 ± 4.4, p = 0.53, n =4).

**FIGS. 11A-D show that genetic inhibition of eIF4E phosphorylation rescues pain-related cognitive impairment in an attentional rule-shifting task.** (FIG. 11A) Illustration of the rule-shifting protocol. Mice were habituated to the maze over 3 days. On day 4, mice were assessed for preference to turn right or left over 7 trials (Turn bias). The following day, mice were trained to turn against their turn bias in order to retrieve food reward and to ignore the pseudorandomly placed visual cue (Response). Approximately three weeks after SNI, the animals were assessed for retention of the task (Retest). The next day, mice were trained to shift strategies so that they now had to turn towards the arm containing the visual cue to obtain food reward (Rule-shifting). (FIG. 11B) Male 4EKI SNI mice showed no impairment on retest day, indicating that disruption of eiF4E phosphorylation has no effect on retention of response training. Male 4EKI SNI mice displayed no impairment on rule-shifting day. (FIG. 11C) Male 4EKI SNI mice made significantly less never-reinforced errors (NRE) and regressive errors (REG), but not perseverative errors (PER), compared to WT SNI animals. (FIG. 11D) Male and female 4EKI mice displayed no differences on rule-shifting day. Two-way ANOVA with Bonferroni multiple comparisons ****p<0.0001, ***p<0.001, **p<0.01, *p<0.05.

**FIGS. 12A-B show that disruption of eIF4E phosphorylation in 4EKI mice prevents PNI-induced spontaneous pain.** (FIG. 12A) WT and 4EKI SNI and sham mice underwent a CPP paradigm in which clonidine (10 μg i.t.) was paired with the white chamber over 3 conditioning days. WT SNI mice spent significantly more time in the analgesic-paired chamber compared to WT sham mice. However, 4EKI SNI animals and 4EKI sham mice showed no difference in time in spent in the analgesic-paired chamber. These data suggest that 4EKI mice have reduced spontaneous pain. (FIG. 12B) Clonidine (10 μg i.t.) reversed mechanical withdrawal thresholds when assessed 2 hours after injection on each CPP conditioning day. One or two-way ANOVA with Bonferroni multiple comparisons ****p<0.0001, *p<0.05.

**FIGS. 13A-F show that pharmacological and genetic inhibition of MNK reverses PNI-induced impairment in attentional rule-shifting.** (FIG. 13A) WT SNI mice were treated with the MNK1/2 inhibitor, tomivosertib (10 mg/kg, p.o.), or vehicle once daily for 7 days. Rule-shifting took place 1 hour after the final injection. Tomivosertib-treated mice showed no impairment on rule-shifting day. (FIG. 13B) WT SNI vehicle-treated animals made significantly more never-reinforced errors (NRE) and regressive errors (REG), but not perseverative errors (PER) compared to all other groups. (FIG. 13C) Tomivosertib-treatment had no effect on mechanical withdrawal thresholds when assessed 1 or 3 hours after treatment on days 1 and 5 of the 7-day treatment schedule. (FIG. 13D) MNK1 KOs displayed a significant impairment in retention of response training on retest day, suggesting that disruption of MNK1 signaling causes issues with long-term memory. However, the MNK1 KO SNI mice showed no impairment on rule-shifting day. (FIG. 13E) MNK1 KO SNI animals also made significantly fewer NRE and REG when compared to WT SNI mice. (FIG. 13F) MNK1 KO SNI animals showed a slight attenuation in tactile allodynia on day 4 post surgery compared to WT SNI animals but showed no differences to WT SNI animals on days 7, 11, 14, and 17 post SNI. Two-way ANOVA with Bonferroni multiple comparisons ****p<0.0001, ***p<0.001, **p<0.01, *p<0.05.

**FIGS. 14A-D show that pharmacological inhibition of MNK1/2 with tomivosertib reverses PNI-induced spontaneous pain.** (FIG. 14A) Western blot analysis of eIF4e phosphorylation was conducted on mPFC, DRG, and sciatic nerve collected from animals 1 hour after administration of tomivosertib (10 mg/kg; i.p or p.o.) or vehicle. Phosphorylation of eIF4E was significantly decreased in mPFC, DRG and sciatic nerve after tomivosertib treatment. (FIG. 14B) WT SNI animals were tested in a CPP paradigm using tomivosertib (10 mg/kg i.p.) as the conditioning-drug. SNI animals treated with tomivosertib spent ~40% more time in the white chamber compared to SNI animals that received only vehicle. This 40% increase in time was significantly different from the change in time between tomivosertib and vehicle conditioned sham mice, suggesting that tomivosertib diminishes spontaneous pain caused by PNI. (FIG. 14C) Microelectrode array recordings of cultured mouse DRG neurons exposed to interleukin-6 (IL-6) showed enhanced spontaneous firing which was attenuated by co-treatment with tomivosertib (25 nM). MEA dishes that were only treated with tomivosertib showed a reduction in firing compared to the IL-6 only group. For the tomivosertib group, there was no significant difference in firing rate between any of the treatment time points and the baseline recording (p>0.05). **IL6 only vs IL6 + tomivosertib; #IL6 only vs tomivosertib only. (FIG. 14D) Western blot analysis revealed that eIF4E phosphorylation was unchanged in mPFC after SNI in male and female mice. One-way ANOVA with Bonferroni multiple comparisons (blot and MEA data), unpaired t-test (CPP) ****p<0.0001, **p<0.01, *p<0.05.

**FIGS. 15A-E show that inhibition of eIF4E signaling reverses PNI-induced changes in AIS length in the infralimbic cortex.** (FIG. 15A) Representative images of AIS staining in the infralimbic cortex of WT, 4EKI, MNK1 KO, and tomivosertib-treated (10 mg/kg p.o. daily for 7 days) animals. (FIG. 15B) AIS length was significantly decreased in L5/L6 of the IL in WT SNI male. SNI did not alter AIS length in (FIG. 15C) 4EKI SNI mice nor (FIG. 15D) MNK1 KO SNI animals. SNI-induced changes in AIS length were reversed by (FIG. 15E) tomivosertib treatment. Image scale bar: 20 μm and 500 nm, respectively. Two-way ANOVA with Bonferroni multiple comparisons ****p<0.0001, **p<0.01.

**FIGS. 16A-C show that Genetic inhibition of the MNK-eIF4E signaling axis causes obsessive compulsive-like behaviors.** (FIG. 16A) MNK1 KO SNI animals took significantly longer to complete rule-shifting with each trial taking ~1.5-2 minutes. (FIG. 16B) Tomivosertib-treated mice did not display the same type of behavior in the maze as the MNK1 KOs. (FIG. 16C) To test if these behaviors were OCD-like, MNK1 KOs, 4EKI and tomivosertib-treated (10 mg/kg p.o. daily for 7 days) mice were assessed in a marble burying task. MNK1 KOs and 4EKI mice buried significantly more marbles compared to WT naive controls, while tomivosertib-treated mice performed similarly to naive and vehicle-controls. These data suggest that disruption of eIF4E signaling from development leads to an OCD phenotype. One or two-way ANOVA with Bonferroni multiple comparisons (FIGS. 16A-B) and comparison to WT naive (FIG. 16C) ****p<0.0001, ***p<0.001, **p<0.01, *p<0.05.

**FIGS. 17A-C show the effects of intraperitoneal (i.p) dosing of tomivosertib on locomotor behaviors.** WT male and female mice were given a single injection of tomivosertib (10 mg/kg i.p.; 4mg/mL stock), vehicle or nothing 1 hour prior to being placed in an open field or on a rotarod. (FIG. 17A) Mice that received vehicle or tomivosertib moved significantly slower in the open field than naive mice. (FIG. 17B) However, vehicle and tomivosertib mice were still conscious and very active in the open field as exemplified in their representative motor maps. (FIG. 17C) Mice that received tomivosertib or vehicle showed no significant difference to naive mice in the rotarod test. These data support that the mice were still awake and active in the conditioning chamber during our CPP-tomivosertib experiments. One or two-way ANOVA with Bonferroni multiple comparisons *p<0.05.

**FIGS. 18A-L show that Type I IFNs induce mechanical nociceptive hypersensitivity responses via a peripheral action in male and female mice.** (FIGS. 18A-D) In male mice, intraplantar (i.pl.) administration of IFN-$\alpha$ (300 U/25 $\mu$L) or IFN-$\beta$ (300 U/25 $\mu$L) increased paw mechanical hypersensitivity (g) to von Frey stimulation (FIGS. 18A-B) with no significant changes in paw withdrawal latency (s) to thermal stimulation (FIGS. 18C-D). n=9 (vehicle groups) and n=12 (IFN groups) in FIGS. 18A-B. n=6 (vehicle groups) and n=12 (IFN groups) in FIGS. 1C-D. (FIGS. 18E-H) In female mice, i.pl. administration of IFN-$\alpha$ (300 U/25 $\mu$L) and IFN-$\beta$ (300 U/25 $\mu$L) increased paw mechanical sensitivity to von Frey stimulation (FIGS. 18E-F) with no significant changes in paw withdrawal latency (s) to thermal stimulation (FIGS. 18G-H). n=6 per group. (FIGS. 1I-L) When comparing male versus female, no significant sex differences in the development of mechanical hypersensitivity (FIGS. 18I-J) or the presence of thermal sensitivity (FIGS. 18K-L) were observed between groups following either IFN-$\alpha$ (300 U/25 $\mu$L) or IFN-$\beta$ (300 U/25 $\mu$L) i.pl. administration. Data are presented as mean$\pm$SEM. Group differences were assessed using two-way ANOVA for FIGS. 18A-B and 18E-F followed by Bonferroni's multiple comparisons test. *p < 0.05, **p < 0.01, ***p < 0.001, ****p < 0.001.

**FIGS. 19A-J present data showing expression of IFNRs in DRG sensory neurons.** (FIG. 19A) Single DRG neuron-sequencing tSNE clusters showing expression of both *Ifnar1* (IFNR1) and *Ifnar2* (IFNR1) mRNAs with the neuronal marker *rbfox3* (NeuN). (FIG. 19B) *Ifnar1* and *Ifnar2* mRNAs expression overlaps with the small/medium-sized neuron sub-population expressing *Prph* (peripherin) and *Scn10a* (Nav1.8). (FIG. 19C) *Ifnar1* and *Ifnar2* mRNAs distribution across DRG sensory neurons of peptidergic [*Trpv1* (TRPV1), *Calca* (CGRP)] and non-peptidergic [*P2rx3* (P2X3)] sub-clusters. (FIG. 19D) *Ifnar1* and *Ifnar2* mRNAs expression differences in a subpopulation that express *F2rl1* (PAR2) and *Nppb* (NPPB). (FIG. 19E) *In situ* hybridization of *Ifnr1* and *Ifnr2* mRNA in neurons expressing *Calca, P2rx3* and NF200. (FIG. 19F) Co-expression analysis of *Ifnr1* and *Ifnr2* across the nociceptive population in the DRG. (FIG. 19G) Quantification of % *Ifnr1* and *Ifnr2* mRNA subunits based on total DRG neuronal population. n=3. (FIGS. 19I-J) Frequency analysis of *Ifnr1* and *Ifnr2* in the DRG based on neuronal size.

**FIGS. 20A-C show downstream signaling events associated with IFNRs activation.** (FIG. 20A) Direct stimulation of IFNRs with IFN-$\alpha$ (300 U/mL) and IFN-$\beta$ (300 U/mL) activated downstream JAK/STAT signaling pathways in cultured DRG neurons. (FIG. 20B) Neither IFN-$\alpha$ (300 U/mL) nor IFN-$\beta$ (300 U/mL) induced mTOR or RS6 phosphorylation in DRG cultures over a time course of 6 h. (FIG. 20C) Time course of the effects produced by IFN-$\alpha$ (300 U/mL) and IFN-$\beta$ (300 U/mL) on ERK, eIF4E and AKT phosphorylation. Data are presented as mean $\pm$SEM. n=3-6 per group for WB analysis. Group differences (treated vs vehicle) in FIGS. 20A-C were assessed using one-way ANOVA followed by Dunnett's multiple comparisons test. *p < 0.05, **p < 0.01, ***p < 0.001, ****p < 0.0001

**FIGS. 21A-E present data showing Type I IFNRs activity in cultured DRG neurons is neither associated with BiP-PKR-eIF2$\alpha$ stimulation nor suppressed by the small-molecule ISR inhibitor ISRIB.** (FIGS. 21A-B) Application of either IFN-$\alpha$ (300 U/mL) (FIG. 21A) or IFN-$\beta$ (300 U/mL) (FIG. 21B) for 1-6 h did not modify BiP expression or signaling via PKR and downstream p-eIF2$\alpha$ in cultured DRG neurons. (FIG. 21C) Long exposure (24 h) to IFN-$\alpha$ (300 U/mL) (FIG. 21A) or IFN-$\beta$ (300 U/mL) did not modify PKR phosphorylation in cultured DRG neurons. Likewise, no changes on PKR phosphorylation after a 24 h IFN-$\alpha$ (300 U/mL) treatment were observed in the presence of the integrated stress response inhibitor ISRIB (200 nM). (FIGS. 21D-E) The integrated stress response (ISR) inhibitor ISRIB (200 nM) did not modulate components of the signaling pathways that are activated after either IFN-$\alpha$ (300 U/mL) (FIG. 21D) or IFN-$\beta$ (300 U/mL) (FIG. 21E) application. n=3 per group. Data are presented as mean$\pm$SEM.

**FIGS. 22A-D present data showing IFN-$\alpha$ treatment causes DRG neuron hyperexcitability.** (FIG. 22A) Small- and medium sized DRG neurons were sampled for patch clamp electrophysiology experiments. The resting membrane potential was similar across the groups, with no significant effect of IFN-$\alpha$ treatment. (FIG. 22B)

Representative traces of action potential firing in the control (n = 8 cells) and IFN-α (n = 6 cells) groups. Action potentials were elicited by slowly depolarizing ramp currents of varying intensities. (FIG. 22C) Mean number of action potentials were higher in the IFN-α group at each ramp intensity. (FIG. 22D) IFN-α treatment significantly shortened the latency to the first spike. Group differences were assessed using two-way ANOVA followed by Fisher's LSD test. * $P < 0.05$, ** $P < 0.01$.

**FIGS. 23A-I present data showing genetic and pharmacological targeting of the MNK-eIF4E signaling axis attenuates type I IFN-induced pain hypersensitivity.** (FIGS. 23A-B) One h stimulation with either IFN-α (300 U/mL) or IFN-β (300 U/mL) phosphorylated the translation initiation factor eIF4E at serine S209 ($eIF4E^{S209}$, in red) in cultured DRG neurons expressing NeuN (blue) and peripherin (green). Scale bar 50 μm. Data are presented as mean±SEM. Group differences were assessed using one-way ANOVA followed by Dunnett's multiple comparisons test. (FIG. 23C) $MNK1^{-/-}$ mouse genotyping. (FIGS. 23D-E) Mechanical hypersensitivity produced by intraplantar (i.pl.) administration of either IFN-α (300 U/25 μL) (FIG. 23D) or IFN-β (300 U/25 μL) (FIG. 23E) was attenuated in mice lacking MNK1 (*Mknk1*$^{-/-}$), the specific kinase that phosphorylates eIF4E. Data are presented as mean±SEM. n=6 per group. Group differences were assessed using two-way ANOVA followed by Bonferroni's multiple comparisons test. *$p < 0.05$, **$p < 0.01$, ***$p < 0.001$, ****$p < 0.0001$. (FIG. 23F) $eIF4E^{S209A}$ mouse genotyping. (FIG. 23G) Mice lacking the phosphorylation site at Serine 209 ($eIF4E^{S209A}$) showed absence of eIF4E phosphorylation in L5 DRGs demonstrating antibody specificity. Scale bar=50 μm. (FIGS. 23H-I) Mechanical hypersensitivity was attenuated in $eIF4E^{S209A}$ mice compared to WT mice following an i.pl. injection of either IFN-α (300 U/25 μL) (FIG. 23H) or IFN-β (300 U/25 μL). (FIG. 23I) Data are presented as mean ± SEM. n=6 per group. Group differences were assessed using two-way ANOVA followed by Bonferroni's multiple comparisons test. *$p < 0.05$, **$p < 0.01$, ***$p < 0.001$

**FIGS. 24A-J present data showing that endogenous type I IFN induction with poly (I:C) produces pain sensitization in mice via MNK1-eIF4E signaling.** (FIGS. 24A-B) The synthetic analog of a double stranded RNA (dsRNA), poly (I:C) (1mg/kg, i.p.), injected for two consecutive days, produced mechanical (FIG. 24A) and thermal hypersensitivity (FIG. 24B) in mice. n=6 per group. *$p < 0.05$, **$p < 0.01$, compare to vehicle. $^{\#}p < 0.05$, $^{\#\#}p < 0.01$, $^{\#\#\#}p < 0.001$, $^{\#\#\#\#}p < 0.0001$ compare to baseline. Group differences were assessed using two-way ANOVA followed by Dunnett's (#) or Bonferroni's (*) multiple comparisons tests. (FIG. 24C) Changes in body temperature produced by intraperitoneal poly (I:C) (1 mg/kg) administration. n=6 per group. **$p < 0.01$, ****$p < 0.0001$ compared to vehicle. Group differences were assessed using unpaired t-test. (FIG. 24D) Intraperitoneal poly (I:C) administration increased phospho, but not total, eIF4E in L5 DRGs of WT mice at day 2 (3 hr post 2$^{nd}$ poly I:C injection). Scale bar=50 μm. n=3 per group. *$p < 0.05$ compared to vehicle. Group differences were assessed using unpaired t-test. (FIGS. 24E-F) Mechanical (FIG. 24E) and thermal (FIG. 24F) hypersensitivity produced by i.pl. administration of poly (I:C) (1 mg/kg) were partially attenuated in $MNK1^{-/-}$ mice compared to WT mice. ***$p < 0.001$, compare to WT mice. $^{\#}p < 0.05$, $^{\#\#}p < 0.01$, $^{\#\#\#}p < 0.001$, $^{\#\#\#\#}p < 0.001$ compared to baseline. n=12 (WT) and n=6 ($MNK1^{-/-}$) per group. Group differences were assessed using two-way ANOVA followed by Dunnett's (#) or Bonferroni's (*) multiple comparisons tests. (FIGS. 24G-H) Mechanical (FIG. 24G) and thermal (FIG. 24H) hypersensitivity produced by i.pl. administration of poly (I:C) (1 mg/kg) were attenuated in $eIF4E^{S209A}$ mice compared to WT mice. n=12 (WT) and n=6 ($eIF4E^{S209A}$) per group. *$p < 0.05$, ***$p < 0.001$ compared to WT mice, $^{\#}p < 0.05$, $^{\#\#}p < 0.01$, $^{\#\#\#}p < 0.001$ compared to baseline. Group differences were assessed using two-way ANOVA followed by Dunnett's (#) or Bonferroni's (*) multiple comparisons tests. (FIG. 24I) Lumbar DRGs (L4-L5), lumbar spinal dorsal horn (SDH) and sciatic nerve from $MNK^{-/-}$ and $eIF4E^{S209A}$ mice showed decrease and absence, respectively, on eIF4E phosphorylation compared to WT mice following i.p. poly I:C administration [day 2, 3 h post 2$^{nd}$ poly (I:C) injection]. n=3 per group. *$p < 0.05$, **$p < 0.01$, ***$p < 0.001$, ****$p < 0.001$ compared to WT. Differences were assessed using one-way ANOVA followed by Dunnett's multiple comparisons test. (FIG. 24J) Application of poly (I:C) (10 μg/mL) did not increase p-ERK, p-eIF4E, p-PKR or p-eIF2α in cultured DRG neurons. n= 3 per group.

**FIG. 25 shows a schematic model of downstream signaling events associated with type I interferon actions in nociceptors.** The inventors' work demonstrates that type I IFN production rapidly induced nociceptor hyperexcitability and mechanical pain sensitization via IFNR activation, likely on sensory neurons. The signaling mechanisms are dependent on MNK-eIF4E with no apparent integrated stress response (ISR) induction. Therefore, MNK-eIF4E signaling is a key translation regulation pathway that modulates viral infection-induced pain.

## DESCRIPTION OF ILLUSTRATIVE EMBODIMENTS

The present disclosure provides a MNK inhibitor, eFT508, for use in treating, inhibiting, preventing or reversing neuropathic pain in a subject.

Neuropathic pain is a major clinical problem that is remarkably difficult to treat. MNK-eIF4E signaling is a key driver of neuropathic pain induced by peripheral nerve injury in mice. Targeting MNK with eFT508, which is a very specific inhibitor of this kinase, can reverse established neuropathic pain. eFT508 treatment may also reverse cognitive deficits and prefrontal cortex pathology that are comorbid with neuropathic pain. In particular, chemotherapy-induced peripheral

neuropathy is driven by MNK-eIF4E signaling, and notoriously challenging to treat, much less prevent. The inventors have shown that targeting MNK with eFT508, can reverse established chemotherapy-induced peripheral neuropathy.

### I. Pain

Pain is an unpleasant feeling often caused by intense or damaging stimuli. The International Association for the Study of Pain's widely used definition states: "Pain is an unpleasant sensory and emotional experience associated with actual or potential tissue damage, or described in terms of such damage."

Pain motivates the individual to withdraw from damaging situations, to protect a damaged body part while it heals, and to avoid similar experiences in the future. Most pain resolves promptly once the painful stimulus is removed and the body has healed, but sometimes pain persists despite removal of the stimulus and apparent healing of the body; and sometimes pain arises in the absence of any detectable stimulus, damage or disease.

Pain is the most common reason for physician consultation in the United States. It is a major symptom in many medical conditions, and can significantly interfere with a person's quality of life and general functioning. Psychological factors such as social support, hypnotic suggestion, excitement, or distraction can significantly modulate pain's intensity or unpleasantness.

The International Association for the Study of Pain (IASP) has classified pain according to specific characteristics: (a) region of the body involved (*e.g.,* abdomen, lower limbs), (b) system whose dysfunction may be causing the pain (*e.g.,* nervous, gastrointestinal), (c) duration and pattern of occurrence, (d) intensity and time since onset, and (e) etiology. This system has been criticized by Clifford J. Woolf and others as inadequate for guiding research and treatment. According to Woolf, there are three classes of pain: nociceptive pain (see hereunder), inflammatory pain which is associated with tissue damage and the infiltration of immune cells, and pathological pain which is a disease state caused by damage to the nervous system (neuropathic pain, see hereunder) or by its abnormal function (dysfunctional pain, like in fibromyalgia, irritable bowel syndrome, tension type headache, *etc.*).

### A. Chronic Pain

Pain is usually transitory, lasting only until the noxious stimulus is removed or the underlying damage or pathology has healed, but some painful conditions, such as rheumatoid arthritis, peripheral neuropathy, cancer and idiopathic pain, may persist for years. Pain that lasts a long time is called chronic, and pain that resolves quickly is called acute. Traditionally, the distinction between acute and chronic pain has relied upon an arbitrary interval of time from onset; the two most commonly used markers being 3 months and 6 months since the onset of pain, though some theorists and researchers have placed the transition from acute to chronic pain at 12 months. Others apply acute to pain that lasts less than 30 days, chronic to pain of more than six months duration, and subacute to pain that lasts from one to six months. A popular alternative definition of chronic pain, involving no arbitrarily fixed durations is "pain that extends beyond the expected period of healing." Chronic pain may be classified as cancer pain or benign.

### B. Nociceptive Pain

Nociceptive pain is caused by stimulation of peripheral nerve fibers that respond only to stimuli approaching or exceeding harmful intensity (nociceptors), and may be classified according to the mode of noxious stimulation; the most common categories being "thermal" (heat or cold), "mechanical" (crushing, tearing, *etc.*) and "chemical" (iodine in a cut, chili powder in the eyes). As subset of nocicipetive pain is called "inflammatory" pain, as it results from tissue damage and the response of innate inflammatory responses. Nociceptive pain may also be divided into "visceral," "deep somatic" and "superficial somatic" pain. Visceral structures are highly sensitive to stretch, ischemia and inflammation, but relatively insensitive to other stimuli that normally evoke pain in other structures, such as burning and cutting. Visceral pain is diffuse, difficult to locate and often referred to a distant, usually superficial, structure. It may be accompanied by nausea and vomiting and may be described as sickening, deep, squeezing, and dull. Deep somatic pain is initiated by stimulation of nociceptors in ligaments, tendons, bones, blood vessels, fasciae and muscles, and is dull, aching, poorly localized pain. Examples include sprains and broken bones. Superficial pain is initiated by activation of nociceptors in the skin or other superficial tissue, and is sharp, well-defined and clearly located. Examples of injuries that produce superficial somatic pain include minor wounds and minor (first degree) burns.

### C. Neuropathic Pain

Neuropathic pain is pain caused by damage or disease that affects the somatosensory system. It may be associated with abnormal sensations called dysesthesia, and pain produced by normally non-painful stimuli (allodynia). Neuropathic pain may have continuous and/or episodic (paroxysmal) components. The latter are likened to an electric

shock. Common qualities include burning or coldness, "pins and needles" sensations, numbness and itching. Nociceptive pain, by contrast, is more commonly described as aching.

Neuropathic pain may result from disorders of the peripheral nervous system or the central nervous system (brain and spinal cord). Thus, neuropathic pain may be divided into peripheral neuropathic pain, central neuropathic pain, or mixed (peripheral and central) neuropathic pain. Central neuropathic pain is found in spinal cord injury, multiple sclerosis, and some strokes. Aside from diabetes (see diabetic neuropathy) and other metabolic conditions, the common causes of painful peripheral neuropathies are herpes zoster infection, HIV-related neuropathies, nutritional deficiencies, toxins, remote manifestations of malignancies, immune mediated disorders and physical trauma to a nerve trunk.

Neuropathic pain is common in cancer as a direct result of cancer on peripheral nerves (*e.g.,* compression by a tumor), or as a side effect of chemotherapy, radiation injury or surgery. After a peripheral nerve lesion, aberrant regeneration may occur. Neurons become unusually sensitive and develop spontaneous pathological activity, abnormal excitability, and heightened sensitivity to chemical, thermal and mechanical stimuli. This phenomenon is called "peripheral sensitization."

The (spinal cord) dorsal horn neurons give rise to the spinothalamic tract (STT), which constitutes the major ascending nociceptive pathway. As a consequence of ongoing spontaneous activity arising in the periphery, STT neurons develop increased background activity, enlarged receptive fields and increased responses to afferent impulses, including normally innocuous tactile stimuli. This phenomenon is called central sensitization. Central sensitization is an important mechanism of persistent neuropathic pain.

Other mechanisms, however, may take place at the central level after peripheral nerve damage. The loss of afferent signals induces functional changes in dorsal horn neurons. A decrease in the large fiber input decreases activity of interneurons inhibiting nociceptive neurons, *i.e.,* loss of afferent inhibition. Hypoactivity of the descending antinociceptive systems or loss of descending inhibition may be another factor. With loss of neuronal input (deafferentation) the STT neurons begin to fire spontaneously, a phenomenon designated "deafferentation hypersensitivity." Neuroglia ("glial cells") may play a role in central sensitization. Peripheral nerve injury induces glia to release proinflammatory cytokines and glutamate-which, in turn influence neurons.

## II. MNK and MNK-eIF4E Signaling

### A. MNKs

Mitogen-activated protein (MAP) kinases interacting kinases (MNKs) phosphorylate the eukaryotic translation initiation factor 4E (eIF4E) and factors that bind to AU-rich elements in the 3'-untranslated region of certain mRNAs. MNKs apparently regulate the expression of a specific set of proteins rather than global protein synthesis, and are involved in growth control, inflammation and viral translation. Therefore, they represent interesting targets for pharmaceutical intervention.

MNKs comprise a subfamily of Ser/Thr kinases, phylogenetically considered $Ca^{2+}$/calmodulin-dependent kinases (CaMK). MNKs are activated through phosphorylation by the growth factor-stimulated Ras/extracellular signal-regulated kinase pathway and the stress-induced p38 pathway. There are two human MNK genes, *mnk1* and *mnk2,* each of is alternatively spliced in to different isoforms (Mnk1a, Mnk1b, Mnk2a and Mnk2b) of 347-465 residues. All four proteins contain a stretch of basic residues at their N-termini that functions as a nuclear localization signal and as a binding region for the eukaryotic initiation factor 4G. Mnk1a and Mnk2a contain putative nuclear export motifs and MAP kinase-binding sites C-terminal of the central kinase domains, both of which are removed by alternative splicing in Mnk1b and Mnk2b.

### B. eIF4F Involvement with MNK Signaling

Eukaryotic initiation factor 4F (eIF4F) is a heterotrimeric protein complex that binds the 5' cap of messenger RNAs (mRNAs) to promote eukaryotic translation initiation. The eIF4F complex is composed of three non-identical subunits: the DEAD-box RNA helicase eIF4A, the cap-binding protein eIF4E, and the large "scaffold" protein eIF4G. The mammalian eIF4F complex was first described in 1983 and has been a major area of study into the molecular mechanisms of cap-dependent translation initiation ever since. eIF4F is important for recruiting the small ribosomal subunit (40S) to the 5' cap of mRNAs during cap-dependent translation initiation. Components of the complex are also involved in cap-independent translation initiation; for instance, certain viral proteases cleave eIF4G to remove the eIF4E-binding region, thus inhibiting cap-dependent translation.

Structures of eIF4F components have been solved individually and as partial complexes by a variety of methods, but no complete structure of eIF4F is currently available. In mammals, the eIF4E•G•A trimeric complex can be directly purified from cells, while only the two subunit eIF4E•G can be purified from yeast cells. eIF4E binds the $m^7G$ 5' cap and the eIF4G scaffold, connecting the mRNA 5' terminus to a hub of other initiation factors and mRNA. The interaction of eIF4G•A

is thought to guide the formation of a single-stranded RNA landing pad for the 43S preinitiation complex (43S PIC) via eIF4A's RNA helicase activity.

The eIF4F proteins interact with a number of different binding partners, and there are multiple genetic isoforms of eIF4A, eIF4E, and eIF4G in the human genome. In mammals, eIF4F is bridged to the 40S ribosomal subunit by eIF3 via eIF4G, while budding yeast lacks this connection. Interactions between eIF4G and PABP are thought to mediate the circularization of mRNA particles. The eIF4E subunit of eIF4F is an important target of mTOR signaling through the eIF4E binding protein (4E-BP). Phosphorylation of 4E-BPs by mTOR prevents their binding to eIF4E, freeing eIF4E to bind eIF4G and participate in translation initiation.

Hyperphosphorylation of the 4E-BPs by the mTOR kinase in response to a variety of environmental cues results in the release of eIF4E, allowing the cap-binding protein to associate with eIF4G and assemble an active eIF4F complex. In addition, the cap-binding protein eIF4E is phosphorylated by the eIF4G-associated kinase MNK-1.

It is known that inhibiting activity-dependent mRNA translation through mechanistic target of rapamycin and mitogen-activated protein kinase (MAPK) pathways blocks the development of nociceptor sensitization. These pathways convergently signal to the eukaryotic translation initiation factor (eIF) 4F complex to regulate the sensitization of nociceptors, but the details of this process are ill defined. Moy *et al.* (2017) investigated whether the phosphorylation of eIF4E by its specific kinase MAPK interacting kinases (MNKs) 1/2 was involved in nociceptor sensitization and chronic pain. Phosphorylation of ser209 on eIF4E regulates the translation of a subset of mRNAs, and the authors showed that pronociceptive and inflammatory factors, such as nerve growth factor (NGF), interleukin-6 (IL-6), and carrageenan, produced decreased mechanical and thermal hypersensitivity, decreased affective pain behaviors, and strongly reduced hyperalgesic priming in mice lacking eIF4E phosphorylation (*$eIF4E_{S209A}$*). Moreover, in patch-clamp electrophysiology and $Ca^{2+}$- imaging experiments on dorsal root ganglion neurons, NGF- and IL-6-induced increases in excitability were attenuated in neurons from *$eIF4E_{S209A}$* mice. These effects were recapitulated in *$Mnk1/2^{-/-}$*mice and with the MNK1/2 inhibitor cercosporamide. The authors also found that cold hypersensitivity induced by peripheral nerve injury is reduced in *$eIF4E_{S209A}$* and *$Mnk1/2^{-/-}$* mice and following cercosporamide treatment. They concluded that the MNK1/2-eIF4E signaling axis is an important contributing factor to mechanisms of nociceptor plasticity and the development of neuropathic pain.

### III. Pharmaceutical Formulations and Routes of Administration

#### A. MNK Inhibitors

The MNK inhibitor eFT508 may be used in the therapeutic and other methods described herein. eFT508, also call Tomivosertib, is a potent, highly selective, and orally bioavailable MNK1 and MNK2 inhibitor, with $IC_{50}$s of 1-2 nM against both isoforms. Its structure is shown below:

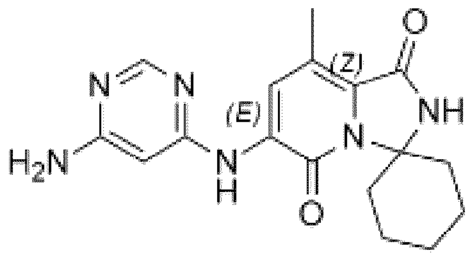

(I).

*In vitro,* eFT508 reduces eIF4E phosphorylation dose-dependently at serine 209 ($IC_{50}$ = 2-16 nM) in tumor cell lines. eFT508 has shown anti-proliferative activity against multiple DLBCL cell lines. Sensitivity to eFT508 in TMD8, OCI-Ly3 and HBL1 DLBCL cell lines is associated with dose-dependent decreases in production of pro-inflammatory cytokines including TNFα, IL-6, IL-10 and CXCL10. eFT508's mechanism of action appears to be decreased TNFα production correlateing with a 2-fold decrease in TNFα mRNA half-life. *In vivo,* eFT508 shows significant anti-tumor activity in multiple models, both of which harbor activating MyD88 mutations. eFT508 also combines effectively with components of R-CHOP and with novel targeted agents, including ibrutinib and venetoclax, in human lymphoma models.

PCT Application Publication No. WO2017117052 describes methods of assessing whether a human subject having a hyperproliferative disease is likely to respond to treatment with a MNK inhibitor or of identifying a human subject as a candidate for treating a hyperproliferative disease with a MNK inhibitor, as well as methods of treating such disease. WO2018218038 describes compositions and methods for cellular immunotherapy by, for example, generating modified antigen-specific T cells for use with MNK-specific inhibition, as well as methods for generating or increasing central

memory, antigen-specific T cells, improving cytotoxic T lymphocyte (CTL) activity of such T cells, or both by MNK-specific inhibition. Both applications describe eFT508 in further detail, and the relevant disclosures from each are incorporeated herein by reference with respect to that molecule.

## B. Formulations and Routes

Where clinical applications in treating pain are contemplated, it will be necessary to prepare pharmaceutical compositions in a form appropriate for the intended application. Generally, this will entail preparing compositions that are essentially free of pyrogens, as well as other impurities that could be harmful to humans or animals.

One will generally desire to employ appropriate salts and buffers to render materials stable and allow for uptake by target cells. Aqueous compositions of the present disclosure comprise an effective amount of the agent to cells, dissolved or dispersed in a pharmaceutically acceptable carrier or aqueous medium. Such compositions also are referred to as inocula. The phrase "pharmaceutically or pharmacologically acceptable" refers to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the compositions of the present disclosure, its use in therapeutic compositions is contemplated. Supplementary active ingredients also can be incorporated into the compositions.

The active compositions of the present disclosure may include classic pharmaceutical preparations. Administration of these compositions according to the present disclosure will be via any common route so long as the target tissue is available via that route. Such routes include intravenous, oral, nasal, buccal, rectal, vaginal or topical route. Alternatively, administration may be by orthotopic, transdermal, intradermal, subcutaneous, intramuscular, intraperitoneal, intrathecal or intravenous injection. Such compositions would normally be administered as pharmaceutically acceptable compositions, described *supra.* Of particular interest is transdermal, intraperitoneal, intravenous, subcutaneous or oral administration.

With regard to transdermal delivery, a patch is particularly contemplated. There are five main types of transdermal patches. In the Single-layer Drug-in-Adhesive, the adhesive layer of this system also contains the drug. In this type of patch, the adhesive layer not only serves to adhere the various layers together, along with the entire system to the skin, but is also responsible for the releasing of the drug. The adhesive layer is surrounded by a temporary liner and a backing. In Multi-layer Drug-in-Adhesive, the multi-layer drug-in adhesive patch is similar to the single-layer system in that both adhesive layers are also responsible for the releasing of the drug. One of the layers is for immediate release of the drug and other layer is for control release of drug from the reservoir. The multi-layer system is different however that it adds another layer of drug-in-adhesive, usually separated by a membrane (but not in all cases). This patch also has a temporary liner-layer and a permanent backing.

Unlike the Single-layer and Multi-layer Drug-in-adhesive systems, the reservoir transdermal system has a separate drug layer. The drug layer is a liquid compartment containing a drug solution or suspension separated by the adhesive layer. This patch is also backed by the backing layer. In this type of system, the rate of release is zero order. The Matrix system has a drug layer of a semisolid matrix containing a drug solution or suspension. The adhesive layer in this patch surrounds the drug layer partially overlaying it. This is also known as a monolithic device. In Vapor Patches, the adhesive layer not only serves to adhere the various layers together but also to release vapour. The vapor patches are new on the market and they release essential oils for up to 6 hours. Vapor patches release essential oils and are used in cases of decongestion mainly. Other vapor patches on the market are controller vapour patches that improve the quality of sleep. Vapor patches that reduce the quantity of cigarettes that one smokes in a month are also available on the market.

The active compounds may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid,

thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For oral administration the polypeptides of the present disclosure may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient may be added in a therapeutically effective amount to a paste dentifrice that may include water, binders, abrasives, flavoring agents, foaming agents, and humectants.

The compositions of the present disclosure may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media, which can be employed, will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences," 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### C. Subjects

The methods of the disclosure can be applied to a wide range of species, *e.g.,* humans, non-human primates (*e.g.,* monkeys, baboons, or chimpanzees), horses, cattle, pigs, sheep, goats, dogs, cats, rabbits, guinea pigs, gerbils, hamsters, rats, and mice.

## IV. Therapies

### A. Monotherapies

MNK inhibitors may be used to treat neuropathic pain. This neuropathic pain may include neuropathic pain related to cancer, chemotherapy-induced neuropathy, diabetic neuropathy, autoimmune neuropathy, and traumatic neuropathy. Additional uses include chronic pain such as rheumatoid arthritis. A therapy will provide relief of one or more symptoms of the pain, where such relief is either short term, such as with a single administration, or long term, such as with repeated or chronic administration.

### B. Agents for Use in Combination with MNK Inhibitors

Treating pain is a major issue in clinical medicine. A goal of current research is to find ways to improve the efficacy of pain relief using traditional known therapies, and one-way is by combining such traditional therapies with the therapies of the present disclosure. In the context of the present disclosure, it is contemplated that the MNK inhibitor may be used in a combination therapy with a second agent for treating pain.

The therapies would be provided in a combined amount effective to reduce pain. This process may involve contacting the patient with the agents/therapies at the same time. This may be achieved by contacting the patient with a single composition or pharmacological formulation that includes both agents, or by contacting the patient with two distinct compositions or formulations, at the same time, wherein one composition includes the MNK inhibitor and the other includes the second agent.

Alternatively, the treatment according to the present disclosure may precede or follow the other treatment by intervals ranging from minutes to weeks. In embodiments where the second therapy and the MNK inhibitor are applied separately to the subject, one would generally ensure that a significant period of time did not expire between each delivery, such that the therapies would still be able to exert an advantageously combined effect on the subject. In such instances, it is contemplated that one would administer both modalities within about 12-24 hours of each other, within about 6-12 hours of each other, or with a delay time of only about 12 hours. In some situations, it may be desirable to extend the time period for treatment significantly; however, where several days (2, 3, 4, 5, 6 or 7) to several weeks (1, 2, 3, 4, 5, 6, 7 or 8) lapse between the respective administrations.

It also is conceivable that more than one administration of either the MNK inhibitor or the second therapy will be desired. Various combinations may be employed, where the MNK inhibitor is "A," and the second therapy is "B," as exemplified below:

| | | | | | | | |
|---|---|---|---|---|---|---|---|
| A/B/A | B/A/B | B/B/A | A/A/B | B/A/A | A/B/B | B/B/B/A | B/B/A/B |
| A/A/B/B | A/B/A/B | | A/B/B/A | B/B/A/A | B/A/B/A | B/A/A/B | B/B/B/A |
| A/A/A/B | B/A/A/A | | A/B/A/A | A/A/B/A | A/B/B/B | B/A/B/B | B/B/A/B |

Other combinations, including chronic and contiuous dosing of one or both agents, are contemplated. Exemplary but non-limiting examples of suitable therapies for combination with the comopsitions and methods of the present disclosure are set out below.

Opioids, also known as narcotics, are increasingly recognized as important treatment options for chronic pain. Opioids, along with anticonvulsants and antidepressants are the most consistently effective class of drugs for neuropathic pain. Opioids must be used only in appropriate individuals and under close medical supervision. Several opioids, particularly methadone, and ketobemidone possess NMDA antagonism in addition to their $\mu$-opioid agonist properties. Methadone does so because it is a racemic mixture; only the l-isomer is a potent $\mu$-opioid agonist. The d-isomer does not have opioid agonist action and acts as an NMDA antagonist; d-methadone is analgesic in experimental models of chronic pain. Clinical studies are in progress to test the efficacy of d-methadone in neuropathic pain syndromes.

The following is a non-limiting list of opioids that can be administered in combination with MNK inhibitor in accordance with the present disclosure: Morphine, Opium, Hydromorphone, Nicomorphine, Oxycodone, Dihydrocodeine, Diamorphine, Papaveretum, Codeine, Phenylpiperidine derivatives, Ketobemidone, Pethidine, Fentanyl, Pethidine, Diphenylpropylamine derivatives, Piritramide, Dextropropoxyphene, Bezitramide, Methadone, Dextropropoxyphene, Benzomorphan derivatives, Pentazocine, Phenazocine, Oripavine derivatives, Buprenorphine, Etorphine, Oripavine derivatives, Morphinan derivatives, Butorphanol, Nalbuphine, Tilidine, Tramadol and Dezocine.

Gabapentinoids are 3-substituted derivatives of the neurotransmitter $\gamma$-aminobutyric acid (GABA) which selectively block $\alpha_2\delta$-containing voltage-dependent calcium channels. Clinically-used gabapentinoids include gabapentin (Neurontin) and pregabalin (Lyrica), as well as a gabapentin prodrug, gabapentin enacarbil (Horizant). Another analogue mirogabalin is in clinical trials but has not yet been approved. Other compounds from this family used in research but not developed for medical use include atagabalin, 4-methylpregabalin and PD-217,014. Gabapentinoids are used clinically in the treatment of conditions including epilepsy, neuropathic pain, fibromyalgia, anxiety, and restless legs syndrome, among others. The OTC (Anglo-sphere) and Russian pharmaceutical nootropic Phenibut, having its origins deriving from Soviet-engineered cosmonaut medicine, and its Vitamin B-modulated cousin, Picamilon, may also said to be "gabapentinoids" - scientifically crossing the blood brain barrier after the manner of this class - despite the international confusion regarding the precise clinical and medicinal usages and interrelated governmental categorizations of these compounds in terms of "liceity."

The following is a discussion of different therapies currently applied against nociceptive pain conditions. Such is exemplary and not limiting. Currently, there are a wide number of agents effective at treating nociceptive pain. These include salicylates, such as Aspirin (acetylsalicylic acid), Diflunisal and Salsalate, Propionic acid derivatives (Ibuprofen,

Dexibuprofen, Naproxen, Fenoprofen, Ketoprofen, Dexketoprofen, Flurbiprofen, Oxaprozin, Loxoprofen), Acetic acid derivatives, (Indomethacin, Tolmetin, Sulindac, Etodolac, Ketorolac, Diclofenac, Nabumetone), Enolic acid (Oxicam) derivatives (Piroxicam, Meloxicam, Tenoxicam, Droxicam, Lornoxicam, Isoxicam), Fenamic acid derivatives or "Fenamates" (Mefenamic acid, Meclofenamic acid, Flufenamic acid, Tolfenamic acid), Selective COX-2 inhibitors (Celecoxib, Rofecoxib, Valdecoxib, Parecoxib, Lumiracoxib, Etoricoxib, Firocoxib), Sulphonanilides such as Nimesulide, and a range of other compounds (Licofelone, Lysine clonixinate, Hyperforin, Figwort).

## V. Examples

The following examples are included to demonstrate particular embodiments of the disclosure. It should be appreciated by those of skill in the art that the techniques disclosed in the examples which follow represent techniques discovered by the inventor to function well in the practice of the disclosure, and thus can be considered to constitute particular modes for its practice. However, those of skill in the art should, in light of the present disclosure, appreciate that many changes can be made in the specific embodiments which are disclosed and still obtain a like or similar result without departing from the spirit and scope of the disclosure. In particular, features of the specific embodiments in these examples, alone or in the combinations dislosed in the examples, may be combined with other elements disclosed in the specification. In addition, variations of the specific embodiments in these examples that lack one or more features of the specific example, such as a particular dosage amount or regimen or non-therapeutic aspects of the specific example, are also contemplated by this disclosure.

### Example 1

### Materials and Methods

**Antibodies.** Antibodies for TRAP (HtzGFP-19F7 and HtzGFP-19C8) were obtained from Sloan Memorial Kettering Centre, after establishing Material Transfer Agreements (MTAs) with the laboratory of Prof. Nathaniel Heintz at The Rockefeller University. All animal procedures were approved by the Institutional Animal Care and Use Committee of University of Texas at Dallas.

**Transgenic animals.** *$Nav1.8^{cre}$/$Rosa26^{fsTRAP}$* mice. $Rosa26^{fsTRAP}$ mice were generated as described previously (Zhou *et al*., 2013) and purchased from Jackson Laboratory (Stock number: 022367). Transgenic mice expressing Cre recombinase under the control of the *Scn10a* (Nav1.8) promoter were obtained initially from Professor John Wood (University College London) but are commercially available from Infrafrontier (EMMA ID: 04582). The initial characterization of these mice demonstrated that the introduction of the Cre recombinase in heterozygous animals does not affect pain behavior, and their DRG neurons have normal electrophysiological properties (Stirling *et al*., 2005).

$Nav1.8^{cre}$ mice on a C57BL/6J genetic background were maintained and bred at the University of Texas at Dallas. Upon arrival, $Rosa26^{fsTRAP}$ mice were crossed to *$Nav1.8^{cre}$* to generate the Nav1.8-TRAP mice that express a fused EGFP-L10a protein in Nav1.8 expressing neurons. All experiments were carried out using male and female littermates aged 8 to 12 weeks old. Mice were group housed (4 maximum) in non-environmentally enriched cages with food and water *ad libitum* in a 12h light-dark cycle. Room temperature was maintained at 21 $\pm$ 2°C.

*$eIF4E^{S209A}$* and *$Mnk1^{-/-}$* mice. *$eIF4E^{S209A}$* mice on a C57BL/6J background were generated in the Sonenberg laboratory at McGill University, as described previously (Furic *et al*., 2010), and bred at The University of Texas Dallas to generate experimental animals. All animals were genotyped using DNA from ear clips taken at the time of weaning and all animals were backcrossed to C57BL/6J background for at least 10 generations prior to experiments. Behavioral experiments using *$eIF4E^{S209A}$* and WT mice were performed using mice between the ages of 8 and 12 weeks.

*$Mnk1^{-/-}$* mice on a C57BL/6J background were provided by the Sonenberg laboratory at McGill University and generated as described previously (Ueda *et al*., 2004).

**Translating Ribosome Affinity Purification (TRAP).** Nav1.8-TRAP male and female mice were decapitated and dorsal root ganglia (DRG) rapidly dissected in ice-cold dissection buffer (1X HBSS (Invitrogen 14065006), 2.5 mM HEPES, 35 mM Glucose, 4 mM NaHCO3, 100 μg/ml cycloheximide, 0.001 V 2 mg/ml emetine). DRGs were transferred to ice-cold polysome buffer (20 mM HEPES, 12 mM $MgCl_2$, 150 mM KCl, 0.5 mM DTT, 100 μg/ml cyclohexamide, 20 μg/ml emetine, 40U/ml SUPERase IN (Promega), 1 μl DNAse and protease inhibitor and homogenized using a dounce homogenizer. Samples were centrifuged at 3,000 x g for 10 min to prepare post-nuclear fraction (S1). Then, 1% NP-40 and 30 mM DHPC were added to the S1 fraction and then centrifuged at 15,000 x g for 15 min to generate a post-mitochnodrial fraction (S20). A 200 μl sample of S20 was removed for use as Input, and 800 μl of S20 was incubated with protein G-coated Dynabeads (Life Technologies) bound to 50 μg of anti-GFP antibodies (HtzGFP-19F7 and HtzGFP-19C8, Memorial Sloan Kettering Centre) for 3 hrs at 4°C with end-over-end mixing. Anti-GFP beads were washed with high salt buffer (20 mM HEPES, 5 mM $MgCl_2$, 350 mM KCl, 1% NP-40, 0.5 mM DTT and 100 μg/ml cyclohexamide) and RNA was eluted from all samples using a Direct-zol kit (Zymo Research) according to the

manufacturer's instructions. RNA yield was quantified using a Nanodrop system (ThermoFisher Scientific) and RNA quality was determined by a fragment analyzer (Advanced Analytical Technologies Inc.).

**Library generation and sequencing.** Libraries were generated from 100 ng to 1 μg of total RNA using Quantseq 3' mRNA-Seq library kit (Lexogen) with RiboCop rRNA depletion kit (Lexogen) treatment according to the manufacturer's protocols. The endpoint PCR amplification cycle number for each sample was determined by quantitative PCR (qPCR) assay with PCR Add-on kit for Illumina (Lexogen). The cycle number was selected when the fluorescence value reached 33% of the maximum for each sample. Purified libraries were quantified by Qubit (Invitrogen) and the average size was determined by fragment analyzer (Advanced Analytical Technologies Inc.) with high sensitivity NGS fragment analysis kit. Libraries were then sequenced on an Illumina NextSeq500 sequencer using 50-bp single-end reads.

Sequence files generated by the Illumina NextSeq500 sequencer were downloaded from BaseSpace. An Initial quality check using FastQC 0.11.5 (Babraham Bioinformatics) was done on the sequencing files and then trimming was performed on the server with the FASTQ Toolkit. Sequences were trimmed with optimized parameters (13 bases from 3'-end, 17 bases from 5'-end, and any poly-adenine longer than 2 bases from the 3' side). Trimming parameters were optimized based on FastQC results and mapping rate, as well as manually checking high reads or abundant chromosomal regions with IGV 2.3.80. The trimmed sequencing samples were then processed using TopHat 2.1.1 (with Bowtie 2.2.9) and mapped to the mouse reference genome (NCBI reference assembly GRCm38.p4) and reference transcriptome (Gencode vM10) generating files in ".bam" format. The inventors filtered reads such that uniquely mapped reads were processed for further analysis (Table 1). Processed .bam files were then quantified for each gene using Cufflinks 2.2.1 with gencode.vM10 genome annotation. Since reads only mapped to the 3'UTR of the gene, read counts were not normalized by length by the using the Cufflinks option -- no-length-correction. Relative abundance for the $i^{th}$ gene was determined by calculating TPM (transcripts per million) values as follow: $TPM_i = 10^6 \times \frac{a_i}{\Sigma_j[a_j]}$, where $a_j$ is the Cufflinks reported relative abundance. Finally, TPM values were normalized to upper decile for each biological replicate and udTPM (upper decile TPM) were used for analysis (Glusman *et al.*, 2013), to provide uniform processing for samples with varying sequencing depth and because of varying number of genes in the transcriptome and translatome.

**Antibodies and chemicals.** The peripherin and neurofilament 200 (NF200) antibodies used for immunohistochemistry (IHC) were obtained from Sigma Aldrich (St. Louis, MO). Isolectin $B_4$ ($IB_4$) conjugated to Alexa-Fluor 568 and secondary Alexa-Fluor antibodies were from Life Technologies (Grand Island, NY). Calcitonin gene-related peptide (CGRP) antibody was purchased from Peninsula Laboratories International, Inc. (San Carlos, CA). The phospho- and total-eIF4E, 4EBP1, ERK, Akt, RagA, Actin, S6 and GAPDH antibodies were obtained from Cell Signaling Technology (Danvers, MA). The phospho-eIF4E antibody used for immunohistochemistry (IHC) was purchased from Abcam (Cambridge, UK). AZD8055 was obtained from LC Laboratories (Woburn, MA) and eFT508 was provided by eFFECTOR (San Diego, CA) under an MTA agreement with the University of Texas at Dallas. See Table 2 for additional details on antibodies and chemicals used in this study.

**Thermal and mechanical testing.** Mice were housed on 12-hr light-/dark cycles with lights on at 7:00 AM. Mice had food and water available *ad libitum.* All behavioral experiments were performed between the hours of 9:00 AM and 6:00 PM. Mice were randomized to groups from multiple cages to avoid using mice from experimental groups that were cohabitating. Sample size was estimated as n < 6 using a power calculation with 80% power, expectations of 50% effect size, with alpha set to 0.05. The number of animals used in each experiment was based on available animals of the appropriate sex and age but was at least n = 5 for experiments. Standard deviation for power calculations was based on previously published data using similar models. Mice were habituated for 1 hr to clear acrylic behavioral chambers before beginning the experiment. Mechanical paw withdrawal thresholds were measured using the up-down method (Chaplan *et al.*, 1994) with calibrated Von Frey filaments (Stoelting Company, Wood Dale, IL). Thermal latency was measured using a Hot/Cold plate setting the temperature at 49, 50 and 52°C. For intrathecal (i.t.) injections 10 μg of AZD8055 was injected in a volume of 5 μL via a 30 ½ -gauge needle. eFT508 for systemic injection was made up in 10% DMSO and 90% polypropylene glycol in water and injected intraperitoneally in a volume of 100 μl and adjusted based on the weight. Mice of both sexes were used in most experiments and no significant differences between sexes were noted for drug or genotype in any experiments. The experimenter was blinded to the genotype of the mice and drug condition in all experiments.

**Conditioned Place Preference (CPP).** The inventors used a modified conditioned place preference (CPP) procedure adapted from (Yang *et al.*, 2014). A homemade CPP box was constructed with three chambers with equal levels of dim illumination. The box was made with one black and one white end chamber connected by a striped chamber. On day 1 of testing (Day 7 after the first injection of paclitaxel), each mouse was permitted to explore the striped and black (but not white) chambers. Conditioning trials occurred on days 2-4. Each morning, 0.1 ml of vehicle (saline) was injected intraperitoneally 10 min before confinement in the black chamber for 30 min. Three hours later, the same mouse was injected with retigabine (Alomones Labs, Jerusalem) (10 mg/kg in 0.1 ml of saline (5)). Ten min before confinement in the white chamber for 30 min. On day 5, each mouse was placed in the striped chamber with unrestricted access to all chambers for 10 min. Data are presented as the difference in time spent in the retigabine-paired (white) chamber minus

time in the vehicle-paired (black) chamber during the drug-free day 5 test.

**Co-Immunoprecipitation (Co-IP).** DRGs were transferred to ice-cold polysome buffer (20 mM HEPES, 12 mM $MgCl_2$, 150 mM KCl, 0.5 mM DTT and protease inhibitor and homogenized using a dounce homogenizer, and samples were centrifuged at 3,000 x g for 10 min to prepare the post-nuclear fraction (S1). Then, 1% NP-40 and 30mM DHPC were added S1 was centrifuged at 15,000 x g for 15 min to generate a post-mitochondrial fraction (S20). A 200 μl sample of S20 was removed for use as Input, and 800 μl incubated with protein G-coated Dynabeads (Life Technologies) bound to anti-GFP antibodies (HtzGFP-19F7 and HtzGFP-19C8, Memorial Sloan Kettering Centre) for 3 hrs at room temperature with end-over-end mixing. Anti-GFP beads were washed with high salt buffer (20 mM HEPES, 5 mM $MgCl_2$, 350 mM KCl, 1% NP-40) and proteins were eluted from all samples using RIPA buffer and heated up to 55° C for 5 min. The samples were then run on a 10% SDS-PAGE gel and immunoblot for L10a.

**Quantitative reverse transcriptase - polymerase chain reaction (qRT-PCR).** Lumbar DRGs were isolated from 4 male mice per genotype and flash-frozen on dry ice and stored at -80° C until ready to be processed. Tissues were homogenized using a pestle and total RNA was extracted using RNAqueous Total RNA Isolation kits (ThermoFisher Scientific). RNA was subsequently treated with TURBO DNase (ThermoFisher Scientific) according to the manufacturer's instructions. RNA concentration was measured on a NanoDrop 2000 (ThermoFisher Scientific). cDNA was synthesized using iScript Reverse Transcriptase (Bio-Rad). qRT-PCR was done using a Applied Biosystems Lightcycler 7500 real time PCR system using iTaq Universal SYBR Green Supermix (Bio-Rad) according to the manufacturer's instructions with 3 technical replicates per biological replicate (averages of the technical replicates per biological replicate are reported) using primers pairs: *Gapdh* forward 5'-GACAACTTTGGCATTGTGGA -3' (SEQ ID NO: 9) & *Gapdh* reverse 5'-CATCATACT TGGCAGGTTTCTC - 3' (SEQ ID NO: 10), *Rraga* forward 5'-ACGTCCGATTCTTGGGGAAC -3' (SEQ ID NO: 11) & *Rraga* reverse 5'-TACGGAAGATGTTGTCCCGC -3' (SEQ ID NO: 12), Primers were made by Integrated DNA Technologies (Coralville, IA). Data were analyzed as $2^{-\Delta\Delta C_T}$ and normalized as shown in Results.

**Measurement of Protein Synthesis.** Eight to 12-week-old wild-type and *eiF4E*$^{S209A}$ mice were injected with puromycin (10 mg/kg via the intraperitoneal route, i.p.), and 1 hr later, DRGs were collected and processed for Western blotting using anti-puromycin monoclonal antibody (PMY-2A4, KeraFast). DRGs from mice injected with eFT508 (10 mg/kg i.p, injected 1 hr before puromycin) or vehicle were processed in parallel and used as controls. Protein synthesis was determined by measuring total lane signal from 250-15 KDa and subtracting unlabeled protein control. Signals were quantified using Image Lab (BioRad, Hercules, CA).

**Immunohistochemistry (IHC).** Animals were anesthetized with isoflurane (4%) and euthanized by decapitation and tissues were flash frozen in O.C.T. on dry ice. Spinal cords were pressure ejected using chilled 1X phosphate buffered saline (PBS). Sections of DRG (20 μm) were mounted onto SuperFrost Plus slides (Thermo Fisher Scientific, Waltham, MA) and fixed in ice-cold 10% formalin in 1X PBS for 45 min then subsequently washed 3 times for 5 min each in 1X PBS. Slides were then transferred to a solution for permeabilization made of 1X PBS with 0.2% Triton X-100 (Sigma Aldrich). After 30 min, slides were washed 3 times for 5 min each in 1X PBS. Tissues were blocked for at least 2 hrs in 1X PBS and 10% heat-inactivated normal goat serum. DRG slices were stained with peripherin and NF200 and Immunoreactivity was visualized following 1 hr incubation with goat anti-rabbit, goat anti-mouse, and goat anti-guinea pig Alexa-Fluor antibodies at room temperature. All IHC images are representations of samples taken from 3 animals per genotype. Images were taken using an Olympus FluoView 1200 confocal microscope. Analysis of images was done using ImageJ Version 1.48 for Apple OSX (National Institutes of Health, Bethesda, MD).

**Acute dissociation of mouse DRG neurons.** Thirty mice were deeply anesthetized with isoflurane and the bilateral C4-5, T3-5, and L4-5 ganglia were surgically exposed and removed. The ganglia were placed in a flask containing trypsin (6.25 μg/mL, Hyclone) and type IA collagenase (0.25 mg/mL, Sigma-Aldrich) in DMEM and shaken for 50 min in a heated (37°C) bath. After the cells were washed and mechanically dispersed with a fire polished Pasteur pipette, they were plated on poly-L-lysine-coated glass sheets and held in culture dishes with DMEM (10% FBS) until use within 6 hours after plating.

**Whole-cell patch recording of spontaneous and evoked action potential in acutely dissociated mice DRG neurons.** A previously described protocol was used (Li *et al.*, 2017) for current-clamp recording. The internal solution contained 135 mM K-gluconate, 5 mM KCl, 5 mM Mg-ATP, 0.5 mM $Na_2$GTP, 5 mM HEPES, 2 mM $MgCl_2$, 5 mM EGTA, and 0.5 mM $CaCl_2$ adjusted to pH 7.4 with KOH and the bath ACSF solution. DRG neurons were held at 0 pA, and the action potential current threshold was evoked using a series of 300-ms depolarizing current injections in 10-pA steps from -50 pA. The current that induced the first action potential that was defined as 1X rheobase. Only neurons with a resting membrane potential of at least - 40 mV, stable baseline recordings, and evoked spikes that overshot 0 mV were used for further experiments and analysis. The DRG neurons were then held at 0 pA to record spontaneous action potential for 5 min. The different times rheobase injections for 500 ms was applied to induce action potential spike-burst responses. Series resistance (Rs) was compensated to above 70% for the recorded DRG neurons. All recordings were made at room temperature.

**Western blotting.** Male and female mice were used for all Western blotting experiments and were sacrificed by decapitation following anesthesia and tissues were flash frozen on dry ice. Frozen tissues were homogenized in lysis

buffer (50 mM Tris pH 7.4, 150 mM NaCl, 1 mM EDTA pH 8.0, and 1% Triton X-100) containing protease and phosphatase inhibitors (Sigma Aldrich), and homogenized using a pestle. *In vitro* studies consisted of cultured primary DRG neurons from ~ 4 week-old mice. Mice were anesthetized with isoflurane and euthanized by decapitation. DRGs were dissected and placed in chilled Hank's Balanced Salt Solution (HBSS, Invitrogen) until processed. DRGs were then digested in 1 mg/mL collagenase A (Roche) for 25 min at 37° C then subsequently digested in a 1:1 mixture of 1 mg/mL collagenase D and papain (Roche) for 20 min at 37° C. DRGs were then triturated in a 1:1 mixture of 1 mg/mL trypsin inhibitor (Roche) and bovine serum albumin (BioPharm Laboratories, LLC), then filtered through a 70 μm cell strainer (Corning). Cells were pelleted, then resuspended in Dulbecco's Modified Eagle's Medium (DMEM) / F12 + Glutamax (Life Technologies) containing 10% fetal bovine serum (FBS, Life Technologies), 1% penicillin and streptomycin, and 3 μg/ml 5-fluorouridine with 7 μg/ml uridine to inhibit mitosis of non-neuronal cells and distributed evenly in a 6-well plate coated with poly-D lysine (Becton Dickenson [BD]). DRG neurons were maintained in a 37° C incubator containing 5% $CO_2$ with a media change every other day. On day 5, DRG neurons were treated as indicated in the results section, and cells were rinsed with chilled 1X PBS and harvested in lysis buffer containing protease and phosphatase inhibitors (Sigma-Aldrich), and then sonicated for 10 sec. To clear debris, samples were centrifuged at 14,000 rpm for 15 min at 4° C. Fifteen μg of protein was loaded into each well and separated by a 10-12% SDS-PAGE gel. Proteins were transferred to a 0.45 μm PVDF membrane (Millipore, Billierca, MA) at 25 V overnight at 4° C. Subsequently, membranes were blocked with 5% non-fat dry milk (NFDM) in 1X Tris buffer solution containing Tween 20 (TTBS) for 3 hrs. Membranes were washed in 1X TTBS 3 times for 5 min each, then incubated with primary antibody overnight at 4° C. The following day, membranes were washed 3 times in 1X TTBS for 5 min each, then incubated with the corresponding secondary antibody at room temperature for 1 hr. Membranes were then washed with 1X TTBS 5 times for 5 min each. Signals were detected using Immobilon Western Chemiluminescent HRP substrate (Millipore). Bands were visualized using film (Kodak; Rochester, NY) or with a Bio-Rad (Hercules, CA) ChemiDoc Touch. Membranes were stripped using Restore Western Blot Stripping buffer (Thermo Fisher Scientific), and re-probed with another antibody. Analysis was performed using Image Lab (Bio-Rad).

**Motif analysis.** For each gene in the upregulated translation gene list, the inventors created one 5'UTR sequence per gene. For each gene, this was done by first extracting 5' UTR sequences from all isoforms based on the Gencode annotation. Isoforms with 5' UTRs shorter than 20 bases, or with sequence that is part of an ORF in another isoform were discarded. The remaining isoforms were collapsed into a single sequence in order of their location in the genome, with overlapping regions present exactly once in the sequence. Based on the set of 5'UTRs, the motif finding tool MEME (world-wide-web at ncbi.nlm.nih.gov/pubmed/19458158) was used to identify motifs of length 6 to 12 bps, having at least 10 instances in the inventors' set of sequences. The background sequence set was simulated by MEME based on a 0th order model of nucleotide composition based on the 5'UTRs to avoid motifs identified as a result of nucleotide compositional biases, the 3 statistically significantly enriched motifs identified are shown, along with the genes they occur in, as well as the e values (expected number of instances for the motif, statistical significance threshold for e - value used is 0.01).

**Statistics.** All data are represented as mean ± standard error of the mean (SEM). All analysis was done using GraphPad Prism 6 v 6.0 for Mac OS X. For qPCR and WB experiments significant outliers (> 2 SD from the mean) were removed using one-sided Dixon's test at a significance ($p < 0.05$). Single comparisons were performed using Student's t-test and multiple comparisons were performed using a one-way ANOVA with post-hoc tests for between group comparisons. For behavioral experiments, two-way ANOVA (time X treatment) was used to measure effects across time between different groups. If significant effects were found by ANOVA, post hoc analyses were performed. Multiples comparisons between/within groups were performed using Bonferroni correction. Statistical results can be found in the figure legends. Electrophysiological recordings were analyzed using an unpaired t-test. DRG intracellular current injection neurophysiology data were analyzed with 2-way ANOVA (treatment X rheobase times) followed by Bonferroni *post hoc* tests (corrected $P < 0.05$). $P < 0.05$ was considered statistically significant.

**Statistics for RNA sequencing.** Differential expression analysis was performed using Matlab scripts. Briefly, TPM (transcripts per million) values were normalized to their $90^{th}$ percentile to generate udTPM and the probability density function of the udTPM was used to set the threshold value for further analysis. Genes showing consistent expression above the set threshold across biological replicates were then used to generate lists of differential expressed genes. Standard t-test was first performed assuming unequal variances between experimental groups generating p-values for each gene as follow. A q-value for the $i^{th}$ test was then calculated using Benjamini-Hochberg correction for multiple comparisons as follows: $Qvalue_i = \frac{pvalue_i \times N}{rank_{pvalue_i}}$ where N is the number of tests. Finally, the cumulative density function of the fold change was plotted and used to set the fold change for the input and TRAP fraction. Gene set enrichment analysis were performed with Enrichr (Kuleshov *et al.*, 2016) using the Gene Ontology molecular function (2015) and biological process (2015) and the Reactome (2015) libraries.

**Data and software availability.** Raw RNA sequencing data are available through GEO number: GSE113941. All

raw data and code is available upon request.

**Results**

To generate nociceptor-enriched ribosome-tagged mice, Nav1.8$^{cre}$ mice were crossed with Rosa26$^{fs-TRAP}$ (Zhou *et al*., 2013), to express eGFP fused to the ribosomal L10a protein in Nav1.8-positive neurons (FIG. 1A). To ensure the specificity of their approach, the inventors characterized expression of the transgene in the DRG. The inventors found that eGFP-L10a-positive neurons primarily co-localized with small diameter peripherin-positive neurons (81% ± 3.1) while a smaller proportion of eGFP-labelled neurons co-expressed NF200, a marker of large diameter neurons (40.1% ± 2.8) (FIG. 1B). The percentage of eGFP-L10a-positive neurons was equally distributed between non-peptidergic (86.7% ± 2.3) and peptidergic neurons (78.7% ± 2.7) (FIG. 1C). The inventors did not observe eGFP-L10a expression in non-neuronal cells in the DRG. Thus, this approach specifically labels sensory neurons including a substantial subset of neurons that are involved in nociception. They refer to these mice as Nav1.8-TRAP mice because they express tagged ribosomes specifically in the Nav1.8 population of DRG neurons.

The inventors then used Nav1.8-TRAP mice to preliminarily characterize the translatome of DRG neurons that express Nav1.8 using mRNAs isolated with immunoprecipitation (IP) of translating ribosomes (Heiman *et al*., 2014). First, the inventors determined that IP of eGFP-L10a from DRGs of control and Nav1.8-TRAP mouse strains yielded a specific IP of eGFP-L10a using eGFP antibodies (Extended FIG. 1-1A). The inventors also used IP combined with RT-qPCR for several mRNAs that are known to be enriched in nociceptors (Corder *et al*., 2017) to demonstrate integrity of the IP-mRNA complex (Extended FIGS. 1-1B-D). These experiments demonstrate that the Nav1.8-TRAP approach can be used to tag ribosomes and capture mRNAs from nociceptive DRG neurons.

One outcome of these characterization experiments was the realization that a large amount of starting material is required to successfully isolate ribosome-associated mRNAs from Nav1.8-TRAP cells. The inventors determined that DRGs from cervical, thoracic and lumbar levels from 4 animals were minimally required for a single biological replicate for accurate TRAP-seq in this tissue. Since a goal of the experiments was to assess the nociceptor translatome using RNA sequencing (Nav1.8-TRAP-seq) in neuropathic pain, this prompted us to utilize a neuropathy model where all DRGs are affected by the treatment. Paclitaxel-induced CIPN is a common form of neuropathic pain with devastating consequences for patients (Ma *et al*., 2018). The common descriptions of this form of CIPN include a "stocking and glove" distribution (Seretny *et al*., 2014; Ma *et al*., 2018) questioning whether pathology is found at all DRG levels from the drug treatment. Given that this would have obvious consequences for the inventors' experimental approach, the inventors sought to assess whether electrophysiological changes were found at all DRG levels in mice treated with paclitaxel. They treated Nav1.8-TRAP mice with the commonly used chemotherapeutic agent, paclitaxel (4 mg/kg) every other day, intraperitoneally, for a total of 8 days. They isolated cervical, thoracic and lumbar DRGs on day 10 after initiation of treatment from mice treated with vehicle or paclitaxel and performed patch clamp electrophysiology. Paclitaxel treatment caused spontaneous activity and increased nociceptor excitability (FIGS. 2A-C) at all DRG levels.

Having determined that paclitaxel affects nociceptors in all DRGs, the inventors pooled samples from 4 mice (2 male and 2 female) per treatment, per biological replicate for high-throughput sequencing. Subsequent behavioral and biochemical experiments were carried out in males and females (Table 3). On day 10 after the start of paclitaxel or vehicle treatment, the inventors collected eGFP-L10a IPs from Nav1.8-TRAP mice for RNA sequencing. From all biological replicates the inventors sequenced input mRNA, equivalent to the DRG transcriptome, and TRAP mRNAs associated with translating ribosomes in the Nav1.8 subset of DRG neurons (TRAP-seq). This approach allowed us to make comparisons of transcriptional and translational changes in response to paclitaxel in a cell-type specific fashion. The inventors observed strong correlation coefficients between biological replicates for the Input and TRAP samples demonstrating high reproducibility across experiments (FIG. 3A). Principal component analysis did not distinguish between treatment conditions in input samples whereas robust differences were found in the TRAP-seq samples suggesting that the key differences driving neuropathic pain generated by paclitaxel are happening at the translational level in Nav1.8-positive neurons (FIGS. 3B-D).

As an additional control for the specificity of this approach, the inventors analyzed a subset of genes that are known to be enriched in different cell populations in the DRG. This analysis demonstrated that TRAP-seq allowed the isolation of translating mRNAs in a cell-type specific manner. Neuronal mRNAs such as *Calca* (CGRP$\alpha$), *Calcb* (CGRP$\beta$) or *Scn10a* (Nav1.8) were enriched in TRAP fractions whereas glial markers (*Aldh1l1, Gfap*) and endothelial specific genes (*Cldn5, Esam)* were depleted. (FIG. 4A). For subsequent genome wide analysis, gene expression values (transcripts per million (TPMs)) were normalized to the 90$^{th}$ percentile for each biological replicate and the empirical probability density function (PDF) of the normalized expression level (upper decile (ud)TPM) was plotted for the input and TRAP fractions (FIG. 4B). The PDF function displayed 2 peaks (FIG. 4B) and the inflexion point was used to set the threshold expression values according to the sequencing depth. After further filtering, based on consistent expression among biological replicates, the inventors included a total of 8270 genes in the final analysis. The inventors then plotted the cumulative frequency distribution as a function of the log 2-fold change for each of these genes in vehicle-versus paclitaxel-treated

biological replicates and the 95th percentile was used to set the threshold fold change values for the input and TRAP fractions (FIG. 4C). Input transcriptome analysis revealed that only 4 genes were up-regulated (*Dpep2, Car3, Iah1* and *Arhgef4*) and 1 was down-regulated (*Plcb3*) in the DRG by paclitaxel treatment when looking at the whole transcriptome of the DRG at day 10 after treatment (FIG. 4D and Extended Table 4-1). This finding suggests that at this time point there are relatively few transcription changes induced by paclitaxel treatment in the whole DRG. In stark contrast, and consistent with the notion that transcriptional and translational changes are frequently decoupled (Liu *et al*., 2016), which is shown graphically for the inventors' dataset in FIG. 4E, the inventors detected 230 genes up-regulated (FIG. 4D and FIG. 4E and Extended Table 4-2) and 222 down regulated (FIG. 4D and FIG. 4E and Extended Table 4-3) by paclitaxel treatment in the TRAP-seq data set. These differences between transcriptome and translatome demonstrate the utility of the TRAP approach for identifying changes in gene expression that cannot be accounted for with traditional transcriptomic analysis.

The inventors then investigated what classes of genes were significantly enriched in the up and down regulated mRNA TRAP-seq dataset using the EnrichR tool (Kuleshov *et al*., 2016). First, validating that this approach can identify pathological changes in nociceptors that have already been linked to CIPN (Bennett *et al*., 2014), reactive oxygen species and mitochondrial dysregulation signatures were significantly enriched in the paclitaxel-treated TRAP-seq dataset. Likewise, several translationally up-regulated mRNAs found in the inventors' dataset have already been identified in the CIPN literature using independent methods, such as caspase 6 (Berta *et al*., 2017). The inventors also found a significant enrichment of genes involved in the regulation of cap-dependent translation, the lysosomal complex and mTORC1 signaling (FIG. 4F).

Combining this dataset with single cell RNA sequencing from existing data sources (Usoskin *et al*., 2015; Hu *et al*., 2016) allowed the inventors to infer translation efficiencies (TEs) for all mRNAs translated in Nav1.8 neurons creating a rich resource for understanding steady state translation of mRNAs expressed in nociceptors (world-wide-web at utdallas.edu/bbs/painneurosciencelab/sensoryomics/cipntrap/browse.html). They strictly limited themselves to calculating TEs for mRNAs that are found in the Nav1.8 population of neurons as defined by single cell RNA sequencing because high expression in other cell types would bias TEs toward less translation efficiency because the translating ribosome associated mRNA sequencing is restricted to the Nav1.8 population (FIG. 5A). The inventors examined families of genes for any systematic differences in TEs for mRNAs expressed in Nav1.8-positive neurons (e.g., ion channels, G-protein coupled receptors and kinases). Interestingly the inventors observed that ion channels and GPCRs tended to show higher TEs compared to other families such as kinases, RNA-binding proteins or transcription factors. When, the inventors looked at specific genes in each family they found a clear increase in TE for *Trpv1* (FIG. 5B), which has been shown to be up-regulated after paclitaxel (Li *et al*., 2015) and translationally upregulated by p38 signaling in the DRG in an inflammatory pain model (Ji *et al*., 2002). Also, they observed a decrease in TE for *Chrna6* associated with an increase in TE for *P2rx2* after CIPN (FIG. 5B). Interestingly, previous studies have demonstrated that *Chrna6* is a negative regulator of *P2rx2* and that *Chrna6* mutations that decrease this functional inhibition are correlated with chronic pain (Wieskopf *et al*., 2015). Therefore, decreased *Chrna6* expression in CIPN may sensitize nociceptors to ATP released from a variety of different sources via enhanced function of P2X2/P2X3 receptors.

Having established alterations in the nociceptor translatome with paclitaxel treatment, the inventors then sought to examine whether there were motifs in the 5' untranslated regions (UTRs) of mRNAs up-regulated after CIPN. They found a clear enrichment in 3 motifs. Most prominent among them was a TOP-like element (FIG. 6). TOP elements are found in the 5'UTRs of mRNAs that are regulated by mTORC1 signaling (Thoreen *et al*., 2012). This finding is consistent with the inventors' gene ontology results (FIG. 4F) and reinforces the concept that mTORC1-regulated mRNA translation is a critical component of neuronal plasticity after paclitaxel treatment. They also found an enrichment in an AG-rich motif with unknown function and a G-quartet-like motif (FIG. 6). The G-quartet motif suggests translation control by the RNA helicase, eIF4A (Wolfe *et al*., 2014). Interestingly, a specific eIF4A isoform, *eIF4a3,* was found as being highly translated after CIPN (Table 5) and *Mtor,* which was also translationally increased by CIPN (FIG. 7A), contains a G-quartet-like motif in its 5'UTR. This suggests that the RNA helicase eIF4A could regulate the translation of the *Mtor* mRNA.

The inventors then turned to biochemical methods to validate selected TRAP-seq results. They confirmed changes in steady state protein levels with paclitaxel treatment for proteins including mTOR (FIG. 7B), and Esyt1 (Extended FIGS. 7-1A-C). The concordance between measurements indicates that TRAP-seq can reliably predict increases in protein expression in DRG after CIPN. Based on increased mTOR protein levels the inventors also examined downstream and associated targets of mTOR where they noted increases in p-4EBP1 expression (FIG. 7B) but no differences in phosphorylation or total protein levels for ERK, AKT or, surprisingly, ribosomal protein S6 (Extended FIGS. 7-2A-B). They also found that eIF4E phosphorylation was increased with paclitaxel treatment (FIG. 7B). These findings suggest a selective increase in signaling via a subset of proteins that control translation of genes involved in neuronal plasticity (Gkogkas *et al*., 2014; Moy *et al*., 2017). To test this hypothesis, the inventors asked if mTOR inhibition or genetic loss of the phosphorylation site for MNK on the cap-binding protein eIF4E (eIF4E$^{S209A}$) would impair CIPN.

The inventors used the ATP competitive mTORC1/2 inhibitor AZD8055 to examine the influence of mTOR inhibition on paclitaxel-induced pain. While a robust reduction in mechanical hypersensitivity occurred with the first intrathecal injection of AZD8055, the magnitude of this effect was decreased upon subsequent treatments (FIG. 7C). The

inventors attribute this rapid onset, tolerance-like effect to the known phenomena of feedback signaling in the mTOR pathway (Melemedjian *et al*., 2013; Soares *et al*., 2015). Although they see clear signs of augmented mTORC1 signaling in this CIPN model, this limited efficacy with repeated dosing makes mTORCT1 a less attractive target for therapeutic intervention in CIPN.

The inventors then turned to a genetic approach to examine the possibility of targeting MNK-eIF4E signaling in CIPN. The inventors' previous work on this target suggests a key role in certain types of pain sensitization but effects in the spared nerve injury model of neuropathic pain were not as compelling (Moy *et al*., 2017; Moy *et al*., 2018). Given that the TRAP-seq data points to a clear role of altered translation regulation signaling in nociceptors in CIPN, the inventors hypothesized that MNK-eIF4E signaling may play a crucial role in this model. In support of this hypothesis, mice harboring a mutation at the MNK phosphorylation site on eIF4E (eIF4E$^{S209A}$ mice) treated with paclitaxel showed decreased mechanical (FIG. 7D) and thermal hypersensitivity (FIG. 7E) compared to their WT littermates. While this suggests decreased CIPN pain in the absence of eIF4E phosphorylation, the inventors determined this empirically using the conditioned place preference (CPP) paradigm (King *et al*., 2009) with retigabine as the conditioning agent, as has been described previously (Yang *et al*., 2014). Importantly, WT mice had a clear preference for the retigabine-paired chamber demonstrating relief of spontaneous pain. On the other hand, *eIF4E$^{S209A}$* mice showed no preference for the retigabine-paired chamber. This indicates an absence of spontaneous pain in these mice (FIG. 7F).

Given the strong behavioral phenotype of eIF4E$^{S209A}$ mice in CIPN, the inventors turned to a clinical candidate drug, eFT508 (Reich *et al*., 2018), that is a potent MNK1/2 inhibitor and blocks eIF4E phosphorylation *in vitro* and *in vivo.* They used this compound to examine whether targeted blockade of the MNK-eIF4E signalling axis inhibits and/or reverses paclitaxel-induced pain. The inventors first tested the effect of eFT508 on cultured DRGs neurons. Treatment with eFT508 reduced eIF4E phosphorylation by 90% (FIG. 8A). Neither loss of eIF4E phosphorylation in eIF4E$^{S209A}$ mice nor eFT508 treatment reduced bulk translation in the DRG *in vivo* (Extended FIGS. 8-1A-B). This parallels previous findings that Mnk1/2 signaling to eIF4E phosphorylation controls the translation of a specific subset of mRNAs (Gkogkas *et al*., 2014; Silva Amorim *et al*., 2018). Paclitaxel-treated mice given eFT508 (10 mg/kg) after full development of CIPN showed a reversal of mechanical (FIG. 8B) and thermal hypersensitivity (FIG. 8C). The inventors tested the specificity of the effect of eFT508 with dosing to WT and eIF4E$^{S209A}$ mice. The also used this experiment to assess the potential development of tolerance to repeated dosing of eFT508. eFT508 (1 mg/kg) produced robust relief of mechanical hypersensitivity at this lower dose, and the effect was completely absent in eIF4E$^{S209A}$ mice demonstrating the specificity (FIG. 8D). No signs of tolerance were observed in this experiment. Consistent with a key role for this signaling pathway in nociceptors, 80% of eGFP-L10a+ neurons exhibited strong p-eIF4E signal (FIG. 8F and FIG. 8G). No signal for the p-eIF4E antibody was observed in the eIF4E$^{S209A}$ mice, indicative of the specificity of the antibody (Extended FIG. 8-2).

These findings suggest a link between increased translation of mTOR complex proteins and MNK-mediated eIF4E phosphorylation as a causative factor in paclitaxel-induced neuropathy. However, mechanisms through which eIF4E phosphorylation might control mTOR signaling are not established. A possible connection between these parallel pathways (Richter and Sonenberg, 2005) is reciprocal translational regulation of mRNAs that encode signaling factors of the RagA-Ragulator complex mediated by MNK-dependent eIF4E phosphorylation. The RagA-Ragulator complex controls mTORC1 activity in response to amino acid flux across the lysosomal membrane (Sancak *et al*., 2010; Wolfson and Sabatini, 2017) and requires activity of the RagA, but not the RagB, GTPase (Efeyan *et al.,* 2013; Efeyan *et al*., 2014). RagA exists in a complex with RagC while RagB forms a complex with RagD (Efeyan *et al*., 2013; Efeyan *et al*., 2014). The TRAP-seq comparison demonstrates that genes corresponding to the components of the RagA-Ragulator-mTORC1 complex are preferentially translated after paclitaxel treatment (Fig. 9A). In particular, there was a 3.5-fold increase in the number of normalized reads mapping to the *Rraga* mRNA, encoding the RagA GTPase amino acid sensor for mTORC1 (FIGS. 9B-C).

Other components of the RagA-Ragulator-mTORC1 complex including *Lamtor5, Lamp2, Atp6v1a, Fkbp1a, Fkbp1b, Ywhaq* and *Mtor* also showed a consistent increase in translation with paclitaxel treatment (FIGS. 9A-D). Interestingly, known negative regulators of mTORC1 including *Akt1s1,* encoding PRAS40, and the $\gamma$2 subunit of AMPK (*Prkag2*) showed decrease translation efficiency (FIG. 9A and FIG. 9D). These findings indicate a fundamental change in the mTORC1 signaling network in Nav1.8 expressing neurons in CIPN that could be indicative of altered protein biosynthesis or related to other functions of mTORC1, such as lipid homeostasis (Saxton and Sabatini, 2017). Given the behavioral observations with eIF4E$^{S209A}$ mice, the effect of eFT508 in CIPN, the inventors further focused on translation regulation mechanisms as the key causative factor in paclitaxel-induced neuropathic pain.

The inventors validated that RagA protein levels were increased in DRG by paclitaxel treatment (FIG. 9E). They also found that RagA protein expression was significantly lower in eIF4E$^{S209A}$ mice compared to WT mice, although mRNA levels were equal in the two strains of mice (FIG. 9F). These findings suggested that eIF4E phosphorylation controls RagA translation in CIPN. To test this, the inventors treated eIF4E$^{S209A}$ mice with paclitaxel and failed to observe an effect from paclitaxel on RagA protein in DRGs from these mice (FIG. 9G). Moreover, the MNK1/2 inhibitor eFT508 decreased RagA protein levels both in cultured DRG neurons (FIG. 10A) and *in vivo* (FIG. 10B). 4EBP1 phosphorylation was also decreased in the DRG of eFT508-treated mice linking altered RagA translation to downstream mTORC1 activity

(FIG. 10B).

Two MNK isoforms are known to phosphorylate eIF4E, however, MNK2 shows constitutive kinase activity while MNK1 is induced by cellular stimuli (Ueda *et al*., 2004). The inventors reasoned that MNK1-mediated phosphorylation of eIF4E might control RagA protein expression and regulate paclitaxel-induced CIPN. *MNK1*$^{-/-}$ mice showed a 40% reduction in RagA protein levels in DRG compared to WT and a significant reduction in eIF4E phosphorylation while upstream signaling pathways were unchanged (FIG. 10C, Extended FIG. 10-1). When the inventors co-examined eIF4E phosphorylation with RagA expression in mouse DRG, they observed correlated expression for RagA and p-eIF4E. This was increased by paclitaxel treatment and strongly suppressed by eFT508 with an 80% correlation coefficient indicating that increases or decreases in eIF4E phosphorylation can reliably influence the level of RagA translation (FIG. 10D). The inventors then assessed paclitaxel-induced CIPN in *MNK1*$^{-/-}$ mice and observed a profound deficit in the development of mechanical hypersensitivity (FIG. 10E) consistent with observations in *eIF4E*$^{S209A}$ mice. Finally, the inventors examined whether transient MNK 1/2 inhibition could decrease spontaneous activity in DRG neurons taken from paclitaxel treated mice. These neurons showed robust spontaneous activity that was strongly suppressed by eFT508 treatment (FIG. 10F). Approximately 20 min of treatment with eFT508 was needed to reduce spontaneous activity in most neurons and this effect was reversible, indicating that eFT508 is not toxic to DRG neurons *in vitro.*

**Discussion**

The inventors used Nav1.8-positive neuronal translational profiling in the paclitaxel model of CIPN pain to comprehensively characterize the nociceptor translatome in mice with and without neuropathic pain. The data provide a unique resource for probing translational control in pain plasticity and reveal high quality targets for the potential generation of disease modifying therapies for a currently intractable disease (Seretny *et al*., 2014; Ma *et al*., 2018). This work demonstrates broad changes in regulation of the mTORC1 and MNK-eIF4E signaling network in CIPN that reveals a complex interplay between eIF4E-mediated translation control of *RragA* mRNA and mTORC1 function (FIG. 10G). These experiments support a model wherein MNK1-mediated phosphorylation of eIF4E controls the translation of *RragA* mRNA creating a previously unknown link between these distinct signaling pathways. In the context of CIPN, increased eIF4E phosphorylation leads to enhanced *Rraga* mRNA translation and RagA protein levels driving mTORC1 activation and neuropathic pain. These findings demonstrate that MNK and eIF4E phosphorylation can be targeted genetically or with eFT508 (also known as Tomivosertib), a drug in late phase clinical trials - serendipitously for cancer - as a means to prevent or reverse CIPN pain. These discoveries can potentially be used to enhance the efficacy of cancer chemotherapy treatment by reducing the primary dose-limiting side effect of chemotherapy (Seretny *et al*., 2014; Ma *et al*., 2018). These findings could also improve the quality of life of people who suffer from CIPN that continues after treatment since the inventors have shown that blocking MNK-eIF4E signaling plays a role in initiating and maintaining CIPN.

The global profile the inventors report of translation provides an independent confirmation for a variety of findings in the CIPN and chronic pain literature. This speaks to the powerful nature of the use of the TRAP-seq technique to gain insight into disease mechanisms in a cell-type specific fashion. In the inventors' gene ontology analysis, they identified both reactive oxygen signaling (Doyle *et al*., 2012; Duggett *et al*., 2016) and mitochondrial function as key changes occurring in CIPN. Both of these are well known mechanisms of CIPN with a great deal of recent focus in the field placed on mitochondrial dysfunction (Flatters and Bennett, 2006; Flatters, 2015; Griffiths and Flatters, 2015). This work provides a comprehensive snapshot of changes in translation of mRNAs that regulate mitochondrial function in CIPN. The inventors also confirm the upregulation of translation of several mRNAs that have also been linked to functional pathology in CIPN. Chief among these are Trpv1 (Li *et al*., 2015), an ion channel well known to contribute to many different types of pain, and caspase 6, an enzyme that has been linked to many different types of chronic pain where it seems to regulate tumor necrosis factor $\alpha$ signaling via an extracellular mechanism (Berta *et al*., 2014). The TRAP-seq findings independently support the conclusion that enhanced nociceptor translation of caspase 6 contributes to CIPN (Berta *et al*., 2017).

CIPN has traditionally been viewed as a length-dependent neuropathy that primarily affects the hands and feet, mainly in the glabrous skin areas (Seretny *et al*., 2014). There is support for distinct mechanisms of altered excitability in nociceptors that innervate glabrous skin caused by chemotherapy treatment that potentially explain this relatively selective pain phenotype (Yilmaz and Gold, 2016). However, several recent clinical studies have demonstrated that pain is more widespread in CIPN, although usually more intense in the hands and feet (Eckhoff *et al*., 2015; Ventzel *et al*., 2016). These studies also indicate that this widespread pain is persistent in many people who continue to suffer from CIPN more than a year after cessation of treatment (Eckhoff *et al*., 2015). The inventors examined DRG neuron excitability in mice treated with paclitaxel at all DRG levels, including thoracic DRGs that innervate the trunk. Consistent with the clinical studies cited above, they found that DRG neuron excitability is increased at all vertebral levels with paclitaxel treatment. Moreover, the TRAP-seq data with DRGs taken from all levels showed strong changes in translation regulation in DRG nociceptors. If only a subset of DRG neurons were influenced by paclitaxel treatment alteration in ribosome bound mRNAs in CIPN would likely not have been detectable with the inventors' experimental approach. These studies were undertaken soon after the cessation of paclitaxel treatment. In future work it will be interesting to assess whether these electrophysiological and translatome changes persist at later stages of CIPN. Nevertheless, this work supports the conclusion that the effects of chemotherapy on DRG neurons are widespread and not limited to the longest axons.

Translation regulation is a key contributor to chronic pain (Jimenez-Diaz *et al*., 2008; Geranton *et al*., 2009; Obara *et al*., 2012; Moy *et al*., 2017; Barragan-Iglesias *et al*., 2018; Khoutorsky and Price, 2018). These findings offer unprecedented insight into how translation regulation signaling is enhanced in Nav1.8-positive neurons in neuropathic pain. Previous studies have shown that peripheral nerve injury enhances mTORC1 signaling in sensory neurons (Geranton *et al*., 2009; Melemedjian OK, 2011), and a very recent study demonstrates that local translation of the *Mtor* mRNA is a key regulatory of the intrinsic neuronal response to DRG neuron axonal injury (Terenzio *et al*., 2018). The inventors' previous work demonstrated that MNK-eIF4E signaling is important for many types of pain sensitization (Moy *et al*., 2017; Moy *et al*., 2018). However, in the spared nerve injury model of neuropathic pain, MNK-eIF4E signaling primarily contributes to cold hypersensitivity with a substantially smaller effect on mechanical hypersensitivity (Moy *et al*., 2017). Here the inventors show a strong impact of MNK-eIF4E signaling on mechanical and heat hypersensitivity. Additionally, they show a contribution to spontaneous pain, a clinically relevant consideration that can be readily reversed in the paclitaxel model of CIPN. A key aspect of the current work is the demonstration of an integrated signaling network of mTORC1 and MNK-eIF4E that is persistently activated in CIPN. An important remaining question is how this signaling network is driven. The inventors discovered a large number of translationally upregulated mRNAs that compose the interface of mTORC1 signaling with the lysosome (Efeyan *et al*., 2012; Bar-Peled and Sabatini, 2014) (Fig 10G). This identifies lysosomal amino acid efflux as a likely contributor to CIPN and demonstrates how this fundamental cellular process is linked to changes in gene expression that drive increased DRG neuron excitability in this form of neuropathic pain. Importantly, the inventors identify many ion channels (TRPV1, $\alpha$6 nicotinic receptors (down-regulated), P2X2), GPCRs (Mrgprd) and enzymes (caspase 6) that are translationally regulated in Nav1.8-positive neurons by paclitaxel treatment providing a link between enhanced mRNA translation and neuronal excitability.

The inventors' electrophysiological findings demonstrate that eFT508 suppresses nociceptor spontaneous activity in DRG neurons isolated from mice treated with paclitaxel within approximately 20 min. Nociceptor spontaneous activity has been linked to spontaneous pain, which is a key behavioral manifestation of neuropathic pain in humans and preclinical models (Price and Gold, 2018). Consistent with their electrophysiological findings, the inventors did not observe signs of CIPN-induced spontaneous pain using the CPP model in mice lacking eIF4E phosphorylation. The rapid onset of effect of eFT508 in electrophysiological and behavioral experiments could point to an effect of MNK signaling on factors other than eIF4E phosphorylation. However, the inventors do not think this is the case because there were no additional effects of eFT508 when it was given to eIF4E phosphorylation mutant mice. The inventors' findings suggest that nociceptor excitability is controlled by the translation of a subset of mRNAs that encode proteins with short half-lives. The inventors hypothesize that these plasticity-mediating proteins may be scaffolding proteins that regulate rapid trafficking of ion channels to and from the cell membrane (Jeske, 2012; 2015). Identifying these proteins will be a goal moving ahead. Importantly, the TRAP-seq datasets identify a number of candidates including *Arl6, Dlg2* and *Ywhaq* genes.

The key finding emerging from this work is that inhibiting MNK-eIF4E signaling disrupts a novel translational circuit driving enhanced mTORC1 activity and nociceptor excitability in neuropathic pain. The inventors propose a model wherein this circuit is driven by MNK1-eIF4E mediated translation of *Rraga* mRNA. Since MNK inhibitors are in phase II clinical trials (Reich *et al*., 2018), this could provide a path for rapid development of a neuropathic pain therapeutic.

**Table 1: Details of each biological replicate showing the next generation assay type, input / immunoprecipitation pairing between replicates, number of mice used and their sex, and alignment quality statistics (number of mapped reads and number of reads mapping to many (>20) places in the genome)**

| | **Biosample details** | | | **Alignment quality** | |
|---|---|---|---|---|---|
| Internal ID | **Library type** | **Biosample #** | **Animal makeup** | **No of reads mapped** | **High ambiguity mapping (>20)** |
| IN2 | Vehicle Input | 1 | 2M+2F | 38,427,220 | 220,558 |
| IN3 | Vehicle Input | 2 | 2M+2F | 23,854,278 | 134,944 |
| IN5 | Vehicle Input | 3 | 2M+2F | 37,406,138 | 304,324 |
| IN6 | Vehicle Input | 4 | 2M+2F | 18,419,246 | 71,160 |
| IP2 | Vehicle IP | 1 | 2M+2F | 25,953,906 | 137,013 |
| IP3 | Vehicle IP | 2 | 2M+2F | 34,757,473 | 207,750 |
| IP5 | Vehicle IP | 3 | 2M+2F | 27,206,084 | 185,472 |
| IP6 | Vehicle IP | 4 | 2M+2F | 20,008,549 | 125,338 |

(continued)

| | Biosample details | | | Alignment quality | |
|---|---|---|---|---|---|
| Internal ID | **Library type** | **Biosample #** | **Animal makeup** | **No of reads mapped** | **High ambiguity mapping (>20)** |
| IN1_PAC | Paclitaxel treated input | 5 | 2M+2F | 29,990,168 | 287,912 |
| IN3_PAC | Paclitaxel treated input | 6 | 2M+2F | 37,060,526 | 316,835 |
| IN4+7_PAC | Paclitaxel treated input | 7 | 6M+2F | 44,559,848 | 457,455 |
| IN8_PAC | Paclitaxel treated input | 8 | 2M+2F | 21,387,544 | 166,589 |
| IP1_PAC | Paclitaxel treated IP | 5 | 2M+2F | 19,369,857 | 126,590 |
| IP3_PAC | Paclitaxel treated IP | 6 | 2M+2F | 43,695,404 | 288,640 |
| IP4+7_PAC | Paclitaxel treated IP | 7 | 6M+2F | 34,048,549 | 382,070 |
| IP8_PAC | Paclitaxel treated IP | 8 | 2M+2F | 20,293,108 | 188,669 |

**Table 2: Antibodies and chemicals used in the study**

| Reagent or Ressources | Source | Identifier |
|---|---|---|
| **Antibodies** | | |
| Anti-Akt produced in rabbit | Cell Signalling | 9272 |
| Anti-p-Akt produced in rabbit | Cell Signalling | 4060 |
| Anti-mTOR produced in rabbit | Cell Signalling | 2972 |
| Anti-p-mTOR produced in rabbit | Cell Signalling | 5536 |
| Anti-p-eiF4E produced in rabbit | Cell Signalling | 9741 |
| Anti-eiF4E produced in rabbit | Cell Signalling | 9742 |
| Anti-p-4EBP1 produced in rabbit | Cell Signalling | 2855 |
| Anti-4EBP1 produced in rabbit | Cell Signalling | 9452 |
| Anti-ERK1/2 produced in rabbit | Cell Signalling | 4695 |
| Anti-p-ERK1/2 produced in rabbit | Cell Signalling | 4366 |
| Anti-S6 produced in rabbit | Cell Signalling | 2217 |
| Anti-p-S6 produced in rabbit | Cell Signalling | 5364 |
| Anti-RagA produced in rabbit | Cell Signalling | 4357 |
| Anti-RPL10a produced in rabbit | Abcam | Ab174318 |
| Anti-Atp6v1a produced in rabbit | Thermo Fisher | PA5-29191 |
| Anti-CGRP produced in rabbit | Peninsula Laboratories | S3006.0001 |
| Anti-Peripherin produced in rabbit | Sigma-Aldrich | SAB4502419 |
| Isolectin B4 | Thermo Fisher | I21413 |
| Anti-NF200 produced in mouse | Sigma-Aldrich | N5389 |
| Anti-p-eIF4E produced in rabbit | Abcam | AB76256 |
| Htz-GFP produced in mouse | Memorial Sloan Kettering | 19F8 |
| Htz-GFP produced in mouse | Memorial Sloan Kettering | 19C7 |
| Anti-rabbit IgG, HRP linked antibody | Jacskon Laboratories | 111-035-003 |
| Anti-mouse IgG, HRP linked antibody | Jacskon Laboratories | 115-035-003 |
| Anti- Puromycin | DSHB | PMY2A4 |

(continued)

| Chemicals, Drugs | | |
|---|---|---|
| AZD8055 | LC Laboratories | A-2345 |
| eFT508 | eFFECTOR | MTA |
| Paclitaxel | Sigma-Aldrich | Y0000598 |
| Retigabine | Alomone Labs | R-100 |
| Emetine | Sigma-Aldrich | E-2375 |
| Cycloheximide | Sigma-Aldrich | C7698 |
| **Commercial Assavs** | | |
| Direct-zol RNA MicroPrep | Zymo Research | R2062 |
| Quantseq 3' mRNA-Seq library kit | Lexogen | 047 |
| RiboCop rRNA depletion kit | Lexogen | 042.08 |
| Qubit | Lexogen | Q32854 |
| iScript cDNA synthesis kit | Bio-Rad | 1708890 |
| SybR Green Supermix | Bio-Rad | 1725270 |
| Clarity ECL Substrate | Bio-Rad | |
| Immobilion Western Chemiluminescent | Millipore | WBKLS0500 |
| **Deposited Data** | | |
| RNA sequencing data | This paper | GEO...... |
| **Experimental Model: Organism/Strains** | | |
| Rosa26$^{fsTRAP}$ | Jackson Laboratory | 022367 |
| Nav1.8$^{Cre}$ | EMMA Infrafrontier | EM04582 |
| eIF4E$^{S209A}$ | Nahum Sonenberg | N/A |
| Mnk1$^{-/-}$ | Nahum Sonenberg | N/A |
| **Oligonucleotides** | | |
| GACAACTTTGGCATTGTGGA (SEQ ID NO: 9) | IDT | GAPDH-F |
| CATCATACTTGGCAGGTTTCTC (SEQ ID NO: 10) | IDT | GAPDH-R |
| ACGTCCGATTCTTGGGGAAC (SEQ ID NO: 11) | IDT | Rraga -F |
| TACGGAAGATGTTGTCCCGC (SEQ ID NO: 12) | IDT | Rraga -R |
| **Software and Algorythms** | | |
| GraphPad Prism v.6 | GraphPad Software | N/A |
| TopHat 2.1.1 with Bowtie 2.2.9 | Linux | N/A |
| GRCm38.p4 (genecode annotation .vM10) | Linux | N/A |
| Samtools 1.3.1 | Linux | N/A |
| Cufflinks 2.2.1 | Linux | N/A |
| FastQ 0.11.5 | Linux | N/A |

**Table 3: Sex of animals by genotype in behavioral experiments in this study.**

| Test | WT Males | WT Females | *eIF4E*$^{S209A}$ Males | *eIF4E*$^{S209A}$ Females | *MNK1*$^{-/-}$ Males | *MNK1*$^{-/-}$ Females |
|---|---|---|---|---|---|---|
| von Frey (Fig. 3C) | 4 | 2 | 2 | 5 | - | - |

(continued)

| Test | WT Males | WT Females | *eIF4E*$^{S209A}$ Males | *eIF4E*$^{S209A}$ Females | *MNK1*$^{-/-}$ Males | *MNK1*$^{-/-}$ Females |
|---|---|---|---|---|---|---|
| von Frey (Fig 3D) | 6 | - | 6 | - | - | - |
| Hot plate (Fig. 3E) | 2 | 4 | 3 | 3 | - | - |
| CPP (Fig. 3F) | 10 | 5 | 8 | 7 | - | - |
| von Frey (Fig 4B) | 5 | 7 | | | - | - |
| Hot plate (Fig. 4C) | 5 | 7 | - | - | - | - |
| von Frey (Fig. 5E) | - | 6 | | | 2 | 3 |

**Extended Table 4-1: 5 differentially expressed genes identified genes in the transcriptome after CIPN related to the data shown in Figure 1F and 1G**

| Gene name | Log2FC | p-value | FDR |
|---|---|---|---|
| Dpep2 | 1.982358644 | 0.001354256 | 0.147364404 |
| Car3 | 1.233007165 | 0.001452244 | 0.14827231 |
| Iah1 | 1.605613252 | 0.001345709 | 0.148386889 |
| Arhgef4 | 1.001632902 | 0.001388384 | 0.149116006 |
| Plcb3 | -0.772132393 | 0.001289632 | 0.148128566 |

**Extended Table 4-2: 230 identified genes significantly upregulated in Nav1.8-TRAP after paclitaxel related to the data shown in Figure 1E, 1F and 1G (Log2 Fold Change (FC) > 1.7)**

| Gene name | Log2FC | p-value | FDR | Gene name | Log2FC | p-value | FDR |
|---|---|---|---|---|---|---|---|
| 2010107E04Ri k | 2.11964072 6 | 0.00073768 3 | 0.08134185 | Mok | 7.25744564 3 | 0.00071369 | 0.08197525 6 |
| 2310039H08R ik | 5.14270083 8 | 0.00066146 3 | 0.08547341 5 | Mpp4 | 3.46529353 2 | 0.00419475 6 | 0.09201758 4 |
| 2700094K13Ri k | 3.30223529 | 0.00281463 3 | 0.08850574 5 | Mpv17 | 1.72999924 6 | 0.00256274 3 | 0.08757802 7 |
| 4833439L19Ri k | 2.13150191 9 | 0.01547348 | 0.13744971 2 | Mrpl18 | 1.68309886 1 | 0.00170538 5 | 0.08345287 5 |
| Aars2 | 2.41755279 8 | 0.00793134 5 | 0.10986971 6 | Mrpl34 | 2.37783917 8 | 0.01257151 6 | 0.12883077 5 |
| Acadvl | 1.92675832 4 | 0.00058347 9 | 0.09104480 2 | Mrpl36 | 2.80480474 3 | 0.00739987 6 | 0.10736311 9 |
| Acap3 | 3.49802213 9 | 0.00271064 6 | 0.08860492 2 | Mrpl42 | 1.78484920 6 | 0.00257689 5 | 0.08733985 |
| Acp1 | 1.94198898 4 | 0.00239343 5 | 0.08643542 1 | Mrps36 | 2.58451418 9 | 0.01365759 | 0.13148808 8 |
| Adamts8 | 3.56121540 6 | 0.00957817 | 0.11614584 8 | mt-Nd1 | 1.74029482 1 | 0.01590263 9 | 0.13887520 9 |
| Adcy6 | 2.71102654 4 | 0.00646199 7 | 0.09988919 2 | Mtor | 1.97324856 9 | 0.01687462 2 | 0.14124810 1 |
| Alkbh3 | 6.47573131 1 | 0.00100475 1 | 0.08566279 | Mxd3 | 1.90459758 6 | 0.00578653 5 | 0.09667605 5 |
| Amacr | 2.95482044 9 | 0.00501364 7 | 0.09597884 7 | Ndufa4 | 2.04080831 4 | 0.01079639 1 | 0.12098394 6 |
| Amd1 | 3.37101016 2 | 0.00741791 2 | 0.10724848 2 | Ndufa8 | 1.69912936 8 | 0.00298764 2 | 0.08919783 2 |
| Amer2 | 2.70584476 3 | 0.01053699 1 | 0.12052684 9 | Ndufaf7 | 2.35841053 4 | 0.00960321 5 | 0.11577054 2 |
| Anapc13 | 3.57501968 2 | 0.00344125 1 | 0.08921361 2 | Ndufs8 | 2.83709005 6 | 0.00046761 6 | 0.09915856 4 |
| Angptl2 | 2.18868846 6 | 0.01695005 8 | 0.14159291 | Nedd9 | 3.02264277 1 | 0.00440386 7 | 0.09362462 9 |
| Ankrd13b | 2.38497801 6 | 0.00981427 | 0.11711978 7 | Ngfr | 1.80476366 | 0.00488487 2 | 0.09572960 2 |
| Anxa5 | 1.87339426 | 0.00210273 7 | 0.08482750 7 | Nipsnap3b | 2.05711200 3 | 0.01618967 4 | 0.13874466 9 |
| Ap2m1 | 1.80168208 1 | 0.00272843 7 | 0.08848695 7 | Nme2 | 3.66005120 4 | 0.01610178 4 | 0.13899974 5 |
| Apip | 3.74612670 7 | 0.00471771 1 | 0.09633448 8 | Ntrk1 | 2.20019915 5 | 0.00978790 9 | 0.11697400 1 |
| Arl3 | 1.89073238 9 | 0.00370163 2 | 0.08898982 7 | Omg | 2.19442866 2 | 0.00616616 2 | 0.09787747 |
| Arl6 | 2.81623590 3 | 0.01272734 3 | 0.12883124 3 | Ost4 | 1.71816633 9 | 0.00362830 6 | 0.08903883 6 |
| Arpc1b | 2.30886994 4 | 0.00052129 9 | 0.09580310 5 | Pafah2 | 2.28266033 1 | 0.01646073 3 | 0.13962078 4 |
| Asna1 | 2.93889567 8 | 0.00643301 1 | 0.10000187 6 | Parm1 | 1.73204460 2 | 0.00166180 7 | 0.08329176 3 |
| Atg4c | 2.34694889 3 | 0.01426693 | 0.13407671 7 | Pcbd2 | 2.68724184 3 | 0.00597669 5 | 0.09710661 3 |
| Atp5c1 | 2.20831720 9 | 0.01552069 9 | 0.13742631 4 | Pcdhb9 | 2.51874047 8 | 0.01258540 9 | 0.12865430 4 |

(continued)

| Gene name | Log2FC | p-value | FDR | Gene name | Log2FC | p-value | FDR |
|---|---|---|---|---|---|---|---|
| Atp5j2 | 1.98751625 3 | 0.00059275 6 | 0.08912887 9 | Pex11b | 3.62835345 3 | 0.00378297 2 | 0.08913155 3 |
| Avpi1 | 2.24053012 4 | 0.00329213 4 | 0.08839593 3 | Pgrmc2 | 1.76016081 8 | 0.00141776 1 | 0.08496291 1 |
| Bcl3 | 1.88982403 2 | 0.01765920 8 | 0.14331859 3 | Phpt1 | 1.81360463 8 | 0.00355257 2 | 0.08957247 6 |
| Bloc1s1 | 1.94415053 7 | 0.00530593 5 | 0.09477340 2 | Plpp1 | 2.87358750 3 | 0.01050630 1 | 0.12067654 3 |
| Btbd2 | 1.76027356 6 | 0.00856392 7 | 0.11259725 8 | Pnrc2 | 1.66756306 1 | 0.01661878 4 | 0.14052898 3 |
| C77080 | 2.16857152 8 | 0.00321844 | 0.08901839 7 | Polr1c | 2.73089411 1 | 0.00838699 3 | 0.11187167 |
| Calca | 1.64318089 8 | 0.00198741 5 | 0.08177074 5 | Polr2j | 3.51815163 1 | 0.00240392 3 | 0.08606252 2 |
| Camk1 | 1.85649455 4 | 0.00721679 1 | 0.10619725 9 | Ppdpf | 2.03777342 8 | 0.00303307 8 | 0.08894878 9 |
| Caml | 1.86495203 | 0.00896828 1 | 0.11392885 3 | Ppp1ca | 2.09359172 2 | 0.01547785 8 | 0.13734107 5 |
| Card19 | 1.75334916 6 | 0.0018977 | 0.08048193 8 | Praf2 | 2.33504029 9 | 0.00344036 | 0.08947099 7 |
| Casp6 | 5.71964470 6 | 0.00010552 5 | 0.07272439 5 | Prdx5 | 2.17409024 6 | 0.01803478 2 | 0.14536807 7 |
| Ccm2 | 1.64317927 | 0.00962894 2 | 0.11574324 4 | Prg4 | 3.24683705 3 | 0.00330683 | 0.08850319 4 |
| Cct3 | 1.63849823 9 | 0.01004261 8 | 0.11780489 6 | Prkrir | 2.09638252 | 0.00226505 8 | 0.08592673 7 |
| Cd1d1 | 3.34481660 1 | 0.01645815 3 | 0.13974222 2 | Prkx | 2.20148643 8 | 0.00127140 4 | 0.08762095 7 |
| Cdc42ep3 | 3.24891594 4 | 0.00247252 4 | 0.08701180 3 | Psma2 | 2.07026813 7 | 0.00768086 | 0.10839712 2 |
| Cdpf1 | 1.81509371 7 | 0.00509841 4 | 0.09560971 5 | Ptdss1 | 1.68116169 3 | 0.01674109 8 | 0.14070008 2 |
| Cenpf | 2.90167870 4 | 0.00531075 7 | 0.09465508 4 | Ptp4a3 | 2.24753373 2 | 0.01698581 2 | 0.14160551 3 |
| Cfap206 | 3.05541283 9 | 0.00465661 8 | 0.09579660 3 | Ptprf | 1.74161147 4 | 0.00612631 6 | 0.09761972 5 |
| Cfd | 2.20059923 9 | 0.01493180 3 | 0.13614775 3 | Pxdc1 | 3.57815078 3 | 0.00337342 9 | 0.08970501 8 |
| Chchd10 | 3.15287777 | 0.00717890 7 | 0.10601706 8 | Rabac1 | 1.84080667 1 | 0.00553607 8 | 0.09498622 9 |
| Chp1 | 1.91607287 3 | 0.00088790 4 | 0.08638786 7 | Rad54l | 4.96484446 8 | 0.00047060 3 | 0.09729725 8 |
| Cisd2 | 2.37545533 3 | 0.00281807 1 | 0.08761446 4 | Rad9a | 1.78619251 3 | 0.00136805 7 | 0.08636514 1 |
| Cln5 | 3.44676748 | 0.00287860 2 | 0.08882849 6 | Rcn3 | 2.08496913 6 | 0.01096104 3 | 0.12216687 9 |
| Cnih4 | 1.92646175 7 | 0.00247115 | 0.08733509 1 | Rft1 | 3.18278907 | 0.00452525 5 | 0.09450468 7 |
| Cnrip1 | 2.13621667 5 | 0.00628949 5 | 0.09851159 6 | Rho | 5.35238538 5 | 0.00010110 4 | 0.08361275 9 |
| Cntfr | 2.49518557 8 | 0.01021026 4 | 0.11876073 2 | Rnase4 | 2.35608790 8 | 0.00185651 5 | 0.08080724 5 |

(continued)

| Gene name | Log2FC | p-value | FDR | Gene name | Log2FC | p-value | FDR |
|---|---|---|---|---|---|---|---|
| Coq3 | 4.10483523 | 0.00111672 3 | 0.08472753 6 | Rnh1 | 2.82669471 2 | 0.00114519 3 | 0.08532201 7 |
| Cox18 | 2.80620275 7 | 0.00371531 8 | 0.08905994 2 | Romo1 | 2.00209634 4 | 0.00300412 3 | 0.08936727 |
| Cox6b1 | 1.81750102 7 | 0.00010112 3 | 0.07602579 5 | Rpl10a | 2.76390890 2 | 0.00990016 1 | 0.11713065 5 |
| Cox7a2 | 2.17016476 9 | 0.00274474 6 | 0.08832316 5 | Rpl23a | 1.72820407 4 | 0.00210333 7 | 0.08443978 7 |
| Cox7b | 2.56601636 | 0.00484199 7 | 0.09579739 7 | Rpl37 | 2.31363840 4 | 0.00344938 8 | 0.08831715 6 |
| Crlf2 | 3.21383046 1 | 0.00525702 7 | 0.09534125 7 | Rpl39 | 2.27869440 1 | 0.00027618 9 | 0.09517009 1 |
| Dad1 | 2.45720146 2 | 0.01763613 2 | 0.14341279 6 | Rprm | 1.70270133 3 | 0.01465143 8 | 0.13508070 1 |
| Ddt | 1.82276948 3 | 0.00617687 3 | 0.09785965 4 | Rps2 | 2.44162350 8 | 0.01753806 8 | 0.14346174 |
| Ddx59 | 3.57724930 7 | 0.00310416 3 | 0.08944747 6 | Rps29 | 2.99446300 6 | 0.00170270 5 | 0.08381769 8 |
| Dhrs1 | 1.83251016 9 | 0.00203859 | 0.08346110 3 | Rraga | 1.84480102 1 | 0.01128066 9 | 0.12614300 4 |
| Dlg2 | 2.7339704 | 0.00174778 8 | 0.08259545 7 | Rtn4rl1 | 2.40725771 | 0.00260519 2 | 0.08687475 2 |
| Dnajb1 | 1.8875061 | 0.00341376 1 | 0.08962477 6 | Rwdd1 | 2.51054523 9 | 0.00066756 5 | 0.08364789 |
| Dnajc5 | 1.67245585 8 | 0.00772349 9 | 0.10862812 3 | Sag | 1.94768753 8 | 0.01890348 8 | 0.14776166 7 |
| Dnal4 | 3.15599501 5 | 0.00624720 1 | 0.09840828 5 | Scpep1 | 2.92347185 2 | 0.01440460 9 | 0.13460578 5 |
| Dolk | 3.02282260 9 | 0.00288093 2 | 0.08856990 6 | Sdhb | 3.23633494 3 | 0.00847037 9 | 0.11262063 4 |
| Dpm3 | 2.92710087 6 | 0.00355567 3 | 0.08937817 1 | Sec14l1 | 2.12902841 5 | 0.01792988 8 | 0.14480486 |
| Dzank1 | 2.22581967 3 | 0.01292894 3 | 0.12960285 6 | Serp2 | 2.80481868 6 | 0.00582203 6 | 0.09648945 7 |
| Edf1 | 2.36242869 7 | 0.00264442 6 | 0.08712909 5 | Serpina3n | 2.54941929 8 | 0.00601609 4 | 0.09717401 3 |
| Efcc1 | 3.20064701 | 0.01157719 3 | 0.12369946 3 | Serping1 | 3.00200183 9 | 0.00295518 4 | 0.08952149 3 |
| Eif3l | 1.62208847 2 | 0.01894994 8 | 0.14770600 1 | Shisa5 | 2.13722939 1 | 0.00516646 | 0.09601487 7 |
| Eif4a3 | 2.26891316 8 | 0.00890850 2 | 0.11369338 7 | Slc25a3 | 2.64131650 2 | 0.00300700 5 | 0.08881404 2 |
| Elk4 | 1.92757347 6 | 0.01317037 5 | 0.13059832 8 | Slc30a6 | 2.80945548 5 | 0.00989425 3 | 0.11722847 5 |
| Emd | 2.13526098 1 | 0.01021746 5 | 0.11867757 4 | Slc3a2 | 2.84090517 6 | 0.00033955 1 | 0.09360302 3 |
| Endov | 2.65238425 7 | 0.00641705 1 | 0.09994164 8 | Slc48a1 | 1.97747570 8 | 0.00386175 2 | 0.08896013 7 |
| Esd | 1.65840172 6 | 0.01036702 5 | 0.12007744 9 | Slc6a15 | 1.97929033 4 | 0.01825414 1 | 0.14642264 6 |
| Esyt1 | 3.65689506 7 | 0.00089190 6 | 0.08478231 1 | Smdt1 | 2.01612921 2 | 0.00068702 2 | 0.08355395 5 |

(continued)

| Gene name | Log2FC | p-value | FDR | Gene name | Log2FC | p-value | FDR |
|---|---|---|---|---|---|---|---|
| Fabp7 | 2.36549619 1 | 0.00237216 6 | 0.08680447 7 | Smug1 | 4.75437715 3 | 0.00022841 1 | 0.09941885 9 |
| Fads3 | 2.61857819 1 | 0.00359057 5 | 0.08971013 8 | Snrpg | 1.65651455 6 | 0.00056214 9 | 0.09115637 9 |
| Fam132a | 3.45301725 5 | 0.00258786 6 | 0.08699859 1 | Soat2 | 4.06584736 4 | 8.95589E-05 | 0.08229467 |
| Fam172a | 2.094451 | 0.00449401 1 | 0.09432860 3 | Sod1 | 1.77013120 8 | 0.00510321 5 | 0.09548322 9 |
| Fam189b | 1.80035378 7 | 0.01746499 7 | 0.14328920 9 | Sorbs1 | 2.94151137 | 0.01806739 6 | 0.14534762 9 |
| Fam43b | 4.13198984 7 | 0.00141826 9 | 0.08438191 9 | Spink10 | 3.16907658 1 | 0.00837490 8 | 0.11189092 9 |
| Fam89a | 3.21590679 2 | 0.00413053 2 | 0.09133555 2 | Stx2 | 1.89593708 7 | 0.00162887 3 | 0.08264283 1 |
| Fbxo2 | 2.96458674 4 | 0.01122893 6 | 0.12316087 8 | Synb | 3.10038479 9 | 0.00644476 9 | 0.09999669 2 |
| Fkbp1b | 1.71407666 6 | 0.01304274 5 | 0.13011279 2 | Syngr3 | 1.70064057 6 | 0.00972702 6 | 0.11641462 8 |
| Fsd1 | 3.04419895 4 | 0.00025108 4 | 0.09438490 1 | Tagln3 | 2.10051563 | 0.00698634 7 | 0.10429078 1 |
| Fxyd2 | 1.65339993 1 | 0.00560398 1 | 0.09496911 2 | Tdrp | 2.33485080 4 | 0.00987038 4 | 0.11711345 6 |
| Gcsh | 1.61000862 3 | 0.01159398 9 | 0.12371908 8 | Tgm2 | 2.73897453 3 | 0.01294542 8 | 0.12945427 6 |
| Gdf1 | 2.41948818 7 | 0.01368249 4 | 0.13142186 3 | Thoc7 | 2.07028927 3 | 0.00249897 6 | 0.08720055 1 |
| Gla | 1.79269112 6 | 0.00388051 7 | 0.08840738 1 | Timm8b | 2.81498039 4 | 0.00483063 | 0.09626340 2 |
| Gm20721 | 2.09285915 | 0.00154272 | 0.08449200 6 | Timm9 | 1.83762326 8 | 0.01331737 | 0.13033686 8 |
| Gm43518 | 4.85975263 9 | 0.00536096 3 | 0.09493611 6 | Tmem106 b | 1.89108101 7 | 0.0083182 | 0.11167454 2 |
| Gmppa | 2.03328933 7 | 0.01407816 2 | 0.13290685 2 | Tmem120 b | 3.03250478 6 | 0.00906510 8 | 0.11445564 |
| Gpr75 | 3.80981263 1 | 0.00132776 4 | 0.08646148 5 | Tmem126 a | 2.74434906 4 | 0.01086948 2 | 0.12130986 8 |
| Gstm1 | 3.15469558 5 | 0.01356616 8 | 0.13152662 7 | Tmem129 | 2.65495815 9 | 0.00471657 5 | 0.09654968 7 |
| H2-T23 | 3.90367597 1 | 0.00575957 1 | 0.09642035 7 | Tmem14c | 1.65739430 7 | 0.00177850 5 | 0.08171240 6 |
| H2afj | 2.76048297 2 | 0.00179509 8 | 0.08201913 8 | Tmem53 | 2.85138268 5 | 0.00628685 1 | 0.09865703 5 |
| H2afz | 1.71676827 5 | 0.00280261 | 0.08880301 1 | Tmem9b | 1.81776303 | 0.01713133 | 0.14210240 4 |
| Hagh | 3.06707340 9 | 0.00280425 | 0.08851584 2 | Tnfaip8l3 | 4.38009095 | 0.00033997 1 | 0.09069539 6 |
| Hhatl | 3.91536840 3 | 0.00065393 2 | 0.08865608 1 | Tonsl | 1.7622861 | 0.00125285 1 | 0.08855625 4 |
| Hist3h2ba | 2.22263837 6 | 0.00646377 6 | 0.09973027 7 | Tpgs2 | 2.07580629 | 0.01143821 6 | 0.12316932 8 |
| Hspa8 | 1.66596847 4 | 0.00079037 | 0.08273876 7 | Tram1 | 2.17403152 8 | 0.01935788 | 0.14933738 |

(continued)

| Gene name | Log2FC | p-value | FDR | Gene name | Log2FC | p-value | FDR |
|---|---|---|---|---|---|---|---|
| Idh3b | 1.90500836 3 | 0.01830362 8 | 0.14625217 9 | Trap1 | 2.44732077 3 | 0.01068536 2 | 0.12088638 4 |
| Idh3g | 1.61429583 3 | 0.0032334 | 0.08913407 1 | Trim11 | 2.39514756 3 | 0.01687593 5 | 0.14111626 5 |
| Ift122 | 2.37832088 5 | 0.00581928 | 0.09663744 7 | Trpv1 | 1.65660668 2 | 0.00156531 | 0.08298150 2 |
| Igtp | 2.40981472 5 | 0.00614124 1 | 0.09766934 9 | Tspan17 | 2.33744627 6 | 0.01866930 7 | 0.14676346 6 |
| Il15ra | 3.55097762 8 | 0.00333577 8 | 0.08898994 5 | Tubb4b | 2.11137386 9 | 0.00341205 5 | 0.08986527 1 |
| Kenip2 | 2.30825735 8 | 0.00696498 4 | 0.10415989 | Tyro3 | 2.92746978 5 | 0.01946921 | 0.14963788 7 |
| Kenip4 | 1.68021640 5 | 0.00140488 8 | 0.08735656 4 | Ucp1 | 3.79497463 1 | 0.00101285 1 | 0.08547223 3 |
| Krt28 | 5.48076752 7 | 0.00027629 5 | 0.09139838 2 | Ufsp2 | 1.92749828 6 | 0.00473204 7 | 0.09638922 6 |
| L3mbtl2 | 1.69062557 4 | 0.01372741 2 | 0.13170034 6 | Uqcrc2 | 3.51422686 | 0.00055884 9 | 0.09243367 9 |
| Lamtor5 | 1.97454465 1 | 0.00351057 2 | 0.08960627 6 | Uqcrq | 1.82523702 3 | 0.00092346 3 | 0.08485600 2 |
| Lcmt1 | 1.71217993 1 | 0.00709047 1 | 0.10527503 3 | Use1 | 1.74815534 8 | 0.01011239 2 | 0.11828781 1 |
| Lingo2 | 2.24682315 1 | 0.01270438 6 | 0.12875646 5 | Usmg5 | 1.79843985 1 | 0.00153449 8 | 0.08516976 9 |
| Litaf | 2.88755099 3 | 0.00263977 9 | 0.08767457 7 | Vezf1 | 1.93325450 6 | 0.01743537 2 | 0.14333054 1 |
| Lrrc55 | 5.43282743 2 | 0.00053124 9 | 0.09152971 3 | Wbp2 | 1.68938612 5 | 0.00029028 6 | 0.09233343 6 |
| Lxn | 2.11878645 2 | 0.00170960 6 | 0.08316731 7 | Wdr35 | 2.67952249 1 | 0.01904270 8 | 0.14814975 9 |
| Lyrm2 | 2.83188446 9 | 0.01523807 6 | 0.13653183 7 | Wdsub1 | 3.62115339 7 | 0.00295315 4 | 0.09012022 1 |
| Med6 | 2.47250793 9 | 0.01886851 8 | 0.14762785 4 | Ythdf2 | 1.86573178 3 | 0.01358296 9 | 0.13138147 |
| Mien1 | 1.84739187 6 | 0.00437164 1 | 0.09341981 8 | Ywhaq | 1.83695230 6 | 7.46045E-05 | 0.10282985 6 |
| Mmd | 2.02557151 7 | 0.01805585 6 | 0.14539623 3 | Zfp386 | 3.02220095 | 0.00535934 2 | 0.09511107 2 |
| Mob1a | 2.68284228 2 | 0.01074706 | 0.12075840 8 | Zfp467 | 2.1471192 | 0.00595383 8 | 0.09730877 8 |
| Mob3b | 2.17933076 2 | 0.00982338 2 | 0.11689117 3 | Zyx | 2.07968163 5 | 0.00938769 2 | 0.11553007 3 |

**Extended Table 4-3: 222 identified genes significantly downregulated in Nav1.8-TRAP after paclitaxel related to the data shown in Figure 1E, 1F and 1G (Log2FC < -1.2)**

| Gene name | Log2FC | p-value | FDR | Gene name | Log2FC | p-value | FDR |
|---|---|---|---|---|---|---|---|
| Abca7 | -1.41 | 0.006692702 | 0.101185819 | Kif3a | -1.68 | 0.007461962 | 0.107322479 |
| Abhd17c | -1.71 | 0.002248298 | 0.086080664 | Kif3c | -1.70 | 0.001800154 | 0.081351201 |
| Acin1 | -1.32 | 0.008363256 | 0.111916067 | Kin | -1.81 | 0.003259304 | 0.08808643 |
| Adamts15 | -1.59 | 0.018585454 | 0.147223856 | Klhdc4 | -1.28 | 0.013557817 | 0.131599938 |
| Adap1 | -1.42 | 0.004316945 | 0.092730213 | Kmt2d | -1.29 | 0.003097241 | 0.089874325 |
| Adora2a | -1.78 | 6.99304E-05 | 0.115664924 | Larp1 | -1.29 | 8.83803E-05 | 0.091363119 |
| Adrbk1 | -1.33 | 0.012685296 | 0.128878865 | Lbh | -1.24 | 0.00134447 | 0.08619202 |
| AI597479 | -1.41 | 0.001772906 | 0.081910231 | Leo1 | -1.59 | 0.008507391 | 0.112569799 |
| Akap1 | -1.26 | 0.012826459 | 0.129201967 | Lhfp | -1.28 | 0.012838918 | 0.129170132 |
| Akt1s1 | -2.67 | 7.80086E-05 | 0.092161583 | Lmbr1l | -1.38 | 0.005498462 | 0.095329732 |
| Anp32b | -1.99 | 0.00192581 | 0.080436593 | Maea | -1.27 | 0.002643502 | 0.087447048 |
| Ap1s1 | -1.47 | 0.001800716 | 0.080934345 | Map1a | -1.31 | 0.004743313 | 0.096145101 |
| Aplf | -1.68 | 0.000876585 | 0.086301894 | Med16 | -1.67 | 0.009586335 | 0.115735754 |
| Arf2 | -2.04 | 0.01478222 | 0.135530999 | Med29 | -1.51 | 0.008983431 | 0.11377178 |
| Arhgap26 | -1.23 | 0.005924719 | 0.097024609 | Mon1a | -1.46 | 0.000962477 | 0.086518296 |
| Arhgef25 | -1.58 | 0.014386449 | 0.134740579 | Mrpl14 | -1.81 | 0.00579203 | 0.096572754 |
| Armc8 | -1.27 | 0.001927092 | 0.08008567 | Mrpl2 | -1.25 | 0.013045365 | 0.129982128 |
| Arnt | -1.20 | 0.01945096 | 0.149636691 | Msn | -1.30 | 0.003104843 | 0.089156441 |
| Arrdc1 | -1.47 | 0.001861411 | 0.080596184 | Mterf4 | -1.37 | 0.001538656 | 0.084831246 |
| Arrdc3 | -1.24 | 0.001297219 | 0.087219545 | Myh9 | -1.44 | 0.004384805 | 0.093459622 |
| Atp2b1 | -1.52 | 0.003728914 | 0.089127517 | Nampt | -1.29 | 0.00143319 | 0.084060132 |
| Atp2b4 | -1.63 | 0.017324012 | 0.142841056 | Napg | -1.47 | 0.001238223 | 0.088276745 |
| Atp6v0a1 | -1.34 | 0.000438198 | 0.097943136 | Nat9 | -2.12 | 0.001545271 | 0.083525411 |
| Bak1 | -1.63 | 0.018344049 | 0.146433669 | Ncl | -1.20 | 0.019403721 | 0.149551515 |
| BC005561 | -1.40 | 0.007344367 | 0.106932948 | Ndufs5 | -1.63 | 0.01618359 | 0.138980574 |
| Bloc1s5 | -1.43 | 0.007028529 | 0.104543049 | Nefm | -1.60 | 0.003082514 | 0.089761949 |
| Borcs6 | -1.28 | 0.015970212 | 0.138587254 | Neurl4 | -1.43 | 0.014555907 | 0.134801068 |
| Btg1 | -1.60 | 0.015889365 | 0.13905296 | Nfe2l1 | -1.27 | 0.008177772 | 0.111051185 |
| Capn1 | -1.43 | 0.000404168 | 0.095499207 | Noct | -2.14 | 0.007502965 | 0.107166705 |
| Cars | -1.40 | 0.003947399 | 0.088950918 | Nuak1 | -1.69 | 0.00741882 | 0.107074413 |
| Ccdc157 | -1.74 | 0.014002661 | 0.132496577 | Nup54 | -1.41 | 0.013948404 | 0.132590004 |
| Ccdc34 | -1.50 | 0.009583869 | 0.116044792 | Nup88 | -1.51 | 0.003793576 | 0.089127482 |
| Ccdc71l | -1.29 | 0.007264112 | 0.106326022 | Nyap1 | -2.06 | 0.001083717 | 0.085355624 |
| Cct8 | -2.60 | 0.002518992 | 0.087163434 | Peo1 | -2.01 | 0.003534612 | 0.089666389 |
| Cdk11b | -1.90 | 0.005284205 | 0.09520779 | Pex6 | -1.84 | 0.006120603 | 0.097716965 |
| Cetn2 | -1.57 | 0.001586461 | 0.083038164 | Pfdn2 | -1.28 | 0.016349632 | 0.139249697 |
| Chfr | -1.62 | 0.011799391 | 0.124783846 | Pgls | -1.26 | 0.011094328 | 0.123154482 |
| Ciz1 | -1.65 | 0.013588972 | 0.131285982 | Phc2 | -2.17 | 0.00223677 | 0.08603761 |
| Ckb | -2.11 | 0.00086907 | 0.086592874 | Pih1d1 | -1.40 | 0.004490884 | 0.094502832 |

(continued)

| Gene name | Log2FC | p-value | FDR | Gene name | Log2FC | p-value | FDR |
|---|---|---|---|---|---|---|---|
| Clcn2 | -2.07 | 0.003594756 | 0.089544074 | Plcb3 | -1.72 | 0.00014362 | 0.079182519 |
| Cldn19 | -1.65 | 0.003025942 | 0.089055314 | Plec | -1.57 | 0.000329166 | 0.093869083 |
| Clip2 | -2.03 | 0.002341852 | 0.086848065 | Plxnd1 | -1.74 | 0.011440935 | 0.123038403 |
| Coq7 | -1.35 | 0.014384145 | 0.134871747 | Pom121 | -1.48 | 0.014452012 | 0.134441103 |
| Cpeb3 | -1.44 | 0.000508455 | 0.097788824 | Prcc | -1.28 | 0.019005702 | 0.148001088 |
| Ctns | -1.40 | 0.018267681 | 0.146247555 | Prkag2 | -1.43 | 0.000256281 | 0.092149623 |
| Ctu2 | -1.94 | 0.008866738 | 0.113686708 | Prph | -1.41 | 0.013068271 | 0.130053668 |
| Cysltr2 | -3.02 | 0.008954458 | 0.113928257 | Prrt2 | -1.23 | 0.00649133 | 0.099968907 |
| Ddx19b | -1.97 | 0.001409203 | 0.085691948 | Prune | -1.31 | 0.003972028 | 0.088780186 |
| Ddx56 | -1.27 | 0.000492122 | 0.096901116 | Psmb10 | -1.28 | 0.01734215 | 0.142706047 |
| Dgkz | -1.28 | 0.000635627 | 0.093868555 | Psmb3 | -1.64 | 0.019495979 | 0.149704497 |
| Dmtn | -1.22 | 0.014689549 | 0.135281259 | Psmd13 | -1.54 | 0.007686774 | 0.108295783 |
| Dock6 | -3.17 | 0.005845394 | 0.096682811 | Psmd4 | -1.37 | 0.005302218 | 0.094911989 |
| Dock9 | -2.20 | 0.005011128 | 0.096153208 | Psmd9 | -1.98 | 0.008564018 | 0.112419729 |
| Dpp8 | -1.67 | 0.002472905 | 0.086656463 | Ptms | -1.87 | 0.004952191 | 0.095912449 |
| Drg2 | -1.91 | 0.010681327 | 0.121006268 | Ptrf | -1.45 | 0.017633602 | 0.143533356 |
| Ebpl | -1.68 | 0.004208776 | 0.09183794 | Rassf5 | -1.26 | 0.000376847 | 0.091662472 |
| Eef1d | -1.74 | 0.001481955 | 0.083943613 | Rbm10 | -1.56 | 0.005533053 | 0.095131703 |
| Ehmt2 | -1.62 | 0.003685787 | 0.088867224 | Rbm34 | -1.45 | 0.012281394 | 0.127277105 |
| Eif2b4 | -1.36 | 0.007288884 | 0.106500127 | Rcc2 | -1.23 | 0.009652492 | 0.115857918 |
| Eif2b5 | -1.48 | 0.000466474 | 0.101519508 | Rfc2 | -1.35 | 0.000525879 | 0.092532325 |
| Eif3b | -1.26 | 0.00366993 | 0.089265653 | Rfwd3 | -1.58 | 0.014463557 | 0.134246484 |
| Elmod2 | -1.90 | 0.004877658 | 0.095815284 | Rltpr | -1.50 | 0.006639013 | 0.101113508 |
| Esf1 | -1.26 | 0.013093814 | 0.130151252 | Rnf2 | -1.56 | 0.010540868 | 0.120404669 |
| Ewsr1 | -1.46 | 0.005414213 | 0.095470241 | Rpl24 | -1.90 | 0.002129218 | 0.084251824 |
| Fahd1 | -2.10 | 0.011475018 | 0.123244674 | Rpl8 | -1.26 | 0.008285035 | 0.111410149 |
| Fam120c | -1.47 | 0.004280995 | 0.092438206 | Rpsa | -1.66 | 0.007804782 | 0.108845782 |
| Fam189a2 | -1.37 | 0.015532862 | 0.13724014 | Rragb | -2.50 | 0.007843241 | 0.109197983 |
| Fam195b | -1.54 | 0.001026891 | 0.084923916 | Rsl1d1 | -1.42 | 0.004249056 | 0.092230161 |
| Fam213a | -1.62 | 0.003097269 | 0.089560884 | Rundc1 | -1.57 | 0.009447339 | 0.115576177 |
| Fam98a | -1.31 | 0.003826615 | 0.089395774 | Sart3 | -1.38 | 0.002157935 | 0.084578771 |
| Fam98b | -1.46 | 0.005552546 | 0.094875107 | Sf3a3 | -1.51 | 0.009585507 | 0.115894944 |
| Fbxo31 | -1.21 | 0.003234606 | 0.08887108 | Sfxn4 | -1.50 | 0.015019562 | 0.136346629 |
| Fhdc1 | -3.22 | 0.005279909 | 0.095338097 | Slc17a7 | -1.61 | 0.006714951 | 0.101152354 |
| Flywch1 | -1.48 | 0.004302609 | 0.09266297 | Slc36a4 | -1.86 | 0.004741303 | 0.096340486 |
| Foxj2 | -1.51 | 0.01894315 | 0.147792314 | Slc37a1 | -1.36 | 0.005853033 | 0.096423473 |
| Frg1 | -1.29 | 0.003200447 | 0.088817768 | Slc4a10 | -1.59 | 0.015909677 | 0.138790113 |
| Gdap1l1 | -1.54 | 0.009179673 | 0.11467658 | Slc7a5 | -2.20 | 2.31413E-05 | 0.095689094 |
| Gng10 | -1.21 | 0.002588582 | 0.086670333 | Smad5 | -1.61 | 0.004901503 | 0.09560242 |

(continued)

| Gene name | Log2FC | p-value | FDR | Gene name | Log2FC | p-value | FDR |
|---|---|---|---|---|---|---|---|
| Golga7b | -1.87 | 0.001909364 | 0.080563454 | Smg6 | -1.24 | 0.017127473 | 0.142213057 |
| Gpank1 | -1.64 | 0.019102915 | 0.148339067 | Snf8 | -1.34 | 0.005170006 | 0.095437396 |
| Gpr179 | -2.00 | 0.01498054 | 0.136291604 | Sox4 | -1.27 | 0.007765699 | 0.108851405 |
| Gpx4 | -2.51 | 0.00018998 | 0.092419454 | Spef1 | -1.92 | 0.004990134 | 0.096196761 |
| Grcc10 | -1.79 | 0.001563515 | 0.083962787 | Sppl3 | -1.81 | 0.002555951 | 0.087708344 |
| Gtf3c1 | -1.36 | 0.004817553 | 0.096234699 | Srgap1 | -1.35 | 0.009156267 | 0.114904893 |
| Gtf3c3 | -1.88 | 0.003606567 | 0.089300329 | Supt16 | -1.58 | 0.003835092 | 0.088840936 |
| Gys1 | -1.76 | 0.000245862 | 0.096822605 | Syp | -1.87 | 0.018382069 | 0.146454441 |
| H2-D1 | -1.77 | 0.0135443 | 0.131778069 | Taco1 | -2.57 | 0.001744262 | 0.082902549 |
| H2afy2 | -2.12 | 0.001308043 | 0.086540124 | Tanc2 | -1.70 | 0.008503234 | 0.112695099 |
| Hccs | -1.64 | 0.011663701 | 0.123983039 | Taok2 | -1.38 | 0.009259735 | 0.114809608 |
| Hddc2 | -1.60 | 0.005092846 | 0.095722351 | Tap1 | -1.31 | 0.007978487 | 0.109787173 |
| Hdgf | -1.62 | 0.010634103 | 0.120636529 | Tarbp2 | -1.55 | 0.002815519 | 0.087865429 |
| Hint2 | -1.43 | 0.005168414 | 0.095621447 | Tbc1d10b | -1.74 | 0.000373055 | 0.09348976 |
| Hjurp | -1.21 | 0.013971147 | 0.132349811 | Tfe3 | -1.48 | 0.008762503 | 0.113405158 |
| Hmbox1 | -1.61 | 0.011079272 | 0.123152657 | Timmdc1 | -1.46 | 0.001802237 | 0.08056486 |
| Hmg20b | -1.56 | 0.008445314 | 0.112468193 | Tln1 | -1.29 | 0.001280647 | 0.087528499 |
| Hnrnpa2b1 | -1.50 | 0.004863143 | 0.095986144 | Tlx3 | -1.29 | 0.015054738 | 0.136366579 |
| Hnrnpd | -1.22 | 0.002308404 | 0.086382353 | Tmem164 | -1.34 | 0.013223175 | 0.130185311 |
| Hoxb4 | -1.63 | 0.000825516 | 0.083256348 | Tmem183a | -1.33 | 0.001889003 | 0.080943295 |
| Hps5 | -1.48 | 0.001772401 | 0.082346956 | Tmem263 | -1.43 | 0.01504702 | 0.136446113 |
| Hsp90b1 | -1.22 | 0.008208576 | 0.111104626 | Tnpo2 | -1.70 | 0.001442271 | 0.083409633 |
| Htr7 | -3.05 | 0.002583886 | 0.087219324 | Tomm34 | -1.25 | 0.003830744 | 0.088989486 |
| Ibtk | -1.59 | 0.007488668 | 0.107519591 | Tpm3 | -1.70 | 0.001824402 | 0.080683449 |
| Ift52 | -1.51 | 0.008697761 | 0.113276348 | Trp53bp2 | -1.59 | 0.001627938 | 0.083105248 |
| Irgq | -2.00 | 0.002412281 | 0.08598949 | Tsc22d3 | -1.32 | 0.009496045 | 0.115829337 |
| Jmy | -1.64 | 0.007407929 | 0.107291723 | Tsfm | -1.53 | 0.018760531 | 0.147340541 |
| Jph2 | -1.64 | 0.005233086 | 0.095746953 | Usp33 | -1.88 | 0.002397784 | 0.086215971 |
| Jund | -1.43 | 0.001675411 | 0.082967978 | Vps39 | -1.53 | 0.014451723 | 0.134589809 |
| Kat6a | -1.22 | 0.014624891 | 0.134986439 | Wbscr16 | -1.92 | 0.008014624 | 0.110101231 |
| Katna1 | -1.67 | 0.001203531 | 0.086549571 | Zbtb18 | -1.69 | 0.005474102 | 0.095106768 |
| Kdm1a | -1.25 | 0.008659278 | 0.113131483 | Zbtb38 | -1.30 | 0.007939486 | 0.109798572 |
| Kdm2a | -1.30 | 0.002986327 | 0.089481618 | Zkscan6 | -1.27 | 0.004774232 | 0.096065441 |

**Example 2**

**Materials and Methods**

**Animals.** Animals were housed on a 12 h light/dark cycle and had food and water available ad libitum. For experiments involving rule-shifting, animals were individually housed for the purposes of food restriction. All procedures were performed during the light cycle. All experiments were approved by the Institutional Animal Care and Use Committee at the University of Texas at Dallas and conducted in accordance with the National Institutes of Health and International

Association for the Study of Pain guidelines. 4EKI and MNK1 KO mice were a gift of the Sonenberg laboratory at McGill University (Furic *et al.* 2010; Ueda *et al.,* 2019) and bred at the University of Texas at Dallas (UTD) to generate experimental animals. Genotype was confirmed at weaning using DNA from ear clips. Experimental C57BL6/J wild-type (WT) animals were obtained from an internally maintained C57BL/6J colony at the University of Texas at Dallas. Twelve-week-old mice were used for all histology and rule-shifting experiments due to food restriction requirements (Jackson-Laboratory, 2019). All western blot, marble burying, and CPP behavioral experiments were conducted in 4-8-week-old mice with SNI being conducted at ~4-5 weeks old, and all behavioral end points being observed at 7-8 weeks old. Male mice were used in all rule-shifting and immunohistochemistry experiments, except for a single experiment comparing male and female 4EKI mice, due to the inventors' previously published work showing a sex difference in rule-shifting performance in which males are significantly more impaired than females [6]. As the inventors have never observed a sex difference in pain behaviors associated with pharmacological or genetic manipulations of the MNK-eIF4E system (Moy *et al.,* 2017; 2018a; 2018b; Megat *et al.,* 2019), they chose to use male and/or female mice in their experiments. The sex of the animal used in each experiment is indicated on the graphs. Experimenters performing behavioral experiments were blinded to genotypes and drug treatments.

**Drugs.** Tomivosertib (also known as eFT508) was provided by eFFECTOR under a Material Transfer agreement with UTD. Per eFFECTOR instructions, tomivosertib was dissolved in 10% 1-Methyl-2-pyrrolidinone, anhydrous (NMP; Sigma Cat# 328634), 90% Propylene Glycol (PG; Fisher Cat# P355-1) with ~1 eq. of HCl. Tomivosertib was given orally to WT mice at 10 mg/kg daily for 7 d for all rule-shifting, marble burying, von Frey, and histology experiments with the last injection occurring 1 hour before behavioral testing. For the CPP experiment, tomivosertib was given intraperitoneally (i.p.) at 10 mg/kg, but at a lower volume to avoid sedation from the drug vehicle (~50 μL per injection). The inventors confirmed this volume of tomivosertib, or vehicle did not anesthetize the animals by conducting rotarod and open field on a mixed-sex cohort of naive, vehicle, and tomivosertib-treated mice (FIGS. 11A-C). Intrathecal (i.t.) injections of clonidine (Sigma) were administered at 10 μg/5 μL, dissolved in 0.9% sterile saline.

**Conditioned Place Preference.** A previously published CPP protocol from the Walters laboratory protocol was used (Yang *et al.,* 2014). The CPP box consisted of one black and one white chamber connected by a striped chamber. CPP was conducted in a dark room with a single low lumens light source (Maia 69E120-WH). The middle chamber of the CPP box was brightly illuminated to discourage mice from spending time in the middle chamber. The black and white end chambers had a different floor texture and odor (scented chapsticks applied to the ceiling). On day 1 of testing (preconditioning), each mouse was permitted to explore the middle and black (but not white) chambers for 30 minutes. Conditioning occurred on days 2-4, except for a single experiment using tomivosertib which had only one conditioning day. On conditioning days, the mice were given either vehicle (tomivosertib experiments; i.p.) or nothing (clonidine experiments) and then 1h later they were confined to the black chamber for 30 minutes. Three hours later, the mice were given an injection of tomivosertib or vehicle (i.p., 10 mg/kg at 4mg/mL) or clonidine (i.t., 10 μg/5 μL), and 1 hour later they were confined to the white chamber for 30 minutes. On the last day of CPP, each mouse was placed in the middle chamber with unrestricted access to all chambers and video-recorded for 10 min. Data are presented as the percent change in time spent in the drug-paired chambered compared to the control (vehicle or sham for each genotype or surgery). This was calculated by dividing each animal's time spent in the white box by the control average, multiplying by 100 and then subtracting 100.

**Surgery.** Spared nerve injury (SNI) was performed by the ligation and cutting of the tibial and peroneal branches of the left sciatic nerve trifurcation, leaving the sural branch intact as previously described (Decosterd and Woolf, 2000). Sham surgeries were performed the same way but without cutting the nerve. Mice were allowed to recover for 2 weeks following surgery.

**Mechanical withdrawal threshold.** Tactile sensitivity was measured by probing the left outer surface of the left hindpaw with a series of calibrated von Frey filaments. Mechanical withdrawal thresholds were assessed in all animals before (baseline) and after (2 weeks) surgery to confirm neuropathy. Withdrawal thresholds were calculated using the up-down method (Chaplan *et al.,* 1994).

**Attentional rule-shifting.** Habituation: Mice were handled and gradually food restricted to 90% of their baseline weight over 3 weeks. Habituation took place over 3 days in which food rewards (Cheerio bits) were placed along each arm of a plusmaze. Mice were allowed to freely explore the maze and consume the food for 4 trials per day with a maximum time of 30 minutes per trial. When the mouse had consumed all of the food, the trial ended, and the mouse was returned to a holding cage while the maze was rebaited. The number of food pellets in each arm decreased from 4 to 2 to 1 on each consecutive day of habituation. Turn bias: On the fourth day the inventors assessed the animals' turn bias. Therefore, the plus maze was converted into a T-maze by blocking off one of the arms. Mice were placed in the stem arm (starting arm) and allowed to turn left or right to obtain a food pellet. After the mouse consumed the reward, it was returned to the stem arm and allowed to make another choice. If the mouse chose the same arm as on the initial choice, it was returned to the stem arm until it chose the other arm and consumed the food pellet. The direction of the initial turn chosen four or more times over seven trials was considered the turn bias. Response Discrimination: On the next day, mice were trained on a response discrimination task that required them to always turn into one arm (left or right, chosen opposite to the direction of their turn bias) to obtain the food reward. The location of the stem arm (starting arm) was pseudorandomly alternated among 3 arms

(East, West, and South) to prevent mice from using an allocentric spatial strategy to locate the food. A visual cue was placed close to the entrance of one of the choice arms. Placement of this cue into the right or left arm varied pseudorandomly to balance the frequency of occurrences in each arm across blocks of 12 consecutive trials. Similarly, the order of the stem arms alternated pseudorandomly in a balanced fashion across blocks of 12 trials. Training continued until the mouse made 9 correct choices over 10 consecutive trials or up to a maximum of 200 trials. When animals achieved this acquisition criterion, a probe trial was administered. In the probe trial the previously unused stem arm (North) was used as the starting arm. If the mice performed the probe trial correctly, Response Discrimination training was completed. If an incorrect turn occurred, response training continued until the mouse made another five consecutive correct choices, and then another probe trial was administered. Upon completion, the total number of trials was tallied, and animals were sorted into counterbalanced groups based on response values. SNI or sham surgeries were then conducted, followed by a 2-week recovery period. Retest: Approximately 3 weeks after surgery the mice were tested for retention of the response discrimination strategy. Retest day was a repeat of the response discrimination training day and took place on day 20 post surgery for all groups. Shift-to-Visual-Cue Discrimination: On the final day (day 21 post-surgery), mice were trained to shift their strategy to now follow the visual cue in order to obtain food rewards. The location of the visual cue and the position of the start arm were again varied pseudorandomly so that their frequency was balanced across blocks of 12 consecutive trials. The training and response criteria for the Shift-to-Visual-Cue Discrimination were identical to those during Response Discrimination. For the tomivosertib group, the final injection was delivered 1 hour prior to rule-shifting. Animals were timed while undergoing rule-shifting to account for any differences in locomotor or motivational behaviors due to analgesic treatment. Performance and Error Analysis: For each of the two test days, the inventors analyzed the total number of trials to criterion and the number of probe trials required to reach criterion. For the Shift-to-Visual-Cue Discrimination, errors were scored as entries into arms that did not contain the visual cue, and they were further broken down into three subcategories to determine whether the animals' treatment altered the ability to either shift from the previously learned strategy (perseverative errors), or to maintain the new strategy after perseveration had ceased (regressive errors, or never-reinforced errors). In order to detect shifts in the strategies that animals used, trials were separated into consecutive blocks of four trials each. A perseverative error (PER) occurred when a mouse made the same egocentric response as required during the Response Discrimination, but which was opposite to the direction of the arm containing the visual cue. Six of every 12 consecutive trials required the mouse to respond in this manner. A perseverative error was scored when the mouse entered the incorrect arm on three or more trials per block of 4 trials where a perseverative error opportunity was possible. Once the mouse made fewer than three perseverative errors in a block, all subsequent errors of the same type were now scored as regressive errors (REG) because at this point the mouse was following an alternative strategy at least half of the time. So-called never-reinforced errors (NRE) were scored when a mouse entered the incorrect arm on trials where the visual cue was placed on the same side that the mouse had been trained to enter on the previous day. Together, these error types provide insight into whether SNI and/or eIF4E manipulation alters the ability of animals to shift strategies (PE) and/or to maintain the new strategy (NRE, REG). Experimental design and data presentation: All rule-shifting experiments were done on cohorts of male and/or female animals that were run over the course of 12 months. There were 10 experimental groups: Male WT Sham + vehicle, Male WT SNI + vehicle, Male WT Sham + tomivosertib, Male WT SNI + tomivosertib, Male 4EKI Sham, Male 4EKI SNI, Female 4EKI Sham, Female 4EKI SNI, Male MNK1 KO Sham, Male MNK1 KO SNI. Animals in each group were staggered and groups were run in a balanced manner. The same WT SNI + vehicle (WT SNI) and sham + vehicle (WT Sham) groups are shown in each figure for comparison to genetically-modified and tomivosertib-treated animals.

**Marble burying task.** The marble burying task was used to assess obsessive compulsive disorder (OCD)-like behaviors as previously described [28]. Nineteen marbles were placed on top of 5 cm of standard bedding in a clean cage. A mouse was placed into the cage for 30 minutes and then the number of buried marbles was counted.

**Immunohistochemistry.** All mice were perfused at 3 weeks post-surgery (the day after rule-shifting). Mice were deeply anesthetized with a mixture of ketamine (80 mg/kg) and xylazine (12 mg/kg; i.p.) and then transcardially perfused with 1X phosphate buffered saline (PBS; 4°C, pH 7.4) followed by 10% formalin in 0.1 M phosphate buffer (PB; 4°C, pH 7.4; 25 mL at a flow rate of 5 mL/min) using a perfusion pump. Brains were dissected and transferred to a 30% sucrose in 0.1M PB. Once the brains sunk in sucrose, they were frozen in OCT over dry ice. Coronal sections (30 μm) were cut using a cryostat and a hole was placed in the left hemisphere to demarcate the left (ipsilateral) hemisphere. Free floating sections were then washed in 0.1M PB, and then incubated in blocking solution (10% Normal Goat Serum, 0.3% Triton-X 100 in 0.1M PB) for 1 hr at room temperature with gentle rocking/agitation. Sections were incubated in primary antibody (mouse-anti-AnkyrinG; UC Davis/NIH NeuroMab Facility, clone N106/36 RRID:AB_10673030; 2 μg/ml working concentration) diluted in blocking solution for 3 hrs at room temperature. Sections were washed 5 times in 0.1 M PB, and then incubated in secondary antibody (goat-anti-mouse 488 IgG2a, Thermo Fisher Scientific, A21131;1:2000) diluted in blocking solution containing DAPI (1:5000) for 1 hr at room temperature. Sections were washed 5 times in 0.1 M PB, mounted onto glass slides, cover-slipped using Prolong Gold Antifade (Thermo Fisher Scientific, P36930) and sealed with nail polish once cured.

**Rotarod.** Motor function was assessed using a rotarod (IITC Life Science rotarod series 8) with ramp speed

(20rpm acceleration) at a starting speed of 4pm for a maximum of 120s. The time until each animal fell was recorded for each trial. Naive mice were trained for 1 trial, and then 2 baseline trials were conducted and scores for both trials were averaged. Tomivosertib (10 mg/kg i.p ~50 μL) or vehicle was then injected, and 1 hour later all mice were tested twice more, and their scores were averaged.

**Open field.** Naive, tomivosertib (10 mg/kg i.p. ~50 μL) or vehicle-treated mice were tested in an open field (60 cm x 60 cm white box) 1 hour after injection. Movement in the maze was video recorded for 10 minutes via ceiling-mounted cameras and tracked using a custom script in MATLAB-2015b. The script outputs total distance traveled and path tracking.

**Image Analysis.** Axon initial segments (AIS - Ankyrin-G antibody): A minimum of 3 z-stacked images of infralimbic cortex (IL) were acquired at 40X magnification for each animal using an Olympus confocal microscope (FV1200). Images were converted to tiff format with randomized file names (for blinding). Images were brightened and contrasted with the same settings across all groups. For each image, 30 segments were analyzed in layers 2/3 and layers 5/6 of the ipsilateral and contralateral IL in CellSens (Olympus) using the polyline measure tool. Length measurements were averaged (3 images per animal). 100X images are also shown in the figures.

**Western blotting.** Naive WT mice were decapitated following anesthesia 1 hour after tomivosertib (p.o. or i.p.) or vehicle injection. The mPFC and DRGs were rapidly microdissected under a dissecting microscope and then flash frozen on dry ice. The mPFC was similarly collected from a separate group of WT male and female sham and SNI mice at 21 days post injury (the same time point as all rule-shifting experiments). Frozen tissues were homogenized with a pestle in lysis buffer (50 mM Tris, pH 7.4, 150 mM NaCl, 1 mM EDTA, pH 8.0, and 1% Triton X-100) containing protease and phosphatase inhibitors (Sigma-Aldrich), and then sonicated briefly. Samples were centrifuged at 14,000 rpm for 15 min at 4°C. 10 μg of protein was loaded into each well and separated by a 10%-12% SDS-PAGE gel. Proteins were transferred to a 0.45 μm PVDF membrane (Millipore) at 30 V overnight at 4°C. The next day, membranes were blocked with 5% non-fat dry milk (NFDM) in 1X Tris buffer solution containing Tween 20 (TTBS) for 1 h. Membranes were washed in 1X TTBS 3 times for 5 min each, then incubated with primary antibody overnight at 4°C. The primary antibodies used were: phospho-eIF4E (abcam ab76256; 1:1000), GAPDH (Cell Signaling Technology 2118S; 1:10000), total eIF4E (Cell Signaling Technology 9742S; 1:1000). The following day, membranes were washed 3 times in 1X TTBS for 5 min each, then incubated with the corresponding secondary antibody at room temperature for 1 h. Membranes were then washed with 1× TTBS 5 times for 5 min each. Signals were detected using Immobilon Western Chemiluminescent HRP substrate (Millipore). Bands were visualized using a Bio-Rad ChemiDoc Touch. Membranes were stripped using Restore Western Blot Stripping buffer (Thermo Fisher Scientific) and reprobed with another antibody. Analysis was performed using Image Lab (Bio-Rad). Data are presented as p-eIF4E/total eIF4E normalized to control.

**Extracellular electrophysiology.** Substrate-integrated microelectrode array (MEA) recordings were performed using PDL and laminin pre-treated 12-well MEA plates (Axion Biosystems, USA) and an Axion Maestro recording system. DRG sensory neurons were dissected, dissociated, and seeded as previously described (Moy *et al.,* 2017). Tomivosertib experiments were carried out 15 days after DRG culture seeding. Cultures were maintained at 37 °C, 5% $CO_2$, and ≥90% humidity throughout the recordings. Briefly, 30 min baseline recordings were carried out following a 30 min acclimation period. Following the baseline recording period, cultures were treated with either interleukin-6 (IL-6; 100 ng/ml final concentration; n=11 wells) only, IL-6 plus tomivosertib (25 nM final concentration; n=11 wells), or tomivosertib only (n=7 wells). Recordings were then collected continuously for 3 hours. IL-6 and tomivosertib were added at 1:1000 as high-concentration boluses. Online and offline MEA data analysis was performed using Axion's AxIS, NeuralMetrics Tools, and NeuroExplorer (NeuroExplorer, USA). Physiologically relevant extracellular action potentials were defined as previously described [29].

**Data Analysis and Statistics.** Graphs and statistical analyses were generated using GraphPad Prism version 7.01, except for the MEA experiment, which was assessed using Axion's AxIS, NeuralMetrics Tools, and NeuroExplorer. Statistical differences between groups were assessed using one- and two-way ANOVAs followed by Bonferroni corrected multiple t-tests for analysis with more than three groups or unpaired t-tests for tests with only two groups as indicated in the text and figure captions. All statistics including F and exact p-values are shown in Tables 6A, 6B, and 6C). All data are represented as mean ± SEM with $p < 0.05$ considered significant. The sample size and sex of all subjects are indicated in the graphs and/or figure captions.

## Results

**Genetic inhibition of eIF4E phosphorylation rescues pain-related cognitive impairment in an attentional rule-shifting task.** The inventors used a rule-shifting task (FIG. 17A) to assess neuropathic pain-related impairments in cognitive flexibility in WT and 4EKI mice, a genetically modified mouse strain in which the MNK phosphorylation site on eIF4E is mutated to render it phosphorylation null. On response training day, groups showed no differences in acquisition of the response training strategy (FIG. 11B). The next day, the animals were given SNI or sham surgeries and they were then retested on the original response strategy 20 days post-surgery. Male 4EKI and WT animals showed no deficits on retest day, indicating that neither surgery nor eIF4E phosphorylation disrupts retention of the initial response strategy (FIG.

11B). On day 21 post-surgery, all groups were required to switch strategies (rule-shifting) to receive food reward. Male WT SNI mice were significantly impaired on rule-shifting day when compared to WT sham, 4EKI SNI, and 4EKI sham mice (FIG. 11B). Male SNI WT mice made significantly more never-reinforced (NRE) and regressive (REG) type errors, but not perseverative errors (PER), compared to WT sham controls and 4EKI sham and SNI mice, suggesting there was an issue with maintenance of the new strategy (FIG. 11C). The inventors have previously shown a large sex difference in rule-shifting performance with males being significantly more impaired than females in the attentional rule-shifting task (Shiers *et al.,* 2018), so they focused most of the rule-shifting experiments on male mice. However, when they tested female 4EKI mice, female SNI mice showed no impairment on response, retest, nor rule-shifting day when compared to female sham or male sham or SNI mice (FIG. 11D).

**Disruption of eIF4E phosphorylation prevents PNI-induced spontaneous pain.** Female WT and 4EKI mice with sham or SNI surgeries were assessed for ongoing pain in a CPP apparatus in which the conditioning drug was clonidine, an $\alpha 2$ adrenergic receptor agonist (King *et al.,* 2009). Following conditioning, WT SNI animals spent significantly more time (~30% more time) in the clonidine-paired chamber compared to WT sham controls, suggesting that the chamber was rewarding due to analgesia. 4EKI SNI mice showed no significant difference in time spent in the drug-paired chamber compared to 4EKI sham controls (FIG. 12A), indicating that the loss of eIF4E phosphorylation protects against the development of spontaneous pain following PNI (FIG. 12A). WT and 4EKI SNI animals were mechanically hypersensitive when withdrawal thresholds were assessed 14 days post-surgery (FIG. 12B). Importantly, clonidine significantly attenuated mechanical hypersensitivity when assessed 2 hours after injection on each CPP conditioning day (FIG. 12B), demonstrating that clonidine is pharmacologically active in both genotypes. These experiments indicate that although 4EKI mice show mechanical allodynia after PNI, they fail to display signs of spontaneous pain.

**Pharmacological and genetic inhibition of MNK reverses PNI-induced impairments in rule-shifting.** Experiments in 4EKI mice implicate eIF4E phosphorylation in PNI-induced spontaneous pain and rule-shifting deficits but they do not determine whether these effects can be reversed via inhibition of the MAPK signaling pathway once PNI-neuropathic pain is well-established. The FIG. hypothesized that pharmacological inhibition of MNK1/2, the only two known kinases responsible for phosphorylation of eIF4E, would fully reverse rule-shifting deficits. Before surgery and drug treatment naive WT male animals were trained on the response strategy. There was a significant difference in baseline performance in this cohort of mice; however, these differences disappeared once animals successfully learned the original discrimination, and accordingly no behavioral differences were observed on Retest Day (FIG. 13A). Two weeks following surgery, WT SNI and sham animals were given a daily treatment of either vehicle or tomivosertib (10 mg/kg p.o.) for 7 days. Retest took place on day 6 of injections, and all groups showed no differences in retention of response training (FIG. 13A). Rule-shifting took place one hour after the final injection on day 7. SNI animals treated with tomivosertib showed no cognitive impairment on rule-shifting day when compared to SNI + vehicle animals (FIG. 13A). Tomivosertib-treated SNI animals made significantly fewer NRE and REG type errors compared to the SNI + vehicle group (FIG. 13B). Despite the profound effect on PNI-induced rule-shifting deficits, tomivosertib-treatment had no effect on mechanical withdrawal thresholds when assessed 1 or 3 hours after treatment on days 1 and 5 of the 7-day treatment schedule (FIG. 13C).

Tomivosertib acts via both MNK1 and 2. The inventors' previous experiments in chemotherapy-induced neuropathic pain suggest that MNK1 is a key isoform driving pain induced by chemotherapy (Megat *et al.,* 2019). To test whether MNK1 plays a similar dominant role in PNI-induced neuropathic pain, the inventors used a full-body knockout strain for MNK1. They tested these mice in the rule-shifting task. Naive WT and MNK1 KOs mice showed no differences in performance on response training day, indicating knockout of the MNK1 gene does not impair acquisition of the initial response strategy (FIG. 13D). However, when tested 20 days after surgery (retest day), the MNK1 KO animals showed significantly impaired retention of response training, suggesting that MNK1 may be important for long-term memory (FIG. 13D). However, MNK1 KO mice with SNI showed no deficit in rule-shifting performance (FIG. 13D) and made significantly fewer NRE and REG errors when compared to WT SNI animals (FIG. 13E). Similar to previous reports in neuropathic 4EKI mice (Moy *et al.,* 2017), MNK1 KO SNI animals showed a slight attenuation in tactile allodynia 4 days after surgery compared to WT SNI animals but showed no differences to WT SNI animals on days 7, 11, 14, and 17 post SNI (FIG. 13F).

While the number of trials to criterion during rule-shifting did not differ from WT sham mice, MNK1 KO SNI animals took significantly longer to complete the rule-shift, with each trial taking ~1.5-2 minutes on average to complete (FIG. 16A). WT mice took less than 1 min, on average. These MNK1 KO animals appeared distracted in the maze and were grooming, staring at walls, or repetitively jumping to escape the maze, leading to the prolonged rule-shifting time. The inventors did not observe the same type of behaviors during rule-shifting in either the 4EKI mice (not shown), nor in the tomivosertib-treated animals (FIG. 16B). They used a marble burying task to test if these behaviors were reflective of obsessive-compulsive disorder (OCD)-like traits. The tomivosertib-treated and vehicle controls showed no differences in marble burying compared to the WT naïve group (FIG. 16C); however, the 4EKI and MNK1 KO mice buried significantly more marbles than WT naive mice, suggesting that disruption of eIF4e signaling throughout development causes OCD-like symptoms (FIG. 16C). Additionally, the inventors observed another OCD-like behavior in 4EKI and MNK1 KO mice (but not WT animals) in which these animals display stereotypic jumping behaviors, especially during their dark cycle (data not shown).

**Pharmacological targeting of MNK1/2 reverses PNI-induced spontaneous pain.** The pharmacokinetics of tomivosertib have been described in detail (Reich *et al.,* 2018); however, in order to understand if tomivosertib can reduce spontaneous pain in PNI mice, the inventors examined the onset of tomivosertib effects after p.o. or i.p. dosing. As elevated eIF4E phosphorylation has been previously observed in DRG neurons and their axons in chronic pain conditions (Melemedjian *et al.,* 2011; Megat *et al.,* 2019), the inventors investigated how tomivosertib affected eIF4E phosphorylation in lumbar (L3-L5) DRG, sciatic nerve, and mPFC. Western blot analysis of mPFC, DRG, and sciatic nerve from naive mice revealed a rapid and profound decrease in eIF4E phosphorylation 1 hour after tomivosertib treatment via both administration routes (FIG. 14A).

Having determined that tomivosertib acts rapidly to inhibit eIF4E phosphorylation after i.p. dosing, the inventors sought to determine if the drug could induce CPP in animals with SNI. To do this they used a single conditioning day of tomivosertib or vehicle (i.p) pairings with the white chamber. SNI animals that received tomivosertib in the white chamber spent ~40% more time in that chamber on test day compared to SNI animals that only received vehicle. This 40% increase in time was significantly different than the change in time in the sham-tomivosertib treated animals (FIG. 14B). This result supports the conclusion that MNK1/2 inhibition with tomivosertib can reduce spontaneous pain caused by PNI.

Although the inventors used oral dosing of tomivosertib for the rule-shifting experiment, they used i.p. injections in their CPP experiment to avoid a potential stressaversion to the conditioning chamber brought about by the gavage. The concentration of the drug was raised in order to deliver a smaller volume of vehicle and drug as the vehicle given at high volumes intraperitoneally can anesthetize the animal (note: oral injections do not have this effect). The mice appeared active in their cages 1 hr after drug-administration at the lower volume and the inventors confirmed this observation by open field (FIG. 17A) and by rotarod (FIG. 17C). No significant differences were seen between naive, vehicle nor tomivosertib-treated mice in the rotarod test. Total distance traveled and movement speed was decreased in the vehicle and tomivosertib-treated mice compared to naive controls in the open field; however, they were still active as exemplified in their exploration maps (FIG. 17B).

Given the CPP effect, the invnetors assessed whether tomivosertib could reduce nociceptor firing and elucidate a mechanism for the reduction in spontaneous pain. They used microelectrode array recordings of cultured mouse DRG neurons exposed to interleukin 6 (IL-6), a cytokine that evokes pain via an action that is dependent on nociceptor expression of its receptor gp130 and that depends on eIF4E phosphorylation. Tomivosertib significantly reduced spontaneous firing induced by IL-6 treatment (FIG. 14C).

Finally, the inventors assessed if eIF4E phosphorylation is changed in the mPFC after SNI in male or female mice. The inventors' previous work has demonstrated that eIF4E phosphorylation is profoundly upregulated in the DRG of chemoneuropathic mice (Megat *et al.,* 2019) and in the sciatic nerve of SNI mice and rats over a similar time course to experiments performed here (Melemedjian *et al.,* 2011). In contrast to the peripheral changes in eIF4E phosphorylation brought about by PNI, they did not observe a difference in eIF4E phosphorylation in the mPFC 3 weeks after injury (FIG. 14D).

**Inhibition of eIF4E signaling reverses PNI-induced changes in AIS length in the infralimbic cortex.** Structural changes in AIS length are linked to changes in neuronal excitability and homeostatic plasticity. Increased or decreased presynaptic input will shorten or lengthen, respectively, the AIS and shift the action potential initiation site to reduce or augment neuronal excitability (Grubb & Burrone, 2010; Kuba *et al.,* 2010; Grubb *et al.,* 2011). The inventors have previously shown that SNI leads to a reduction in AIS length in layer 5/6 of the infralimbic cortex ipsilateral and contralateral to the site of SNI. This AIS shrinkage correlates to deficits in rule-shifting performance caused by PNI (Shiers *et al.,* 2018). They assessed if this structural plasticity was also present in 4EKI, MNK1 KO, and tomivosertib-treated animals. Tomivosertib-treated animals were given p.o. treatments (10 mg/kg) for 7 days starting on day 14 after SNI. The inventors observed axon initial segment shrinkage in layer 5/6 of the ipsilateral and contralateral infralimbic cortices (FIG. 15A) in WT SNI (FIG. 15B) male mice, but not male 4EKI (FIG. 15C), male MNK1 KO (FIG. 15D), nor tomivosertib treated male SNI mice (FIG. 15E). This finding correlates with behavioral performance after SNI in these treatment groups and strongly supports the idea that spontaneous pain may drive rule-shifting deficits and mPFC pathology after PNI.

**Discussion**

Cognitive dysfunction is a debilitating comorbidity of chronic pain which diminishes quality of life in patients. Commonly prescribed analgesics often have no effect or even worsen pain-related cognitive impairment, indicating that there is a disconnect between the management of pain and its comorbidities (Eccleston, 1995; Povedano *et al.,* 2007; Schiltenwolf *et al.,* 2014; Richards *et al.,* 2018; APF, 2006). Human brain neuroimaging (Apkarin *et al.,* 2004; Schmidt-Wilcke *et al.,* 2005; Davis *et al.,* 2008; Kuchinad *et al.,* 2007) and preclinical studies (Shiers *et al.,* 2018; Metz *et al.,* 2009; Seminowicz *et al.,* 2009; Ji *et al.,* 2010; Zhang *et al.,* 2015; Kelly *et al.,* 2016; Cheriyan & Sheets, 2018) have revealed that chronic pain causes functional and morphological reorganization of prefrontal cortex subregions which may contribute to pain-induced cognitive dysfunction. However, the underlying mechanisms behind these changes are still unknown, making it difficult to generate new therapeutics that can simultaneously target pain and comorbidities of the disease. The

inventors' findings show that targeting MNK1-eIF4E signaling inhibits and reverses rule-shifting deficits associated with PNI, and it reduces spontaneous pain, which is the most important clinical complaint of neuropathic pain patients. The inventors conclude that the specific MNK1/2 inhibitor tomivosertib has great potential to impact neuropathic pain.

An important question is whether the effects the inventors have observed here are mediated by a central or peripheral site of action. The inventors hypothesize that the site of action is likely peripheral. Previous studies in multiple neuropathic pain models in both rats and mice have demonstrated that eIF4E phosphorylation is increased in DRG neurons and their axons over the course of neuropathic pain (Melemedjian *et al.,* 2011; Megat *et al.,* 2019). Moreover, electrophysiological experiments using tomivosertib and other MNK inhibitors, as well as genetic experiments in 4EKI mice, have shown that many cytokines and growth factors associated with neuropathic pain fail to sensitize nociceptors when MNK signaling is blocked (Moy *et al.* 2017). This also includes reversal of spontaneous activity in nociceptors from mice treated with chemotherapy (Megat *et al.,* 2019). Insofar as spontaneous activity in nociceptors is likely the key driver of spontaneous pain in rodent models and in patients suffering from neuropathic pain, MNK inhibition might be particularly suited to address this most salient feature of neuropathic pain. In support of this conclusion, the inventors found that tomivosertib-treatment resolved ectopic activity after IL-6 treatment in cultured DRG neurons and induced CPP in neuropathic mice.

Genetic and pharmacological targeting of eIF4E also reversed deficits in rule-switching performance. The inventors' interpretation of these findings is that disruption of MNK-eIF4E signaling in the DRG is leading to a resolution of ectopic activity in nociceptors, and of ascending nociceptive signaling to the brain, resulting in improved cognitive function. Indeed, ascending nociceptive projections terminate in several key brain regions that project to the mPFC and are implicated in cognitive dysfunction in pain. The most compelling evidence supports a central and basolateral amygdala (CeA-BLA) projection in which nociceptive information arising from the spino-parabrachio-amygdala pain pathway (Gauriau & Bernard, 2002) leads to enhanced activity in CeA (Goncalves & Dickenson, 2012) and BLA neurons after injury (Ji *et al.,* 2010; Neugebauer *et al.,* 2004). BLA hyperactivity in models of arthritic pain leads to enhanced glutamatergic signaling to the PrL resulting in augmented feedforward inhibition, PrL deactivation and cognitive impairment (Ji *et al.,* 2010). It is likely that resolution of nociceptor ectopic activity via MNK-eIF4E inhibition corrects this enhanced nociceptive drive, leading to restoration of cognitive function in the rule-shifting task.

Importantly, the inventors did not see signs of increased MNK-eIF4E signaling in the mPFC of SNI mice, suggesting that the mPFC is unlikely to be the site of action of genetic or pharmacological interventions. However, tomivosertib clearly crosses the blood-brain-barrier as evidenced by western blot analyses of eIF4E phosphorylation after drug-treatment. Therefore, it is possible that a decrease in eIF4E phosphorylation from baseline levels could be responsible for restoration of cognitive function after SNI; however, the inventors do not have evidence to support this conclusion. Additionally, it is possible that eIF4E signaling could be disrupted in other regions that project to the mPFC. To distinguish between central and peripheral contributions, nociceptor-specific manipulation of MNK1 and development of a peripherally-restricted MNK1/2 inhibitor will be a priority for future investigations.

While the inventors favor a peripheral hypothesis, the inventors cannot rule out an effect of eIF4E phosphorylation in the CNS. eIF4E dysregulation has been identified in a variety of neuropsychiatric disorders including autism, fragile x syndrome, schizophrenia, and depression/anxiety [50]. MNK-eIF4E signaling appears to regulate serotonin neurotransmission in the brain, and deficits in eIF4E phosphorylation can lead to depressive phenotypes (Amorim *et al.,* 2018). Those effects can be recapitulated with pharmacological treatments in adult animals (Aguilar-Valles *et al.,* 2018). While genetic manipulation of MNK-eIF4E caused OCD-like behaviors in the inventors' experiments, pharmacological inhibition of MNK using tomivosertib, even over a week of treatment, did not have an effect. This suggests that the OCD-like phenotype the inventors observed in both genotypes is developmentally regulated and is unlikely to arise with pharmacological treatment in adults.

It is notable that the inventors observed strong effects on spontaneous pain and attentional rule shifting without any clear effect on mechanical allodynia. This is likely explained by a difference in the type of afferent fibers that are required for mechanical allodynia in different models. Mechanical allodynia, as measured with punctate stimulation with traditional von Frey filaments, is absent in inflammatory mouse models when all nociceptors are ablated, whereas SNI-induced mechanical allodynia persists in the absence of these fibers (Abrahamsen *et al.,* 2008). This indicates that Aβ fibers are sufficient to mediate mechanical allodynia following SNI. On the other hand, cold allodynia requires the TRPM8-population of nociceptors (Knowlton *et al.,* 2013). In MNK KO and 4EKI mice many forms of inflammatory mechanical allodynia and cold hypersensitivity after SNI are strongly attenuated (Moy *et al.,* 2017). This indicates that nociceptors are most strongly affected by inhibition of MNK-eIF4E signaling. Insofar as nociceptor spontaneous activity is a key driver of spontaneous neuropathic pain (North *et al.,* 2019), and inhibition of MNK-eIF4E signaling also reduces this form of intrinsic plasticity in these cells (Megat *et al.,* 2019), this effect likely explains why MNK inhibition reduces spontaneous pain. This effect on spontaneous pain occurs within 1.5 hrs *in vivo* as shown by the CPP experiment time course. The inventors' previous work demonstrates that MNK inhibition leads to resolution of spontaneous activity in nociceptors taken from mice with neuropathic pain within 20 minutes (Megat *et al.,* 2019). It is interesting that such a strong effect on spontaneous pain cannot influence mechanical allodynia in neuropathic pain, but this may reflect a lack of effect of MNK-eIF4E signaling on

spinal disinhibition which is a key driver of Aβ-fiber driven allodynia after PNI (Price & Prescott, 2015).

Change in AIS length is a homeostatic mechanism for neurons to maintain neuronal excitability by regulating the expression of voltage-gated channels (Leterrier, 2018; Kuba *et al.,* 2015). The inventors observed AIS shrinkage in bilateral layer 5/6 IL neurons which correlated to poor performance in the rule-shifting task. While they did not use cell-specific markers to identify which cortical neurons the inventors were measuring, they are confident that the majority, if not all, of the AISs measured were from pyramidal neurons. Pyramidal cells have a very unique, organized and defined cytoarchitecture in which their axons travel distally from the cell body while interneurons have axons that can stem in any direction. The inventors only measured axons that showed a pyramidal neuron-like AIS label. AIS shrinkage is a sign of enhanced presynaptic input. Previous studies found that IL neurons show no changes in functional activity 1 week after SNI [46,57] but this may change at later time points where AIS shrinkage is clearly occurring in the IL.

The inventors speculate that this AIS loss may partially underlie frontal cortex gray matter loss seen in chronic pain patients. Others have observed changes in dendritic morphology (Metz *et al.,* 2009; Kelly *et al.,* 2016; Mitric *et al.,* 2019) in rodent mPFC after SNI, suggesting that neuronal structural reorganization may underlie cortical thinning. It is important to note that there are only two methods known to reverse cortical thinning in patients: cognitive behavioral therapy (Seminowicz *et al.,* 2013) and effective pain management (Rodriguez-Raecke *et al.,* 2009; 2013; Seminowicz *et al.,* 2011). Cortical gray matter restoration has been observed in patients with hip osteoarthritic pain whose pain dissipated after undergoing total hip replacement surgery Rodriguez-Raecke *et al.,* 2009; 2013) and in chronic low back pain patients after spinal surgery or facet joint injections (Seminowicz *et al.,* 2011). Increases in cortical thickness in chronic low back pain patients, particularly in the left dorsolateral prefrontal cortex, the rodent equivalent of the mPFC, correlated to a reduction in pain and physical disability and improved cognitive performance in an attentional-demanding task (Seminowicz *et al.,* 2011). Similarly, in the inventors' neuropathic mice, effective, long-term pain management via both tomivosertib and metformin (Shiers *et al.,* 2018), an AMPK activator and upstream regulator of eIF4E activity, attenuated both cognitive deficits and AIS shrinkage in the IL.

The inventors conclude that MNK-eIF4E signaling plays a crucial role in the generation of spontaneous pain and rule-shifting deficits that develop as a consequence of PNI. Surprisingly, these important features of neuropathic pain can be mitigated with genetic or pharmacological approaches without having any effect on mechanical allodynia. Because mechanical allodynia is the most commonly utilized test for neuropathic pain in preclinical models, these results demonstrate that this approach can overlook potential therapeutic approaches that target the most salient complaints of neuropathic pain: spontaneous pain and cognitive comorbidities. These results make a strong case for further exploration of tomivosertib, which is already in human clinical trials for cancer, as a novel neuropathic pain treatment.

**Table 4 - Two-Way ANOVA**

| | | Source of Variation Surgery/Treatment/Genotype | | | Time/Day, Location or Error type | | | Interaction | | |
|---|---|---|---|---|---|---|---|---|---|---|
| Fig | Panel | dfn,dfd | F | p | dfn,dfd | F | p | dfn,dfd | F | p |
| 1 | B | 3,78 | 16.68 | <0.0001 | 2,78 | 63.98 | <0.0001 | 6,78 | 7.211 | <0.0001 |
| | C | 3,78 | 18.06 | <0.0001 | 2,78 | 11.23 | <0.0001 | 6,78 | 2.642 | 0.0219 |
| | D | 3,96 | 0.3953 | 0.7567 | 2,96 | 52.52 | <0.0001 | 6,96 | 1.217 | 0.3045 |
| 2 | B | 3,155 | 5.38 | 0.0015 | 4,155 | 13.19 | <0.0001 | 12,155 | 2.562 | 0.0040 |
| 3 | A | 3,60 | 8.841 | <0.0001 | 2,60 | 59.44 | <0.0001 | 6,60 | 7.07 | <0.0001 |
| | B | 3,60 | 11.81 | <0.0001 | 2,60 | 14.32 | <0.0001 | 6,60 | 0.979 | 0.4475 |
| | C | 1,36 | 0.205 | 0.6535 | 5,36 | 184.5 | <0.0001 | 5,36 | 0.4662 | 0.7988 |
| 4 | C | 1,10 | 0.6314 | 0.3430 | 1,10 | 0.9907 | 0.3430 | 1,10 | 0.9907 | 0.3430 |
| 5 | A | 3,60 | 5.411 | 0.0023 | 2,60 | 23.72 | <0.0001 | 6,60 | 13.76 | <0.0001 |
| | B | 3,60 | 16.71 | <0.0001 | 2,60 | 16.69 | <0.0001 | 6,60 | 1.396 | 0.2311 |
| | C | 1,72 | 2.997 | 0.0877 | 5,72 | 272.5 | <0.0001 | 5,72 | 1.552 | 0.1848 |
| 6 | A | 3,54 | 27.38 | <0.0001 | 2,54 | 0.9642 | 0.3877 | 6,54 | 1.359 | 0.2477 |
| | B | 3,54 | 6.283 | 0.0010 | 2,54 | 3.78 | 0.0291 | 6,54 | 0.6796 | 0.6667 |
| 7 | B | 1,32 | 23.13 | <0.0001 | 3,32 | 9.854 | <0.0001 | 3,32 | 6.103 | 0.0021 |
| | C | 1,32 | 0.3533 | 0.5565 | 3,32 | 2.468 | 0.0799 | 3,32 | 0.6801 | 0.5707 |
| | D | 1,28 | 1.11 | 0.3010 | 3,28 | 0.1937 | 0.8998 | 3,28 | 0.3788 | 0.7690 |

(continued)

| | | Source of Variation Surgery/Treatment/Genotype | | | Time/Day, Location or Error type | | | Interaction | | |
|---|---|---|---|---|---|---|---|---|---|---|
| Fig | Panel | dfn,dfd | F | p | dfn,dfd | F | p | dfn,dfd | F | p |
| | E | 1,16 | 86.16 | <0.0001 | 3,16 | 33.56 | <0.0001 | 3,16 | 16.75 | <0.0001 |

**Table 5 - One-Way ANOVA with Bonferroni or Unpaired T-test as Indicated**

| One-way ANOVA | | | | | | | | |
|---|---|---|---|---|---|---|---|---|
| | | Between treatment | Within treatment | | | | | Bonferroni |
| Fig | Panel | MS | MS | dfn,dfd | F | p | Comparison | p |
| 2 | A | 7881 | 841.3 | 3,31 | 9.369 | 0.0001 | Sham WT vs SNI WT | 0.0126 |
| | | | | | | | Sham WT vs Sham 4EKI | >0.9999 |
| | | | | | | | Sham WT vs SNI 4EKI | 0.3913 |
| | | | | | | | SNI WT vs Sham 4EKI | 0.0126 |
| | | | | | | | SNI WT vs SNI 4EKI | <0.0001 |
| | | | | | | | Sham 4EKI vs SNI 4EKI | 0.3911 |
| 4 | A-mPFC | 6012 | 103.2 | 2,9 | 58.26 | <0.0001 | Veh vs tom p.o. | <0.0001 |
| | | | | | | | Veh vs tom i.p. | <0.0001 |
| | | | | | | | Tom p.o. vs tom i.p | 0.2535 |
| 4 | A-DRG | 8839 | 1069 | 2,9 | 8.268 | 0.0092 | Veh vs tom p.o. | 0.0217 |
| | | | | | | | Veh vs tom i.p. | 0.0176 |
| | | | | | | | Tom p.o. vs tom i.p | >0.9999 |
| 4 | B-0h | 0.169 | 0.303 | 2,26 | 0.558 | 0.5786 | IL-6 + tom vs IL6 only | 0.12 |
| | | | | | | | Tom only vs IL6 only | 0.99 |
| | | | | | | | IL6 + tom vs tom only | 0.42 |
| | B-1h | 1.836 | 0.339 | 2.26 | 5.408 | 0.0108 | IL-6 + tom vs IL6 only | 0.032 |
| | | | | | | | Tom only vs IL6 only | 0.039 |
| | | | | | | | IL6 + tom vs tom only | 0.96 |
| | B-3h | 1.802 | 0.319 | 2,26 | 5.655 | 0.009 | IL-6 + tom vs IL6 only | 0.01 |
| | | | | | | | Tom only vs IL6 only | 0.041 |

(continued)

| One-way ANOVA | | | | | | | | |
|---|---|---|---|---|---|---|---|---|
| | | Between treatment | Within treatment | | | | | Bonferroni |
| Fig | Panel | MS | MS | dfn,dfd | F | p | Comparison | p |
| | | | | | | | IL6 + tom vs tom only | 0.67 |
| 6 | C | 4107 | 273.6 | 6.48 | 15.01 | <0.0001 | WT naïve vs MNK1 KO Sham | 0.0001 |
| | | | | | | | WT naïve vs MNK1 KO naïve | 0.0164 |
| | | | | | | | WT naïve vs MNK1 KO SNI | 0.0012 |
| | | | | | | | WT naïve vs 4EKI naïve | <0.0001 |
| | | | | | | | WT naïve vs WT vehicle | >0.9999 |
| | | | | | | | WT naïve vs WT tom | >0.9999 |
| Unpaired t-test | | | | | | | | |
| Fig | Panel | Comparison | | | | | | p |
| 4 | D | SNI + veh vs SNI + tom | | | | | | 0.0382 |

## Example 3

### Materials and Methods

**Animals.** Male eIF4E$^{S209A}$ and MNK1$^{-/-}$ mice were a gift of the Sonenberg laboratory at McGill University (Ueda *et al.,* 2004; Furic *et al.,* 2010) and bred at the University of Texas at Dallas (UTD) to generate experimental animals. Genotype was confirmed at weaning using DNA from ear clips. Experimental C57BL/6J wild-type (WT) animals were obtained from an internally maintained C57BL/6J colony at UTD. Electrophysiological experiments using WT mice were performed using mice between the ages of 4 and 6 weeks at the start of the experiment. Behavioral experiments using *eIF4E$^{S209A}$,* MNK1$^{-/-}$ (knockout for the *Mknk1* gene) and WT mice were performed using mice between the ages of 8 and 12 weeks, weighing ~20-25 g. All animal procedures were approved by the Institutional Animal Care and Use Committee at The University of Texas at Dallas and were performed in accordance with the guidelines of the International Association for the Study of Pain.

**Antibodies and chemicals.** Rabbit primary antibodies against p-eIF4E$^{S209}$ (cat # ab76256, 1:500) and p-PKR (ab32036, 1:1000) were procured from Abcam. Mouse anti-NeuN antibody (cat # MAB377, 1:500) was obtained from Millipore. Chicken (for ICC, cat # CPCA-Peri, 1:500) and mouse (for IHC, cat # MAB1527, 1;1000) primary antibodies against peripherin were obtained from Encor Biotechnology Inc. and Sigma-Aldrich, respectively. Rabbit primary antibodies against p-JAK1 (cat # 3331S, 1:1000), JAK1 (cat # 3332S, 1;1000), p-STAT1 (cat # 9171, 1:000), STAT1 (cat # 9172S, 1:1000), p-STAT3 (cat # 9134S, 1:1000), STAT3 (cat # 91325, 1:1000), p-mTOR (cat # 2976S, 1:1000), mTOR (cat # 2983S, 1:1000), BiP (cat # 3177, 1:1000), PKR (cat # 3072S, 1:1000), p-eIF2$\alpha^{Ser51}$ (cat # 9721, 1:1000), eIF2$\alpha$ (cat # 9722, 1: 1000), p-ERK (cat # 9101S, 1:1000), ERK (cat # 9102S, 1:1000), p-AKT (cat # 2965S, 1:1000), AKT (cat # 4691S, 1:1000), p-RS6 (cat # 2317, 1:1000), RS6 (cat # 2317, 1:1000), eIF4E (cat # 9742S, 1:1000), $\beta$-actin (cat # 4967S; 1:10,000), and GAPDH (cat # 2118, 1:10,000) were obtained from Cell Signaling Technology. Alexa Fluor- and HRP-conjugated secondary antibodies were obtained from Life Technologies. The integrated stress response inhibitor, ISRIB (cat # SML0843), was purchased from Sigma. Recombinant mouse IFN-$\alpha$ protein (cat # 12100-1, lot # 6454) was procured from R&D Systems. Recombinant mouse IFN-$\beta$ protein (cat # IF011, lot # SLBX5164) and poly (I:C) (cat # P1530) were purchased from Millipore-Sigma.

**Primary cell culture of mouse DRG neurons and Western blot.** Cultured primary DRG neurons were used to test the effects of IFN-$\alpha$ [(300 units (U)/mL] and IFN-$\beta$ [(300 units (U)/mL] application. For these experiments, mice were

anesthetized with isoflurane and killed by decapitation. Then DRGs, from all spinal levels, were dissected and placed in chilled Hanks balanced-salt solution (HBSS, Invitrogen) until processed. Dorsal root ganglia were digested in 1 mg/mL collagenase A (Roche, Mannheim, Germany) for 25 minutes at 37 °C then subsequently digested in a 1:1 mixture of 1 mg/mL collagenase D and papain (Roche) for 20 minutes at 37°C. After this step, dorsal root ganglia were then triturated in a 1:1 mixture of 1 mg/mL trypsin inhibitor (Roche) and bovine serum albumin (BioPharm Laboratories, Bluffdale, UT), then filtered through a 70 $\mu$m cell strainer (Corning, NY). Cells were pelleted then resuspended in DMEM/F12 with GlutaMAX (Thermo Fisher Scientific, MA) containing 10% fetal bovine serum (FBS; Thermo Fisher Scientific), 5 ng/mL NGF, 1% penicillin and streptomycin, and 3 mg/mL 5-fluorouridine with 7 mg/mL uridine to inhibit mitosis of non-neuronal cells and were distributed evenly in a 6-well plate coated with poly-D-lysine (Becton Dickinson, NJ). DRG neurons were maintained in a 37 °C incubator containing 5% $CO_2$ with a media change every other day. On day 6, DRG neurons were treated with either vehicle, IFN-$\alpha$ or IFN-$\beta$ for 1, 3, 6 and 24h. Following treatments, cells were rinsed with chilled 1X PBS buffer, harvested in 200 $\mu$L of RIPA lysis buffer (50 mM Tris, pH 7.4, 150 mM NaCl, 1 mM EDTA, pH 8.0, and 1% Triton X-100) containing protease and phosphatase inhibitors (Sigma-Aldrich), and then sonicated for 5 seconds. To clear debris, samples were centrifuged at 14,000 rpm for 15 min at 4°C. Ten to 15 $\mu$g of protein was loaded into each well and separated by a 10% SDS-PAGE gel. Proteins were transferred to a 0.45 PVDF membrane (Millipore, MA) at 30 V overnight at 4 °C. Subsequently, membranes were blocked with 5% non-fat dry milk in 1X Tris buffer solution containing Tween 20 (TTBS) for at least 2 h. Membranes were washed 3 times for 10 minutes each (3 x 10) in 1X TTBS, then incubated with primary antibodies overnight at 4°C. The following day, membranes were washed 3 x 10 each then incubated with the corresponding secondary antibodies at room temperature for 1 h. After incubation, membranes were washed with 1X TTBS 3 x 10 each. Signals were detected using Immobilon Western Chemiluminescent HRP Substrate (Millipore) and then visualized with Bio-Rad ChemiDoc Touch. Membranes were stripped using Restore Western Blot Stripping buffer (Thermo Fisher Scientific) and reprobed with another antibody. Analysis was performed using Image lab 6.0.1 software for Mac (Bio-Rad, Hercules, CA).

To perform western blot analysis (WB) from tissues, animals were anesthetized with isoflurane and killed by decapitation. Lumbar spinal dorsal horn, L4-L5 DRGs and sciatic nerve were immediately frozen on dry ice and then sonicated for at least 10 seconds in 300 $\mu$L of RIPA lysis buffer containing protease and phosphatase inhibitors. To clear debris, samples were centrifuged at 14,000 rpm for 15 min at 4 °C. Samples were processed for WB using the experimental protocol and analysis described above.

**Immunofluorescence.** For primary neuronal cultures, DRG neurons were harvested and cultured for 6 days according to the protocol described above with the exception that cells were distributed evenly on poly-D-lysine-coated 8-well chamber slide with removable wells (cat # 12-565-8, Fisher Scientific). After treatments, cells were fixed in ice-cold 10% formalin in 1X PBS for 1 h and processed for immunocytochemistry (ICC). Cells were washed with 1X PBS and permeabilized in 1X PBS containing 10% heat-inactivated normal goat serum (NGS; Atlanta Biologicals, Atlanta, GA) and 0.02% Triton X-100 (Sigma) in 1X PBS for 30 min and then blocked in 10% NGS in 1X PBS for 2 h. Primary antibodies were applied overnight at 4°C and the next day appropriate secondary antibodies (Alexa Fluor; Invitrogen) were applied for 1 h. After additional 1X PBS washes, coverslips were mounted on Superfrost plus slides with ProLong Gold antifade (Invitrogen). Images were taken using an Olympus FluoView 1200 confocal microscope and analyzed with FIJI for Mac OS X. Images shown are representative of samples taken from 3 separate wells and presented as projections of Z stacks. Using ImageJ, the corrected total cell fluorescence (CTCF) was calculated to determine the intensity of the signal between experimental groups. To do so, the integrated density and the area, as well as the background noise was measured and the CTCF calculated as equal to: the integrated density - (area of selected cell x mean fluorescence of background readings). CTCF values from all experimental treatment groups were normalized to vehicle groups and expressed as normalized CTCF.

For tissues, animals were anesthetized with isoflurane and killed by decapitation, and tissues were frozen in O.C.T. on dry ice. Spinal cords were pressure ejected using chilled 1X PBS. Sections of L4-L5 DRGs (20 $\mu$m) were mounted onto SuperFrost Plus slides (Thermo Fisher Scientific) and fixed in ice-cold 10% formalin in 1X PBS for 1 h then subsequently washed 3 x 10 each in 1X PBS and processed for immunohistochemistry (IHC). Slides were permeabilized in 50% ethanol for 30 min. After 30 min, slides were washed 3 x 10 each in 1X PBS. Tissues were blocked for at least 2 h in 1XPBS and 10% NGS. Primary antibodies against NeuN, peripherin, p-eIF4E$^{S209}$ and eIF4E were applied and incubated with DRG sections on slides at 4°C overnight. Immunoreactivity was visualized after 1 h incubation with Alexa Fluor secondary antibodies at room temperature. Images were taken using an Olympus FluoView 1200 confocal microscope. Images are presented as projections of Z stacks, and they are representative of samples taken from 3 animals.

**Single cell data.** Single cell mouse DRG sequencing data from previously published work (Li *et al.,* 2016) was used to generate Figures 2A-2D. Seurat package 2.2.1 (Butler *et al.,* 2018) was used to cluster the single-cell data and visualization (van der Maaten and Hinton, 2008).

**RNAscope *in situ* hybridization.** RNAscope *in situ* hybridization multiplex version 1 was performed as instructed by Advanced Cell Diagnostics (ACD). Fresh frozen lumbar DRGs from male C57BL/6J mice were rapidly dissected, frozen in cryomolds with O.C.T (Fisher Scientific; Cat# 23-730-571) over dry ice and sectioned at 20 $\mu$m onto

charged slides. The sections were fixed in cold (4°C) 10% formalin for 15 minutes and then dehydrated in 50% ethanol (5 min), 70% ethanol (5 min) and 100% ethanol (10 min) at room temperature. The slides were air dried briefly and then boundaries were drawn around each section using a hydrophobic pen (ImmEdge PAP pen; Vector Labs). When hydrophobic boundaries had dried, the sections were incubated in protease IV reagent for 2 minutes and then washed in 1X phosphate buffered saline (PBS). Each slide was then placed in a prewarmed humidity control tray (ACD) containing dampened filter paper and incubated in a mixture of Channel 1 (*Ifnar1* ACD Cat# 512971 or *Ifnar2* ACD Cat# 846831), Channel 2 (*Calca;* ACD Cat#417961), and Channel 3 (*P2rx3;* ACD Cat# 521611) probes for 2 hours at 40°C. This was performed one slide at a time to avoid liquid evaporation and section drying. Following probe incubation, the slides were washed two times in 1X RNAscope wash buffer and returned to the oven for 30 minutes after submersion in AMP-1 reagent. Washes and amplification were repeated using AMP-2, AMP-3 and AMP-4B reagents with a 15-min, 30-min, and 15-min incubation period, respectively. Slides were then washed two times in 0.1M phosphate buffer (PB, pH7.4) and then submerged in blocking reagent (10% Normal Goat serum and 0.3% Triton-X 100 in 0.1M PB) for 1 hour at room temperature. Slides were incubated in primary antibody (mouse-anti-Neurofilament 200; clone N52; Sigma) at 1:500 in blocking buffer overnight at 4°C. The next day, slides were washed two times in 0.1M PB, and then incubated in secondary antibody (goat-anti-mouse H&L 405; 1:2000) for 1 hour at room temperature. Sections were washed two times in 0.1M PB, air dried, and cover-slipped with Prolong Gold Antifade (Fisher Scientific; Cat# P36930) mounting medium.

DRG sections were imaged on an Olympus FV3000 confocal microscope at 20X magnification. One image was acquired of each mouse DRG section, and 3 sections were imaged per mouse (total: 9 images/experiment for an n = 3). The raw image files were brightened and contrasted equally in Olympus CellSens software (v1.18), and then analyzed manually one neuron at a time for expression of *Ifnar1* or *Ifnar2* with the neuronal markers *Calca* (peptidergic neurons), *P2rx3* (non-peptidergic neurons) and/or NF200 (Aβ neurons). Soma diameter was measured using the polyline tool.

**Patch-clamp electrophysiology.** Cell cultures for patch clamp electrophysiology were prepared as previously described (Moy *et al.,* 2017). Male C57BL/6J mice (average age of 43 days) were anesthetized with 5% isoflurane and sacrificed by decapitation. DRGs were dissected and placed in ice-cold HBSS (divalent free) and incubated at 37°C for 15 min in 20 U/mL Papain (Worthington, Lakewood, NJ) followed by 15 min in 3 mg/ml Collagenase Type II (Worthington). After trituration through a fire-polished Pasteur pipette of progressively smaller opening sizes, cells were plated on poly-D-lysine and laminin (Sigma, St. Louis, MO)-coated plates. Cells were allowed to adhere for several hours at room temperature in a humidified chamber and then nourished with Liebovitz L-15 medium (Life Technologies, Grand Island, NY) supplemented with 10% fetal bovine serum (FBS), 10 mM glucose, 10 mM HEPES and 50 U/ml penicillin/streptomycin. The following day (within 24 h of dissociation), changes in neuronal excitability were tested after incubating the neurons with IFN-α (300 U/mL) for 1 h. To do so, whole-cell patch-clamp experiments were performed using a MultiClamp 700B (Molecular Devices) patch-clamp amplifier and PClamp 9 acquisition software (Molecular Devices) at room temperature. Recordings were sampled at 20 kHz and filtered at 3 kHz (Digidata 1550B, Molecular Devices). Pipettes (outer diameter, 1.5 mm; inner diameter, 1.1 mm, BF150-110-10, Sutter Instruments) were pulled using a PC-100 puller (Narishige) and heat polished to 3-5 MΩ resistance using a microforge (MF-83, Narishige). Series resistance was typically 7 MΩ and was compensated up to 60%. Data were analyzed using Clampfit 10 (Molecular Devices). Data are from 3 independent mice cultured on separate days. The purpose of this experimental protocol was to consider both biological and experimental variability, potentially coming from the culturing process. All neurons included in the analysis had a resting membrane potential more negative than -40 mV. The RMP was recorded 1-3 min after achieving whole-cell configuration. In current-clamp mode, cells were held at -60 mV and action potentials were elicited by injecting slow ramp currents from 100 to 700 pA with Δ 200 pA over 1 s to mimic slow depolarization. Only cells that responded to the ramp depolarization - at least one spike at the maximum 700 pA, were considered for further analysis. The pipette solution contained the following (in mM): 120 K-gluconate, 6 KCl, 4 ATP-Mg, 0.3 GTP-Na, 0.1 EGTA, 10 HEPES and 10 phosphocreatine, pH 7.4 (adjusted with N-methyl glucamine), and osmolarity was ~285 mOsm. The external solution contained the following (in mM): 135 NaCl, 2 $CaCl_2$, 1 $MgCl_2$, 5 KCl, 10 glucose, and 10 HEPES, pH 7.4 (adjusted with N-methyl glucamine), and osmolarity was adjusted to ~315 mOsm with sucrose.

**Behavior.** Mice were housed on 12-hour light/dark cycles with food and water available *ad libitum.* Mice were randomized to groups from multiple cages to avoid using mice from experimental groups that were cohabitating. Sample size was estimated by performing a power calculation using G*Power (version 3.1.9.2). With 80% power and an expectation of *d* = 2.2 effect size in behavioral experiments, and α set to 0.05, the sample size required was calculated as n = 6 per group. The inventors therefore sought to have at least n = 6 sample in all behavioral experiments. SD (set at 0.3) for the power calculation was based on previously published mechanical threshold data from the inventors' lab (Moy *et al.,* 2017). Animals were habituated for 1 hour to clear acrylic behavioral chambers before beginning the experiment. For intraplantar injections, drugs were injected in a total volume of 25 μL through a 30-gauge needle. For intraperitoneal injections, drugs were administered in a volume of 100 μL Mechanical paw withdrawal thresholds in mice were measured using the up-down method (Chaplan *et al.,* 1994) with calibrated von Frey filaments (Stoelting Company, WoodDale, IL). Thermal latency was measured using a Hargreaves device (IITC Life Science) (Hargreaves *et al.,* 1988) with heated glass settings of 29°C, 40% active laser power, and 20 sec cutoff were used. The experimenter was blinded to the genotype of the

mice and the drug condition in all experiments.

**Quantification and statistical analysis.** All results are presented as the mean ± SEM. Statistical differences between 2 groups were determined by the Student *t* test. One- or 2-way analysis of variance, followed by Dunnett or Bonferroni test, was used to compare differences between more than 2 groups. Post-hoc testing for electrophysiology data used Fisher's LSD test. Differences were considered to reach statistical significance when $P < 0.05$. Complete statistical analysis is detailed on Table 6D. The *N* for each individual experiment is described in the figure legends. Data analysis was performed using GraphPad Prism 8.0 (GraphPad Software).

**Data and code availability.** The data that support the findings of this study, including specific details of how tSNE plots were generated, are available from the corresponding author upon reasonable request.

## Results

**Characterizing pain behavior responses induced by peripheral administration of type I IFNs.** The inventors first sought to investigate the nociceptive responses produced by type I interferons (α and β) *in vivo* in both sexes. The dose (300 units (U) - approximately 5 ng) of IFNs was chosen based on previous studies showing concentration-dependent effects on cellular signaling pathways (Larner *et al.,* 1986; Hilkens *et al.,* 2003) and studies showing plasma levels of type I IFNs in mice in response to viral infection (~ 1-2 ng/ml) (Gerlach *et al.,* 2006; Shibamiya *et al.,* 2009; Murray *et al.,* 2015; Cheng *et al.,* 2017). In male mice, intraplantar (i.pl.) administration of either IFN-α (300 U/25 μL) or IFN-β (300 U/25 μL), but not vehicle (saline), produced a rapid mechanical hypersensitivity, lasting for at least 3 days, to von Frey filament stimulation (FIGS. 18A-B) with no significant changes in paw withdrawal latency to thermal stimulation (FIGS. 18C-D). Likewise, in female mice, i.pl. administration of either IFN-α (300 U/25 μL) or IFN-β (300 U/25 μL) also increased hind paw mechanical hypersensitivity with no significant changes in thermal hypersensitivity (FIGS. 18G-H). Nosex differences in the development of mechanical hypersensitivity (FIGS. 18I-J) or the presence of thermal hypersensitivity (FIGS. 18K-L) were observed between male versus female mice following either IFN-α (300 U/25 μL) or IFN-β (300 U/25 μL) i.pl. administration. Since the inventors did not find any differences in pain responses between males and females, they decided to use only males for all the subsequent experiments. These results show that activation of IFNRs by IFN-α or IFN-β creates a pronociceptive state that is likely produced via a peripheral site of action.

**Identifying IFNRs expression in sensory neurons and their downstream signaling pathways.** Because the inventors observed a pronociceptive effect of IFN-α and IFN-β, they investigated the expression of IFNRs in DRG neurons. The inventors used mouse DRG deeply RNA sequenced single cell data generated by (Li *et al.,* 2016). They generated tSNE plots to show genes expression in specific clusters of cells (van der Maaten and Hinton, 2008). IFN-α and IFN-β bind a heterodimeric transmembrane receptor termed the IFN-α receptor (IFNAR), which is composed of IFNAR1 and IFNAR2 subunits (Schreiber, 2017). The inventors observed expression of both *Ifnar1* (Interferon receptor 1, IFNR1) and *Ifnar2* (Interferon receptor 2, IFNR2) mRNAs with the neuronal marker *rbfox3* (NeuN), indicating their presence in neuronal populations in the mouse DRG (FIG. 19A). *Ifnar1* and *Ifnar2* mRNAs were coexpressed among neurons that are likely to be nociceptors because they also express *Prph* (peripherin) and *Scn10a* (Nav1.8) (FIG. 19B). Additionally, the inventors detected that *Ifnar1* and *Ifnar2* mRNAs are widely distributed across nociceptors of peptidergic [*Trpv1* (TRPV1), *Calca* (CGRP)] and non-peptidergic [*P2rx3* (P2X3)] nature (FIG. 19C). *Ifnar2* mRNA shows higher expression levels than *Ifnar1* in neurons containing *F2rl1* (PAR2) and *Nppb* (NPPB) mRNAs (FIG. 19D).

In order to confirm the presence of IFNR1 and IFNAR2 subunits in the DRG, the inventors performed RNAscope *in situ* hybridization. They found that both *Ifnr1* and *Ifnr2* mRNAs were widely expressed in neurons expressing *Calca* (CGRP) mRNA, *P2rx3* (P2X3) mRNA and NF200 protein (FIG. 19E). Co-expression analysis demonstrated that *Ifnar1* and *Ifnar2* show a similar pattern of expression across peptidergic nociceptors (*Calca*+) and large diameter neurons (NF200+). However, *Ifnar1* is more expressed than *Ifnar2* in non-peptidergic (*P2rx3*+) nociceptors (35.8% vs 26.5%; FIGS. 19F-G). Additionally, approximately 80-90% of total neurons in the DRG express *Ifnar1* and *Ifnar2* (FIG. 19H; see also FIGS. 19E-G). Co-expression analysis based on neuronal size confirmed that both *Ifnar1* and *Ifnar2* are present across the entire neuronal population in the DRG (FIGS. 19I-J). Therefore, these results confirm that IFNRs are present in mouse DRG neurons supporting the idea that their activation could modulate nociceptive signaling events.

The inventors then sought to investigate the downstream signaling events evoked by type I IFN application to cultured DRG neurons. They focused on two major pathways involved in type I IFN signaling in different cell types: transcriptional control via JAK/STAT (Levy and Darnell, 2002; de Weerd and Nguyen, 2012; Stark and Darnell, 2012) and translational control via two distinct pathways. Translation regulation by type I IFNs can occur through stimulation of cap-dependent translation via ERK/MAP kinase-MNK-eIF4E signaling (Walsh *et al.,* 2013; Ivashkiv and Donlin, 2014) or inhibition of cap-dependent translation through induction of PKR-eIF2α signaling (Pindel and Sadler, 2011; Walsh *et al.,* 2013) resulting in activation of the integrated stress response (ISR). Direct application of either IFN-α (300 U/mL) or IFN-β (300 U/mL) rapidly activated downstream JAK/STAT signaling pathways in cultured DRG neurons (FIG. 20A). This signaling cascade involved the phosphorylation of JAK1, STAT1 and STAT3 together with a delayed increase in STAT1 total protein, likely representing a transcriptional change. In addition to STATs, other signaling factors have a role in IFN-mediated activities. These include activation of the AKT/mTOR/ULKl pathway via PI3K and the ERK/MAP kinase pathway (Thyrell *et al.,* 2004; Platanias, 2005; Hjortsberg *et al.,* 2007; Saleiro *et al.,* 2015). The inventors did not observe any changes in mTOR or ribosomal protein S6 phosphorylation (FIG. 20B) but they did observe an increase in ERK and eIF4E phosphorylation that occurred rapidly after type 1 IFN exposure (FIG. 20C). Both IFN-α and IFN-β also stimulated AKT phosphorylation (FIG. 20C). These findings demonstrate that type I IFNs engage cap-dependent translation regulation signaling via eIF4E phosphorylation.

Type 1 IFNs are also known to regulate translation via induction of PKR and activation of the integrated stress response (ISR). The inventors did not observe changes in p-PKR or p-eIF2α levels and no changes were observed in BiP expression (ER chaperone protein) in response to type I IFN exposure (FIGS. 21A-B). The inventors noticed a non statisticallysignificant downregulation of p-eIF2α within the same time course of p-ERK/p-eIF4E activation (FIGS. 21A-B). A possible explanation is that, as type I interferons are enhancing features of cap-dependent translation machinery, a slight down-regulation in the pathway that suppresses cap-dependent translation, p-eIF2α, may also be occurring. Moreover, 24 hr exposure to either IFN-α or IFN-β did not modify PKR phosphorylation or expression suggesting that type 1 IFNs do not induce PKR expression in DRG neurons (FIG. 21C). In further support of these observations, no changes on p-PKR/PKR after a long IFN-α exposure were observed in the presence of the integrated stress response inhibitor ISRIB (FIG. 21C), and ISRIB does not suppress signaling pathways shown to be modulated by IFN-α or IFN-β in the inventors' previous experiments (FIGS. 21D-E). Taken together, these experiments demonstrate that type I IFNs engage MNK-eIF4E signaling in DRG cultures and do not induce PKR activation to suppress cap-dependent translation via eIF2α phosphorylation.

**Patch-clamp electrophysiology on DRG neurons links Type I IFNs activity to neuron hyperexcitability.** To assess whether the effects of type I interferons contribute to nociceptor excitability, the inventors exposed DRG neurons to IFN-α (300 U/mL) for ~1 h (average exposure time: 86.6±7 min) and measured neuronal excitability using patch-clamp electrophysiology. The treatment was present in both the L-15 culture medium and later in the external bath solution until completion of the electrophysiology experiments. Patch clamp electrophysiology was performed from small- and medium-sized populations of neurons in the cultured DRGs in both groups (capacitance: control 23.8 ± 2.9 pF vs IFN-α 24.3 ± 1.5 pF, P = 0.89; diameter: control 26.5 ± 0.56 pF vs IFN-α 26.8 ± 0.53 pF, P = 0.7; FIG. 21A). Resting membrane potential (RMP) was more hyperpolarized than -40 mV in all cells sampled and IFN-α treatment did not alter the RMP compared to the control group (control -51.2 ± 3 mV vs IFN-α -47.5 ± 2.5 mV, P = 0.38; FIG. 22A). In response to ramp current injections mimicking slow depolarizations, DRG neurons exposed to IFN-α showed elevated excitability, measured as the number of action potentials elicited, compared to the control group with a significant main effect of treatment ($F_{(1,48)}$ = 22.9, P < 0.001). Significant differences were observed at each time point of ramp injection tested (FIGS. 22B-C). The inventors further measured the latency to the first spike following ramp current injection and determined that exposure to IFN-α shortened the latency of initiation of the action potential ($F_{(1,48)}$ = 21.02, P < 0.001; FIG. 22D). Therefore, type I IFN exposure rapidly promotes hyperexcitability in small diameter DRG neurons over a time course coinciding with MNK-eIF4E activation.

**MNK-eIF4E signaling links type I IFN actions on sensory neurons to mechanical hypersensitivity.** Since the inventors previously observed that ERK/MNK-eIF4E signaling axis was the primary component contributing to IFN-α and IFN-β effects in DRG neurons, they targeted this pathway using genetic tools to investigate its contribution to type 1 IFN-induced pain hypersensitivity. When ERK is activated, it subsequently phosphorylates MNK1/2 (Waskiewicz *et al.,* 1997) leading to phosphorylation of eIF4E at serine 209 (Waskiewicz *et al.,* 1999). The inventors used immunocytochemistry (ICC) on cultured DRG neurons to assess whether type 1 IFNs impacts eIF4E phosphorylation at single cell resolution. They found that one-hour stimulation with either IFN-α (300 U/mL) or IFN-β (300 U/mL) stimulated phosphorylation of eIF4E mostly in neurons expressing peripherin (FIGS. 23A-B). Almost all neurons in the cultures were peripherin-positive making it difficult to ascertain whether this effect occurs exclusively in putative nociceptors or also in large diameter lowthreshold mechanoreceptive cells. The expression data suggests that both classes of neurons likely express type I IFN receptors (FIGS. 19A-J). To assess the behavioral impact of this signaling, the inventors used $MNK1^{-/-}$ mice (FIG. 23C) and tested mechanical hypersensitivity after i.pl. IFN-α (300 U/25 μL) or IFN-β (300 U/25 μL) administration. Mechanical hypersensitivity was attenuated in $MNK1^{-/-}$ mice compared to WT mice following IFN application (FIGS. 23D-E). Furthermore, mice lacking eIF4E phosphorylation at serine 209 ($eIF4E^{S209A}$, FIG. 23F) showed a complete absence of eIF4E phosphorylation in lumbar (L5) DRGs (FIG. 23G) and a significant reduction in mechanical hypersensitivity following i.pl. IFN injection (FIGS. 23H-I). The inventors conclude that a MNK-eIF4E signaling mechanism strongly contributes to type I IFN-induced pain hypersensitivity.

**Induction of endogenous type I interferon response with poly (I:C) causes MNK-eIF4E-dependent pain hypersensitivity.** Type I IFN responses are caused by viral infections and sustained elevations in type I IFNs have been associated with multiple autoimmune diseases including systemic lupus erythematosus (SLE) and rheumatoid arthritis (Forster, 2012). Moreover, therapeutic IFN-α administration has also been reported as associated with the emergence of somatic symptomatology such as body pain, myalgias, headache, joint pain, abdominal pain (Capuron *et al.,* 2002; Shakoor *et al.,* 2010; Nogueira *et al.,* 2012), and inflammatory hyperalgesia (Fitzgibbon *et al.,* 2019). To investigate how endogenous type I IFN production causes pain sensitization, the inventors intraperitoneally (i.p.) injected mice, for 2

consecutive days, with a synthetic analog of a double-stranded RNA (dsRNA), poly (I:C) (1 mg/kg). Poly (I:C) is well-known to activate a number of transcription factors, including IFN regulatory factor 3 (IRF3) resulting in the production of IFN-α and IFN-β (Kawai and Akira, 2008). The inventors found that mice injected with poly (I:C) developed mechanical hypersensitivity (FIG. 24A) as well as thermal hypersensitivity (FIG. 24B) over a time-course of 3-24 h after the second poly (I:C) administration. Changes in mechanical and thermal hypersensitivity were preceded by an increase in core body temperature, consistent with known physiological effects of poly (I:C) (FIG. 24C). Based on the inventors' previous observations, they hypothesized that the effects seen on thermal and mechanical hypersensitivity would be mechanistically linked to MNK-eIF4E signaling. As predicted, poly (I:C) administration increased phosphorylated eIF4E immunoreactivity in L5 DRGs of WT mice without affecting total eIF4E protein (FIG. 247D). Mechanical (FIG. 24E) and thermal (FIG. 24F) hypersensitivity produced by poly (I:C) was attenuated in $MNK1^{-/-}$ compared to WT mice. Similarly, mechanical (FIG. 24G) and thermal (FIG. 7H) hypersensitivity was decreased in $eIF4E^{S209A}$ mice compared to WTs. Moreover, L4-L5 DRGs, lumbar spinal dorsal horn (SDH) and sciatic nerve from $MNK1^{-/-}$ and $eIF4E^{S209A}$ mice showed a decrease and absence, respectively, of eIF4E phosphorylation compared to WT mice following poly (I:C) administration (FIG. 24I). Finally, the inventors tested whether poly (I:C) had a direct effect on DRG neurons. Direct application of poly (I:C) did not increase p-ERK, p-eIF4E, p-PKR or p-eIF2α in cultured DRG neurons (FIG. 24J), suggesting that effects observed with poly (I:C) *in vivo* are unlikely explained by a direct action of the compound on DRG neurons. Instead, poly (I:C) likely acts via endogenous production of type I IFNs that then act on DRG neurons. These experiments demonstrate that endogenous type I IFN production acts via MNK-eIF4E signaling to induce pain hypersensitivity.

Phosphorylation of the 5' cap-binding protein eIF4E by MAPK interacting kinases MNK1/2 is important for nociceptor sensitization and the development of chronic pain. IL-6 induced DRG nociceptor excitability is attenuated in mice lacking eIF4E phosphorylation, in $MNK1/2^{-/-}$ mice and by the nonselective MNK1/2 inhibitor cercosporamide. The inventors sought to better understand the neurophysiological mechanisms underlying how IL-6 causes nociceptor excitability via MNK-eIF4E signaling using eFT508. Dorsal root ganglion (DRG) neurons were cultured from male and female ICR mice, 4-7 weeks old. DRG cultures were treated with vehicle, IL-6, eFT508 (pretreat) followed by IL-6 or eFT508 alone. Whole-cell patch clamp recordings were done on small diameter neurons (20-30 pF) to measure membrane excitability in response to ramp depolarization. One hr IL-6 treatment resulted in increased action potential firing compared to vehicle at all ramp intensities, an effect that was blocked by pretreatment with eFT508. Basic membrane properties, including resting membrane potential, input resistance and rheobase, were similar across groups. Latency to the first action potential in the ramp protocol was lower in the IL-6 group and rescued by eFT508 pretreatment. They also found that the amplitudes of T-type voltage-gated calcium channels (VGCCs) were increased in the DRG following IL-6 treatment, but not in the eFT508 co-treatment group. These findings are consistent with a model wherein MNK-eIF4E signaling controls the translation of signaling factors that regulate T-type VGCCs in response to IL-6 treatment. Inhibition of MNK with eFT508 disrupts these events, thereby preventing nociceptor hyperexcitability.

## Discussion

The findings reported here provide evidence for a mechanistic link between viral infection, type 1 IFN production and rapid induction of nociceptor hyperexcitability and mechanical pain sensitization. This occurs via a direct action of type I IFN receptors on sensory neurons and is dependent on downstream signaling via MNK-eIF4E. The inventors find no evidence for mTORC1 activation or induction of eIF2α phosphorylation in DRG neurons by type I IFNs, demonstrating that the key translation regulation pathway engaged is eIF4E phosphorylation. A summary of the main findings from this study are highlighted in a schematic diagram in FIG. 25. Collectively, these results provide molecular insight into why one of the first signs of viral infection is body-wide aches and pain.

While it is well known that viral infection can cause pain, very little work has been done to understand the underlying mechanisms driving this effect (Chiu *et al.,* 2016). Aches and pain caused by viral infection have classically been attributed to fever but these aches and pain often begin before the onset of fever. The findings with poly (I:C) treatment in mice show that fever and pain effects are disassociated, but in this case the fever clearly preceded hyperalgesia caused by poly (I:C) treatment. An alternative mechanism for viral infectioninduced pain is upregulation of IDO1 enzyme and consequent increased production of kynurenine. In support of this idea, mice lacking IDO1 show decreased pain sensitization in response to viral infection (Huang *et al.,* 2016). However, subsequent work demonstrates that this IDO1 upregulation occurs via virally-mediated upregulation of IFN-β and that IDO1 expression can be driven by type I IFNs (Gaelings *et al.,* 2017). This work demonstrates that this initial type 1 IFN induction by viral infection can drive a direct sensitization of nociceptors through type 1 IFN receptors expressed by these sensory neurons. This suggests that early pain sensitization caused by viral infection may proceed independently of IDO1 upregulation. A direct effect of type I IFNs on nociceptors and type 1 IFN-mediated upregulation of IDO1 and subsequent kynurenine signaling may act in concert to cause prolonged pain responses that can occur with viral infections.

This work adds to a growing understanding of how pathogens and host-defense responses interact with nociceptors (Chiu *et al.,* 2016). Bacteria can act directly on nociceptors via N-formylated peptides that are agonists of

G protein-coupled formyl peptide receptors (Chiu *et al.,* 2013; Pinho-Ribeiro *et al.,* 2017) that are expressed in mouse and human DRG neurons (Ray *et al.,* 2018). Bacteria also release α-hemolysin which directly excites nociceptors to cause pain (Chiu *et al.,* 2013; Blake *et al.,* 2018). While nociceptors can detect bacterial invasion, rapidly sending an alert signal to the brain, they can also play more nuanced roles in bacterial host defense. For instance, gut-innervating nociceptors have very recently been shown to play an active role in defending against *Salmonella* infection. This happens via an effect of calcitonin gene-related peptide (CGRP) signaling on intestinal microvilli cells and resident microbiome to protect against *Salmonella* invasiveness (Lai *et al.,* 2019). In the case of viruses, the inventors find that a dsRNA mimetic, poly (I:C) does not seem to have a direct effect on nociceptors, suggesting that immune and other somatic cells are likely the first detectors of viral infection. These findings clearly demonstrate, however, that one of the earliest responses to viral infection, production of type 1 IFN, causes robust and rapid sensitization of nociceptors via a specific translation regulation signaling cascade. These findings regarding the action of type I IFNs on nociceptors are opposed to the recent work of Liu *et al.,* (Liu *et al.,* 2016) who found that IFNα causes inhibition of pain signaling at the level of the dorsal horn. These inventors proposed that type I IFNs from astrocytes cause presynaptic inhibition of neurotransmitter release from nociceptors therefore reducing pain signaling. It is possible that CNS-released type I IFNs have a different action on nociceptor central terminals than type 1 IFNs released in the periphery have on nociceptor peripheral ending and cell bodies. Another possibility is that very high doses of type I IFNs (5,000-10,000 units) produce an inhibition of MAPK signaling, as recently shown in the dorsal horn in the context of neuropathic pain (Liu *et al.,* 2019). The inventors show that lower doses of type I IFNs (300 units) produce clear MAPK signaling activation in DRG neurons *in vitro* and *in vivo.* The dose used in the experiments is consistent with earlier studies examining dose-dependent effects of type I IFN signaling on what are now known as canonical signaling pathways (Larner *et al.,* 1986; Hilkens *et al.,* 2003) and with plasma levels induced by virus (Gerlach *et al.,* 2006; Murray *et al.,* 2015; Cheng *et al.,* 2017) or poly (I:C) (Shibamiya *et al.,* 2009).

Translation regulation is a central mechanism driving nociceptor hyperexcitability and mechanical pain (Khoutorsky and Price, 2018). A key antiviral response is activation of PKR and downstream phosphorylation of eIF2α (Balachandran and Barber, 2007). This results in decreased cap-dependent translation and suppression of viral replication capability in host cells. Another upstream eIF2α kinase, PERK, is activated in DRG neurons in diabetic neuropathy (Inceoglu *et al.,* 2015), an effect that is likely mediated by the toxic end glycation byproduct methylglyoxal (Barragan-Iglesias *et al.,* 2019). The inventors initially hypothesized that type I IFNs might induce eIF2α phosphorylation in DRG neurons via PKR activation given the well-established induction of this pathway in other cell types (Pindel and Sadler, 2011). The inventors did not find evidence for type I IFN-induced PKR-eIF2α signaling in DRG neurons, even over long time courses. Instead, the inventors observed clear evidence for rapid activation of MNK1-eIF4E signaling in DRG neurons *in vitro* and *in vivo.* Signaling via eIF4E was also critical for the production of mechanical pain responses by type I IFNs and poly (I:C), which produces endogenous type I IFN production (Yamamoto *et al.,* 2003; Kawai and Akira, 2008). Activation of MNK-eIF4E signaling by type I IFNs has been observed in other cell types where it has been linked to increased immune surveillance (Joshi *et al.,* 2009). Multiple previous studies have demonstrated that activation of MNK-eIF4E-mediated translation events are causative in the production of chronic pain states, including neuropathic pain (Moy *et al.,* 2017; Megat *et al.,* 2019; Shiers *et al.,* 2019). Since both viral infections and prolonged production of type I IFNs can cause neuropathic pain, it is possible that type I IFN receptor signaling to MNK-eIF4E may be a key pathway for production of these types of neuropathies.

**Table 6A - Two-way ANOVA**

| | | Source of Variation Surgery/Treatment/Genotype | | | Time/Day, Location or Error type | | | Interaction | | |
|---|---|---|---|---|---|---|---|---|---|---|
| Fi g | Pane l | dfn,dfd | F | p | dfn,df d | F | p | dfn,df d | F | p |
| 1 | B | 3,78 | 16.68 | <0.0001 | 2,78 | 63.98 | <0.000 1 | 6,78 | 7.211 | <0.000 1 |
| | C | 3,78 | 18.06 | <0.0001 | 2,78 | 11.23 | <0.000 1 | 6,78 | 2.642 | 0.0219 |
| | D | 3,96 | 0.3953 | 0.7567 | 2,96 | 52.52 | <0.000 1 | 6,96 | 1.217 | 0.3045 |
| 2 | B | 3,155 | 5.38 | 0.0015 | 4,155 | 13.19 | <0.000 1 | 12,15 5 | 2.562 | 0.0040 |
| 3 | A | 3,60 | 8.841 | <0.0001 | 2,60 | 59.44 | <0.000 1 | 6,60 | 7.07 | <0.000 1 |
| | B | 3,60 | 11.81 | <0.0001 | 2,60 | 14.32 | <0.000 1 | 6,60 | 0.979 | 0.4475 |
| | C | 1,36 | 0.205 | 0.6535 | 5,36 | 184.5 | <0.000 1 | 5,36 | 0.466 2 | 0.7988 |
| | D | 3,60 | 5.411 | 0.0023 | 2,60 | 23.72 | <0.000 1 | 6,60 | 13.76 | <0.000 1 |
| | E | 3,60 | 16.71 | <0.0001 | 2,60 | 16.69 | <0.000 1 | 6,60 | 1.396 | 0.2311 |

(continued)

| | | Source of Variation Surgery/Treatment/Genotype | | | Time/Day, Location or Error type | | | Interaction | | |
|---|---|---|---|---|---|---|---|---|---|---|
| Fig | Panel | dfn,dfd | F | p | dfn,dfd | F | p | dfn,dfd | F | p |
| | F | 1,72 | 2.997 | 0.0877 | 5,72 | 272.5 | <0.000 1 | 5,72 | 1.552 | 0.1848 |
| 4 | D | 1,10 | 0.6314 | 0.3430 | 1,10 | 0.990 7 | 0.3430 | 1,10 | 0.990 7 | 0.3430 |
| S1 | A | 3,54 | 27.38 | <0.0001 | 2,54 | 0.964 2 | 0.3877 | 6,54 | 1.359 | 0.2477 |
| | B | 3,54 | 6.283 | 0.0010 | 2,54 | 3.78 | 0.0291 | 6,54 | 0.679 6 | 0.6667 |
| 5 | B | 1,32 | 23.13 | <0.0001 | 3,32 | 9.854 | <0.000 1 | 3,32 | 6.103 | 0.0021 |
| | C | 1,32 | 0.3533 | 0.5565 | 3,32 | 2.468 | 0.0799 | 3,32 | 0.680 1 | 0.5707 |
| | D | 1,28 | 1.11 | 0.3010 | 3,28 | 0.193 7 | 0.8998 | 3,28 | 0.378 8 | 0.7690 |
| | E | 1,16 | 86.16 | <0.0001 | 3,16 | 33.56 | <0.000 1 | 3,16 | 16.75 | <0.000 1 |
| S2 | C | 2,24 | 2.188 | 0.1340 | 1,24 | 0.076 2 | 0.7849 | 2,24 | 0.592 0 | 0.5611 |

**Table 6B - Two-way ANOVA Bonferroni comparisons**

| Bonferroni | | | | | |
|---|---|---|---|---|---|
| Fig | Comparison | p | Fig | Comparison | p |
| **1B** | **Response** | | **2B** | | |
| | WT Sham vs WT SNI | >0.9999 | | SNI WT vs Sham 4EKI | >0.9999 |
| | WT Sham vs 4EKI Sham | >0.9999 | | SNI WT vs SNI 4EKI | >0.9999 |
| | WT Sham vs 4EKI SNI | >0.9999 | | Sham 4EKI vs SNI 4EKI | >0.9999 |
| | WT SNI vs 4EKI Sham | 0.1167 | | **D14** | |
| | WT SNI vs 4EKI SNI | 0.1119 | | Sham WT vs SNI WT | 0.0001 |
| | 4EKI Sham vs 4EKI SNI | >0.9999 | | Sham WT vs Sham 4EKI | >0.9999 |
| | **Retest** | | | Sham WT vs SNI 4EKI | 0.0002 |
| | WT Sham vs WT SNI | >0.9999 | | SNI WT vs Sham 4EKI | <0.0001 |
| | WT Sham vs 4EKI Sham | >0.9999 | | SNI WT vs SNI 4EKI | >0.9999 |
| | WT Sham vs 4EKI SNI | >0.9999 | | Sham 4EKI vs SNI 4EKI | <0.0001 |
| | WT SNI vs 4EKI Sham | >0.9999 | | **D1** | |
| | WT SNI vs 4EKI SNI | >0.9999 | | Sham WT vs SNI WT | >0.9999 |
| | 4EKI Sham vs 4EKI SNI | >0.9999 | | Sham WT vs Sham 4EKI | >0.9999 |
| | **Rule shifting** | | | Sham WT vs SNI 4EKI | 0.5044 |
| | WT Sham vs WT SNI | <0.0001 | | SNI WT vs Sham 4EKI | >0.9999 |
| | WT Sham vs 4EKI Sham | 0.2957 | | SNI WT vs SNI 4EKI | >0.9999 |
| | WT Sham vs 4EKI SNI | >0.9999 | | Sham 4EKI vs SNI 4EKI | >0.9999 |
| | WT SNI vs 4EKI Sham | <0.0001 | | **D2** | |
| | WT SNI vs 4EKI SNI | <0.0001 | | Sham WT vs SNI WT | >0.9999 |
| | 4EKI Sham vs 4EKI SNI | >0.9999 | | Sham WT vs Sham 4EKI | >0.9999 |
| **1C** | **PE** | | | Sham WT vs SNI 4EKI | >0.9999 |
| | WT Sham vs WT SNI | >0.9999 | | SNI WT vs Sham 4EKI | >0.9999 |
| | WT Sham vs 4EKI Sham | >0.9999 | | SNI WT vs SNI 4EKI | >0.9999 |

(continued)

| Bonferroni | | | | | |
|---|---|---|---|---|---|
| Fig | Comparison | p | Fig | Comparison | p |
| | WT Sham vs 4EKI SNI | >0.9999 | | Sham 4EKI vs SNI 4EKI | >0.9999 |
| | WT SNI vs 4EKI Sham | 0.7264 | | **D3** | |
| | WT SNI vs 4EKI SNI | >0.9999 | | Sham WT vs SNI WT | >0.9999 |
| | 4EKI Sham vs 4EKI SNI | >0.9999 | | Sham WT vs Sham 4EKI | >0.9999 |
| | **NRE** | | | Sham WT vs SNI 4EKI | >0.9999 |
| | WT Sham vs WT SNI | 0.0048 | | SNI WT vs Sham 4EKI | >0.9999 |
| | WT Sham vs 4EKI Sham | >0.9999 | | SNI WT vs SNI 4EKI | >0.9999 |
| | WT Sham vs 4EKI SNI | >0.9999 | | Sham 4EKI vs SNI 4EKI | >0.9999 |
| | WT SNI vs 4EKI Sham | 0.0001 | **3A** | **Response** | |
| | WT SNI vs 4EKI SNI | 0.0028 | | Sham + veh vs SNI + veh | >0.9999 |
| | 4EKI Sham vs 4EKI SNI | >0.9999 | | Sham + veh vs Sham + tom | 0.6088 |
| | **REG** | | | Sham + veh vs SNI + tom | 0.6657 |
| | WT Sham vs WT SNI | 0.0176 | | SNI + veh vs Sham + tom | 0.0234 |
| | WT Sham vs 4EKI Sham | 0.4905 | | SNI + veh vs SNI + tom | >0.9999 |
| | WT Sham vs 4EKI SNI | 0.3505 | | Sham + tom vs SNI + tom | 0.0083 |
| | WT SNI vs 4EKI Sham | <0.0001 | | **Retest** | |
| | WT SNI vs 4EKI SNI | <0.0001 | | Sham + veh vs SNI + veh | >0.9999 |
| | 4EKI Sham vs 4EKI SNI | >0.9999 | | Sham + veh vs Sham + tom | >0.9999 |
| **1D** | **Response** | | | Sham + veh vs SNI + tom | >0.9999 |
| | F 4EKI Sham vs F 4EKI SNI | >0.9999 | | SNI + veh vs Sham + tom | >0.9999 |
| | F 4EKI Sham v M 4EKI Sham | >0.9999 | | SNI + veh vs SNI + tom | >0.9999 |
| | F 4EKI Sham vs M 4EKI SNI | >0.9999 | | Sham + tom vs SNI + tom | >0.9999 |
| | F 4EKI SNI vs M 4EKI Sham | >0.9999 | | **Rule shifting** | |
| | F 4EKI SNI vs M 4EKI SNI | >0.9999 | | Sham + veh vs SNI + veh | <0.0001 |
| | M 4EKI Sham vs M 4EKI SNI | >0.9999 | | Sham + veh vs Sham + tom | >0.9999 |
| | **Retest** | | | Sham + veh vs SNI + tom | >0.9999 |
| | F 4EKI Sham vs F 4EKI SNI | >0.9999 | | SNI + veh vs Sham + tom | <0.0001 |
| | F 4EKI Sham v M 4EKI Sham | >0.9999 | | SNI + veh vs SNI + tom | <0.0001 |
| | F 4EKI Sham vs M 4EKI SNI | >0.9999 | | Sham + tom vs SNI + tom | >0.9999 |
| | F 4EKI SNI vs M 4EKI Sham | >0.9999 | **3B** | **PE** | |
| | F 4EKI SNI vs M 4EKI SNI | >0.9999 | | Sham + veh vs Sham + tom | >0.9999 |
| | M 4EKI Sham vs M 4EKI SNI | >0.9999 | | Sham + veh vs SNI + veh | >0.9999 |
| | **Rule shifting** | | | Sham + veh vs SNI + tom | >0.9999 |
| | F 4EKI Sham vs F 4EKI SNI | >0.9999 | | Sham + tom vs SNI + veh | >0.9999 |
| | F 4EKI Sham v M 4EKI Sham | 0.8804 | | Sham + tom vs SNI + tom | >0.9999 |
| | F 4EKI Sham vs M 4EKI SNI | >0.9999 | | SNI + veh vs SNI + tom | 0.6728 |
| | F 4EKI SNI vs M 4EKI Sham | 0.0590 | | **NRE** | |
| | F 4EKI SNI vs M 4EKI SNI | >0.9999 | | Sham + veh vs Sham + tom | 0.9554 |

(continued)

| Bonferroni | | | | | |
|---|---|---|---|---|---|
| Fig | Comparison | p | Fig | Comparison | p |
| | M 4EKI Sham vs M 4EKI SNI | 0.7402 | | Sham + veh vs SNI + veh | 0.0096 |
| **2B** | **BL** | | | Sham + veh vs SNI + tom | >0.9999 |
| | Sham WT vs SNI WT | >0.9999 | | Sham + tom vs SNI + veh | 0.5380 |
| | Sham WT vs Sham 4EKI | >0.9999 | | Sham + tom vs SNI + tom | 0.7022 |
| | Sham WT vs SNI 4EKI | >0.9999 | | SNI + veh vs SNI + tom | 0.0038 |
| | SNI WT vs Sham 4EKI | >0.9999 | | **REG** | |
| **Bonferroni** | | | | | |
| Fig | **Comparison** | p | Fig | Comparison | p |
| **3B** | Sham + veh vs Sham + tom | >0.9999 | **4D** | Male Sham vs Male SNI | 0.4137 |
| | Sham + veh vs SNI + veh | 0.0303 | | Female Sham vs Female SNI | 0.9879 |
| | Sham + veh vs SNI + tom | >0.9999 | **5B** | L2/L3 ipsi WT Sham vs SNI | >0.9999 |
| | Sham + tom vs SNI + veh | 0.0247 | | L5/L6 ipsi WT Sham vs SNI | 0.0056 |
| | Sham + tom vs SNI + tom | >0.9999 | | L2/L3 contra Sham vs SNI | 0.6122 |
| | SNI + veh vs SNI + tom | 0.0004 | | L5/L6 contra Sham vs SNI | <0.0001 |
| **3C** | BL: SNI + veh vs SNI + tom | >0.9999 | **5C** | L2/L3 ipsi 4EKI Sham vs SNI | >0.9999 |
| | D14: SNI + veh vs SNI + tom | >0.9999 | | L5/L6 ipsi 4EKI Sham vs SNI | >0.9999 |
| | D1 1h: SNI + veh vs SNI + tom | >0.9999 | | L2/L3 con 4EKI Sham vs SNI | >0.9999 |
| | D1 3h: SNI + veh vs SNI + tom | >0.9999 | | L5/L6 con 4EKI Sham vs SNI | >0.9999 |
| | D5 1h: SNI + veh vs SNI + tom | 0.8982 | **5D** | L2/L3 ipsi MNK Sham vs SNI | >0.9999 |
| | D5 3h: SNI + veh vs SNI + tom | >0.9999 | | L5/L6 ipsi MNK Sham vs SNI | >0.9999 |
| **3D** | **Response** | | | L2/L3 con MNK Sham vs SNI | >0.9999 |
| | WT Sham vs WT SNI | >0.9999 | | L5/L6 con MNK Sham vs SNI | >0.9999 |
| | WT Sham vs MNK1 KO Sham | >0.9999 | **5E** | L2/L3 ipsi SNI veh vs tom | >0.9999 |
| | WT Sham vs MNK1 KO SNI | >0.9999 | | L5/L6 ipsi SNI veh vs tom | <0.0001 |
| | WT SNI vs MNK1 KO Sham | >0.9999 | | L2/L3 con SNI veh vs tom | >0.9999 |
| | WT SNI vs MNK1 KO SNI | >0.9999 | | L5/L6 con SNI veh vs tom | <0.0001 |
| | MNK1 KO Sham vs SNI | >0.9999 | **S2C** | BL: Naïve vs vehicle | >0.9999 |
| | **Retest** | | | BL: Naïve vs tomivosertib | >0.9999 |
| | WT Sham vs WT SNI | >0.9999 | | BL: vehicle vs tomivosertib | >0.9999 |
| | WT Sham vs MNK1 KO Sham | 0.2535 | | Test: Naïve vs vehicle | 0.1619 |
| | WT Sham vs MNK1 KO SNI | 0.0012 | | Test: Naïve vs tomivosertib | 0.3293 |
| | WT SNI vs MNK1 KO Sham | 0.4542 | | Test: vehicle vs tomivosertib | >0.9999 |
| | WT SNI vs MNK1 KO SNI | 0.0014 | | | |
| | MNK1 KO Sham vs SNI | 0.4765 | | | |
| | **Rule shifting** | | | | |
| | WT Sham vs WT SNI | <0.0001 | | | |
| | WT Sham vs MNK1 KO Sham | >0.9999 | | | |
| | WT Sham vs MNK1 KO SNI | 0.0763 | | | |

(continued)

| Bonferroni | | | | | |
|---|---|---|---|---|---|
| Fig | Comparison | p | Fig | Comparison | p |
| | WT SNI vs MNK1 KO Sham | <0.0001 | | | |
| | WT SNI vs MNK1 KO SNI | <0.0001 | | | |
| | MNK1 KO Sham vs SNI | 0.0539 | | | |
| **3E** | **PE** | | | | |
| | WT Sham vs WT SNI | >0.9999 | | | |
| | WT Sham vs MNK1 KO Sham | >0.9999 | | | |
| | WT Sham vs MNK1 KO SNI | >0.9999 | | | |
| | WT SNI vs MNK1 KO Sham | >0.9999 | | | |
| | WT SNI vs MNK1 KO SNI | 0.2818 | | | |
| | MNK1 KO Sham vs SNI | >0.9999 | | | |
| | **NRE** | | | | |
| | WT Sham vs WT SNI | 0.0057 | | | |
| | WT Sham vs MNK1 KO Sham | >0.9999 | | | |
| | WT Sham vs MNK1 KO SNI | >0.9999 | | | |
| | WT SNI vs MNK1 KO Sham | 0.0006 | | | |
| | WT SNI vs MNK1 KO SNI | 0.0002 | | | |
| | MNK1 KO Sham vs SNI | >0.9999 | | | |
| | **REG** | | | | |
| | WT Sham vs WT SNI | 0.0197 | | | |
| | WT Sham vs MNK1 KO Sham | >0.9999 | | | |
| | WT Sham vs MNK1 KO SNI | 0.7359 | | | |
| | WT SNI vs MNK1 KO Sham | 0.1015 | | | |
| | WT SNI vs MNK1 KO SNI | <0.0001 | | | |
| | MNK1 KO Sham vs SNI | 0.2210 | | | |
| **3F** | BL: MNK1 KO SNI vs WT SNI | >0.9999 | | | |
| | D4: MNK1 KO SNI vs WT SNI | 0.0157 | | | |
| | D7: MNK1 KO SNI vs WT SNI | >0.9999 | | | |
| | D11: MNK1 KO SNI vs WT SNI | >0.9999 | | | |
| | D14: MNK1 KO SNI vs WT SNI | >0.9999 | | | |
| | D17: MNK1 KO SNI vs WT SNI | >0.9999 | | | |

**Table 6C - One-way ANOVA with Bonferroni or unpaired t-test as indicated**

| One-way ANOVA | | | | | | | | |
|---|---|---|---|---|---|---|---|---|
| | | Between treatment | Within treatment | | | | | Bonferroni |
| Fig | Panel | MS | MS | dfn,dfd | F | p | Comparison | p |
| 2 | A | 2902 | 300.3 | 3,31 | 9.663 | 0.0001 | Sham WT vs SNI WT | 0.0089 |

(continued)

| One-way ANOVA | | | | | | | | |
|---|---|---|---|---|---|---|---|---|
| | | Between treatment | Within treatment | | | | | Bonferroni |
| Fig | Panel | MS | MS | dfn,dfd | F | p | Comparison | p |
| | | | | | | | Sham WT vs Sham 4EKI | >0.9999 |
| | | | | | | | Sham WT vs SNI 4EKI | 0.4574 |
| | | | | | | | SNI WT vs Sham 4EKI | 0.0089 |
| | | | | | | | SNI WT vs SNI 4EKI | <0.0001 |
| | | | | | | | Sham 4EKI vs SNI 4EKI | 0.4574 |
| 4 | A-mPFC | 6012 | 103.2 | 2,9 | 58.26 | <0.0001 | Veh vs tom p.o. | <0.0001 |
| | | | | | | | Veh vs tom i.p. | <0.0001 |
| | | | | | | | Tom p.o. vs tom i.p | 0.2535 |
| 4 | A-DRG | 8839 | 1069 | 2,9 | 8.268 | 0.0092 | Veh vs tom p.o. | 0.0217 |
| | | | | | | | Veh vs tom i.p. | 0.0176 |
| | | | | | | | Tom p.o. vs tom i.p | >0.9999 |
| 4 | A-Sciatic | 8965 | 1110 | 2,8 | 8.074 | 0.0120 | Veh vs tom p.o. | 0.0275 |
| | | | | | | | Veh vs tom i.p. | 0.0257 |
| | | | | | | | Tom p.o. vs tom i.p | >0.9999 |
| 4 | C-0h | 0.169 | 0.303 | 2,26 | 0.558 | 0.5786 | IL-6 + tom vs IL6 only | 0.12 |
| | | | | | | | Tom only vs IL6 only | 0.99 |
| | | | | | | | IL6 + tom vs tom only | 0.42 |
| | C-1h | 1.836 | 0.339 | 2,26 | 5.408 | 0.0108 | IL-6 + tom vs IL6 only | 0.032 |
| | | | | | | | Tom only vs IL6 only | 0.039 |
| | | | | | | | IL6 + tom vs tom only | 0.96 |
| | C-3h | 1.802 | 0.319 | 2,26 | 5.655 | 0.009 | IL-6 + tom vs IL6 only | 0.01 |
| | | | | | | | Tom only vs IL6 only | 0.041 |
| | | | | | | | IL6 + tom vs tom only | 0.67 |
| S1 | C | 4107 | 273.6 | 6,48 | 15.01 | <0.0001 | WT naïve vs MNK1 KO Sham | 0.0001 |

(continued)

| One-way ANOVA | | | | | | | | |
|---|---|---|---|---|---|---|---|---|
| | | Between treatment | Within treatment | | | | | Bonferroni |
| Fig | Panel | MS | MS | dfn,dfd | F | p | Comparison | p |
| | | | | | | | WT naïve vs MNK1 KO naïve | 0.0164 |
| | | | | | | | WT naïve vs MNK1 KO SNI | 0.0012 |
| | | | | | | | WT naïve vs 4EKI naïve | <0.0001 |
| | | | | | | | WT naïve vs WT vehicle | >0.9999 |
| | | | | | | | WT naïve vs WT tom | >0.9999 |
| S2 | A | 17.23 | 3.153 | 2,11 | 5.465 | 0.0225 | Naïve vs vehicle | 0.0492 |
| | | | | | | | Naïve vs tomivosertib | 0.0231 |
| Unpaired t-test | | | | | | | | |
| Fig | Panel | Comparison | | | | | | p |
| 4 | B | Sham + tomivosertib vs SNI + tomivosertib | | | | | | .0283 |

| Table 6D - Statistical Analvsis | | |
|---|---|---|
| **Figure** | **Test** | **Posthoc comparison** |
| 1A | Two-way RM ANOVA:<br>Interaction: $F_{(6, 114)}$ = 3.367, P=0.0043<br>Time effect: $F_{(6, 114)}$ = 5.570, P<0.0001<br>Treatment effect: $F_{(1, 19)}$ = 29.53, P<0.0001 | Bonferroni's test:<br>Vehicle x IFN-α at 3h: P<0.0001, at 24h: P=0.003, at 3d: P=0.0004 |
| 1B | Two-way RM ANOVA:<br>Interaction: $F_{(6, 114)}$ = 2.908, P=0.0112<br>Time effect: $F_{(6, 114)}$ = 3.223, P=0.0058<br>Treatment effect: $F_{(1, 19)}$ = 63.79, P<0.0001 | Bonferroni's test:<br>Vehicle x IFN-β at 1h: P=0.0007, at 3h: P<0.0001, at 24h: P<0.0001, at 3d: P<0.0001, at 6d: P=0.0006 |
| 1E | Two-way RM ANOVA:<br>Interaction: $F_{(6, 60)}$ = 3.453, P=0.0054<br>Time effect: $F_{(6, 60)}$ = 7.657, P<0.0001<br>Treatment effect: $F_{(1, 10)}$ = 29.05, P=0.0003 | Bonferroni's test: vehicle x IFN-α at 1h: P=0.0002, at 3h: P=0.0430, at 24h: P=0.0018, at 3d: P=0.0020 |
| 1F | Two-way RM ANOVA:<br>Interaction: F (6, 60) = 3.569, P=0.0043<br>Time effect: $F_{(6, 60)}$ = 6.728, P<0.0001<br>Treatment effect: $F_{(1, 10)}$ = 42.45, P<0.0001 | Bonferroni's test: vehicle x IFN-β at 1h: P=0.0109, at 3h: P<0.0001, at 24h: 0.0037, at 3d: P=0.0032 |

(continued)

| Table 6D - Statistical Analvsis | | |
|---|---|---|
| **Figure** | **Test** | **Posthoc comparison** |
| 2E | Ordinary one-way ANOVA: | Dunnett's test: |
| | | IFN-α |
| | pJAK: $F_{(3, 8)}$ = 5.088, P=0.0293<br>pSTAT1: $F_{(3, 8)}$ = 30.42, P=0.0001<br>STAT1: $F_{(3, 8)}$ = 11.71, P=0.0027<br>p-STAT3: $F_{(3, 8)}$ = 5.298, P=0.0264 | Vehicle x IFN-α at 1h: P=0.0163, at 3h: 0.0473<br>Vehicle x IFN-α at 1h: P<0.0001<br>Vehicle x IFN-α at 6h: P= 0.0036<br>Vehicle x IFN-α at 1h: P=0.0281 |
| | IFN-β | IFN-β |
| | pJAK: $F_{(3, 20)}$ = 3.614, P=0.0311<br>pSTAT1: $F_{(3, 8)}$ = 6.424, P=0.0159<br>STAT1: $F_{(3, 8)}$ = 13.7, P=0.0016<br>p-STAT3: $F_{(3, 8)}$ = 9.089, P=0.0059 | Vehicle x IFN-β at 1h: P=0.0190<br>Vehicle x IFN-β at 1h: P=0.0134<br>Vehicle x IFN-β at 6h: P=0.0010<br>Vehicle x IFN-β at 1h: 0.0074 |
| 2G | Ordinary one-way ANOVA: | Dunnett's test: |
| | IFN-α | IFN-α |
| | p-ERK: $F_{(3, 15)}$ = 13.39, P=0.0002<br>p-eIF4E: $F_{(3, 8)}$ = 17.29, P=0.0007<br>pAKT: $F_{(3, 20)}$ = 29.26, P<0.0001 | Vehicle x IFN-α at 1h: 0.0119<br>Vehicle x IFN-α at 1h: 0.0011<br>Vehicle x IFN-α at 1h: 0.0001, at 3h: 0.0001, at 6h: 0.0337 |
| | IFN-β | IFN-β |
| | p-ERK: $F_{(3, 20)}$ = 13.99, P<0.0001<br>p-eIF4E: $F_{(3, 20)}$ = 1.894, P=0.1631<br>pAKT: $F_{(3, 20)}$ = 3.847, P=0.0253 | Vehicle x IFN-β at 1h: P=0.0109<br>Vehicle x IFN-β at 1h: P=0.0343<br>Vehicle x IFN-β at 1h: 0.0168 |
| 4C | Ordinary two-way ANOVA:<br>Interaction: $F_{(3, 48)}$ = 0.7803, P=0.5108<br>pA effect: $F_{(3, 48)}$ = 11.38, P<0.0001<br>Treatment effect: $F_{(1, 48)}$ = 22.98, P<0.0001 | Uncorrected Fisher's LSD. Control vs IFN-α at 300 pA: P=0.0127, at 500 pA: P=0.0053, at 700 pA: P=0.0045 |
| 4D | Two-way ANOVA:<br>Interaction: $F_{(3, 48)}$ = 0.2291, P=0.8757<br>pA effect: $F_{(3, 48)}$ = 5.316, P=0.0030<br>Treatment effect: $F_{(1, 48)}$ = 21.02, P<0.0001 | Uncorrected Fisher's LSD: Control vs IFN-α at 100 pA: P=0.0054, At 300 pA: P=0.0203, at 700 pA: P=0.0498 |
| 5B | Ordinary One-way ANOVA: $F_{(2, 670)}$ = 29.87, P<0.0001 | Dunnett's test. Vehicle x IFN-α: P=0.0002, vehicle x IFN-β: P=0.0001 |
| 5D | Two-way RM ANOVA:<br>Interaction: $F_{(6, 60)}$ = 2.854, P=0.0164<br>Time effect: $F_{(6, 60)}$ = 10.41, P<0.0001<br>Genotyping effect: $F_{(1,10)}$ = 53.35, P<0.0001 | Bonferroni's test. WT+ IFN-α x MNK1$^{-/--}$+ IFN-α at 1h: P=0.0155, at 3h: P=0.0055, at 24h: P=0.0106, at 3d: P=0.0007 |
| 5E | Two-way RM ANOVA:<br>Interaction: $F_{(6, 60)}$ = 6.712, P<0.0001<br>Time effect: $F_{(6, 60)}$ = 10.73, P<0.0001<br>Genotyping effect: $F_{(1, 10)}$ = 48.24, P<0.0001 | Bonferroni's test. WT + IFN-β x MNK1$^{-/-}$+ IFN-β at 3h: P=0.0009, at 24h: P=0.0160, at 3d: P=0.0008 |
| 5H | Two-way RM ANOVA:<br>Interaction: $F_{(6, 60)}$ = 4.514, P=0.0008<br>Time effect: $F_{(6, 60)}$ = 14.40, P<0.0001<br>Genotyping effect: $F_{(1, 10)}$ = 19.43, P=0.0013 | Bonferroni's test. WT+ IFN-α x eIF4E$^{S209A}$+ IFN-α at 1h: P=0.0053, at 3h: 0.0047, at 3d: P=0.0384 |

(continued)

| Table 6D - Statistical Analvsis | | |
|---|---|---|
| Figure | Test | Posthoc comparison |
| 5I | Two-way RM ANOVA: Interaction: $F_{(6, 60)}$ = 3.227, P=0.0082 Time effect: $F_{(6, 60)}$ = 9.718, P<0.0001 Genotyping effect: $F_{(1, 10)}$ = 67.09, P<0.0001 | Bonferroni's test. WT+ IFN-β x eIF4E$^{S209A-/-}$+ IFN-β at 3h: P=0.009, at 24h: P=0.00,41 at 3d: P=0.0006 |
| 6A | Two-way RM ANOVA: Interaction: $F_{(6, 60)}$ = 3.504, P=0.0049 Time effect: $F_{(6, 60)}$ = 4.619, P=0.0006 Treatment effect: $F_{(1, 10)}$ = 18.44, P=0.0016 | Bonferroni's test. Vehicle x Poly (I:C) at 1h (day 2): **P=0.0034, at 6h: *P=0.0247, at 24h: ***P=0.0001. Dunnett's test. Baseline x 1h (Day 1): ###P=0.0009, Baseline x 24h: #P=0.0237, Baseline x 1h (day2): ####P<0.0001, Baseline x 6h: ###P=0.0002, Baseline x 24h: ####P<0.0001 |
| 6B | Two-way RM ANOVA: Interaction: $F_{(4, 40)}$ = 1.499, P=0.2208 Time effect: $F_{(4, 40)}$ = 0.3799, P=0.8216 Treatment effect: $F_{(1, 10)}$ = 6.34, P=0.0305 | Bonferroni's test: Vehicle x Poly (I:C) at 3h (day 2): *P=0.0159. |
| 6C | Unpaired t test. Day 1: t=7.098, P<0.0001; Day 2: t=3.54, P=0.0054 | N/A |
| 6D | Unpaired t test. t=3.519, P=0.0245 | N/A |
| 6E | Two-way RM ANOVA: Interaction: $F_{(6, 96)}$ = 2.163, P=0.0532 Time effect: $F_{(6, 96)}$ = 6.495, P<0.0001 Genotyping effect: $F_{(1, 16)}$ = 14.59, P=0.0015 | Bonferroni's test. WT + Poly (I:C) x MNK1$^{-/-}$ + Poly (I:C) at 24h (day 2): ***P=0.0007. Dunnett's test. Baseline x WT + Poly (I:C) at 1h (day 2): ##P=0.0038, at 6h: ###P=0.0007, ###P=0.0009 |
| 6F | Two-way RM ANOVA: Interaction: $F_{(4, 64)}$ = 1.272, P=0.2901 Time effect: $F_{(4, 64)}$ = 2.133 P=0.0869 Genotyping effect: $F_{(1, 16)}$ = 8.663, P=0.0095 | Dunnett's test. Baseline x WT + Poly (I:C) at 3h (day 2): #P=0.0216 |
| 6G | Two-way RM ANOVA: Interaction: $F_{(6,96)}$ = 1.712, P=0.1263 Time effect: $F_{(6, 96)}$ = 7.252, P<0.0001 Genotyping effect: $F_{(1, 16)}$ = 29.15, P<0.0001 | Bonferroni's test. WT + Poly (I:C) x eIF4E$^{S209A-/-}$+ Poly (I:C) at 1h (day 1): *P=0.0148, at 24h: *P=0.0493, at 6h (day2): ***P=0.0002 Dunnett's test. Baseline x WT + Poly (I:C) at 1h (day 1): ##P=0.0092, at 6h: #P=0.0371, at 24h: #P=0.0144, at 1h (day 2): ###P=0.0006, at 6h: ##P=0.0016, at 24h: #P=0.0041 |
| 6H | Two-way RM ANOVA: Interaction: $F_{(4, 64)}$ = 1.462, P=0.2242 Time effect: $F_{(4, 64)}$ = 0.2079, P=0.9331 Genotyping effect: $F_{(1, 16)}$ = 3.410, P=0.0834 | Dunnett's test: Baseline x WT + Poly (I:C) at 3h (day 2): #P=0.0216 |
| 6I | Ordinary One-way ANOVA: | Dunnett's test: |

(continued)

| Table 6D - Statistical Analvsis | | |
|---|---|---|
| Figure | Test | Posthoc comparison |
| | DRG; $F_{(2, 6)}$ = 42.63, P=0.0003 | P=0.002, WT x eIF4E$^{S209A-/-}$ |
| | SDH; $F_{(2, 6)}$ = 10.16, P=0.0119 | P=0.0441, WT x MNK1$^{-/-}$; P=0.0080, WT x eIF4E$^{S209A-/-}$ |
| | Sciatic Nerve; $F_{(2, 6)}$ = 210.6, P<0.0001 | P<0.0001, WT x MNK1$^{-/-}$; P<0.0001, WT x eIF4E$^{S209A-/-}$ |

All of the compositions and/or methods disclosed and claimed herein can be made and executed without undue experimentation in light of the present disclosure. While the compositions and methods of this disclosure have been described in terms of preferred embodiments, it will be apparent to those of skill in the art that variations may be applied to the compositions and/or methods and in the steps or in the sequence of steps of the method described herein without departing from the concept, spirit and scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the disclosure as defined by the appended claims.

## VI. References

The following references, to the extent that they provide exemplary procedural or other details supplementary to those set forth herein, are specifically incorporated herein by reference:


Moy et al., J. Neurosci. 37(31):7481-7499, 2017.
Remington's Pharmaceutical Sciences, 15th Edition.
WO2017117052
WO2018218038


## References for Example 1

Bar-Peled L, Sabatini DM (2014) Regulation of mTORC1 by amino acids. Trends in cell biology 24:400-406.

Barragan-Iglesias P, Lou TF, Bhat VD, Megat S, Burton MD, Price TJ, Campbell ZT (2018) Inhibition of Poly(A)-binding protein with a synthetic RNA mimic reduces pain sensitization in mice. Nat Commun 9:10.

Bennett GJ, Doyle T, Salvemini D (2014) Mitotoxicity in distal symmetrical sensory peripheral neuropathies. Nat Rev Neurol 10:326-336.

Berta T, Park CK, Xu ZZ, Xie RG, Liu T, Lu N, Liu YC, Ji RR (2014) Extracellular caspase-6 drives murine inflammatory pain via microglial TNF-alpha secretion. J Clin Invest 124:1173-1186.

Berta T, Perrin FE, Pertin M, Tonello R, Liu YC, Chamessian A, Kato AC, Ji RR, Decosterd I (2017) Gene Expression Profiling of Cutaneous Injured and Non-Injured Nociceptors in SNI Animal Model of Neuropathic Pain. Sci Rep 7:9367.

Campbell JN, Meyer RA (2006) Mechanisms of neuropathic pain. Neuron 52:77-92.

Chaplan SR, Bach FW, Pogrel JW, Chung JM, Yaksh TL (1994) Quantitative assessment of tactile allodynia in the rat paw. J Neurosci Methods 53:55-63.

Corder G, Tawfik VL, Wang D, Sypek EI, Low SA, Dickinson JR, Sotoudeh C, Clark JD, Barres BA, Bohlen CJ, Scherrer G (2017) Loss of mu opioid receptor signaling in nociceptors, but not microglia, abrogates morphine tolerance without disrupting analgesia. Nat Med 23:164-173.

Cowie AM, Moehring F, O'Hara C, Stucky CL (2018) Optogenetic Inhibition of CGRPalpha Sensory Neurons Reveals Their Distinct Roles in Neuropathic and Incisional Pain. J Neurosci 38:5807-5825.

Daou I, Beaudry H, Ase AR, Wieskopf JS, Ribeiro-da-Silva A, Mogil JS, Seguela P (2016) Optogenetic Silencing of Nav1.8-Positive Afferents Alleviates Inflammatory and Neuropathic Pain. eNeuro 3.

Denk F, McMahon SB, Tracey I (2014) Pain vulnerability: a neurobiological perspective. Nat Neurosci 17:192-200.

Doyle T, Chen Z, Muscoli C, Bryant L, Esposito E, Cuzzocrea S, Dagostino C, Ryerse J, Rausaria S, Kamadulski A, Neumann WL, Salvemini D (2012) Targeting the overproduction of peroxynitrite for the prevention and reversal of paclitaxel-induced neuropathic pain. J Neurosci 32:6149-6160.

Duggett NA, Griffiths LA, McKenna OE, de Santis V, Yongsanguanchai N, Mokori EB, Flatters SJ (2016) Oxidative

stress in the development, maintenance and resolution of paclitaxel-induced painful neuropathy. Neuroscience 333:13-26.
Eckhoff L, Knoop A, Jensen MB, Ewertz M (2015) Persistence of docetaxel-induced neuropathy and impact on quality of life among breast cancer survivors. Eur J Cancer 51:292-300.
Efeyan A, Zoncu R, Sabatini DM (2012) Amino acids and mTORC1: from lysosomes to disease. Trends Mol Med 18:524-533.
Efeyan A, Schweitzer LD, Bilate AM, Chang S, Kirak O, Lamming DW, Sabatini DM (2014) RagA, but not RagB, is essential for embryonic development and adult mice. Dev Cell 29:321-329.
Efeyan A, Zoncu R, Chang S, Gumper I, Snitkin H, Wolfson RL, Kirak O, Sabatini DD, Sabatini DM (2013) Regulation of mTORC1 by the Rag GTPases is necessary for neonatal autophagy and survival. Nature 493:679-683.
Finnerup NB, Attal N, Haroutounian S, McNicol E, Baron R, Dworkin RH, Gilron I, Haanpaa M, Hansson P, Jensen TS, Kamerman PR, Lund K, Moore A, Raja SN, Rice AS, Rowbotham M, Sena E, Siddall P, Smith BH, Wallace M (2015) Pharmacotherapy for neuropathic pain in adults: a systematic review and meta-analysis. Lancet Neurol 14:162-173.
Flatters SJ (2015) The contribution of mitochondria to sensory processing and pain. Prog Mol Biol Transl Sci 131:119-146.
Flatters SJ, Bennett GJ (2006) Studies of peripheral sensory nerves in paclitaxel-induced painful peripheral neuropathy: evidence for mitochondrial dysfunction. Pain 122:245-257.
Furic L, Rong L, Larsson O, Koumakpayi IH, Yoshida K, Brueschke A, Petroulakis E, Robichaud N, Pollak M, Gaboury LA, Pandolfi PP, Saad F, Sonenberg N (2010) eIF4E phosphorylation promotes tumorigenesis and is associated with prostate cancer progression. Proc Natl Acad Sci U S A 107:14134-14139.
Geranton SM, Jimenez-Diaz L, Torsney C, Tochiki KK, Stuart SA, Leith JL, Lumb BM, Hunt SP (2009) A rapamycin-sensitive signaling pathway is essential for the full expression of persistent pain states. J Neurosci 29:15017-15027.
Gereau RWt, Sluka KA, Maixner W, Savage SR, Price TJ, Murinson BB, Sullivan MD, Fillingim RB (2014) A pain research agenda for the 21st century. J Pain 15:1203-1214.
Gkogkas CG, Khoutorsky A, Cao R, Jafarnejad SM, Prager-Khoutorsky M, Giannakas N, Kaminari A, Fragkouli A, Nader K, Price TJ, Konicek BW, Graff JR, Tzinia AK, Lacaille JC, Sonenberg N (2014) Pharmacogenetic Inhibition of eIF4E-Dependent Mmp9 mRNA Translation Reverses Fragile X Syndrome-like Phenotypes. Cell Rep 9:1742-1755.
Glusman G, Caballero J, Robinson M, Kutlu B, Hood L (2013) Optimal scaling of digital transcriptomes. PLoS One 8:e77885.
Gold MS, Gebhart GF (2010) Nociceptor sensitization in pain pathogenesis. Nat Med 16:1248-1257.
Griffiths LA, Flatters SJ (2015) Pharmacological Modulation of the Mitochondrial Electron Transport Chain in Paclitaxel-Induced Painful Peripheral Neuropathy. J Pain 16:981-994.
Haroutounian S, Nikolajsen L, Bendtsen TF, Finnerup NB, Kristensen AD, Hasselstrom JB, Jensen TS (2014) Primary afferent input critical for maintaining spontaneous pain in peripheral neuropathy. Pain 155:1272-1279.
Haroutounian S, Ford AL, Frey K, Nikolajsen L, Finnerup NB, Neiner A, Kharasch ED, Karlsson P, Bottros MM (2018) How central is central poststroke pain? The role of afferent input in poststroke neuropathic pain: a prospective, open-label pilot study. Pain.
Heiman M, Kulicke R, Fenster RJ, Greengard P, Heintz N (2014) Cell type-specific mRNA purification by translating ribosome affinity purification (TRAP). Nat Protoc 9:1282-1291.
Heiman M, Schaefer A, Gong S, Peterson JD, Day M, Ramsey KE, Suarez-Farinas M, Schwarz C, Stephan DA, Surmeier DJ, Greengard P, Heintz N (2008) A translational profiling approach for the molecular characterization of CNS cell types. Cell 135:738-748.
Hu G, Huang K, Hu Y, Du G, Xue Z, Zhu X, Fan G (2016) Single-cell RNA-seq reveals distinct injury responses in different types of DRG sensory neurons. Sci Rep 6:31851.
Ingolia NT, Brar GA, Rouskin S, McGeachy AM, Weissman JS (2012) The ribosome profiling strategy for monitoring translation in vivo by deep sequencing of ribosome-protected mRNA fragments. Nat Protoc 7:1534-1550.
Jeske NA (2012) Somatosensory scaffolding structures. Front Mol Neurosci 5:2.
Jeske NA (2015) Peripheral scaffolding and signaling pathways in inflammatory pain. Prog Mol Biol Transl Sci 131:31-52.
Ji RR, Chamessian A, Zhang YQ (2016) Pain regulation by non-neuronal cells and inflammation. Science 354:572-577.
Ji RR, Samad TA, Jin SX, Schmoll R, Woolf CJ (2002) p38 MAPK activation by NGF in primary sensory neurons after inflammation increases TRPV1 levels and maintains heat hyperalgesia. Neuron 36:57-68.
Jimenez-Diaz L, Geranton SM, Passmore GM, Leith JL, Fisher AS, Berliocchi L, Sivasubramaniam AK, Sheasby A, Lumb BM, Hunt SP (2008) Local translation in primary afferent fibers regulates nociception. PLoS One 3:e1961.
Khoutorsky A, Price TJ (2018) Translational Control Mechanisms in Persistent Pain. Trends Neurosci 41:100-114.
King T, Vera-Portocarrero L, Gutierrez T, Vanderah TW, Dussor G, Lai J, Fields HL, Porreca F (2009) Unmasking the tonic-aversive state in neuropathic pain. Nature neuroscience 12:1364-1366.

Kuleshov MV, Jones MR, Rouillard AD, Fernandez NF, Duan Q, Wang Z, Koplev S, Jenkins SL, Jagodnik KM, Lachmann A, McDermott MG, Monteiro CD, Gundersen GW, Ma'ayan A (2016) Enrichr: a comprehensive gene set enrichment analysis web server 2016 update. Nucleic Acids Res 44:W90-97.

Li Y, Tatsui CE, Rhines LD, North RY, Harrison DS, Cassidy RM, Johansson CA, Kosturakis AK, Edwards DD, Zhang H, Dougherty PM (2017) Dorsal root ganglion neurons become hyperexcitable and increase expression of voltage-gated T-type calcium channels (Cav3.2) in paclitaxel-induced peripheral neuropathy. Pain 158:417-428.

Li Y, Adamek P, Zhang H, Tatsui CE, Rhines LD, Mrozkova P, Li Q, Kosturakis AK, Cassidy RM, Harrison DS, Cata JP, Sapire K, Zhang H, Kennamer-Chapman RM, Jawad AB, Ghetti A, Yan J, Palecek J, Dougherty PM (2015) The Cancer Chemotherapeutic Paclitaxel Increases Human and Rodent Sensory Neuron Responses to TRPV1 by Activation of TLR4. J Neurosci 35:13487-13500.

Liu Y, Beyer A, Aebersold R (2016) On the Dependency of Cellular Protein Levels on mRNA Abundance. Cell 165:535-550.

Lopes DM, Denk F, McMahon SB (2017a) The Molecular Fingerprint of Dorsal Root and Trigeminal Ganglion Neurons. Front Mol Neurosci 10:304.

Lopes DM, Malek N, Edye M, Jager SB, McMurray S, McMahon SB, Denk F (2017b) Sex differences in peripheral not central immune responses to pain-inducing injury. Sci Rep 7:16460.

Ma J, Kavelaars A, Dougherty PM, Heijnen CJ (2018) Beyond symptomatic relief for chemotherapy-induced peripheral neuropathy: Targeting the source. Cancer.

Manteniotis S, Lehmann R, Flegel C, Vogel F, Hofreuter A, Schreiner BS, Altmuller J, Becker C, Schobel N, Hatt H, Gisselmann G (2013) Comprehensive RNA-Seq expression analysis of sensory ganglia with a focus on ion channels and GPCRs in Trigeminal ganglia. PLoS One 8:e79523.

Melemedjian OK, Khoutorsky A, Sorge RE, Yan J, Asiedu MN, Valdez A, Ghosh S, Dussor G, Mogil JS, Sonenberg N, Price TJ (2013) mTORC1 inhibition induces pain via IRS-1-dependent feedback activation of ERK. Pain.

Melemedjian OK AM, Tillu DV, Sanoja R, Yan J, Lark A, Khoutorsky A, Johnson J, Peebles KA, Lepow T, Sonenberg N, Dussor G, Price TJ. (2011) Targeting adenosine monophosphate-activated protein kinase (AMPK) in preclinical models reveals a potential mechanism for the treatment of neuropathic pain. Molecular Pain.

Moy JK, Khoutorsky A, Asiedu MN, Dussor G, Price TJ (2018) eIF4E Phosphorylation Influences Bdnf mRNA Translation in Mouse Dorsal Root Ganglion Neurons. Front Cell Neurosci 12:29.

Moy JK, Khoutorsky A, Asiedu MN, Black BJ, Kuhn JL, Barragan-Iglesias P, Megat S, Burton MD, Burgos-Vega CC, Melemedjian OK, Boitano S, Vagner J, Gkogkas CG, Pancrazio JJ, Mogil JS, Dussor G, Sonenberg N, Price TJ (2017) The MNK-eIF4E Signaling Axis Contributes to Injury-Induced Nociceptive Plasticity and the Development of Chronic Pain. J Neurosci 37:7481-7499.

Obara I, Geranton SM, Hunt SP (2012) Axonal protein synthesis: a potential target for pain relief? Curr Opin Pharmacol 12:42-48.

Price TJ, Gold MS (2018) From Mechanism to Cure: Renewing the Goal to Eliminate the Disease of Pain. Pain medicine 19:1525-1549.

Price TJ, Basbaum AI, Bresnahan J, Chambers JF, De Koninck Y, Edwards RR, Ji RR, Katz J, Kavelaars A, Levine JD, Porter L, Schechter N, Sluka KA, Terman GW, Wager TD, Yaksh TL, Dworkin RH (2018) Transition to chronic pain: opportunities for novel therapeutics. Nat Rev Neurosci 19:383-384.

Ray P, Torck A, Quigley L, Wangzhou A, Neiman M, Rao C, Lam T, Kim JY, Kim TH, Zhang MQ, Dussor G, Price TJ (2018) Comparative transcriptome profiling of the human and mouse dorsal root ganglia: an RNA-seq-based resource for pain and sensory neuroscience research. Pain 159:1325-1345.

Reich SH et al. (2018) Structure-based Design of Pyridone-aminal eFT508 Targeting Dysregulated Translation by Selective Mitogen-activated Protein Kinase Interacting Kinases 1 and 2 (MNK1/2) Inhibition. J Med Chem.

Richter JD, Sonenberg N (2005) Regulation of cap-dependent translation by eIF4E inhibitory proteins. Nature 433:477-480.

Sancak Y, Bar-Peled L, Zoncu R, Markhard AL, Nada S, Sabatini DM (2010) Ragulator-Rag complex targets mTORC1 to the lysosomal surface and is necessary for its activation by amino acids. Cell 141:290-303.

Saxton RA, Sabatini DM (2017) mTOR Signaling in Growth, Metabolism, and Disease. Cell 168:960-976.

Seretny M, Currie GL, Sena ES, Ramnarine S, Grant R, MacLeod MR, Colvin LA, Fallon M (2014) Incidence, prevalence, and predictors of chemotherapy-induced peripheral neuropathy: A systematic review and meta-analysis. Pain 155:2461-2470.

Silva Amorim I, Kedia S, Kouloulia S, Simbriger K, Gantois I, Jafarnejad SM, Li Y, Kampaite A, Pooters T, Romano N, Gkogkas CG (2018) Loss of eIF4E phosphorylation engenders depression-like behaviors via selective mRNA translation. J Neurosci.

Soares HP, Ming M, Mellon M, Young SH, Han L, Sinnet-Smith J, Rozengurt E (2015) Dual PI3K/mTOR Inhibitors Induce Rapid Overactivation of the MEK/ERK Pathway in Human Pancreatic Cancer Cells through Suppression of mTORC2. Mol Cancer Ther 14:1014-1023.

Stirling LC, Forlani G, Baker MD, Wood JN, Matthews EA, Dickenson AH, Nassar MA (2005) Nociceptor-specific gene deletion using heterozygous NaV1.8-Cre recombinase mice. Pain 113:27-36.
Terenzio M, Koley S, Samra N, Rishal I, Zhao Q, Sahoo PK, Urisman A, Marvaldi L, Oses-Prieto JA, Forester C, Gomes C, Kalinski AL, Di Pizio A, Doron-Mandel E, Perry RB, Koppel I, Twiss JL, Burlingame AL, Fainzilber M (2018) Locally translated mTOR controls axonal local translation in nerve injury. Science 359:1416-1421.
Thakur M, Crow M, Richards N, Davey GI, Levine E, Kelleher JH, Agley CC, Denk F, Harridge SD, McMahon SB (2014) Defining the nociceptor transcriptome. Front Mol Neurosci 7:87.
Thoreen CC, Chantranupong L, Keys HR, Wang T, Gray NS, Sabatini DM (2012) A unifying model for mTORC1-mediated regulation of mRNA translation. Nature 485:109-113.
Ueda T, Watanabe-Fukunaga R, Fukuyama H, Nagata S, Fukunaga R (2004) Mnk2 and Mnk1 are essential for constitutive and inducible phosphorylation of eukaryotic initiation factor 4E but not for cell growth or development. Mol Cell Biol 24:6539-6549.
Usoskin D, Furlan A, Islam S, Abdo H, Lonnerberg P, Lou D, Hjerling-Leffler J, Haeggstrom J, Kharchenko O, Kharchenko PV, Linnarsson S, Ernfors P (2015) Unbiased classification of sensory neuron types by large-scale single-cell RNA sequencing. Nat Neurosci 18:145-153.
Uttam S, Wong C, Amorim IS, Jafarnej SM, Tansley SM, Yang J, Prager-Khoutorsky M, Mogil JS, Gkogkas CG, Khoutorsky A (2018) Translational profiling of dorsal root ganglia and spinal cord in a mouse model of neuropathic pain. Neurobiology of Pain.
Vaso A, Adahan HM, Gjika A, Zahaj S, Zhurda T, Vyshka G, Devor M (2014) Peripheral nervous system origin of phantom limb pain. Pain 155:1384-1391.
Ventzel L, Jensen AB, Jensen AR, Jensen TS, Finnerup NB (2016) Chemotherapy-induced pain and neuropathy: a prospective study in patients treated with adjuvant oxaliplatin or docetaxel. Pain 157:560-568.
Wieskopf JS et al. (2015) The nicotinic alpha6 subunit gene determines variability in chronic pain sensitivity via cross-inhibition of P2X2/3 receptors. Sci Transl Med 7:287ra272. Wolfe AL *et al.* (2014) RNA G-quadruplexes cause eIF4A-dependent oncogene translation in
cancer. Nature.
Wolfson RL, Sabatini DM (2017) The Dawn of the Age of Amino Acid Sensors for the mTORC1 Pathway. Cell Metab 26:301-309.
Yang Q, Wu Z, Hadden JK, Odem MA, Zuo Y, Crook RJ, Frost JA, Walters ET (2014) Persistent pain after spinal cord injury is maintained by primary afferent activity. J Neurosci 34:10765-10769.
Yilmaz E, Gold MS (2016) Paclitaxel-induced increase in NCX activity in subpopulations of nociceptive afferents: A protective mechanism against chemotherapy-induced peripheral neuropathy? Cell Calcium 60:25-31.
Zhou P, Zhang Y, Ma Q, Gu F, Day DS, He A, Zhou B, Li J, Stevens SM, Romo D, Pu WT (2013) Interrogating translational efficiency and lineage-specific transcriptomes using ribosome affinity purification. Proc Natl Acad Sci U S A 110:15395-15400.

**References for Example 2**

Eccleston, C. Chronic pain and distraction: an experimental investigation into the role of sustained and shifting attention in the processing of chronic persistent pain. Behav Res Ther 33, 391-405 (1995).
Povedano, M., Gascon, J., Galvez, R., Ruiz, M. & Rejas, J. Cognitive function impairment in patients with neuropathic pain under standard conditions of care. J Pain Symptom Manage 33, 78-89, doi:10.1016/j.jpainsymman.2006.07.012 (2007).
Schiltenwolf, M. et al. Evidence of specific cognitive deficits in patients with chronic low back pain under long-term substitution treatment of opioids. Pain Physician 17, 9-20 (2014).
Shiers, S. et al. Neuropathic Pain Creates an Enduring Prefrontal Cortex Dysfunction Corrected by the Type II Diabetic Drug Metformin But Not by Gabapentin. J Neurosci 38, 7337-7350, doi:10.1523/JNEUROSCI.0713-18.2018 (2018).
Melemedjian, O. K. et al. Targeting adenosine monophosphate-activated protein kinase (AMPK) in preclinical models reveals a potential mechanism for the treatment of neuropathic pain. Mol Pain 7, 70, doi:10.1186/1744-8069-7-70 (2011).
Moy, J. K. et al. The MNK-eIF4E Signaling Axis Contributes to Injury-Induced Nociceptive Plasticity and the Development of Chronic Pain. J Neurosci 37, 7481-7499, doi:10.1523/JNEUROSCI.0220-17.2017 (2017).
Moy, J. K., Kuhn, J. L., Szabo-Pardi, T. A., Pradhan, G. & Price, T. J. eIF4E phosphorylation regulates ongoing pain, independently of inflammation, and hyperalgesic priming in the mouse CFA model. Neurobiol Pain 4, 45-50, doi:10.1016/j.ynpai.2018.03.001 (2018a).
Inyang, K. E. et al. The antidiabetic drug metformin prevents and reverses neuropathic pain and spinal cord microglial

activation in male but not female mice. Pharmacol Res 139, 1-16, doi:10.1016/j.phrs.2018.10.027 (2019).

Megat, S. et al. Nociceptor Translational Profiling Reveals the Ragulator-Rag GTPase Complex as a Critical Generator of Neuropathic Pain. J Neurosci 39, 393-411, doi:10.1523/JNEUROSCI.2661-18.2018 (2019).

Yang, Q. et al. Persistent pain after spinal cord injury is maintained by primary afferent activity. J Neurosci 34, 10765-10769, doi:10.1523/JNEUROSCI.5316-13.2014 (2014).

Cowie, A. M., Moehring, F., O'Hara, C. & Stucky, C. L. Optogenetic Inhibition of CGRPalpha Sensory Neurons Reveals Their Distinct Roles in Neuropathic and Incisional Pain. J Neurosci 38, 5807-5825, doi:10.1523/JNEUROSCI.3565-17.2018 (2018).

North, R. Y. et al. Electrophysiological and transcriptomic correlates of neuropathic pain in human dorsal root ganglion neurons. Brain 142, 1215-1226, doi:10.1093/brain/awz063 (2019).

Khoutorsky, A. & Price, T. J. Translational Control Mechanisms in Persistent Pain. Trends Neurosci 41, 100-114, doi: 10.1016/j.tins.2017.11.006 (2018).

Uttam, S., Wong, C., Price, T. J. & Khoutorsky, A. eIF4E-Dependent Translational Control: A Central Mechanism for Regulation of Pain Plasticity. Front Genet 9, 470, doi:10.3389/fgene.2018.00470 (2018).

Furic, L. et al. eIF4E phosphorylation promotes tumorigenesis and is associated with prostate cancer progression. Proc Natl Acad Sci U S A 107, 14134-14139, doi:10.1073/pnas.1005320107 (2010).

Gkogkas, C. G. et al. Pharmacogenetic inhibition of eIF4E-dependent Mmp9 mRNA translation reverses fragile X syndrome-like phenotypes. Cell Rep 9, 1742-1755, doi:10.1016/j.celrep.2014.10.064 (2014).

Amorim, I. S. et al. Loss of eIF4E Phosphorylation Engenders Depression-like Behaviors via Selective mRNA Translation. J Neurosci 38, 2118-2133, doi:10.1523/JNEUROSCI.2673-17.2018 (2018).

Moy, J. K., Khoutorsky, A., Asiedu, M. N., Dussor, G. & Price, T. J. eIF4E Phosphorylation Influences Bdnf mRNA Translation in Mouse Dorsal Root Ganglion Neurons. Front Cell Neurosci 12, 29, doi:10.3389/fncel.2018.00029 (2018b).

Ueda, T., Watanabe-Fukunaga, R., Fukuyama, H., Nagata, S. & Fukunaga, R. Mnk2 and Mnk1 are essential for constitutive and inducible phosphorylation of eukaryotic initiation factor 4E but not for cell growth or development. Mol Cell Biol 24, 6539-6549, doi:10.1128/MCB.24.15.6539-6549.2004 (2004).

Jackson-Laboratory. Body Weight Information for C57BL/6J. (2019).

Decosterd, I. & Woolf, C. J. Spared nerve injury: an animal model of persistent peripheral neuropathic pain. Pain 87, 149-158 (2000).

Chaplan, S. R., Bach, F. W., Pogrel, J. W., Chung, J. M. & Yaksh, T. L. Quantitative assessment of tactile allodynia in the rat paw. J Neurosci Methods 53, 55-63 (1994).

Angoa-Perez, M., Kane, M. J., Briggs, D. I., Francescutti, D. M. & Kuhn, D. M. Marble burying and nestlet shredding as tests of repetitive, compulsive-like behaviors in mice. J Vis Exp, 50978, doi: 10.3791/50978 (2013).

Black, B. J. et al. Adult mouse sensory neurons on microelectrode arrays exhibit increased spontaneous and stimulus-evoked activity in the presence of interleukin-6. J Neurophysiol 120, 1374-1385, doi:10.1152/jn.00158.2018 (2018).

King, T. et al. Unmasking the tonic-aversive state in neuropathic pain. Nat Neurosci 12, 1364-1366, doi:10.1038/nn.2407 (2009).

Reich, S. H. et al. Structure-based Design of Pyridone-Aminal eFT508 Targeting Dysregulated Translation by Selective Mitogen-activated Protein Kinase Interacting Kinases 1 and 2 (MNK1/2) Inhibition. J Med Chem 61, 3516-3540, doi:10.1021/acs.jmedchem.7b01795 (2018).

Grubb, M. S. & Burrone, J. Activity-dependent relocation of the axon initial segment fine-tunes neuronal excitability. Nature 465, 1070-1074, doi:10.1038/nature09160 (2010).

Kuba, H., Oichi, Y. & Ohmori, H. Presynaptic activity regulates Na(+) channel distribution at the axon initial segment. Nature 465, 1075-1078, doi:10.1038/nature09087 (2010).

Grubb, M. S. et al. Short- and long-term plasticity at the axon initial segment. J Neurosci 31, 16049-16055, doi:10.1523/JNEUROSCI.4064-11.2011 (2011).

Richards, G. C. et al. Effects of long-term opioid analgesics on cognitive performance and plasma cytokine concentrations in patients with chronic low back pain: a cross-sectional pilot study. Pain Rep 3, e669, doi:10.1097/PR9.0000000000000669 (2018).

APF. Voices of Chronic Pain. (American Pain Foundation, 2006).

Apkarian, A. V. et al. Chronic back pain is associated with decreased prefrontal and thalamic gray matter density. J Neurosci 24, 10410-10415, doi:10.1523/JNEUROSCI.2541-04.2004 (2004).

Schmidt-Wilcke, T. et al. Gray matter decrease in patients with chronic tension type headache. Neurology 65, 1483-1486, doi:10.1212/01.wnl.0000183067.94400.80 (2005).

Davis, K. D. et al. Cortical thinning in IBS: implications for homeostatic, attention, and pain processing. Neurology 70, 153-154, doi:10.1212/01.wnl.0000295509.30630.10 (2008).

Kuchinad, A. et al. Accelerated brain gray matter loss in fibromyalgia patients: premature aging of the brain? J Neurosci 27, 4004-4007, doi:10.1523/JNEUROSCI.0098-07.2007 (2007).

Metz, A. E., Yau, H. J., Centeno, M. V., Apkarian, A. V. & Martina, M. Morphological and functional reorganization of rat medial prefrontal cortex in neuropathic pain. Proc Natl Acad Sci U S A 106, 2423-2428, doi:10.1073/pnas.0809897106 (2009).
Seminowicz, D. A. et al. MRI structural brain changes associated with sensory and emotional function in a rat model of long-term neuropathic pain. Neuroimage 47, 1007-1014, doi:10.1016/j.neuroimage.2009.05.068 (2009).
Ji, G. et al. Cognitive impairment in pain through amygdala-driven prefrontal cortical deactivation. J Neurosci 30, 5451-5464, doi:10.1523/JNEUROSCI.0225-10.2010 (2010).
Zhang, Z. et al. Role of Prelimbic GABAergic Circuits in Sensory and Emotional Aspects of Neuropathic Pain. Cell Rep 12, 752-759, doi:10.1016/j.celrep.2015.07.001 (2015).
Kelly, C. J., Huang, M., Meltzer, H. & Martina, M. Reduced Glutamatergic Currents and Dendritic Branching of Layer 5 Pyramidal Cells Contribute to Medial Prefrontal Cortex Deactivation in a Rat Model of Neuropathic Pain. Front Cell Neurosci 10, 133, doi:10.3389/fncel.2016.00133 (2016).
Cheriyan, J. & Sheets, P. L. Altered Excitability and Local Connectivity of mPFC-PAG Neurons in a Mouse Model of Neuropathic Pain. J Neurosci 38, 4829-4839, doi:10.1523/JNEUROSCI.2731-17.2018 (2018).
Gauriau, C. & Bernard, J. F. Pain pathways and parabrachial circuits in the rat. Exp Physiol 87, 251-258 (2002).
Goncalves, L. & Dickenson, A. H. Asymmetric time-dependent activation of right central amygdala neurones in rats with peripheral neuropathy and pregabalin modulation. Eur J Neurosci 36, 3204-3213, doi:10.1111/j.1460-9568.2012.08235.x (2012).
Neugebauer, V., Li, W., Bird, G. C. & Han, J. S. The amygdala and persistent pain. Neuroscientist 10, 221-234, doi:10.1177/1073858403261077 (2004).
Amorim, I. S., Lach, G. & Gkogkas, C. G. The Role of the Eukaryotic Translation Initiation Factor 4E (eIF4E) in Neuropsychiatric Disorders. Front Genet 9, 561, doi:10.3389/fgene.2018.00561 (2018).
Aguilar-Valles, A. et al. Translational control of depression-like behavior via phosphorylation of eukaryotic translation initiation factor 4E. Nat Commun 9, 2459, doi:10.1038/s41467-018-04883-5 (2018).
Abrahamsen, B. et al. The cell and molecular basis of mechanical, cold, and inflammatory pain. Science 321, 702-705, doi:10.1126/science.1156916 (2008).
Knowlton, W. M. et al. A sensory-labeled line for cold: TRPM8-expressing sensory neurons define the cellular basis for cold, cold pain, and cooling-mediated analgesia. J Neurosci 33, 2837-2848, doi:10.1523/JNEUROSCI.1943-12.2013 (2013).
Price, T. J. & Prescott, S. A. Inhibitory regulation of the pain gate and how its failure causes pathological pain. Pain 156, 789-792, doi:10.1097/j.pain.0000000000000139 (2015).
Leterrier, C. The Axon Initial Segment: An Updated Viewpoint. J Neurosci 38, 2135-2145, doi:10.1523/JNEUROSCI.1922-17.2018 (2018).
Kuba, H., Yamada, R., Ishiguro, G. & Adachi, R. Redistribution of Kv1 and Kv7 enhances neuronal excitability during structural axon initial segment plasticity. Nat Commun 6, 8815, doi:10.1038/ncomms9815 (2015).
Mitric, M. et al. Layer- and subregion-specific electrophysiological and morphological changes of the medial prefrontal cortex in a mouse model of neuropathic pain. Sci Rep 9, 9479, doi:10.1038/s41598-019-45677-z (2019).
Seminowicz, D. A. et al. Cognitive-behavioral therapy increases prefrontal cortex gray matter in patients with chronic pain. J Pain 14, 1573-1584, doi:10.1016/j.jpain.2013.07.020 (2013).
Rodriguez-Raecke, R., Niemeier, A., Ihle, K., Ruether, W. & May, A. Brain gray matter decrease in chronic pain is the consequence and not the cause of pain. J Neurosci 29, 13746-13750, doi:10.1523/JNEUROSCI.3687-09.2009 (2009).
Rodriguez-Raecke, R., Niemeier, A., Ihle, K., Ruether, W. & May, A. Structural brain changes in chronic pain reflect probably neither damage nor atrophy. PLoS One 8, e54475, doi:10.1371/journal.pone.0054475 (2013).
Seminowicz, D. A. et al. Effective treatment of chronic low back pain in humans reverses abnormal brain anatomy and function. J Neurosci 31, 7540-7550, doi:10.1523/JNEUROSCI.5280-10.2011 (2011).

**References for Example 3**

Aziz-Donnelly A, Harrison TB (2017) Update of HIV-Associated Sensory Neuropathies. Curr Treat Options Neurol 19:36.
Balachandran S, Barber GN (2007) PKR in innate immunity, cancer, and viral oncolysis. Methods Mol Biol 383:277-301.
Baral P, Mills K, Pinho-Ribeiro FA, Chiu IM (2016) Pain and Itch: Beneficial or Harmful to Antimicrobial Defense? Cell Host Microbe 19:755-759.
Barragan-Iglesias P, Kuhn J, Vidal-Cantu GC, Salinas-Abarca AB, Granados-Soto V, Dussor GO, Campbell ZT, Price

TJ (2019) Activation of the integrated stress response in nociceptors drives methylglyoxal-induced pain. Pain 160:160-171.
Barrat FJ, Crow MK, Ivashkiv LB (2019) Interferon target-gene expression and epigenomic signatures in health and disease. Nat Immunol 20:1574-1583.
Blake KJ, Baral P, Voisin T, Lubkin A, Pinho-Ribeiro FA, Adams KL, Roberson DP, Ma YC, Otto M, Woolf CJ, Torres VJ, Chiu IM (2018) Staphylococcus aureus produces pain through pore-forming toxins and neuronal TRPV1 that is silenced by QX-314. Nat Commun 9:37.
Brizzi KT, Lyons JL (2014) Peripheral nervous system manifestations of infectious diseases. Neurohospitalist 4:230-240.
Butler A, Hoffman P, Smibert P, Papalexi E, Satija R (2018) Integrating single-cell transcriptomic data across different conditions, technologies, and species. Nat Biotechnol 36:411-420.
Capuron L, Gumnick JF, Musselman DL, Lawson DH, Reemsnyder A, Nemeroff CB, Miller AH (2002) Neurobehavioral effects of interferon-alpha in cancer patients: phenomenology and paroxetine responsiveness of symptom dimensions. Neuropsychopharmacology 26:643-652.
Chaplan SR, Bach FW, Pogrel JW, Chung JM, Yaksh TL (1994) Quantitative assessment of tactile allodynia in the rat paw. J Neurosci Methods 53:55-63.
Cheng L, Yu H, Li G, Li F, Ma J, Li J, Chi L, Zhang L, Su L (2017) Type I interferons suppress viral replication but contribute to T cell depletion and dysfunction during chronic HIV-1 infection. JCI Insight 2.
Chiu IM, Pinho-Ribeiro FA, Woolf CJ (2016) Pain and infection: pathogen detection by nociceptors. Pain 157:1192-1193.
Chiu IM, Heesters BA, Ghasemlou N, Von Hehn CA, Zhao F, Tran J, Wainger B, Strominger A, Muralidharan S, Horswill AR, Bubeck Wardenburg J, Hwang SW, Carroll MC, Woolf CJ (2013) Bacteria activate sensory neurons that modulate pain and inflammation. Nature 501:52-57.
de la Pena JBI, Song JJ, Campbell ZT (2019) RNA control in pain: Blame it on the messenger. Wiley Interdiscip Rev RNA 10:e1546.
de Weerd NA, Nguyen T (2012) The interferons and their receptors--distribution and regulation. Immunol Cell Biol 90:483-491.
Fitzgibbon M, Kerr DM, Henry RJ, Finn DP, Roche M (2019) Endocannabinoid modulation of inflammatory hyperalgesia in the IFN-alpha mouse model of depression. Brain Behav Immun 82:372-381.
Forster S (2012) Interferon signatures in immune disorders and disease. Immunol Cell Biol 90:520-527.
Foster SL, Seehus CR, Woolf CJ, Talbot S (2017) Sense and Immunity: Context-Dependent Neuro-Immune Interplay. Front Immunol 8:1463.
Furic L, Rong L, Larsson O, Koumakpayi IH, Yoshida K, Brueschke A, Petroulakis E, Robichaud N, Pollak M, Gaboury LA, Pandolfi PP, Saad F, Sonenberg N (2010) eIF4E phosphorylation promotes tumorigenesis and is associated with prostate cancer progression. Proc Natl Acad Sci U S A 107:14134-14139.
Gaelings L et al. (2017) Regulation of kynurenine biosynthesis during influenza virus infection. FEBS J 284:222-236.
Gerlach N, Schimmer S, Weiss S, Kalinke U, Dittmer U (2006) Effects of type I interferons on Friend retrovirus infection. Journal of virology 80:3438-3444.
Hadley GR, Gayle JA, Ripoll J, Jones MR, Argoff CE, Kaye RJ, Kaye AD (2016) Post-herpetic Neuralgia: a Review. Curr Pain Headache Rep 20:17.
Hargreaves K, Dubner R, Brown F, Flores C, Joris J (1988) A new and sensitive method for measuring thermal nociception in cutaneous hyperalgesia. Pain 32:77-88.
Hilkens CM, Schlaak JF, Kerr IM (2003) Differential responses to IFN-alpha subtypes in human T cells and dendritic cells. J Immunol 171:5255-5263.
Hjortsberg L, Lindvall C, Corcoran M, Arulampalam V, Chan D, Thyrell L, Nordenskjold M, Grander D, Pokrovskaja K (2007) Phosphoinositide 3-kinase regulates a subset of interferon-alpha-stimulated genes. Exp Cell Res 313:404-414.
Huang L, Ou R, Rabelo de Souza G, Cunha TM, Lemos H, Mohamed E, Li L, Pacholczyk G, Randall J, Munn DH, Mellor AL (2016) Virus Infections Incite Pain Hypersensitivity by Inducing Indoleamine 2,3 Dioxygenase. PLoS Pathog 12:e1005615.
Inceoglu B, Bettaieb A, Trindade da Silva CA, Lee KS, Haj FG, Hammock BD (2015) Endoplasmic reticulum stress in the peripheral nervous system is a significant driver of neuropathic pain. Proc Natl Acad Sci U S A 112:9082-9087.
Ivashkiv LB, Donlin LT (2014) Regulation of type I interferon responses. Nat Rev Immunol 14:36-49.
Joshi S, Kaur S, Redig AJ, Goldsborough K, David K, Ueda T, Watanabe-Fukunaga R, Baker DP, Fish EN, Fukunaga R, Platanias LC (2009) Type I interferon (IFN)-dependent activation of Mnk1 and its role in the generation of growth inhibitory responses. Proc Natl Acad Sci U S A 106:12097-12102.
Kawai T, Akira S (2008) Toll-like receptor and RIG-I-like receptor signaling. Ann N Y Acad Sci 1143:1-20.
Khoutorsky A, Price TJ (2018) Translational Control Mechanisms in Persistent Pain. Trends Neurosci 41:100-114.

Khoutorsky A, Sorge RE, Prager-Khoutorsky M, Pawlowski SA, Longo G, Jafarnejad SM, Tahmasebi S, Martin LJ, Pitcher MH, Gkogkas CG, Sharif-Naeini R, Ribeiro-da-Silva A, Bourque CW, Cervero F, Mogil JS, Sonenberg N (2016) eIF2alpha phosphorylation controls thermal nociception. Proc Natl Acad Sci U S A 113:11949-11954.

Lai NY et al. (2019) Gut-Innervating Nociceptor Neurons Regulate Peyer's Patch Microfold Cells and SFB Levels to Mediate Salmonella Host Defense. Cell.

Larner AC, Chaudhuri A, Darnell JE, Jr. (1986) Transcriptional induction by interferon. New protein(s) determine the extent and length of the induction. J Biol Chem 261:453-459.

Levy DE, Darnell JE, Jr. (2002) Stats: transcriptional control and biological impact. Nat Rev Mol Cell Biol 3:651-662.

Li CL, Li KC, Wu D, Chen Y, Luo H, Zhao JR, Wang SS, Sun MM, Lu YJ, Zhong YQ, Hu XY, Hou R, Zhou BB, Bao L, Xiao HS, Zhang X (2016) Somatosensory neuron types identified by high-coverage single-cell RNA-sequencing and functional heterogeneity. Cell research 26:83-102.

Liu CC, Gao YJ, Luo H, Berta T, Xu ZZ, Ji RR, Tan PH (2016) Interferon alpha inhibits spinal cord synaptic and nociceptive transmission via neuronal-glial interactions. Sci Rep 6:34356.

Liu S, Karaganis S, Mo RF, Li XX, Wen RX, Song XJ (2019) IFNbeta treatment inhibits nerve injury-induced mechanical allodynia and MAPK signaling by activating ISG15 in mouse spinal cord. J Pain.

Liu T, Gao YJ, Ji RR (2012) Emerging role of Toll-like receptors in the control of pain and itch. Neurosci Bull 28:131-144.

Malcangio M (2019) Role of the immune system in neuropathic pain. Scand J Pain.

Megat S, Ray PR, Moy JK, Lou TF, Barragan-Iglesias P, Li Y, Pradhan G, Wanghzou A, Ahmad A, Burton MD, North RY, Dougherty PM, Khoutorsky A, Sonenberg N, Webster KR, Dussor G, Campbell ZT, Price TJ (2019) Nociceptor Translational Profiling Reveals the Ragulator-Rag GTPase Complex as a Critical Generator of Neuropathic Pain. J Neurosci 39:393-411.

Moy JK, Khoutorsky A, Asiedu MN, Black BJ, Kuhn JL, Barragan-Iglesias P, Megat S, Burton MD, Burgos-Vega CC, Melemedjian OK, Boitano S, Vagner J, Gkogkas CG, Pancrazio JJ, Mogil JS, Dussor G, Sonenberg N, Price TJ (2017) The MNK-eIF4E Signaling Axis Contributes to Injury-Induced Nociceptive Plasticity and the Development of Chronic Pain. J Neurosci 37:7481-7499.

Munir M, Berg M (2013) The multiple faces of proteinkinase R in antiviral defense. Virulence 4:85-89.

Murray C, Griffin EW, O'Loughlin E, Lyons A, Sherwin E, Ahmed S, Stevenson NJ, Harkin A, Cunningham C (2015) Interdependent and independent roles of type I interferons and IL-6 in innate immune, neuroinflammatory and sickness behaviour responses to systemic poly I:C. Brain Behav Immun 48:274-286.

Nogueira JB, Sena LC, Quintans Jde S, Almeida JR, Franca AV, Junior LJ (2012) Side effects of the therapy with peginterferon and ribavirin in chronic hepatitis C: a small audit. J Pharm Pract 25:85-88.

Obara I, Hunt SP (2014) Axonal protein synthesis and the regulation of primary afferent function. Dev Neurobiol 74:269-278.

Pindel A, Sadler A (2011) The role of protein kinase R in the interferon response. J Interferon Cytokine Res 31:59-70.

Pinho-Ribeiro FA, Verri WA, Jr., Chiu IM (2017) Nociceptor Sensory Neuron-Immune Interactions in Pain and Inflammation. Trends Immunol 38:5-19.

Platanias LC (2005) Mechanisms of type-I- and type-II-interferon-mediated signalling. Nat Rev Immunol 5:375-386.

Ray P, Torck A, Quigley L, Wangzhou A, Neiman M, Rao C, Lam T, Kim JY, Kim TH, Zhang MQ, Dussor G, Price TJ (2018) Comparative transcriptome profiling of the human and mouse dorsal root ganglia: an RNA-seq-based resource for pain and sensory neuroscience research. Pain 159:1325-1345.

Rodriguez Y, Vatti N, Ramirez-Santana C, Chang C, Mancera-Paez O, Gershwin ME, Anaya JM (2019) Chronic inflammatory demyelinating polyneuropathy as an autoimmune disease. J Autoimmun 102:8-37.

Saleiro D, Mehrotra S, Kroczynska B, Beauchamp EM, Lisowski P, Majchrzak-Kita B, Bhagat TD, Stein BL, McMahon B, Altman JK, Kosciuczuk EM, Baker DP, Jie C, Jafari N, Thompson CB, Levine RL, Fish EN, Verma AK, Platanias LC (2015) Central role of ULK1 in type I interferon signaling. Cell Rep 11:605-617.

Schreiber G (2017) The molecular basis for differential type I interferon signaling. J Biol Chem 292:7285-7294.

Sen GC, Sarkar SN (2007) The interferon-stimulated genes: targets of direct signaling by interferons, double-stranded RNA, and viruses. Curr Top Microbiol Immunol 316:233-250.

Shakoor A, Shafqat F, Mehmud T, Akram M, Riaz S, Iqbal Z, Khan AA (2010) Frequency of depression and somatic symptoms in patients on interferon alpha/ribavirin for chronic hepatitis C. J Ayub Med Coll Abbottabad 22:6-9.

Shibamiya A, Hersemeyer K, Schmidt Woll T, Sedding D, Daniel JM, Bauer S, Koyama T, Preissner KT, Kanse SM (2009) A key role for Toll-like receptor-3 in disrupting the hemostasis balance on endothelial cells. Blood 113:714-722.

Shiers S, Mwirigi J, Pradhan G, Kume M, Black B, Barragan-Iglesias P, Moy JK, Dussor G, Pancrazio JJ, Kroener S, Price TJ (2019) Reversal of peripheral nerve injury-induced neuropathic pain and cognitive dysfunction via genetic and tomivosertib targeting of MNK. Neuropsychopharmacology.

Stark GR, Darnell JE, Jr. (2012) The JAK-STAT pathway at twenty. Immunity 36:503-514. Thyrell L, Hjortsberg L, Arulampalam V, Panaretakis T, Uhles S, Dagnell M, Zhivotovsky B, Leibiger I, Grander D, Pokrovskaja K (2004)

Interferon alpha-induced apoptosis in tumor cells is mediated through the phosphoinositide 3-kinase/mammalian target of rapamycin signaling pathway. J Biol Chem 279:24152-24162.
Ueda T, Watanabe-Fukunaga R, Fukuyama H, Nagata S, Fukunaga R (2004) Mnk2 and Mnk1 are essential for constitutive and inducible phosphorylation of eukaryotic initiation factor 4E but not for cell growth or development. Mol Cell Biol 24:6539-6549.
van der Maaten L, Hinton G (2008) Visualizing Data using t-SNE. Journal of Machine Learning Research 9:2579-2605.
Walsh D, Mathews MB, Mohr I (2013) Tinkering with translation: protein synthesis in virus-infected cells. Cold Spring Harb Perspect Biol 5:a012351.
Waskiewicz AJ, Flynn A, Proud CG, Cooper JA (1997) Mitogen-activated protein kinases activate the serine/threonine kinases Mnk1 and Mnk2. EMBO J 16:1909-1920.
Waskiewicz AJ, Johnson JC, Penn B, Mahalingam M, Kimball SR, Cooper JA (1999) Phosphorylation of the cap-binding protein eukaryotic translation initiation factor 4E by protein kinase Mnk1 in vivo. Mol Cell Biol 19:1871-1880.
Yamamoto M, Sato S, Hemmi H, Hoshino K, Kaisho T, Sanjo H, Takeuchi O, Sugiyama M, Okabe M, Takeda K, Akira S (2003) Role of adaptor TRIF in the MyD88-independent toll-like receptor signaling pathway. Science 301:640-643.

The present invention relates to the following items:

1. A method of treating, inhibiting, preventing or reversing neuropathic pain in a subject comprising administering to said subject an amount of eFT508 sufficient to treat, inhibit, prevent or reverse said neuropathic pain.

2. A method of treating, inhibiting, preventing or reversing a cognitive deficit comorbid with neuropathic pain in a subject comprising administering to said subject an amount of eFT508 sufficient to treat, inhibit, prevent or reverse said cognitive deficit.

3. The method of items 1-2, wherein said subject is a human.

4. The method of items 1-2, wherein said subject is a non-human mammal.

5. The method of items 1-2, wherein said neuropathic pain is chronic neuropathic pain.

6. The method of items 1-2, wherein said neuropathic pain is acute neuropathic pain.

7. The method of items 1-2, wherein eFT508 is administered with a second anti-pain agent.

8. The method of item 7, wherein eFT508 and said second anti-pain agent are co-formulated.

9. The method of item 7, wherein eFT508 and said second anti-pain agent are not co-formulated.

10. The method of item 7, wherein eFT508 and said second anti-pain agent are delivered at distinct times.

11. The method of item 10, wherein eFT508 is delivered before said second anti-pain agent.

12. The method of item 10, wherein eFT508 is delivered after said second anti-pain agent.

13. The method of item 10, wherein eFT508 and said second anti-pain agent are delivered in alternating administrations.

14. The method of item 7, wherein said second anti-pain agent is an opioid or a gabapentanoid.

15. The method of items 1-2, wherein eFT508 is delivered over a period of one week, two weeks, three weeks, four weeks, one month, two months, three months, four months, five months, six months, seven months, eight months, nine months, ten months, eleven months, one year, two years or three years.

16. The method of items 1-2, wherein eFT508 is delivered by continuous infusion.

17. The method of item 16, wherein continuous infusion is provided by an implanted pump.

18. The method of items 1-2, wherein said neuropathic pain is the result of an injury.

19. The method of item 18, wherein said injury is a peripheral nerve injury.

20. The method of item 18, wherein said injury is a penetration wound, a burn, frostbite or a fracture.

21. The method of items 1-2, wherein said neuropathic pain is the result of a disease.

22. The method of item 21, wherein said disease is diabetes, cancer, spinal nerve disease, multiple sclerosis, arthritis, an autoimmune disease, or an infection, such as viral infection, fungal infection or bacterial infection.

23. The methd of items 1-2, wherein said neuropathic pain is the result of chemotherapy.

24. The methd of items 1-2, wherein said neuropathic pain is the result of surgery.

## Claims

**1.** A composition comprising an effective amount of eFT508, or a pharmaceutically acceptable salt thereof, for use in the treatment of neuropathic pain, wherein the neuropathic pain is the result of a peripheral nerve injury.

**2.** A composition comprising an effective amount of eFT508, or a pharmaceutically acceptable salt thereof, for use in the treatment of a cognitive deficit comorbid with neuropathic pain.

**3.** The composition for use of claim 1 or 2, the use further comprising administering a second anti-pain agent.

**4.** The composition for use of any one of claims 1 to 3, wherein said neuropathic pain is chronic neuropathic pain.

**5.** The composition for use of any one of claims 1 to 3, wherein said neuropathic pain is acute neuropathic pain.

**6.** The composition for use of claim 3, wherein the second anti-pain agent is an opioid or a gabapentanoid.

**7.** The composition for use of any one of claims 1 to 6, wherein the treatment is by continuous infusion.

**8.** The composition for use of claim 7, wherein the continuous infusion is provided by an implant pump.

**9.** The composition for use of any one of claims 1, and 3 to 8, wherein the peripheral nerve injury is a penetration wound, a burn, frostbite or a fracture.

**10.** The composition for use of any one of claims 1 to 8, wherein the neuropathic pain is the result of disease.

**11.** The composition for use of claim 10, wherein the disease is diabetes, cancer, spinal nerve disease, multiple sclerosis, arthritis, an autoimmune disease, or an infection.

**12.** The composition for use of any one of claims 1 to 8, wherein the neuropathic pain is the result of chemotherapy.

**13.** The composition for use of any one of claims 1 to 8, wherein the neuropathic pain is the result of surgery.

**14.** The composition for use of claim 3, wherein the neuropathic pain is peripheral nerve injury-induced spontaneous pain.

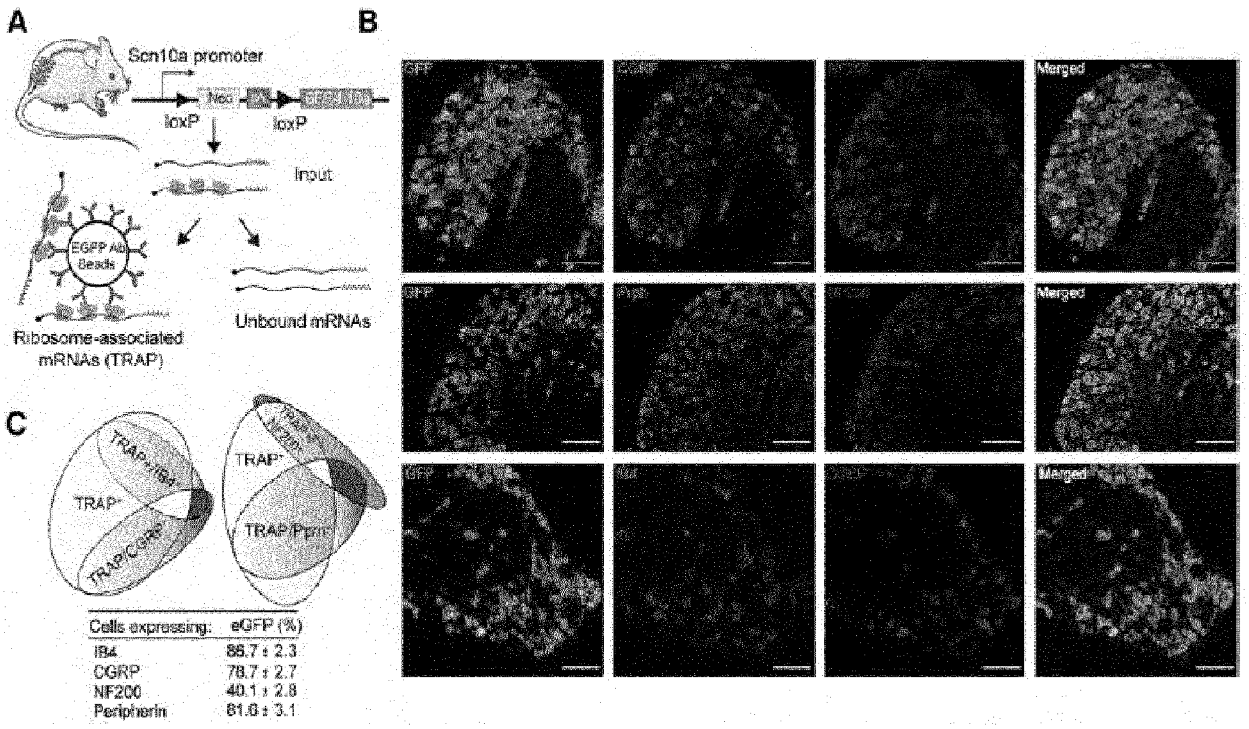
A
Scn10a promoter
Neo
loxP
loxP
Input
EGFP Ab Beads
Ribosome-associated mRNAs (TRAP)
Unbound mRNAs
B
GFP
CGRP
Merged
GFP
Merged
Merged
C
TRAP+/IB4
TRAP+
TRAP/CGRP
TRAP+
TRAP/Ppn
Cells expressing: eGFP (%)
IB4 86.7 ± 2.3
CGRP 78.7 ± 2.7
NF200 40.1 ± 2.8
Peripherin 81.6 ± 3.1

FIGS. 1A-C

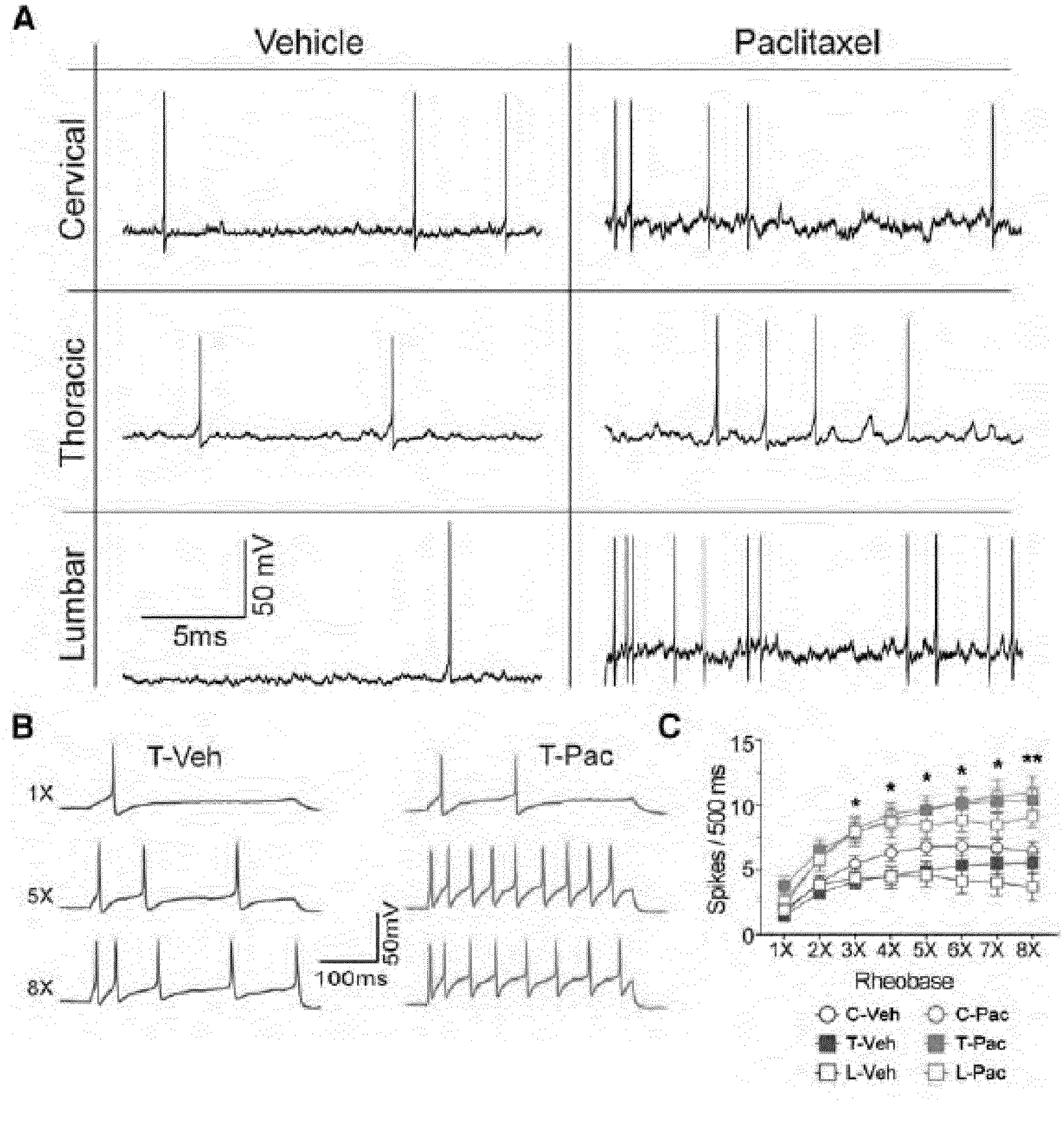
A
Vehicle
Paclitaxel
Cervical
Thoracic
Lumbar
50 mV
5ms
B
T-Veh
T-Pac
1X
5X
8X
50mV
100ms
C
Spikes / 500 ms
Rheobase
1X 2X 3X 4X 5X 6X 7X 8X
C-Veh
C-Pac
T-Veh
T-Pac
L-Veh
L-Pac

FIGS. 2A-C

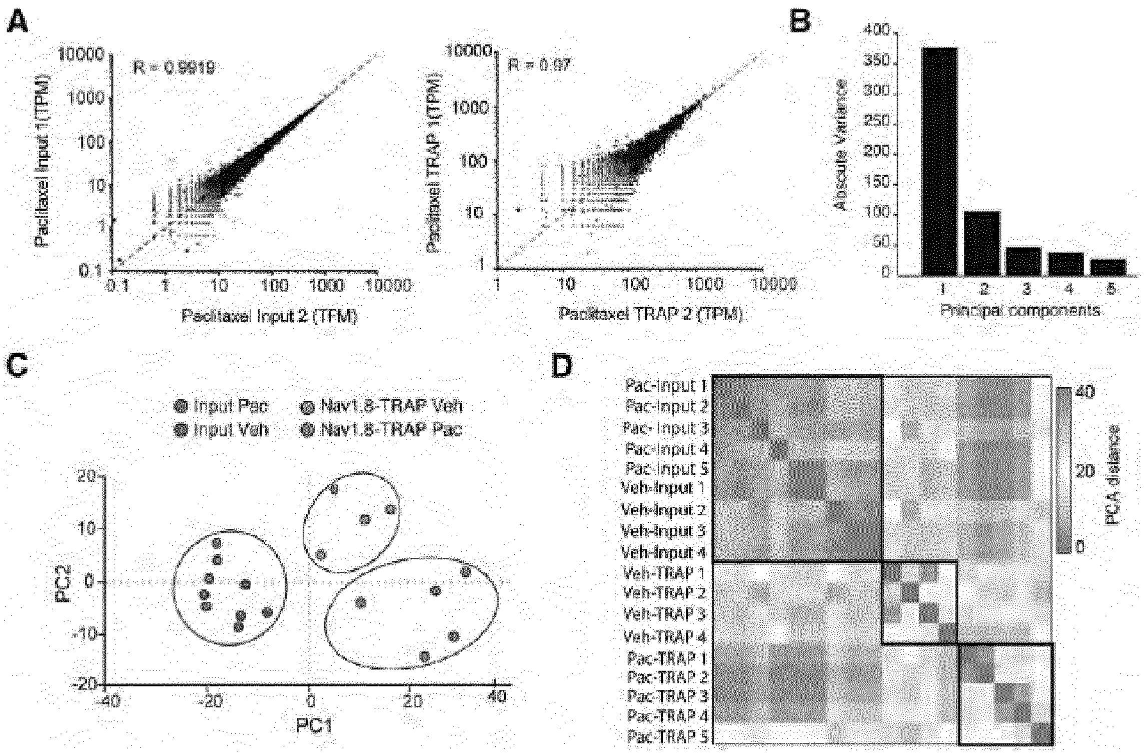
A
R = 0.9919
Paclitaxel Input 1 (TPM)
Paclitaxel Input 2 (TPM)
0.1
1
10
100
1000
10000
R = 0.97
Paclitaxel TRAP 1 (TPM)
Paclitaxel TRAP 2 (TPM)
B
Absolute Variance
Principal components
0
50
100
150
200
250
300
350
400
1
2
3
4
5
C
Input Pac
Input Veh
Nav1.8-TRAP Veh
Nav1.8-TRAP Pac
PC2
PC1
-40
-20
0
20
40
-10
10
D
Pac-Input 1
Pac-Input 2
Pac-Input 3
Pac-Input 4
Pac-Input 5
Veh-Input 1
Veh-Input 2
Veh-Input 3
Veh-Input 4
Veh-TRAP 1
Veh-TRAP 2
Veh-TRAP 3
Veh-TRAP 4
Pac-TRAP 1
Pac-TRAP 2
Pac-TRAP 3
Pac-TRAP 4
Pac-TRAP 5
PCA distance

FIGS. 3A-D

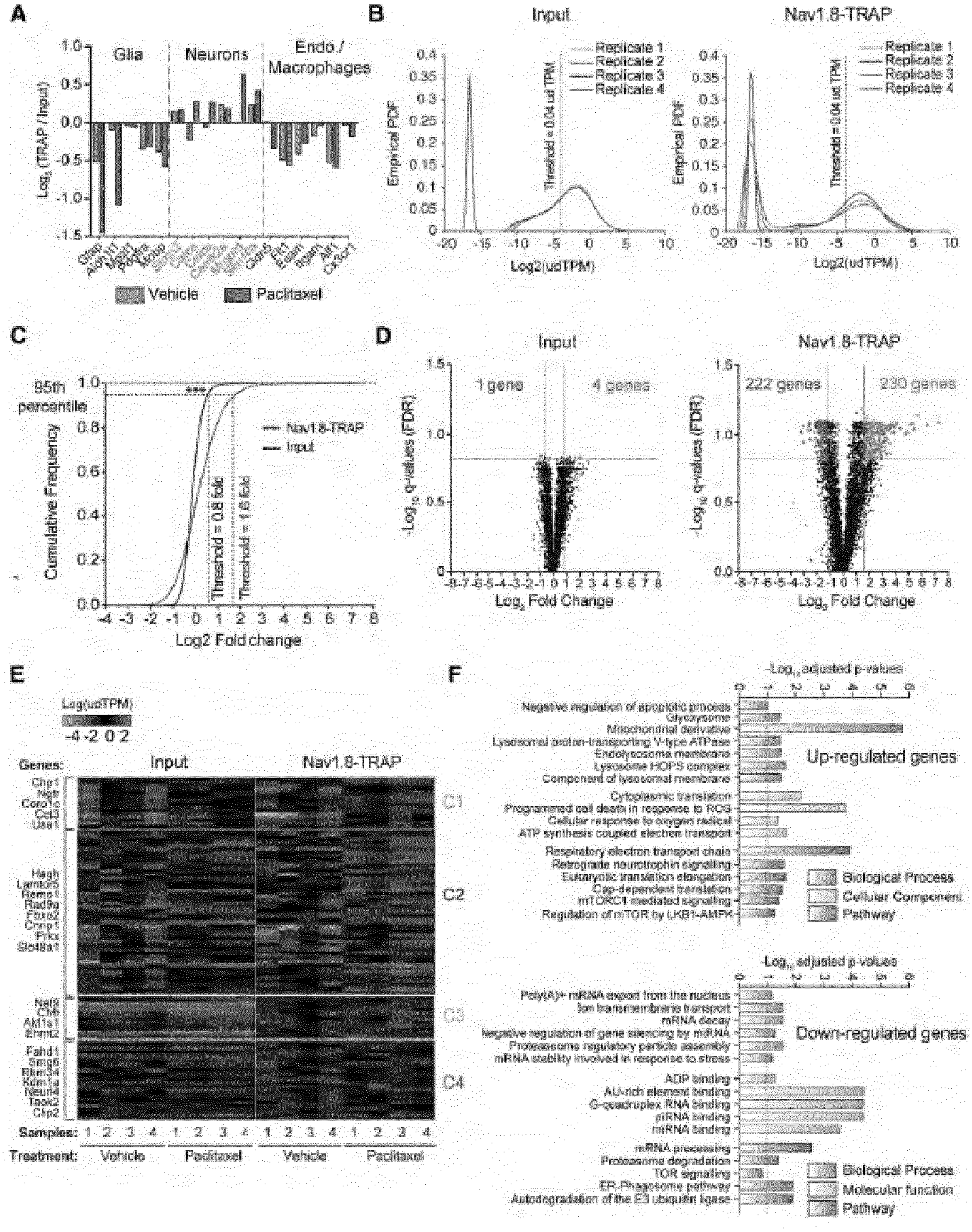
A
Glia
Neurons
Endo./ Macrophages
Log2 (TRAP / Input)
Gfap
Aldh1l1
Mbp
Vehicle
Paclitaxel
B
Input
Nav1.8-TRAP
Replicate 1
Replicate 2
Replicate 3
Replicate 4
Threshold = 0.04 ud TPM
Empirical PDF
Log2(udTPM)
C
95th percentile
***
Nav1.8-TRAP
Input
Threshold = 0.8 fold
Threshold = 1.6 fold
Cumulative Frequency
Log2 Fold change
D
Input
Nav1.8-TRAP
1 gene
4 genes
222 genes
230 genes
-Log10 q-values (FDR)
Log2 Fold Change
E
Log(udTPM)
-4 -2 0 2
Genes:
Input
Nav1.8-TRAP
C1
C2
C3
C4
Samples: 1 2 3 4 1 2 3 4 1 2 3 4 1 2 3 4
Treatment: Vehicle Paclitaxel Vehicle Paclitaxel
F
-Log10 adjusted p-values
Negative regulation of apoptotic process
Glycosome
Mitochondrial derivative
Lysosomal proton-transporting V-type ATPase
Endolysosome membrane
Lysosome HOPS complex
Component of lysosomal membrane
Cytoplasmic translation
Programmed cell death in response to ROS
Cellular response to oxygen radical
ATP synthesis coupled electron transport
Respiratory electron transport chain
Retrograde neurotrophin signalling
Eukaryotic translation elongation
Cap-dependent translation
mTORC1 mediated signalling
Regulation of mTOR by LKB1-AMPK
Up-regulated genes
Biological Process
Cellular Component
Pathway
Poly(A)+ mRNA export from the nucleus
Ion transmembrane transport
mRNA decay
Negative regulation of gene silencing by miRNA
Proteasome regulatory particle assembly
mRNA stability involved in response to stress
ADP binding
AU-rich element binding
G-quadruplex RNA binding
piRNA binding
miRNA binding
mRNA processing
Proteasome degradation
TOR signalling
ER-Phagosome pathway
Autodegradation of the E3 ubiquitin ligase
Down-regulated genes
Biological Process
Molecular function
Pathway

FIGS. 4A-F

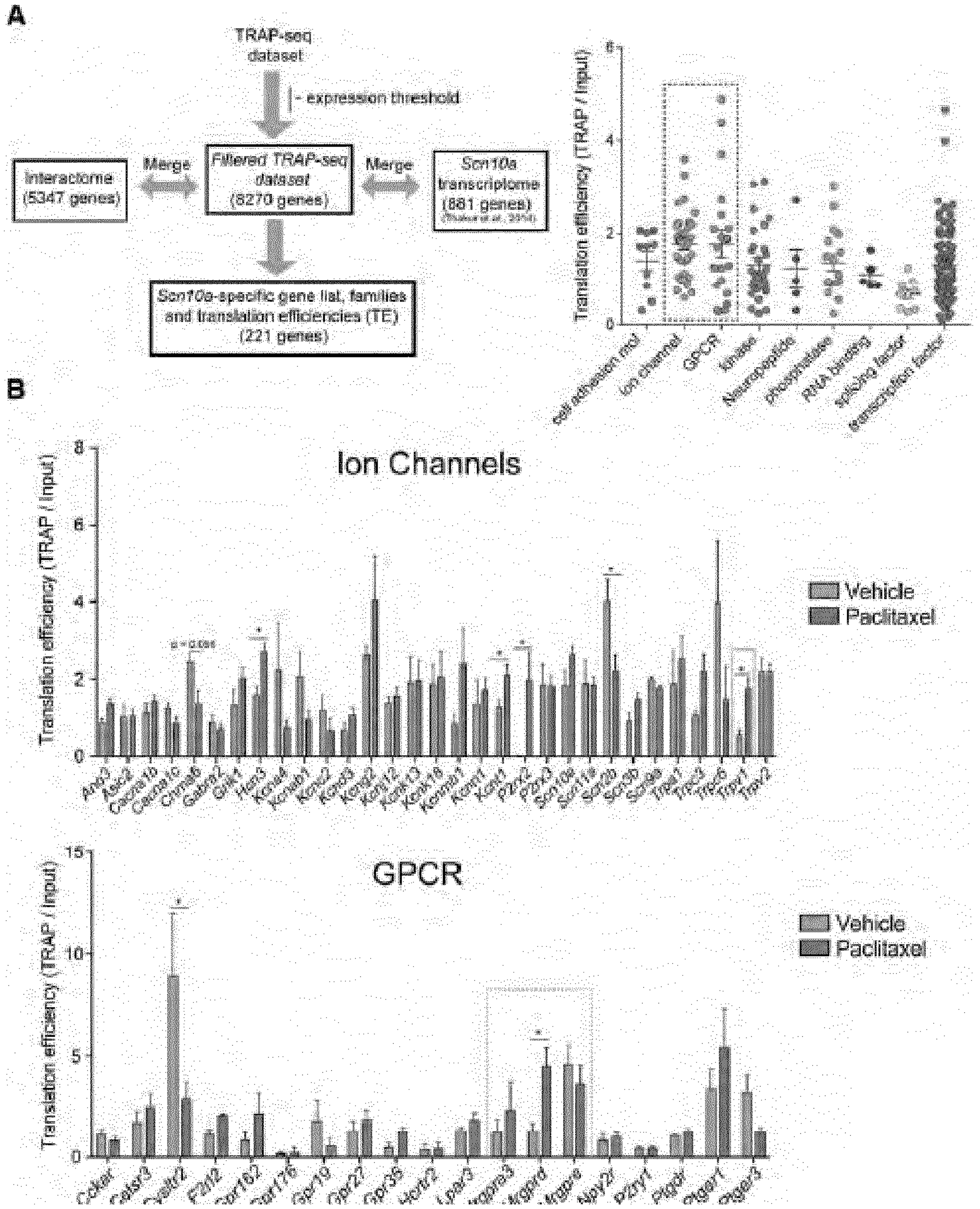
A
TRAP-seq dataset
- expression threshold
Interactome (5347 genes)
Merge
Filtered TRAP-seq dataset (8270 genes)
Merge
Scn10a transcriptome (881 genes)
Scn10a-specific gene list, families and translation efficiencies (TE) (221 genes)
Translation efficiency (TRAP / Input)
cell adhesion mol
ion channel
GPCR
kinase
Neuropeptide
phosphatase
RNA binding
splicing factor
transcription factor
B
Ion Channels
Vehicle
Paclitaxel
GPCR
Vehicle
Paclitaxel

FIGS. 5A-B

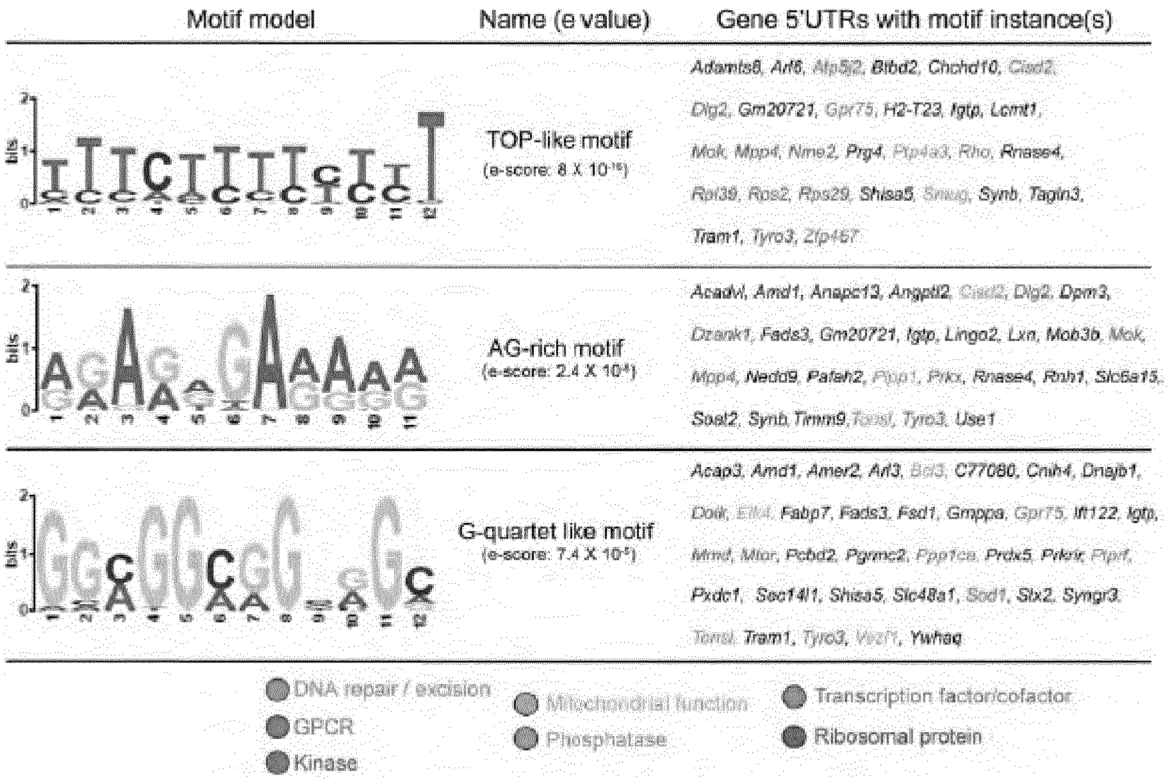


FIG. 6

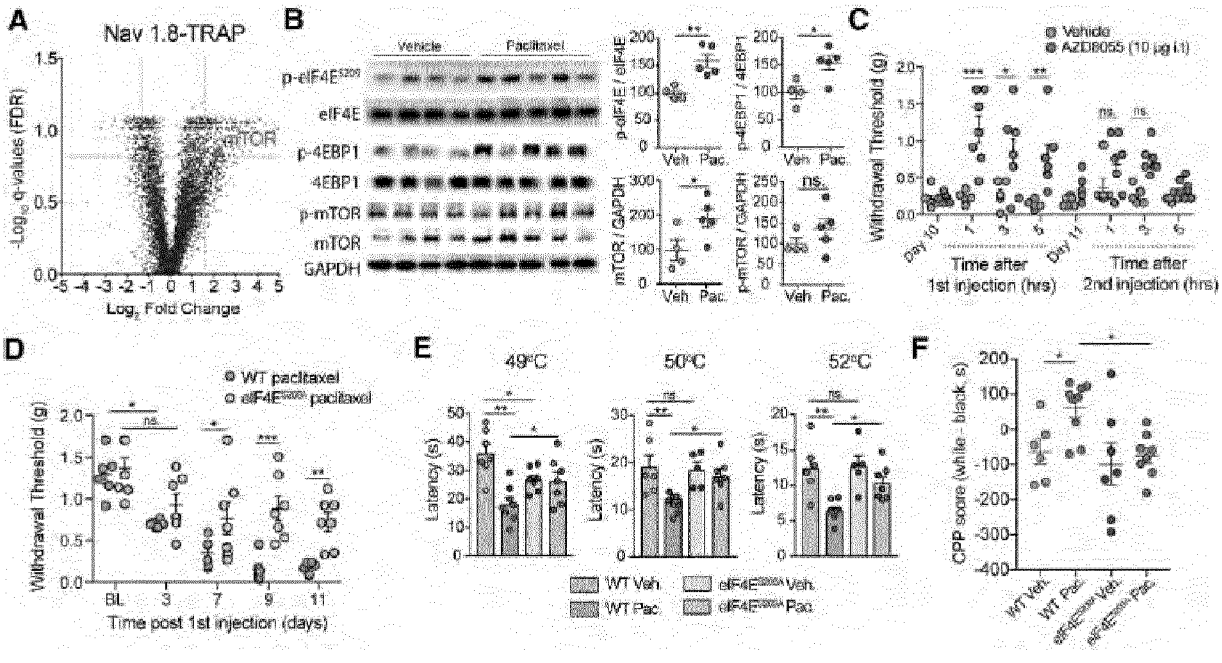


FIGS. 7A-F

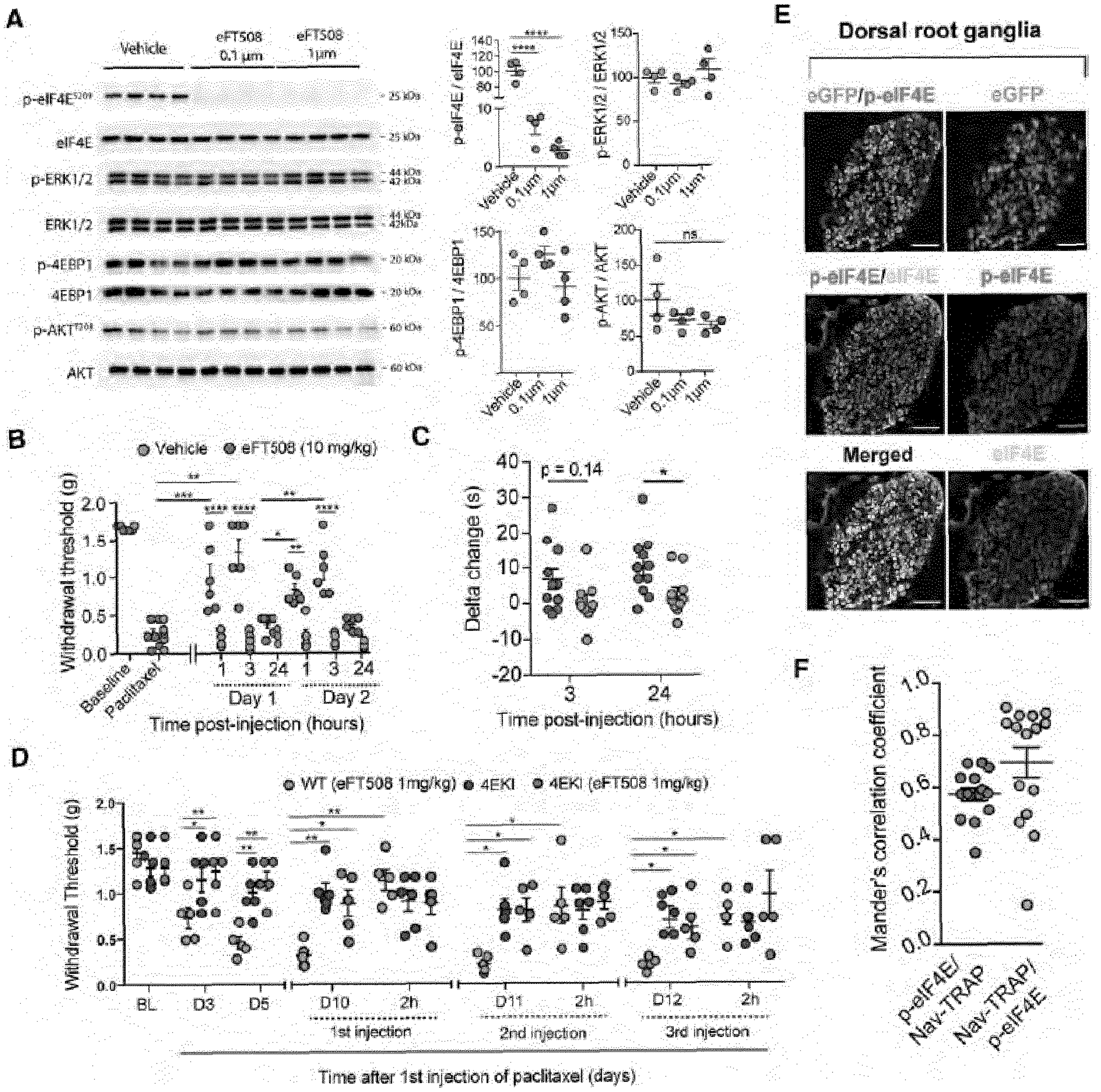
A
Vehicle
eFT508 0.1 μm
eFT508 1μm
p-eIF4E
eIF4E
p-ERK1/2
ERK1/2
p-4EBP1
4EBP1
p-AKT
AKT
B
Vehicle
eFT508 (10 mg/kg)
Withdrawal threshold (g)
Baseline
Paclitaxel
Day 1
Day 2
Time post-injection (hours)
C
p = 0.14
Delta change (s)
Time post-injection (hours)
D
WT (eFT508 1mg/kg)
4EKI
4EKI (eFT508 1mg/kg)
Withdrawal Threshold (g)
BL
D3
D5
D10
D11
D12
2h
1st injection
2nd injection
3rd injection
Time after 1st injection of paclitaxel (days)
E
Dorsal root ganglia
eGFP/p-eIF4E
eGFP
p-eIF4E/eIF4E
p-eIF4E
Merged
eIF4E
F
Mander's correlation coefficient
p-eIF4E/ Nav-TRAP
Nav-TRAP/ p-eIF4E

FIGS. 8A-F

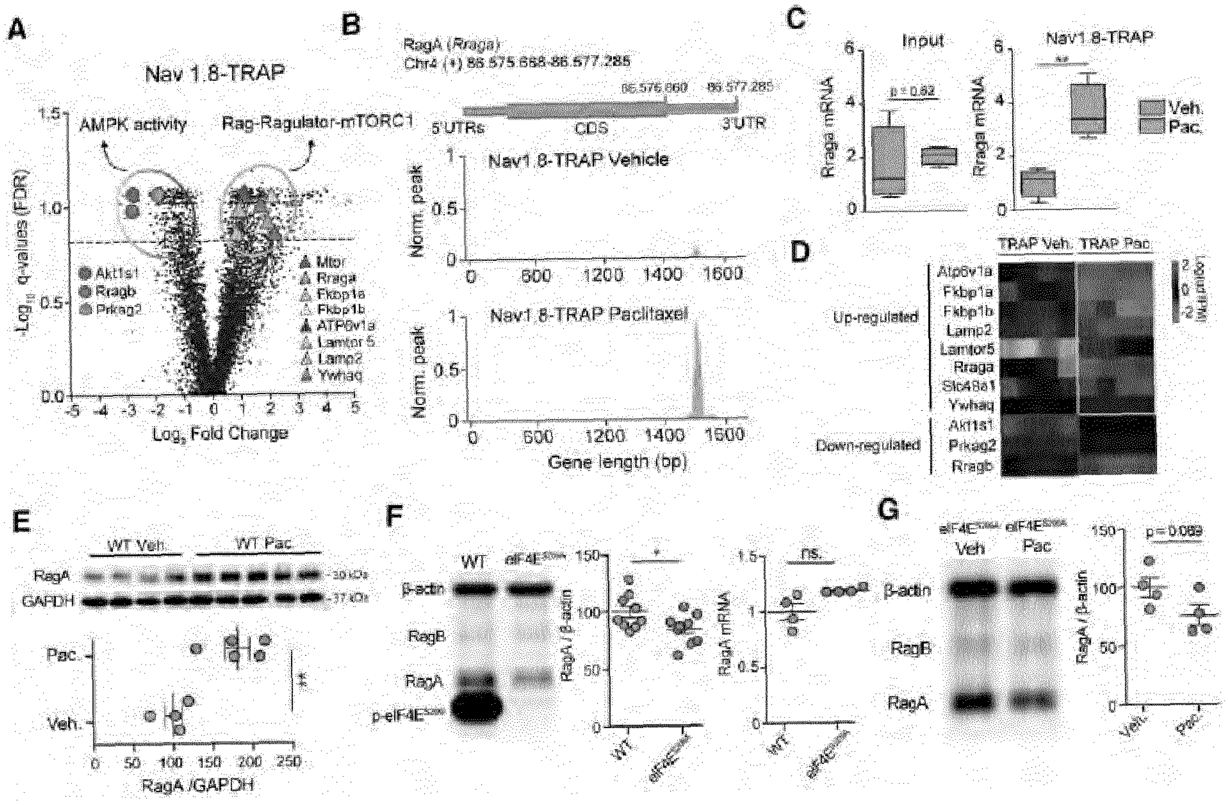
A
Nav 1.8-TRAP
AMPK activity
Rag-Ragulator-mTORC1
-Log10 q-values (FDR)
Log2 Fold Change
Akt1s1
Rragb
Prkag2
Mtor
Rraga
Fkbp1a
Fkbp1b
ATP6v1a
Lamtor 5
Lamp2
Ywhaq
B
RagA (Rraga)
Chr4 (+) 86.575.668-86.577.285
5'UTRs
CDS
3'UTR
Nav1.8-TRAP Vehicle
Nav1.8-TRAP Paclitaxel
Norm. peak
Gene length (bp)
C
Input
Nav1.8-TRAP
Rraga mRNA
p = 0.82
Veh.
Pac.
D
TRAP Veh.
TRAP Pac
Up-regulated
Down-regulated
Atp6v1a
Fkbp1a
Fkbp1b
Lamp2
Lamtor5
Rraga
Slc48a1
Ywhaq
Akt1s1
Prkag2
Rragb
E
WT Veh.
WT Pac.
RagA
GAPDH
Pac.
Veh.
RagA /GAPDH
F
WT
β-actin
RagB
RagA
RagA / β-actin
RagA mRNA
ns.
G
Veh
Pac
β-actin
RagB
RagA
p = 0.089
RagA / β-actin

FIGS. 9A-G

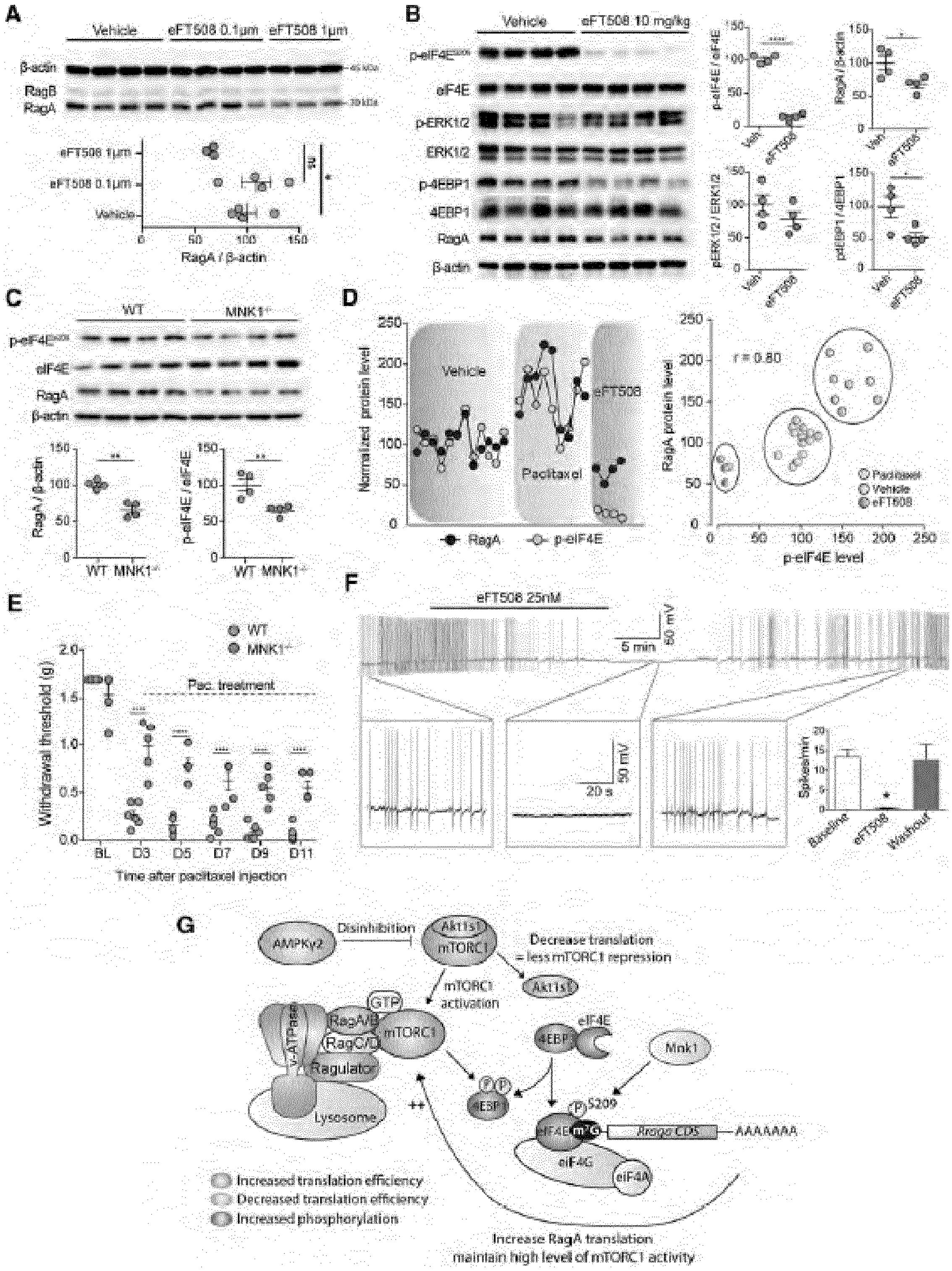
A
Vehicle
eFT508 0.1μm
eFT508 1μm
β-actin
RagB
RagA
RagA / β-actin
B
Vehicle
eFT508 10 mg/kg
p-eIF4E
eIF4E
p-ERK1/2
ERK1/2
p-4EBP1
4EBP1
RagA
β-actin
Veh
eFT508
C
WT
MNK1
p-eIF4E
eIF4E
RagA
β-actin
D
Vehicle
Paclitaxel
eFT508
Normalized protein level
RagA
p-eIF4E
r = 0.80
RagA protein level
p-eIF4E level
E
WT
MNK1
Pac. treatment
Withdrawal threshold (g)
BL
D3
D5
D7
D9
D11
Time after paclitaxel injection
F
eFT508 25nM
5 min
50 mV
20 s
Spikes/min
Baseline
eFT508
Washout
G
AMPKγ2
Disinhibition
Akt1s1
mTORC1
Decrease translation
= less mTORC1 repression
mTORC1 activation
GTP
RagA/B
RagC/D
v-ATPase
Ragulator
Lysosome
4EBP1
eIF4E
Mnk1
S209
eIF4G
eIF4A
AAAAAAA
Increased translation efficiency
Decreased translation efficiency
Increased phosphorylation
Increase RagA translation
maintain high level of mTORC1 activity

FIGS. 10A-G

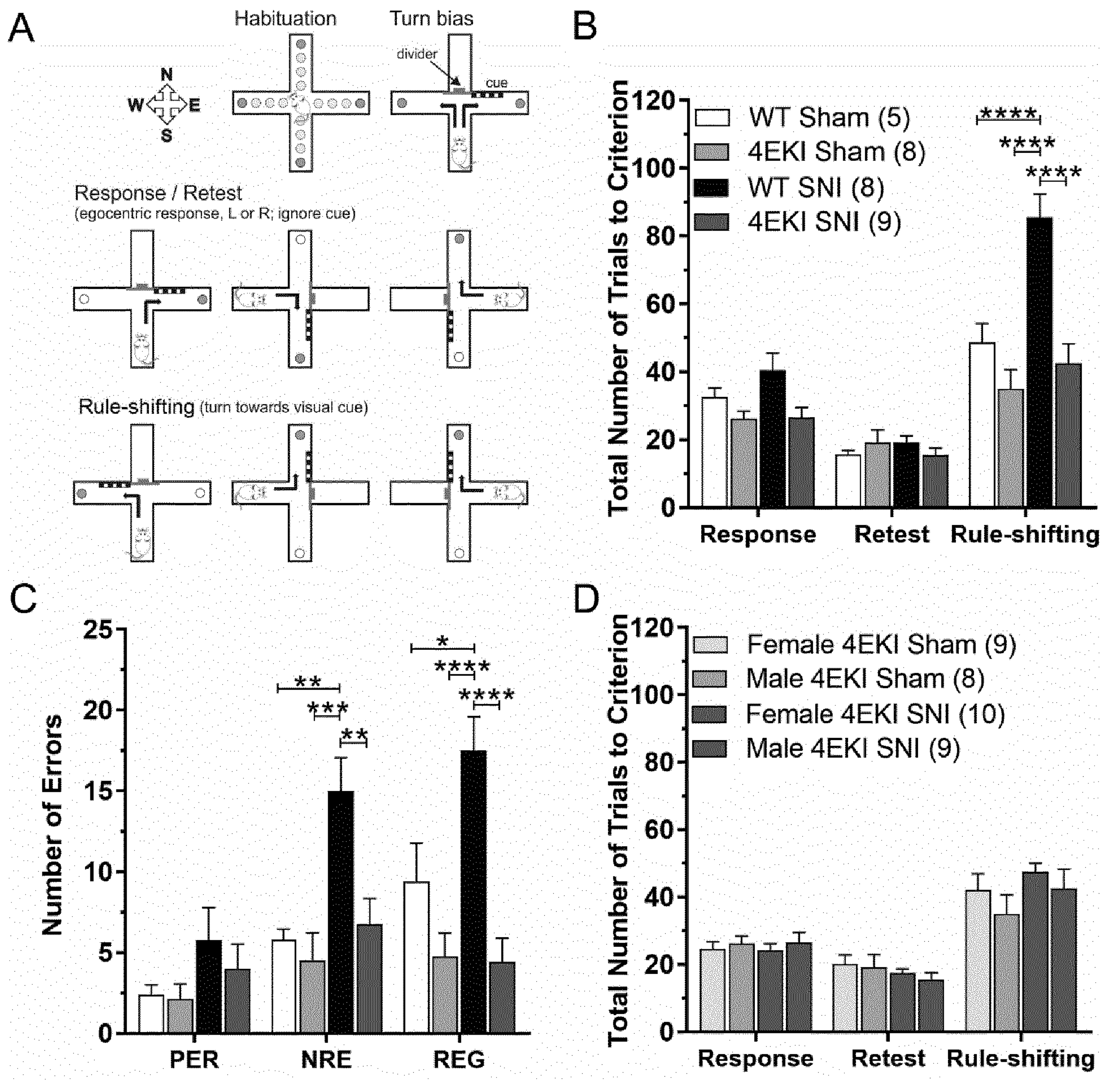
A
Habituation
Turn bias
divider
cue
N
W
E
S
Response / Retest
(egocentric response, L or R; ignore cue)
Rule-shifting (turn towards visual cue)
B
Total Number of Trials to Criterion
WT Sham (5)
4EKI Sham (8)
WT SNI (8)
4EKI SNI (9)
Response
Retest
Rule-shifting
C
Number of Errors
PER
NRE
REG
D
Total Number of Trials to Criterion
Female 4EKI Sham (9)
Male 4EKI Sham (8)
Female 4EKI SNI (10)
Male 4EKI SNI (9)
Response
Retest
Rule-shifting

FIGS. 11A-D

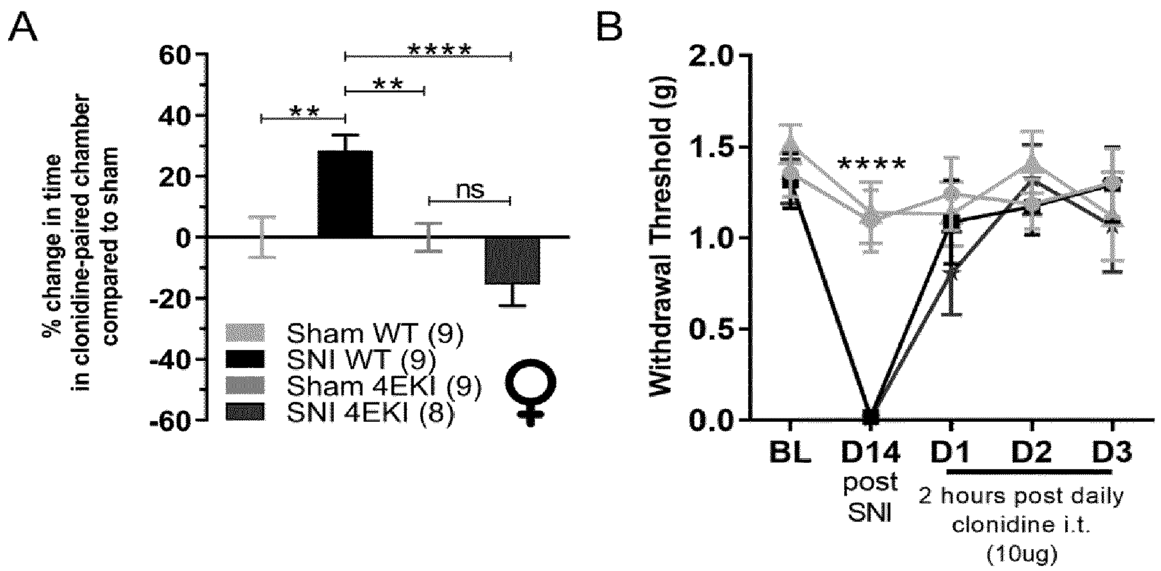


FIGS. 12A-B

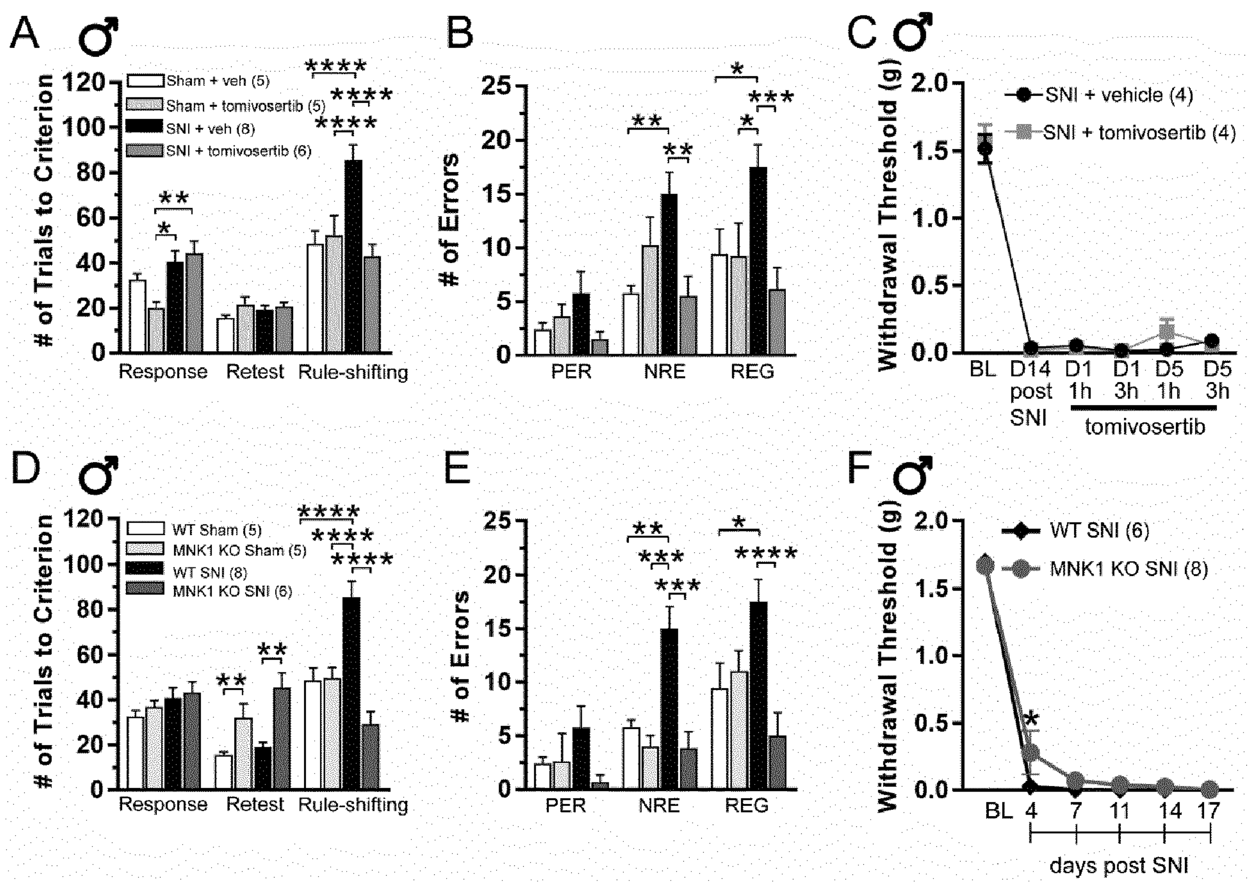


FIGS. 13A-F

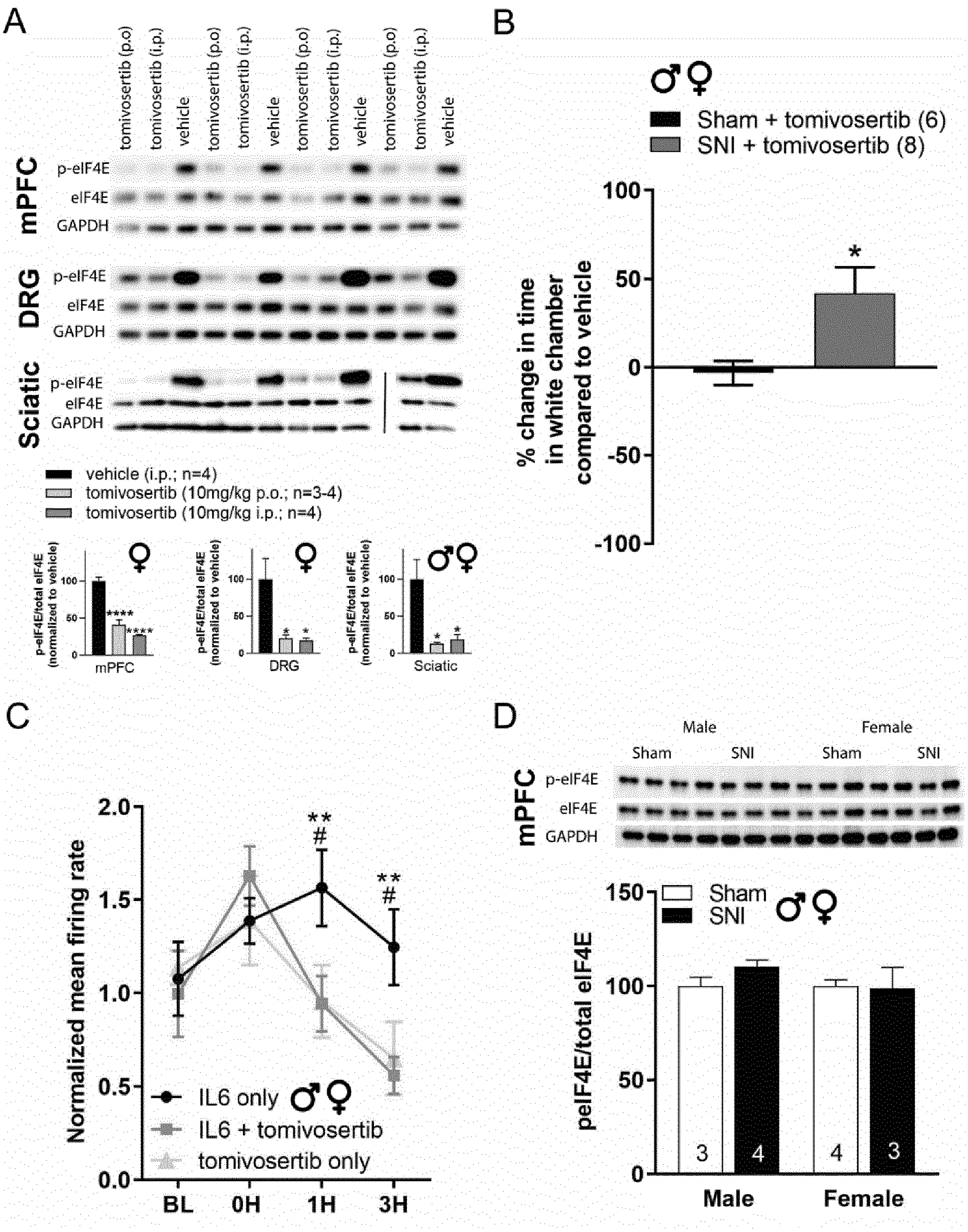
A
tomivosertib (p.o)
tomivosertib (i.p.)
vehicle
mPFC
DRG
Sciatic
p-eIF4E
eIF4E
GAPDH
vehicle (i.p.; n=4)
tomivosertib (10mg/kg p.o.; n=3-4)
tomivosertib (10mg/kg i.p.; n=4)
p-eIF4E/total eIF4E (normalized to vehicle)
****
*
B
♂♀
Sham + tomivosertib (6)
SNI + tomivosertib (8)
% change in time in white chamber compared to vehicle
100
50
0
-50
-100
C
Normalized mean firing rate
2.0
1.5
1.0
0.5
0.0
BL
0H
1H
3H
**
#
IL6 only
IL6 + tomivosertib
tomivosertib only
D
Male
Female
Sham
SNI
peIF4E/total eIF4E
150
3
4

FIGS. 14A-D

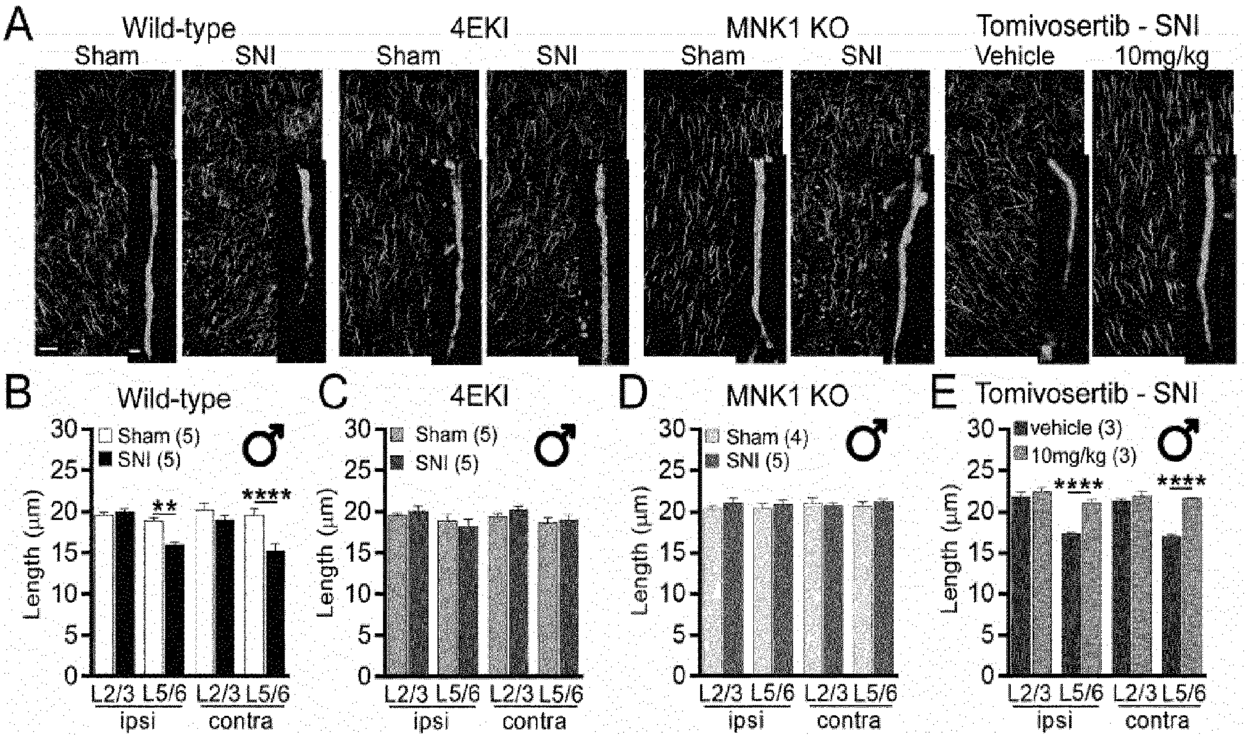
A
Wild-type
Sham
SNI
4EKI
Sham
SNI
MNK1 KO
Sham
SNI
Tomivosertib - SNI
Vehicle
10mg/kg
B
Wild-type
Sham (5)
SNI (5)
Length (μm)
L2/3 L5/6 L2/3 L5/6
ipsi
contra
C
4EKI
Sham (5)
SNI (5)
D
MNK1 KO
Sham (4)
SNI (5)
E
Tomivosertib - SNI
vehicle (3)
10mg/kg (3)

FIGS. 15A-E

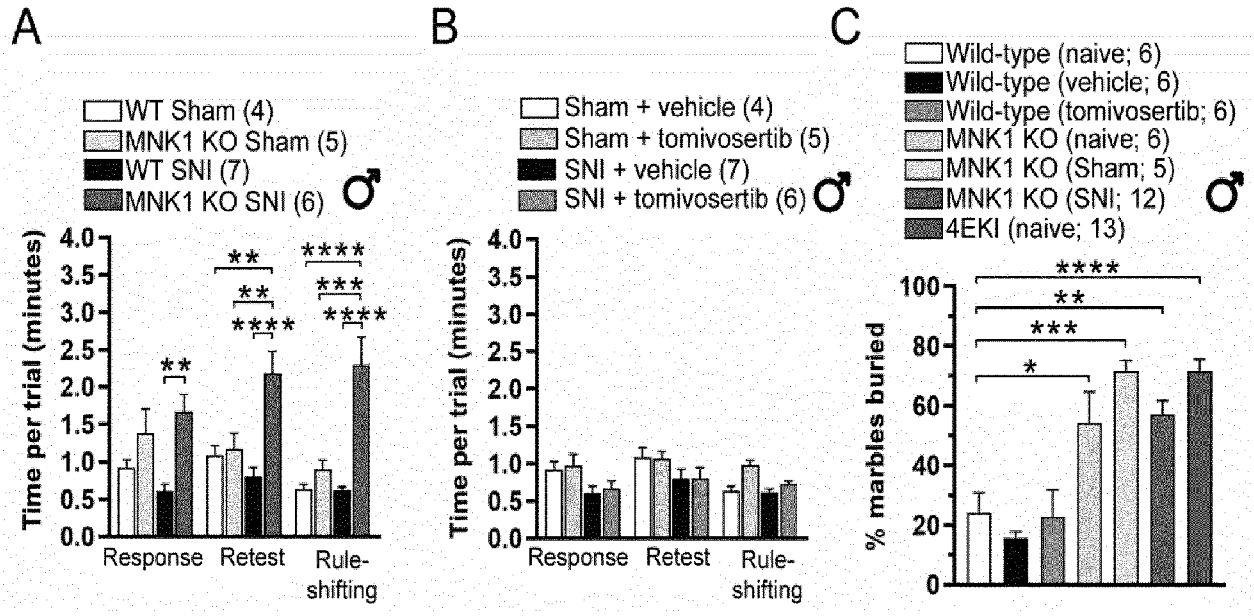
A
WT Sham (4)
MNK1 KO Sham (5)
WT SNI (7)
MNK1 KO SNI (6)
Time per trial (minutes)
4.0
3.5
3.0
2.5
2.0
1.5
1.0
0.5
0.0
**
**
**
****
****
***
****
Response
Retest
Rule-shifting
B
Sham + vehicle (4)
Sham + tomivosertib (5)
SNI + vehicle (7)
SNI + tomivosertib (6)
Time per trial (minutes)
Response
Retest
Rule-shifting
C
Wild-type (naive; 6)
Wild-type (vehicle; 6)
Wild-type (tomivosertib; 6)
MNK1 KO (naive; 6)
MNK1 KO (Sham; 5)
MNK1 KO (SNI; 12)
4EKI (naive; 13)
% marbles buried
100
80
60
40
20
0
****
**
***
*

FIGS. 16A-C

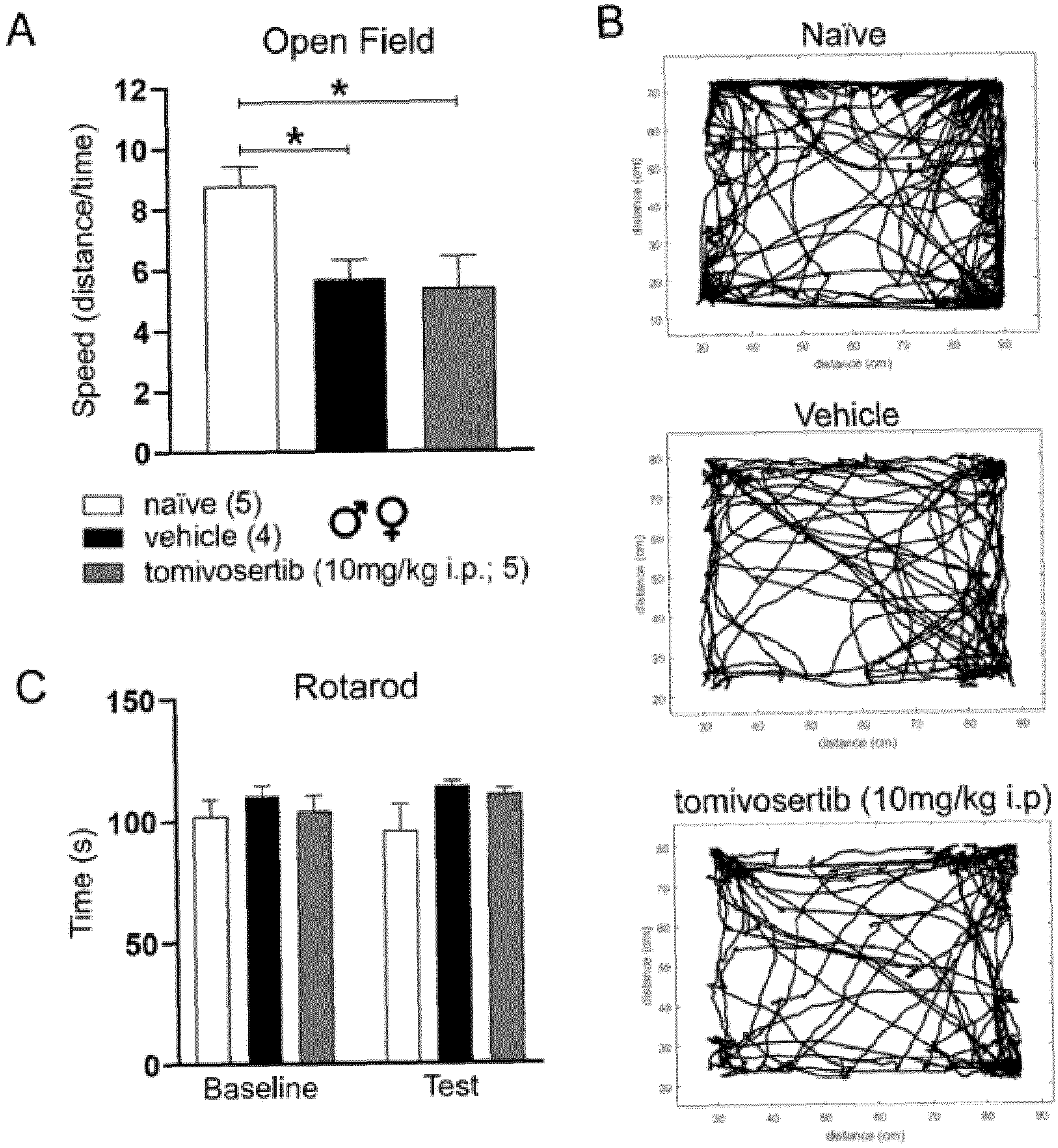
A
Open Field
Speed (distance/time)
naïve (5)
vehicle (4)
tomivosertib (10mg/kg i.p.; 5)
B
Naïve
Vehicle
tomivosertib (10mg/kg i.p)
C
Rotarod
Time (s)
Baseline
Test

FIGS. 17A-C

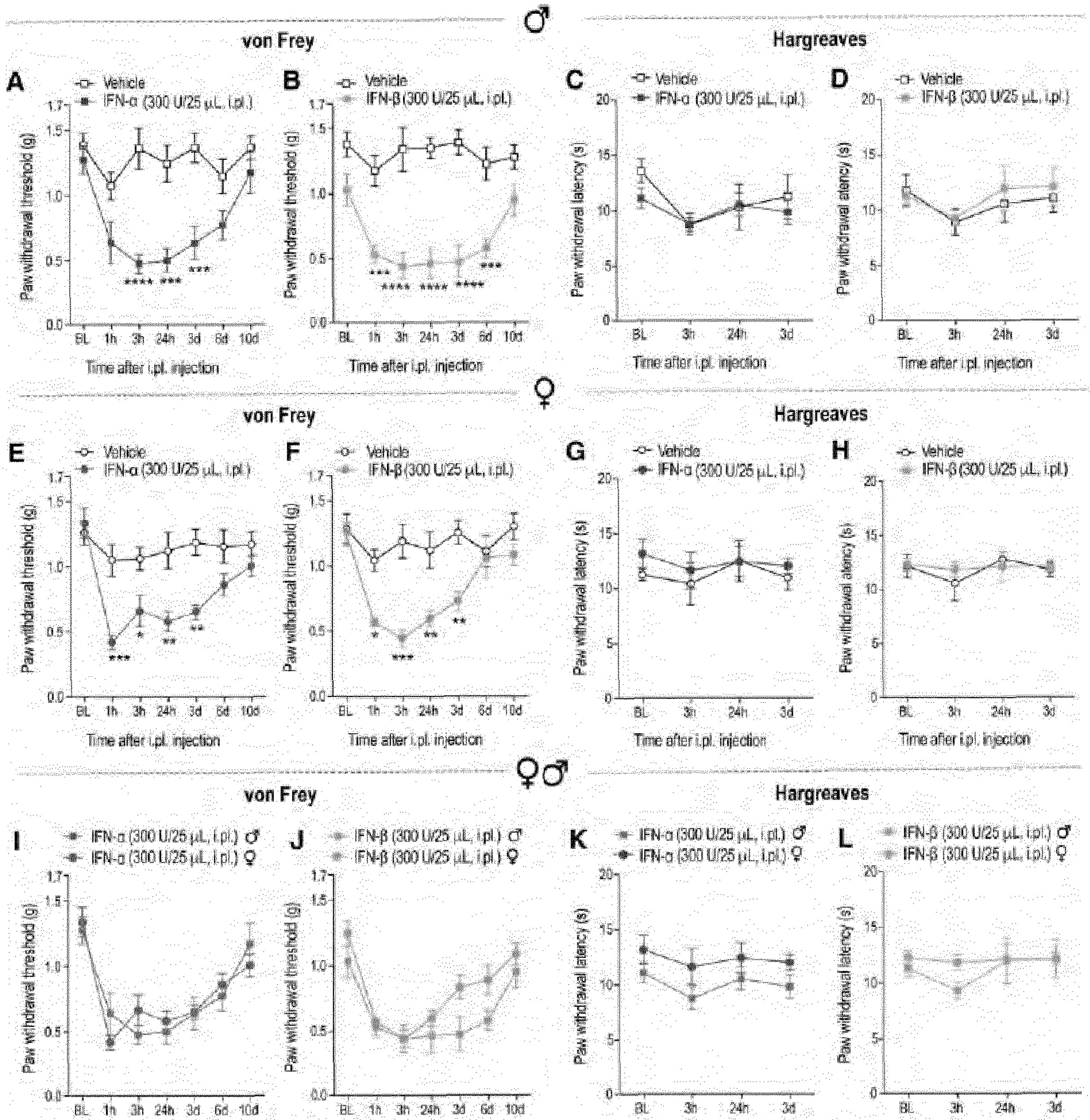

♂
von Frey
Hargreaves
A
B
C
D
Vehicle
IFN-α (300 U/25 μL, i.pl.)
IFN-β (300 U/25 μL, i.pl.)
Paw withdrawal threshold (g)
Paw withdrawal latency (s)
Time after i.pl. injection
BL 1h 3h 24h 3d 6d 10d
BL 3h 24h 3d
♀
von Frey
Hargreaves
E
F
G
H
♀♂
von Frey
Hargreaves
I
J
K
L
IFN-α (300 U/25 μL, i.pl.) ♂
IFN-α (300 U/25 μL, i.pl.) ♀
IFN-β (300 U/25 μL, i.pl.) ♂
IFN-β (300 U/25 μL, i.pl.) ♀


FIGS. 18A-L

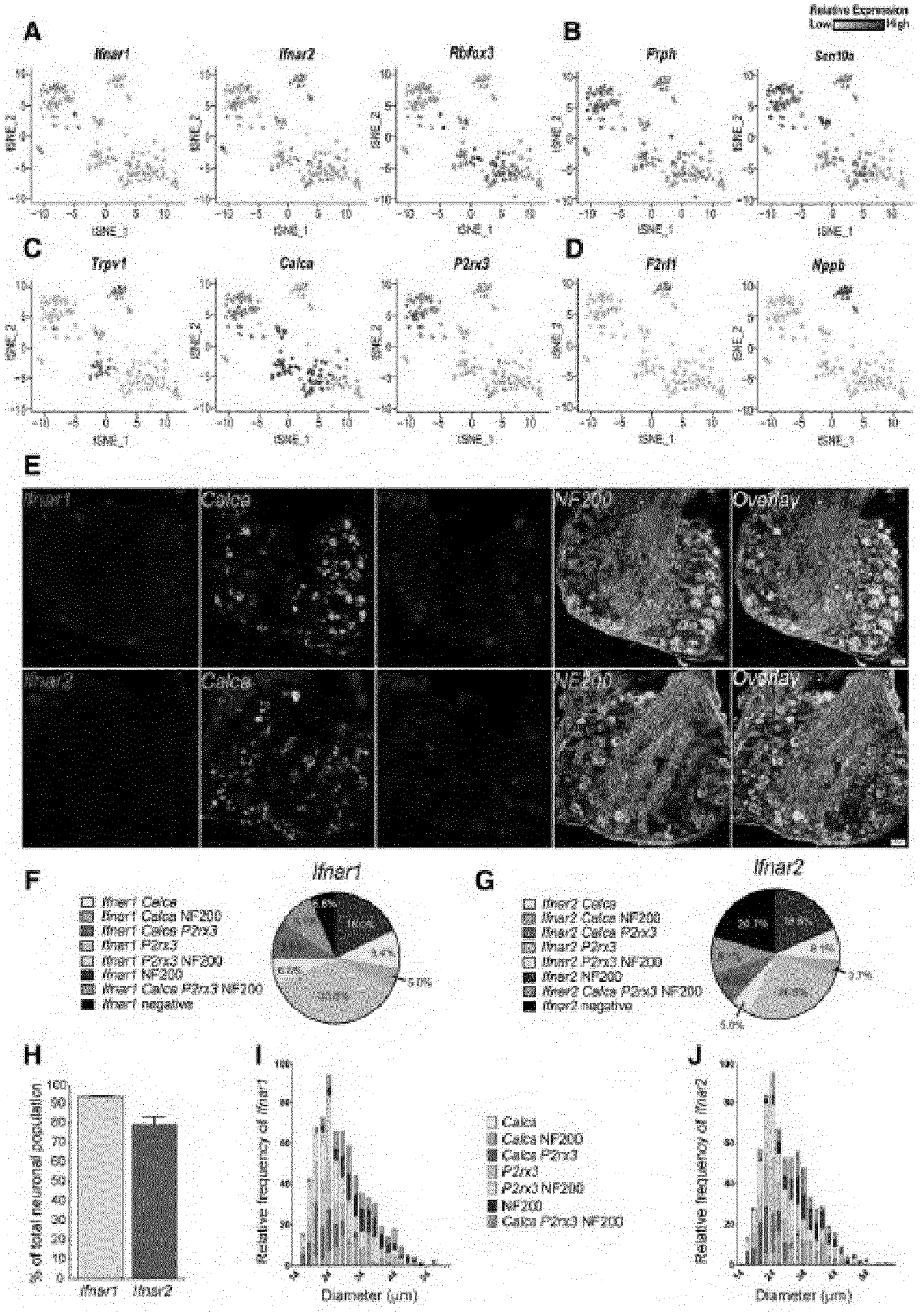
Relative Expression
Low High
A
Ifnar1
Ifnar2
Rbfox3
B
Prph
Scn10a
C
Trpv1
Calca
P2rx3
D
F2rl1
Nppb
tSNE_1
tSNE_2
E
Ifnar1
Calca
P2rx3
NF200
Overlay
Ifnar2
Calca
P2rx3
NF200
Overlay
F
Ifnar1
Ifnar1 Calca
Ifnar1 Calca NF200
Ifnar1 Calca P2rx3
Ifnar1 P2rx3
Ifnar1 P2rx3 NF200
Ifnar1 NF200
Ifnar1 Calca P2rx3 NF200
Ifnar1 negative
G
Ifnar2
Ifnar2 Calca
Ifnar2 Calca NF200
Ifnar2 Calca P2rx3
Ifnar2 P2rx3
Ifnar2 P2rx3 NF200
Ifnar2 NF200
Ifnar2 Calca P2rx3 NF200
Ifnar2 negative
H
% of total neuronal population
Ifnar1
Ifnar2
I
Relative frequency of Ifnar1
Diameter (µm)
Calca
Calca NF200
Calca P2rx3
P2rx3
P2rx3 NF200
NF200
Calca P2rx3 NF200
J
Relative frequency of Ifnar2
Diameter (µm)

FIGS. 19A-J

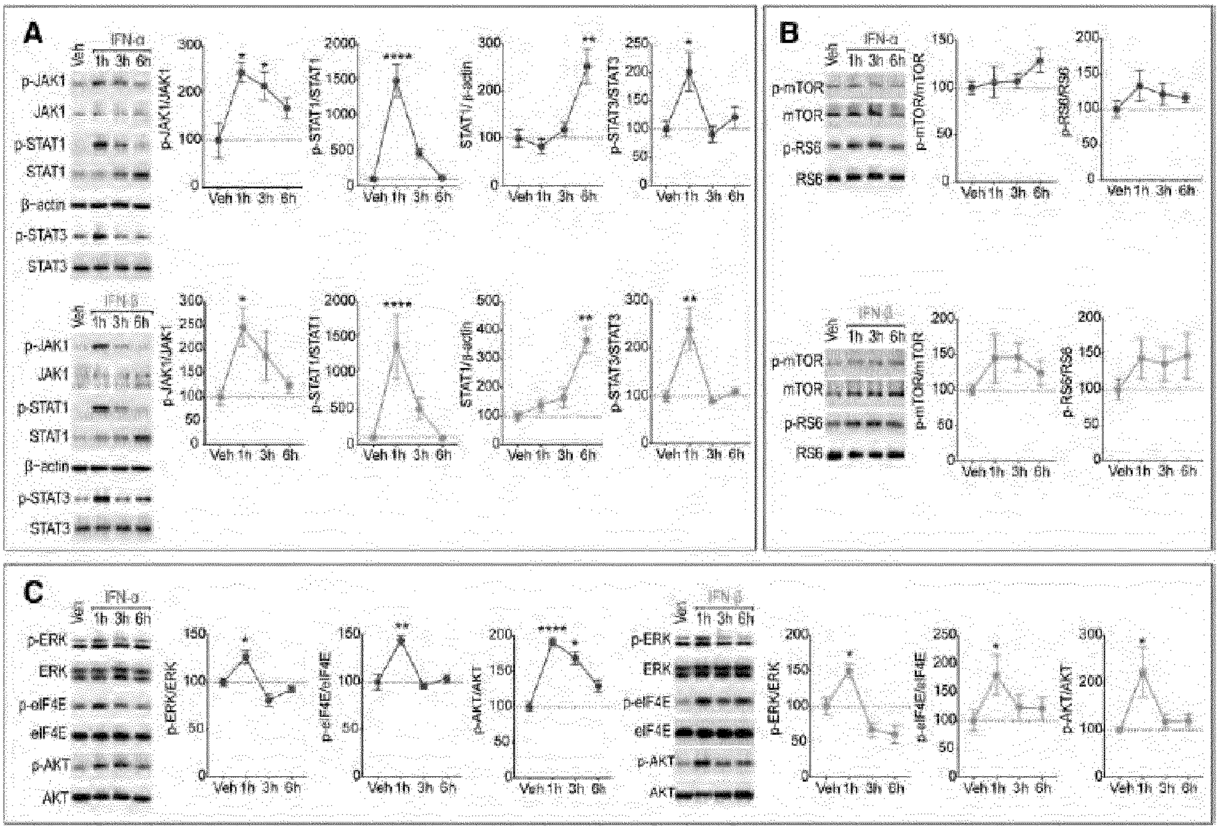
A
Veh
IFN-α
1h 3h 6h
p-JAK1
JAK1
p-STAT1
STAT1
β-actin
p-STAT3
STAT3
p-JAK1/JAK1
p-STAT1/STAT1
STAT1/β-actin
p-STAT3/STAT3
Veh 1h 3h 6h
IFN-β
B
p-mTOR
mTOR
p-RS6
RS6
p-mTOR/mTOR
p-RS6/RS6
C
p-ERK
ERK
p-eIF4E
eIF4E
p-AKT
AKT
p-ERK/ERK
p-eIF4E/eIF4E
p-AKT/AKT

FIGS. 20A-C

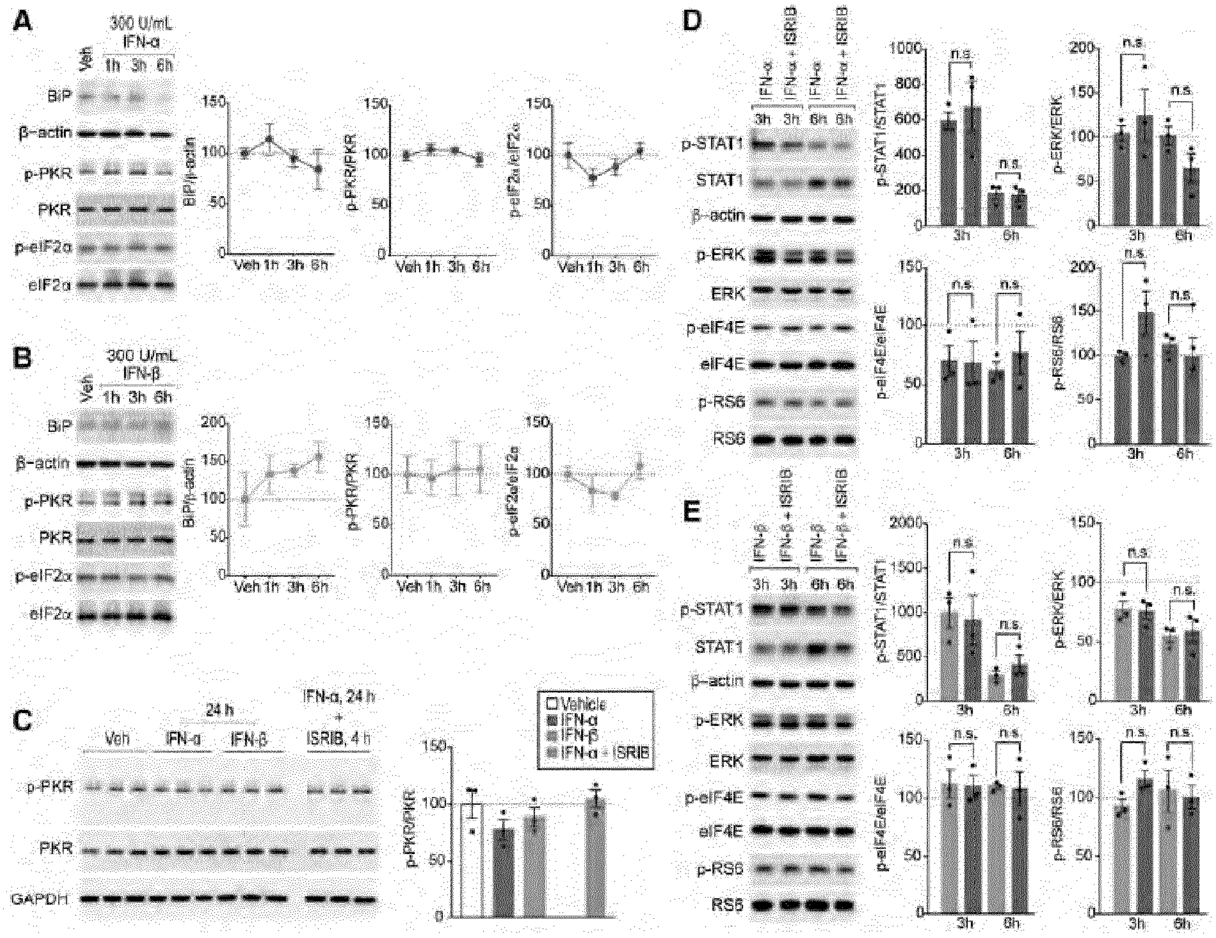
A
300 U/mL IFN-α
Veh 1h 3h 6h
BiP
β-actin
p-PKR
PKR
p-eIF2α
eIF2α
BiP/β-actin
p-PKR/PKR
p-eIF2α/eIF2α
B
300 U/mL IFN-β
C
24 h
Veh IFN-α IFN-β
IFN-α, 24 h + ISRIB, 4 h
GAPDH
Vehicle
IFN-α
IFN-β
IFN-α + ISRIB
D
IFN-α
IFN-α + ISRIB
p-STAT1
STAT1
p-ERK
ERK
p-eIF4E
eIF4E
p-RS6
RS6
p-STAT1/STAT1
p-ERK/ERK
p-eIF4E/eIF4E
p-RS6/RS6
n.s.
E
IFN-β
IFN-β + ISRIB

FIGS. 21A-E

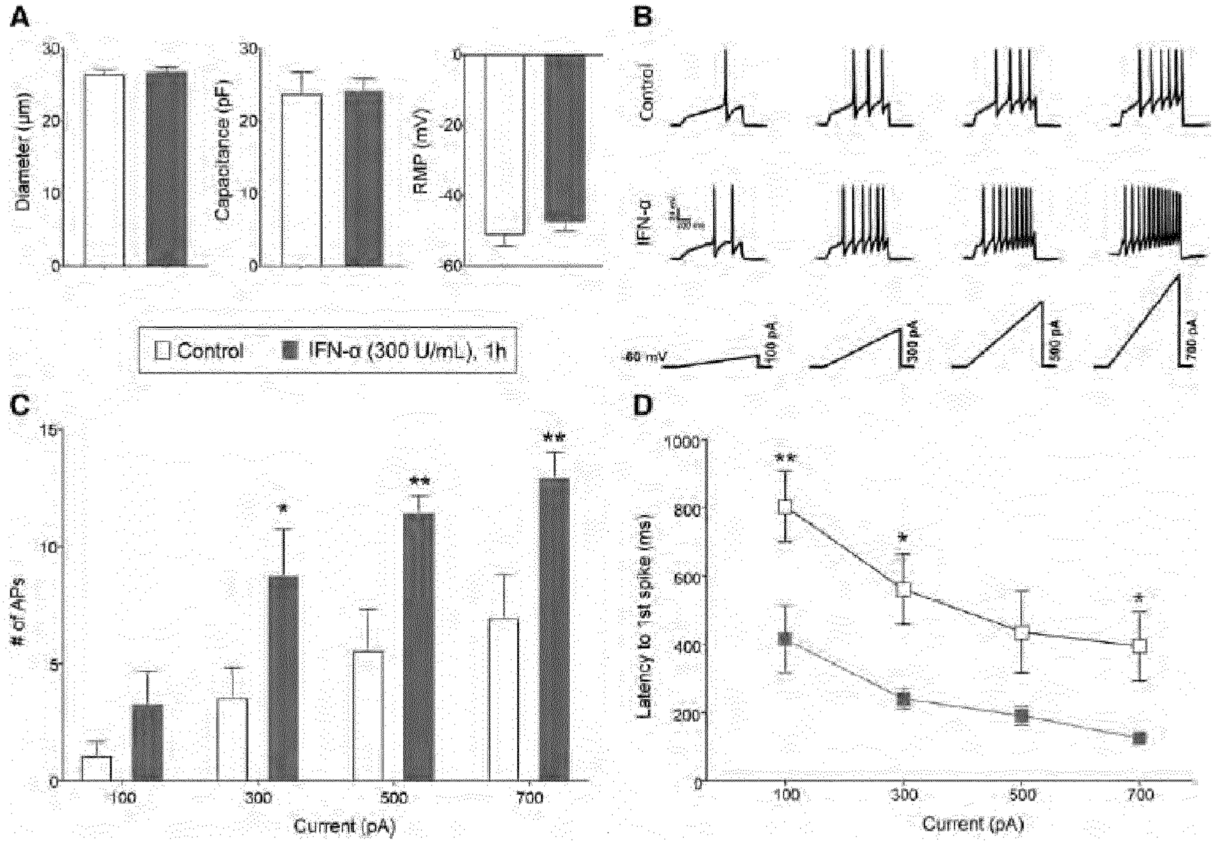
A
Diameter (μm)
Capacitance (pF)
RMP (mV)
B
Control
IFN-α
-60 mV
100 pA
300 pA
500 pA
700 pA
□ Control
IFN-α (300 U/mL), 1h
C
# of APs
Current (pA)
D
Latency to 1st spike (ms)
Current (pA)


FIGS. 22A-D

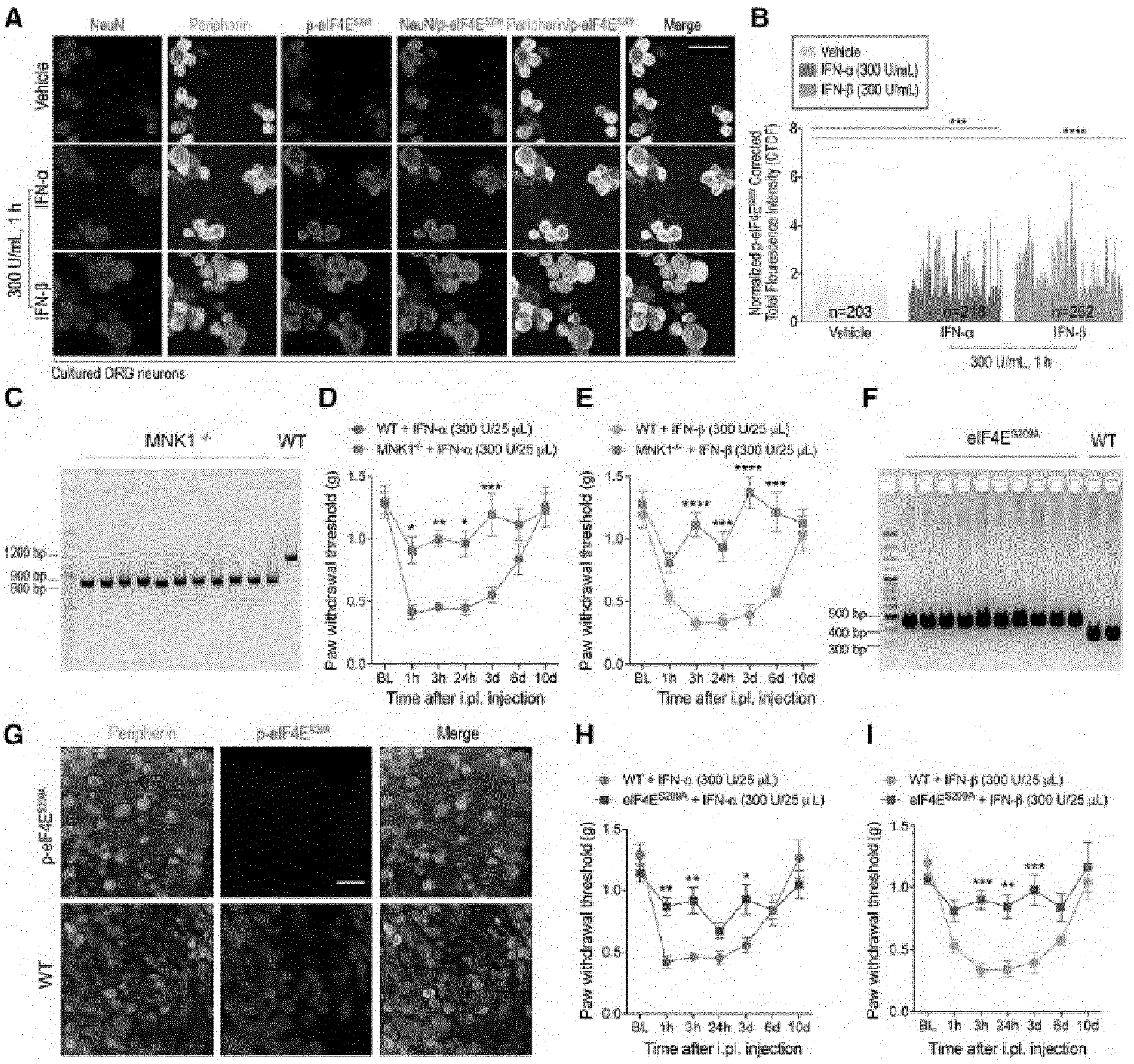
A
NeuN
Peripherin
p-eIF4E S209
Merge
Vehicle
IFN-α
IFN-β
300 U/mL, 1 h
Cultured DRG neurons
B
Vehicle
IFN-α (300 U/mL)
IFN-β (300 U/mL)
Normalized p-eIF4E S209 Corrected Total Flourescence Intensity (CTCF)
n=203
n=218
n=252
300 U/mL, 1 h
C
MNK1 -/-
WT
1200 bp
900 bp
800 bp
D
WT + IFN-α (300 U/25 μL)
MNK1-/- + IFN-α (300 U/25 μL)
Paw withdrawal threshold (g)
Time after i.pl. injection
E
WT + IFN-β (300 U/25 μL)
MNK1-/- + IFN-β (300 U/25 μL)
F
eIF4E S209A
WT
500 bp
400 bp
300 bp
G
p-eIF4E S209A
H
WT + IFN-α (300 U/25 μL)
eIF4E S209A + IFN-α (300 U/25 μL)
I
WT + IFN-β (300 U/25 μL)
eIF4E S209A + IFN-β (300 U/25 μL)
BL 1h 3h 24h 3d 6d 10d

FIGS. 23A-I

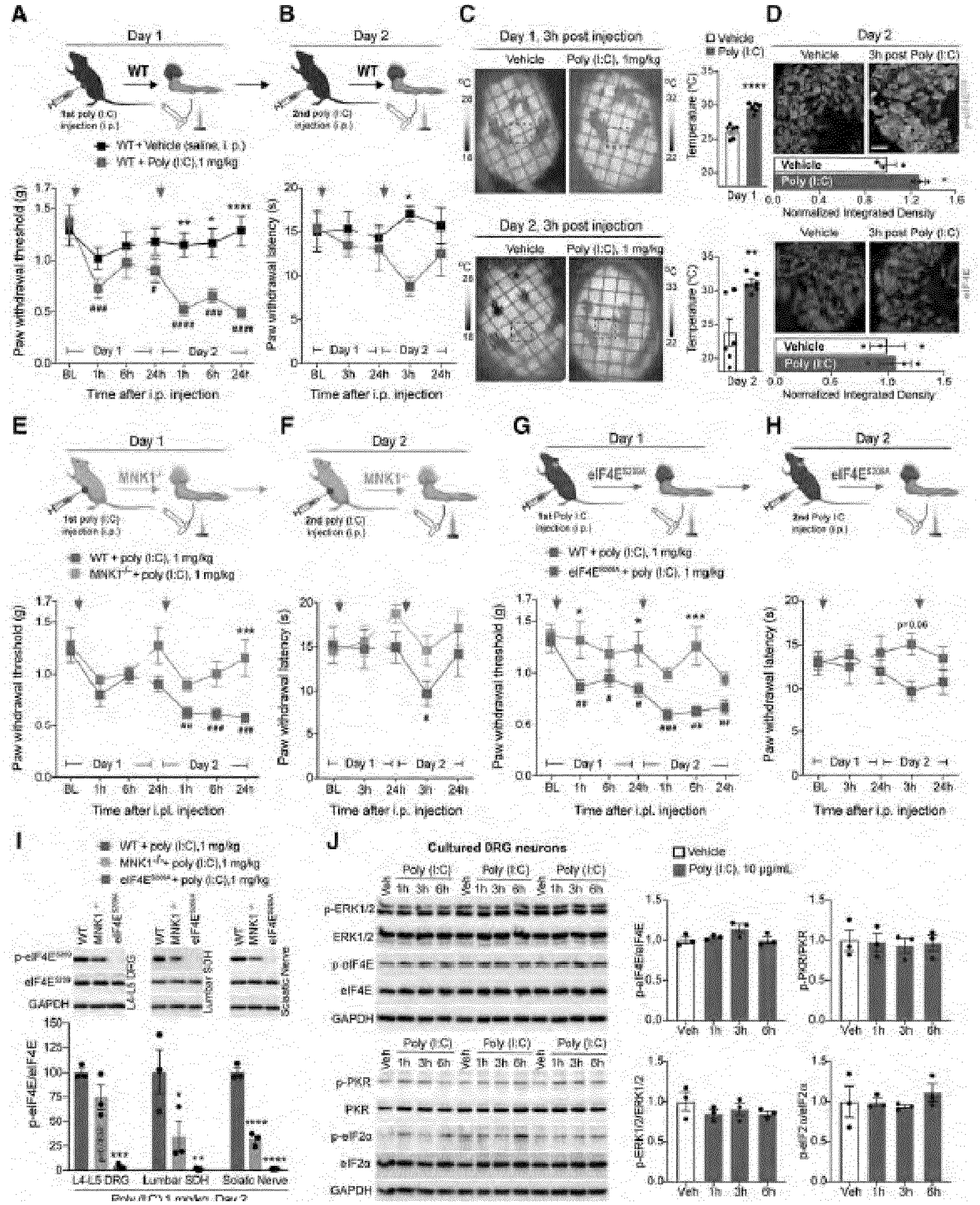

FIGS. 24A-J

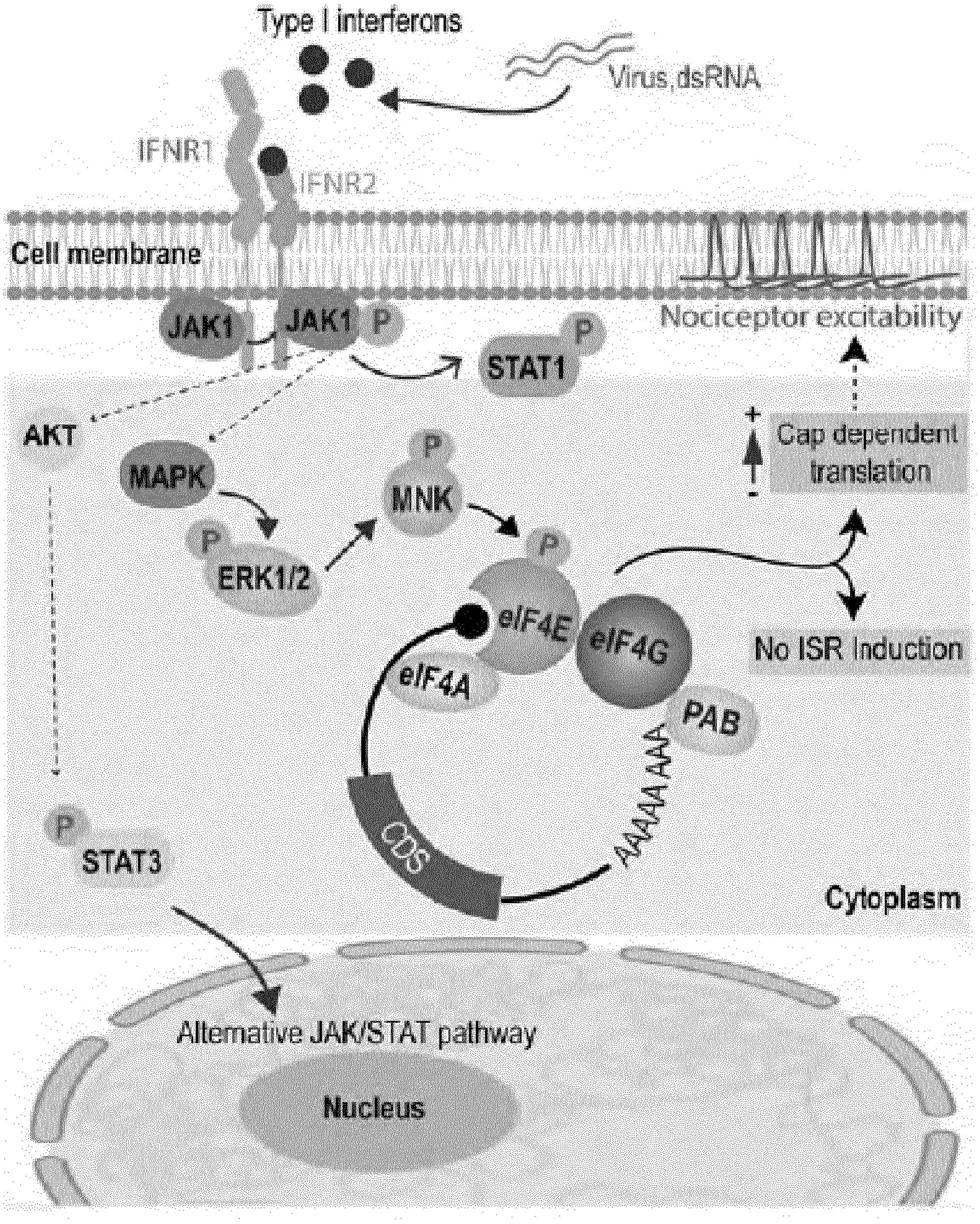
Type I interferons
Virus,dsRNA
IFNR1
IFNR2
Cell membrane
JAK1
JAK1
P
STAT1
P
Nociceptor excitability
AKT
MAPK
P
MNK
P
ERK1/2
P
Cap dependent translation
+
-
eIF4E
eIF4G
eIF4A
No ISR Induction
PAB
AAAAA AAA
CDS
P
STAT3
Cytoplasm
Alternative JAK/STAT pathway
Nucleus

FIG. 25

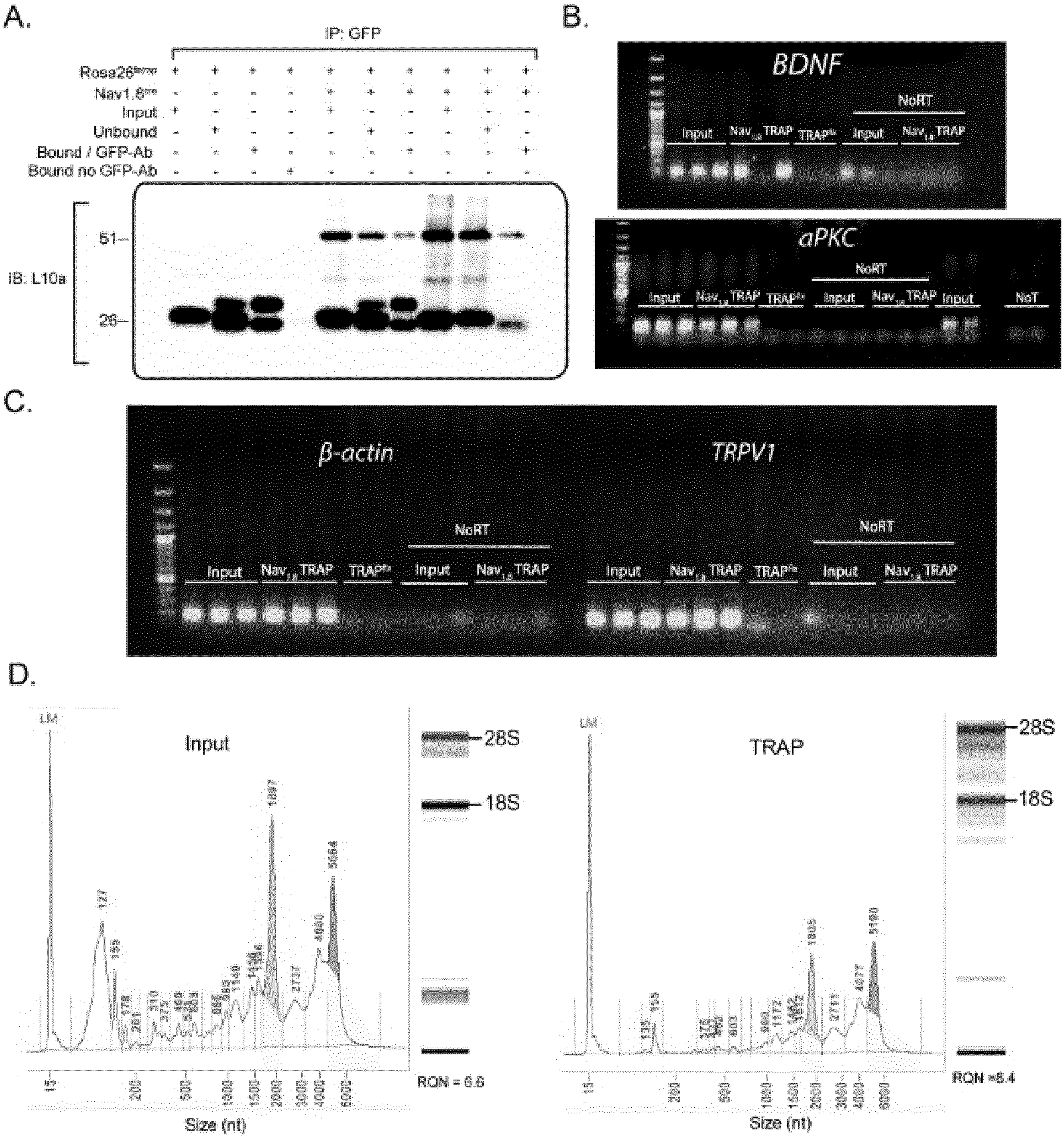


EXTENDED FIG. 1-1A-D

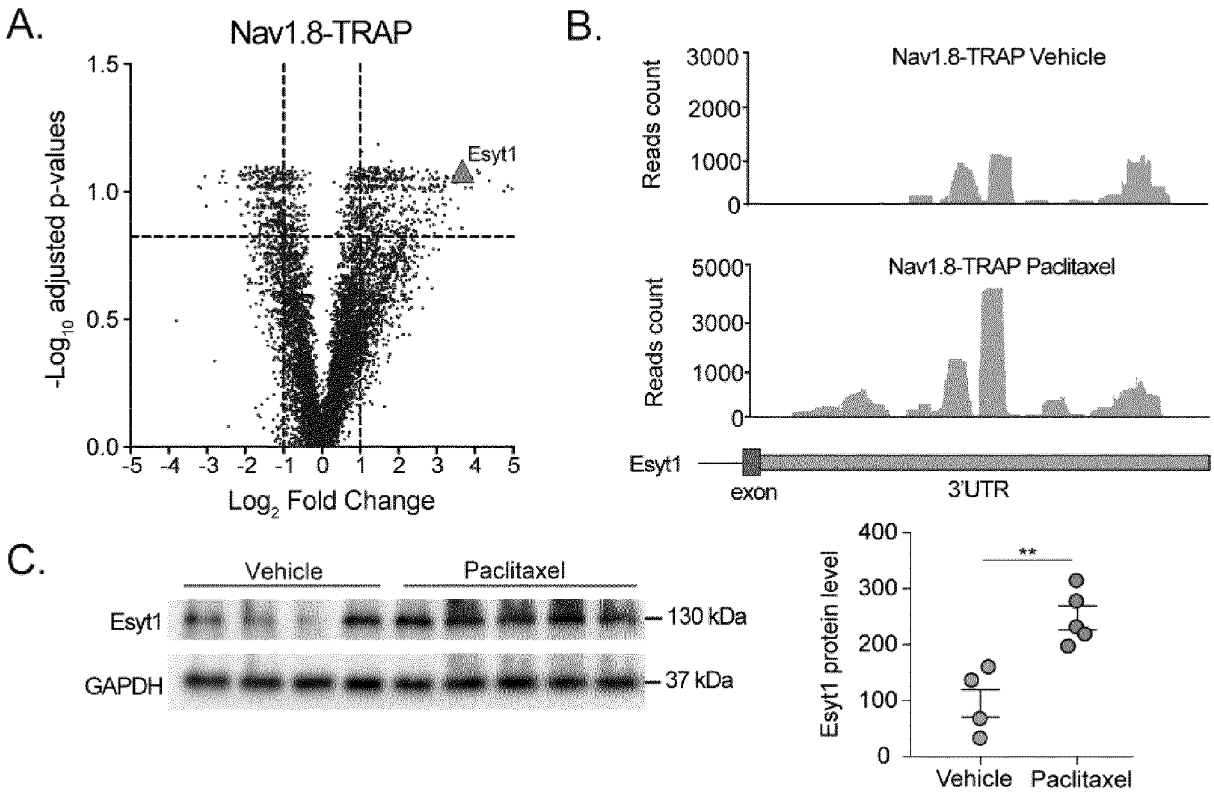


EXTENDED FIGS. 7-1A-C

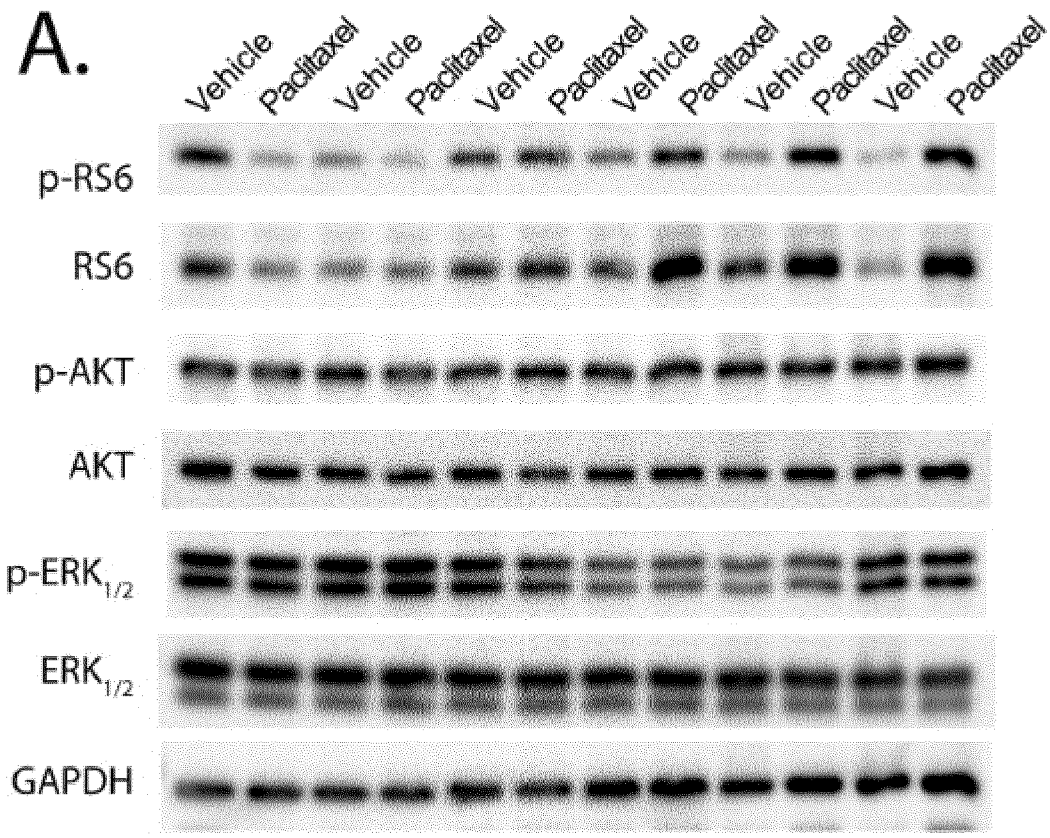


EXTENDED FIG. 7-2A

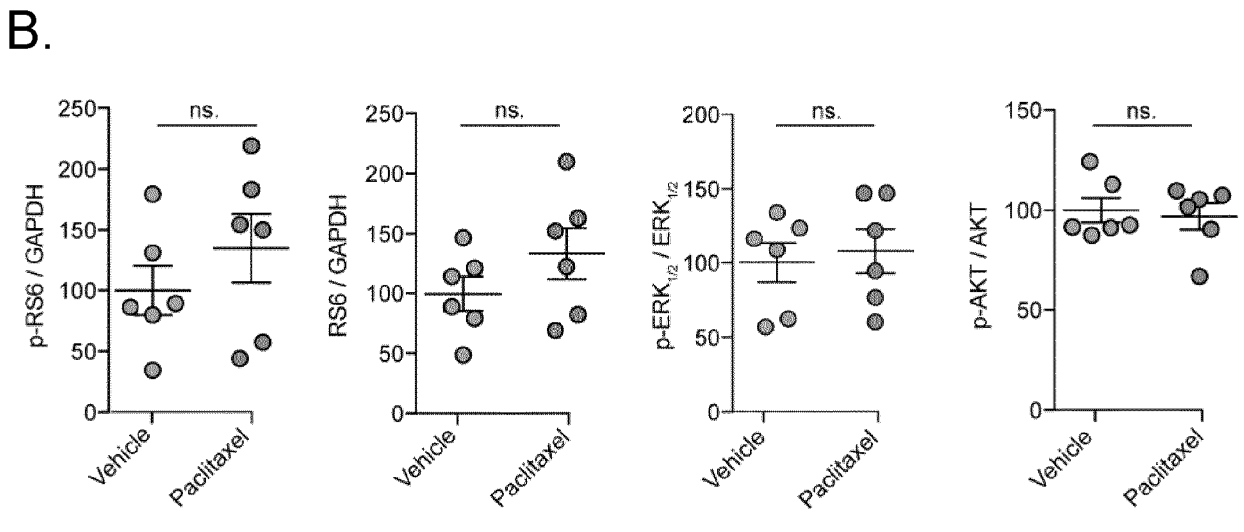


EXTENDED FIG. 7-2B

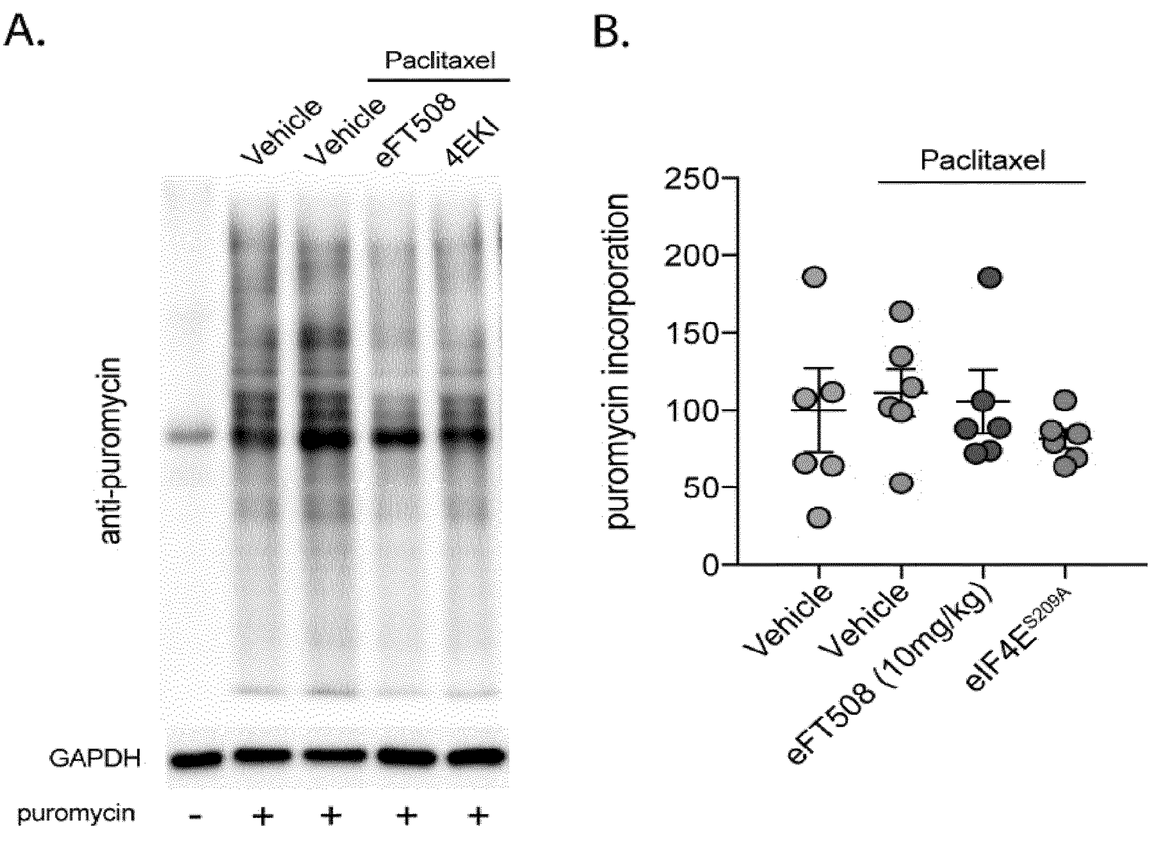


EXTENDED FIGS. 8-1A-B

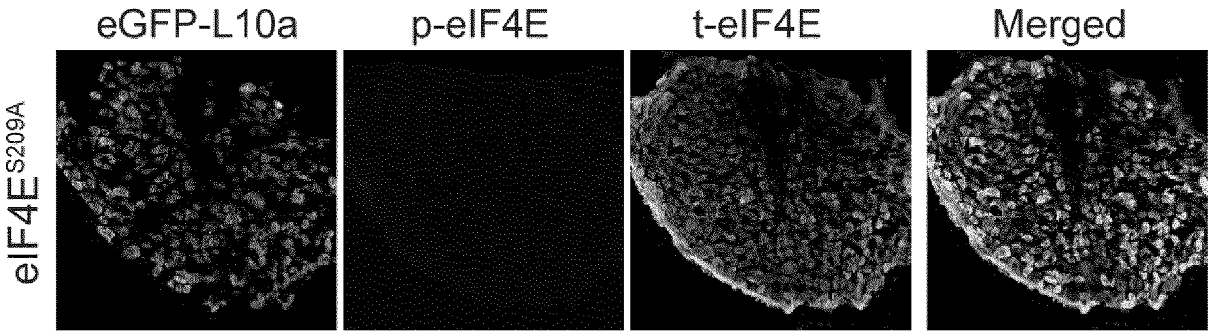


EXTENDED FIG. 8-2

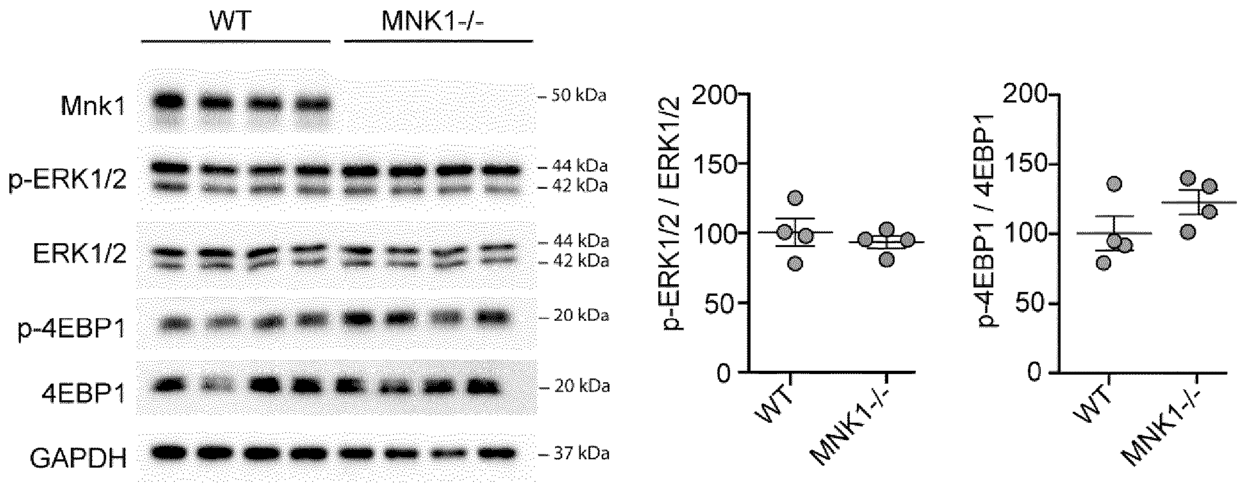


EXTENDED FIG. 10-1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62851897
- WO 2017117052 A
- WO 2018218038 A


### Non-patent literature cited in the description


- Remington's Pharmaceutical Sciences, 1035-1038, 1570-1580
- **MOY et al.** *J. Neurosci.*, 2017, vol. 37 (31), 7481-7499
- Remington's Pharmaceutical Sciences
- **BAR-PELED L** ; **SABATINI DM**. Regulation of mTORC1 by amino acids.. *Trends in cell biology*, 2014, vol. 24, 400-406
- **BARRAGAN-IGLESIAS P** ; **LOU TF** ; **BHAT VD** ; **MEGAT S** ; **BURTON MD** ; **PRICE TJ** ; **CAMPBELL ZT**. Inhibition of Poly(A)-binding protein with a synthetic RNA mimic reduces pain sensitization in mice.. *Nat Commun*, 2018, vol. 9, 10
- **BENNETT GJ** ; **DOYLE T** ; **SALVEMINI D**. Mitotoxicity in distal symmetrical sensory peripheral neuropathies.. *Nat Rev Neurol*, 2014, vol. 10, 326-336
- **BERTA T** ; **PARK CK** ; **XU ZZ** ; **XIE RG** ; **LIU T** ; **LU N** ; **LIU YC** ; **JI RR**. Extracellular caspase-6 drives murine inflammatory pain via microglial TNF-alpha secretion.. *J Clin Invest*, 2014, vol. 124, 1173-1186
- **BERTA T** ; **PERRIN FE** ; **PERTIN M** ; **TONELLO R** ; **LIU YC** ; **CHAMESSIAN A** ; **KATO AC** ; **JI RR** ; **DECOSTERD I**. Gene Expression Profiling of Cutaneous Injured and Non-Injured Nociceptors in SNI Animal Model of Neuropathic Pain. *Sci Rep*, 2017, vol. 7, 9367
- **CAMPBELL JN** ; **MEYER RA**. Mechanisms of neuropathic pain.. *Neuron*, 2006, vol. 52, 77-92
- **CHAPLAN SR** ; **BACH FW** ; **POGREL JW** ; **CHUNG JM** ; **YAKSH TL**. Quantitative assessment of tactile allodynia in the rat paw.. *J Neurosci Methods*, 1994, vol. 53, 55-63
- **CORDER G** ; **TAWFIK VL** ; **WANG D** ; **SYPEK EI** ; **LOW SA** ; **DICKINSON JR** ; **SOTOUDEH C** ; **CLARK JD** ; **BARRES BA** ; **BOHLEN CJ**. Loss of mu opioid receptor signaling in nociceptors, but not microglia, abrogates morphine tolerance without disrupting analgesia.. *Nat Med*, 2017, vol. 23, 164-173
- **COWIE AM** ; **MOEHRING F** ; **O'HARA C** ; **STUCKY CL**. Optogenetic Inhibition of CGRPalpha Sensory Neurons Reveals Their Distinct Roles in Neuropathic and Incisional Pain.. *J Neurosci*, 2018, vol. 38, 5807-5825
- **DAOU I** ; **BEAUDRY H** ; **ASE AR** ; **WIESKOPF JS** ; **RIBEIRO-DA-SILVA A** ; **MOGIL JS** ; **SEGUELA P**. Optogenetic Silencing of Nav1.8-Positive Afferents Alleviates Inflammatory and Neuropathic Pain.. *eNeuro*, 2016, 3
- **DENK F** ; **MCMAHON SB** ; **TRACEY I**. Pain vulnerability: a neurobiological perspective.. *Nat Neurosci*, 2014, vol. 17, 192-200
- **DOYLE T** ; **CHEN Z** ; **MUSCOLI C** ; **BRYANT L** ; **ESPOSITO E** ; **CUZZOCREA S** ; **DAGOSTINO C** ; **RYERSE J** ; **RAUSARIA S** ; **KAMADULSKI A**. Targeting the overproduction of peroxynitrite for the prevention and reversal of paclitaxel-induced neuropathic pain.. *J Neurosci*, 2012, vol. 32, 6149-6160
- **DUGGETT NA** ; **GRIFFITHS LA** ; **MCKENNA OE** ; **DE SANTIS V** ; **YONGSANGUANCHAI N** ; **MOKORI EB** ; **FLATTERS SJ**. Oxidative stress in the development, maintenance and resolution of paclitaxel-induced painful neuropathy.. *Neuroscience*, 2016, vol. 333, 13-26
- **ECKHOFF L** ; **KNOOP A** ; **JENSEN MB** ; **EWERTZ M**. Persistence of docetaxel-induced neuropathy and impact on quality of life among breast cancer survivors.. *Eur J Cancer*, 2015, vol. 51, 292-300
- **EFEYAN A** ; **ZONCU R** ; **SABATINI DM**. Amino acids and mTORC1: from lysosomes to disease.. *Trends Mol Med*, 2012, vol. 18, 524-533
- **EFEYAN A** ; **SCHWEITZER LD** ; **BILATE AM** ; **CHANG S** ; **KIRAK O** ; **LAMMING DW** ; **SABATINI DM**. RagA, but not RagB, is essential for embryonic development and adult mice.. *Dev Cell*, 2014, vol. 29, 321-329

- **EFEYAN A** ; **ZONCU R** ; **CHANG S** ; **GUMPER I** ; **SNITKIN H** ; **WOLFSON RL** ; **KIRAK O** ; **SABATINI DD** ; **SABATINI DM**. Regulation of mTORC1 by the Rag GTPases is necessary for neonatal autophagy and survival.. *Nature*, 2013, vol. 493, 679-683
- **FINNERUP NB** ; **ATTAL N** ; **HAROUTOUNIAN S** ; **MCNICOL E** ; **BARON R** ; **DWORKIN RH** ; **GILRON I** ; **HAANPAA M** ; **HANSSON P** ; **JENSEN TS**. Pharmacotherapy for neuropathic pain in adults: a systematic review and meta-analysis.. *Lancet Neurol*, 2015, vol. 14, 162-173
- **FLATTERS SJ**. The contribution of mitochondria to sensory processing and pain.. *Prog Mol Biol Transl Sci*, 2015, vol. 131, 119-146
- **FLATTERS SJ** ; **BENNETT GJ**. Studies of peripheral sensory nerves in paclitaxel-induced painful peripheral neuropathy: evidence for mitochondrial dysfunction.. *Pain*, 2006, vol. 122, 245-257
- **FURIC L** ; **RONG L** ; **LARSSON O** ; **KOUMAKPAYI IH** ; **YOSHIDA K** ; **BRUESCHKE A** ; **PETROULAKIS E** ; **ROBICHAUD N** ; **POLLAK M** ; **GABOURY LA**. eIF4E phosphorylation promotes tumorigenesis and is associated with prostate cancer progression.. *Proc Natl Acad Sci U S A*, 2010, vol. 107, 14134-14139
- **GERANTON SM** ; **JIMENEZ-DIAZ L** ; **TORSNEY C** ; **TOCHIKI KK** ; **STUART SA** ; **LEITH JL** ; **LUMB BM** ; **HUNT SP**. A rapamycin-sensitive signaling pathway is essential for the full expression of persistent pain states.. *J Neurosci*, 2009, vol. 29, 15017-15027
- **GEREAU RWT** ; **SLUKA KA** ; **MAIXNER W** ; **SAVAGE SR** ; **PRICE TJ** ; **MURINSON BB** ; **SULLIVAN MD** ; **FILLINGIM RB**. A pain research agenda for the 21st century.. *J Pain*, 2014, vol. 15, 1203-1214
- **GKOGKAS CG** ; **KHOUTORSKY A** ; **CAO R** ; **JAFARNEJAD SM** ; **PRAGER-KHOUTORSKY M** ; **GIANNAKAS N** ; **KAMINARI A** ; **FRAGKOULI A** ; **NADER K** ; **PRICE TJ**. Pharmacogenetic Inhibition of eIF4E-Dependent Mmp9 mRNA Translation Reverses Fragile X Syndrome-like Phenotypes.. *Cell Rep*, 2014, vol. 9, 1742-1755
- **GLUSMAN G** ; **CABALLERO J** ; **ROBINSON M** ; **KUTLU B** ; **HOOD L**. Optimal scaling of digital transcriptomes.. *PLoS One*, 2013, vol. 8, e77885
- **GOLD MS** ; **GEBHART GF**. Nociceptor sensitization in pain pathogenesis.. *Nat Med*, 2010, vol. 16, 1248-1257
- **GRIFFITHS LA** ; **FLATTERS SJ**. Pharmacological Modulation of the Mitochondrial Electron Transport Chain in Paclitaxel-Induced Painful Peripheral Neuropathy.. *J Pain*, 2015, vol. 16, 981-994
- **HAROUTOUNIAN S** ; **NIKOLAJSEN L** ; **BENDTSEN TF** ; **FINNERUP NB** ; **KRISTENSEN AD** ; **HASSELSTROM JB** ; **JENSEN TS**. Primary afferent input critical for maintaining spontaneous pain in peripheral neuropathy.. *Pain*, 2014, vol. 155, 1272-1279
- **HAROUTOUNIAN S** ; **FORD AL** ; **FREY K** ; **NIKOLAJSEN L** ; **FINNERUP NB** ; **NEINER A** ; **KHARASCH ED** ; **KARLSSON P** ; **BOTTROS MM**. How central is central poststroke pain? The role of afferent input in poststroke neuropathic pain: a prospective, open-label pilot study.. *Pain*, 2018
- **HEIMAN M** ; **KULICKE R** ; **FENSTER RJ** ; **GREENGARD P** ; **HEINTZ N**. Cell type-specific mRNA purification by translating ribosome affinity purification (TRAP).. *Nat Protoc*, 2014, vol. 9, 1282-1291
- **HEIMAN M** ; **SCHAEFER A** ; **GONG S** ; **PETERSON JD** ; **DAY M** ; **RAMSEY KE** ; **SUAREZ-FARINAS M** ; **SCHWARZ C** ; **STEPHAN DA** ; **SURMEIER DJ**. A translational profiling approach for the molecular characterization of CNS cell types.. *Cell*, 2008, vol. 135, 738-748
- **HU G** ; **HUANG K** ; **HU Y** ; **DU G** ; **XUE Z** ; **ZHU X** ; **FAN G**. Single-cell RNA-seq reveals distinct injury responses in different types of DRG sensory neurons.. *Sci Rep*, 2016, vol. 6, 31851
- **INGOLIA NT** ; **BRAR GA** ; **ROUSKIN S** ; **MCGEACHY AM** ; **WEISSMAN JS**. The ribosome profiling strategy for monitoring translation in vivo by deep sequencing of ribosome-protected mRNA fragments.. *Nat Protoc*, 2012, vol. 7, 1534-1550
- **JESKE NA**. Somatosensory scaffolding structures. *Front Mol Neurosci*, 2012, vol. 5, 2
- **JESKE NA**. Peripheral scaffolding and signaling pathways in inflammatory pain.. *Prog Mol Biol Transl Sci*, 2015, vol. 131, 31-52
- **JI RR** ; **CHAMESSIAN A** ; **ZHANG YQ**. Pain regulation by non-neuronal cells and inflammation.. *Science*, 2016, vol. 354, 572-577
- **JI RR** ; **SAMAD TA** ; **JIN SX** ; **SCHMOLL R** ; **WOOLF CJ**. MAPK activation by NGF in primary sensory neurons after inflammation increases TRPV1 levels and maintains heat hyperalgesia.. *Neuron*, 2002, vol. 36, 57-68
- **JIMENEZ-DIAZ L** ; **GERANTON SM** ; **PASSMORE GM** ; **LEITH JL** ; **FISHER AS** ; **BERLIOCCHI L** ; **SIVASUBRAMANIAM AK** ; **SHEASBY A** ; **LUMB BM** ; **HUNT SP**. Local translation in primary afferent fibers regulates nociception.. *PLoS One*, 2008, vol. 3, e1961
- **KHOUTORSKY A** ; **PRICE TJ**. Translational Control Mechanisms in Persistent Pain.. *Trends Neurosci*, 2018, vol. 41, 100-114

- **KING T** ; **VERA-PORTOCARRERO L** ; **GUTIERREZ T** ; **VANDERAH TW** ; **DUSSOR G** ; **LAI J** ; **FIELDS HL** ; **PORRECA F**. Unmasking the tonic-aversive state in neuropathic pain.. *Nature neuroscience*, 2009, vol. 12, 1364-1366
- **KULESHOV MV** ; **JONES MR** ; **ROUILLARD AD** ; **FERNANDEZ NF** ; **DUAN Q** ; **WANG Z** ; **KOPLEV S** ; **JENKINS SL** ; **JAGODNIK KM** ; **LACHMANN A**. Enrichr: a comprehensive gene set enrichment analysis web server 2016 update.. *Nucleic Acids Res*, 2016, vol. 44, W90-97
- **LI Y** ; **TATSUI CE** ; **RHINES LD** ; **NORTH RY** ; **HARRISON DS** ; **CASSIDY RM** ; **JOHANSSON CA** ; **KOSTURAKIS AK** ; **EDWARDS DD** ; **ZHANG H**. Dorsal root ganglion neurons become hyperexcitable and increase expression of voltage-gated T-type calcium channels (Cav3.2) in paclitaxel-induced peripheral neuropathy.. *Pain*, 2017, vol. 158, 417-428
- **LI Y** ; **ADAMEK P** ; **ZHANG H** ; **TATSUI CE** ; **RHINES LD** ; **MROZKOVA P** ; **LI Q** ; **KOSTURAKIS AK** ; **CASSIDY RM** ; **HARRISON DS**. The Cancer Chemotherapeutic Paclitaxel Increases Human and Rodent Sensory Neuron Responses to TRPV1 by Activation of TLR4.. *J Neurosci*, 2015, vol. 35, 13487-13500
- **LIU Y** ; **BEYER A** ; **AEBERSOLD R**. On the Dependency of Cellular Protein Levels on mRNA Abundance.. *Cell*, 2016, vol. 165, 535-550
- **LOPES DM** ; **DENK F** ; **MCMAHON SB**. The Molecular Fingerprint of Dorsal Root and Trigeminal Ganglion Neurons.. *Front Mol Neurosci*, 2017, vol. 10, 304
- **LOPES DM** ; **MALEK N** ; **EDYE M** ; **JAGER SB** ; **MCMURRAY S** ; **MCMAHON SB** ; **DENK F**. Sex differences in peripheral not central immune responses to pain-inducing injury.. *Sci Rep*, 2017, vol. 7, 16460
- **MA J** ; **KAVELAARS A** ; **DOUGHERTY PM** ; **HEIJNEN CJ**. Beyond symptomatic relief for chemotherapy-induced peripheral neuropathy: Targeting the source.. *Cancer*, 2018
- **MANTENIOTIS S** ; **LEHMANN R** ; **FLEGEL C** ; **VOGEL F** ; **HOFREUTER A** ; **SCHREINER BS** ; **ALTMULLER J** ; **BECKER C** ; **SCHOBEL N** ; **HATT H**. Comprehensive RNA-Seq expression analysis of sensory ganglia with a focus on ion channels and GPCRs in Trigeminal ganglia.. *PLoS One*, 2013, vol. 8, e79523
- **MELEMEDJIAN OK** ; **KHOUTORSKY A** ; **SORGE RE** ; **YAN J** ; **ASIEDU MN** ; **VALDEZ A** ; **GHOSH S** ; **DUSSOR G** ; **MOGIL JS** ; **SONENBERG N**. mTORC1 inhibition induces pain via IRS-1-dependent feedback activation of ERK.. *Pain*, 2013
- **MELEMEDJIAN OK AM** ; **TILLU DV** ; **SANOJA R** ; **YAN J** ; **LARK A** ; **KHOUTORSKY A** ; **JOHNSON J** ; **PEEBLES KA** ; **LEPOW T** ; **SONENBERG N**. Targeting adenosine monophosphate-activated protein kinase (AMPK) in preclinical models reveals a potential mechanism for the treatment of neuropathic pain.. *Molecular Pain.*, 2011
- **MOY JK** ; **KHOUTORSKY A** ; **ASIEDU MN** ; **DUSSOR G** ; **PRICE TJ**. eIF4E Phosphorylation Influences Bdnf mRNA Translation in Mouse Dorsal Root Ganglion Neurons.. *Front Cell Neurosci*, 2018, vol. 12, 29
- **MOY JK** ; **KHOUTORSKY A** ; **ASIEDU MN** ; **BLACK BJ** ; **KUHN JL** ; **BARRAGAN-IGLESIAS P** ; **MEGAT S** ; **BURTON MD** ; **BURGOS-VEGA CC** ; **MELEMEDJIAN OK**. The MNK-eIF4E Signaling Axis Contributes to Injury-Induced Nociceptive Plasticity and the Development of Chronic Pain.. *J Neurosci*, 2017, vol. 37, 7481-7499
- **OBARA I** ; **GERANTON SM** ; **HUNT SP**. Axonal protein synthesis: a potential target for pain relief?. *Curr Opin Pharmacol*, 2012, vol. 12, 42-48
- **PRICE TJ** ; **GOLD MS**. From Mechanism to Cure: Renewing the Goal to Eliminate the Disease of Pain.. *Pain medicine*, 2018, vol. 19, 1525-1549
- **PRICE TJ** ; **BASBAUM AI** ; **BRESNAHAN J** ; **CHAMBERS JF** ; **DE KONINCK Y** ; **EDWARDS RR** ; **JI RR** ; **KATZ J** ; **KAVELAARS A** ; **LEVINE JD**. Transition to chronic pain: opportunities for novel therapeutics.. *Nat Rev Neurosci*, 2018, vol. 19, 383-384
- **RAY P** ; **TORCK A** ; **QUIGLEY L** ; **WANGZHOU A** ; **NEIMAN M** ; **RAO C** ; **LAM T** ; **KIM JY** ; **KIM TH** ; **ZHANG MQ**. Comparative transcriptome profiling of the human and mouse dorsal root ganglia: an RNA-seq-based resource for pain and sensory neuroscience research.. *Pain*, 2018, vol. 159, 1325-1345
- **REICH SH et al.** Structure-based Design of Pyridone-aminal eFT508 Targeting Dysregulated Translation by Selective Mitogen-activated Protein Kinase Interacting Kinases 1 and 2 (MNK1/2) Inhibition.. *J Med Chem.*, 2018
- **RICHTER JD** ; **SONENBERG N**. Regulation of cap-dependent translation by eIF4E inhibitory proteins.. *Nature*, 2005, vol. 433, 477-480
- **SANCAK Y** ; **BAR-PELED L** ; **ZONCU R** ; **MARKHARD AL** ; **NADA S** ; **SABATINI DM**. Ragulator-Rag complex targets mTORC1 to the lysosomal surface and is necessary for its activation by amino acids.. *Cell*, 2010, vol. 141, 290-303
- **SAXTON RA** ; **SABATINI DM**. mTOR Signaling in Growth, Metabolism, and Disease.. *Cell*, 2017, vol. 168, 960-976

- **SERETNY M** ; **CURRIE GL** ; **SENA ES** ; **RAMNARINE S** ; **GRANT R** ; **MACLEOD MR** ; **COLVIN LA** ; **FALLON M**. Incidence, prevalence, and predictors of chemotherapy-induced peripheral neuropathy: A systematic review and meta-analysis.. *Pain*, 2014, vol. 155, 2461-2470
- **SILVA AMORIM I** ; **KEDIA S** ; **KOULOULIA S** ; **SIMBRIGER K** ; **GANTOIS I** ; **JAFARNEJAD SM** ; **LI Y** ; **KAMPAITE A** ; **POOTERS T** ; **ROMANO N**. Loss of eIF4E phosphorylation engenders depression-like behaviors via selective mRNA translation.. *J Neurosci*, 2018
- **SOARES HP** ; **MING M** ; **MELLON M** ; **YOUNG SH** ; **HAN L** ; **SINNET-SMITH J** ; **ROZENGURT E**. Dual PI3K/mTOR Inhibitors Induce Rapid Overactivation of the MEK/ERK Pathway in Human Pancreatic Cancer Cells through Suppression of mTORC2.. *Mol Cancer Ther*, 2015, vol. 14, 1014-1023
- **STIRLING LC** ; **FORLANI G** ; **BAKER MD** ; **WOOD JN** ; **MATTHEWS EA** ; **DICKENSON AH** ; **NASSAR MA**. Nociceptor-specific gene deletion using heterozygous NaV1.8-Cre recombinase mice.. *Pain*, 2005, vol. 113, 27-36
- **TERENZIO M** ; **KOLEY S** ; **SAMRA N** ; **RISHAL I** ; **ZHAO Q** ; **SAHOO PK** ; **URISMAN A** ; **MARVALDI L** ; **OSES-PRIETO JA** ; **FORESTER C**. Locally translated mTOR controls axonal local translation in nerve injury.. *Science*, 2018, vol. 359, 1416-1421
- **THAKUR M** ; **CROW M** ; **RICHARDS N** ; **DAVEY GI** ; **LEVINE E** ; **KELLEHER JH** ; **AGLEY CC** ; **DENK F** ; **HARRIDGE SD** ; **MCMAHON SB**. Defining the nociceptor transcriptome.. *Front Mol Neurosci*, 2014, vol. 7, 87
- **THOREEN CC** ; **CHANTRANUPONG L** ; **KEYS HR** ; **WANG T** ; **GRAY NS** ; **SABATINI DM**. A unifying model for mTORC1-mediated regulation of mRNA translation.. *Nature*, 2012, vol. 485, 109-113
- **UEDA T** ; **WATANABE-FUKUNAGA R** ; **FUKUYAMA H** ; **NAGATA S** ; **FUKUNAGA R**. Mnk2 and Mnk1 are essential for constitutive and inducible phosphorylation of eukaryotic initiation factor 4E but not for cell growth or development.. *Mol Cell Biol*, 2004, vol. 24, 6539-6549
- **USOSKIN D** ; **FURLAN A** ; **ISLAM S** ; **ABDO H** ; **LONNERBERG P** ; **LOU D** ; **HJERLING-LEFFLER J** ; **HAEGGSTROM J** ; **KHARCHENKO O** ; **KHARCHENKO PV**. Unbiased classification of sensory neuron types by large-scale single-cell RNA sequencing.. *Nat Neurosci*, 2015, vol. 18, 145-153
- **UTTAM S** ; **WONG C** ; **AMORIM IS** ; **JAFARNEJ SM** ; **TANSLEY SM** ; **YANG J** ; **PRAGER-KHOUTORSKY M** ; **MOGIL JS** ; **GKOGKAS CG** ; **KHOUTORSKY A**. Translational profiling of dorsal root ganglia and spinal cord in a mouse model of neuropathic pain.. *Neurobiology of Pain*, 2018
- **VASO A** ; **ADAHAN HM** ; **GJIKA A** ; **ZAHAJ S** ; **ZHURDA T** ; **VYSHKA G** ; **DEVOR M**. Peripheral nervous system origin of phantom limb pain.. *Pain*, 2014, vol. 155, 1384-1391
- **VENTZEL L** ; **JENSEN AB** ; **JENSEN AR** ; **JENSEN TS** ; **FINNERUP NB**. Chemotherapy-induced pain and neuropathy: a prospective study in patients treated with adjuvant oxaliplatin or docetaxel.. *Pain*, 2016, vol. 157, 560-568
- **WIESKOPF JS et al.** The nicotinic alpha6 subunit gene determines variability in chronic pain sensitivity via cross-inhibition of P2X2/3 receptors.. *Sci Transl Med*, 2015, vol. 7, 287-272
- **WOLFE AL**. RNA G-quadruplexes cause eIF4A-dependent oncogene translation in cancer. *Nature*, 2014
- **WOLFSON RL** ; **SABATINI DM**. The Dawn of the Age of Amino Acid Sensors for the mTORC1 Pathway.. *Cell Metab*, 2017, vol. 26, 301-309
- **YANG Q** ; **WU Z** ; **HADDEN JK** ; **ODEM MA** ; **ZUO Y** ; **CROOK RJ** ; **FROST JA** ; **WALTERS ET**. Persistent pain after spinal cord injury is maintained by primary afferent activity.. *J Neurosci*, 2014, vol. 34, 10765-10769
- **YILMAZ E** ; **GOLD MS**. Paclitaxel-induced increase in NCX activity in subpopulations of nociceptive afferents: A protective mechanism against chemotherapy-induced peripheral neuropathy?. *Cell Calcium*, 2016, vol. 60, 25-31
- **ZHOU P** ; **ZHANG Y** ; **MA Q** ; **GU F** ; **DAY DS** ; **HE A** ; **ZHOU B** ; **LI J** ; **STEVENS SM** ; **ROMO D**. Interrogating translational efficiency and lineage-specific transcriptomes using ribosome affinity purification.. *Proc Natl Acad Sci U S A*, 2013, vol. 110, 15395-15400
- **ECCLESTON, C.** Chronic pain and distraction: an experimental investigation into the role of sustained and shifting attention in the processing of chronic persistent pain.. *Behav Res Ther*, 1995, vol. 33, 391-405
- **POVEDANO, M.** ; **GASCON, J.** ; **GALVEZ, R.** ; **RUIZ, M.** ; **REJAS, J.** Cognitive function impairment in patients with neuropathic pain under standard conditions of care.. *J Pain Symptom Manage*, 2007, vol. 33, 78-89
- **SCHILTENWOLF, M. et al.** Evidence of specific cognitive deficits in patients with chronic low back pain under long-term substitution treatment of opioids.. *Pain Physician*, 2014, vol. 17, 9-20
- **SHIERS, S. et al.** Neuropathic Pain Creates an Enduring Prefrontal Cortex Dysfunction Corrected by the Type II Diabetic Drug Metformin But Not by Gabapentin.. *J Neurosci*, 2018, vol. 38, 7337-7350

- **MELEMEDJIAN, O. K. et al.** Targeting adenosine monophosphate-activated protein kinase (AMPK) in preclinical models reveals a potential mechanism for the treatment of neuropathic pain.. *Mol Pain*, 2011, vol. 7, 70
- **MOY, J. K. et al.** The MNK-eIF4E Signaling Axis Contributes to Injury-Induced Nociceptive Plasticity and the Development of Chronic Pain.. *J Neurosci*, 2017, vol. 37, 7481-7499
- **MOY, J. K.** ; **KUHN, J. L.** ; **SZABO-PARDI, T. A.** ; **PRADHAN, G.** ; **PRICE, T. J.** eIF4E phosphorylation regulates ongoing pain, independently of inflammation, and hyperalgesic priming in the mouse CFA model.. *Neurobiol Pain*, 2018, vol. 4, 45-50
- **INYANG, K. E. et al.** The antidiabetic drug metformin prevents and reverses neuropathic pain and spinal cord microglial activation in male but not female mice.. *Pharmacol Res*, 2019, vol. 139, 1-16
- **MEGAT, S. et al.** Nociceptor Translational Profiling Reveals the Ragulator-Rag GTPase Complex as a Critical Generator of Neuropathic Pain.. *J Neurosci*, 2019, vol. 39, 393-411
- **YANG, Q. et al.** Persistent pain after spinal cord injury is maintained by primary afferent activity.. *J Neurosci*, 2014, vol. 34, 10765-10769
- **COWIE, A. M.** ; **MOEHRING, F.** ; **O'HARA, C.** ; **STUCKY, C. L.** Optogenetic Inhibition of CGRPalpha Sensory Neurons Reveals Their Distinct Roles in Neuropathic and Incisional Pain.. *J Neurosci*, 2018, vol. 38, 5807-5825
- **NORTH, R. Y. et al.** Electrophysiological and transcriptomic correlates of neuropathic pain in human dorsal root ganglion neurons.. *Brain*, 2019, vol. 142, 1215-1226
- **KHOUTORSKY, A.** ; **PRICE, T. J.** Translational Control Mechanisms in Persistent Pain.. *Trends Neurosci*, 2018, vol. 41, 100-114
- **UTTAM, S.** ; **WONG, C.** ; **PRICE, T. J.** ; **KHOUTORSKY, A.** eIF4E-Dependent Translational Control: A Central Mechanism for Regulation of Pain Plasticity.. *Front Genet*, 2018, vol. 9, 470
- **FURIC, L. et al.** eIF4E phosphorylation promotes tumorigenesis and is associated with prostate cancer progression.. *Proc Natl Acad Sci U S A*, 2010, vol. 107, 14134-14139
- **GKOGKAS, C. G. et al.** Pharmacogenetic inhibition of eIF4E-dependent Mmp9 mRNA translation reverses fragile X syndrome-like phenotypes.. *Cell Rep*, 2014, vol. 9, 1742-1755
- **AMORIM, I. S. et al.** Loss of eIF4E Phosphorylation Engenders Depression-like Behaviors via Selective mRNA Translation.. *J Neurosci*, 2018, vol. 38, 2118-2133
- **MOY, J. K.** ; **KHOUTORSKY, A.** ; **ASIEDU, M. N.** ; **DUSSOR, G.** ; **PRICE, T. J.** eIF4E Phosphorylation Influences Bdnf mRNA Translation in Mouse Dorsal Root Ganglion Neurons.. *Front Cell Neurosci*, 2018, vol. 12, 29
- **UEDA, T.** ; **WATANABE-FUKUNAGA, R.** ; **FUKUYAMA, H.** ; **NAGATA, S.** ; **FUKUNAGA, R.** Mnk2 and Mnk1 are essential for constitutive and inducible phosphorylation of eukaryotic initiation factor 4E but not for cell growth or development.. *Mol Cell Biol*, 2004, vol. 24, 6539-6549
- *Body Weight Information for C57BL/6J*, 2019
- **DECOSTERD, I.** ; **WOOLF, C. J.** Spared nerve injury: an animal model of persistent peripheral neuropathic pain.. *Pain*, 2000, vol. 87, 149-158
- **CHAPLAN, S. R.** ; **BACH, F. W.** ; **POGREL, J. W.** ; **CHUNG, J. M.** ; **YAKSH, T. L.** Quantitative assessment of tactile allodynia in the rat paw.. *J Neurosci Methods*, 1994, vol. 53, 55-63
- **ANGOA-PEREZ, M.** ; **KANE, M. J.** ; **BRIGGS, D. I.** ; **FRANCESCUTTI, D. M.** ; **KUHN, D. M.** Marble burying and nestlet shredding as tests of repetitive, compulsive-like behaviors in mice.. *J Vis Exp*, 2013, 50978
- **BLACK, B. J. et al.** Adult mouse sensory neurons on microelectrode arrays exhibit increased spontaneous and stimulus-evoked activity in the presence of interleukin-6.. *J Neurophysiol*, 2018, vol. 120, 1374-1385
- **KING, T. et al.** Unmasking the tonic-aversive state in neuropathic pain.. *Nat Neurosci*, 2009, vol. 12, 1364-1366
- **REICH, S. H. et al.** Structure-based Design of Pyridone-Aminal eFT508 Targeting Dysregulated Translation by Selective Mitogen-activated Protein Kinase Interacting Kinases 1 and 2 (MNK1/2) Inhibition.. *J Med Chem*, 2018, vol. 61, 3516-3540
- **GRUBB, M. S.** ; **BURRONE, J.** Activity-dependent relocation of the axon initial segment fine-tunes neuronal excitability.. *Nature*, 2010, vol. 465, 1070-1074
- **KUBA, H.** ; **OICHI, Y.** ; **OHMORI, H.** Presynaptic activity regulates Na(+) channel distribution at the axon initial segment.. *Nature*, 2010, vol. 465, 1075-1078
- **GRUBB, M. S. et al.** Short- and long-term plasticity at the axon initial segment.. *J Neurosci*, 2011, vol. 31, 16049-16055
- **RICHARDS, G. C. et al.** Effects of long-term opioid analgesics on cognitive performance and plasma cytokine concentrations in patients with chronic low back pain: a cross-sectional pilot study.. *Pain Rep*, 2018, vol. 3, e669
- Voices of Chronic Pain. American Pain Foundation, 2006
- **APKARIAN, A. V. et al.** Chronic back pain is associated with decreased prefrontal and thalamic gray matter density.. *J Neurosci*, 2004, vol. 24, 10410-10415
- **SCHMIDT-WILCKE, T. et al.** Gray matter decrease in patients with chronic tension type headache.. *Neurology*, 2005, vol. 65, 1483-1486

- **DAVIS, K. D. et al.** Cortical thinning in IBS: implications for homeostatic, attention, and pain processing.. *Neurology*, 2008, vol. 70, 153-154
- **KUCHINAD, A. et al.** Accelerated brain gray matter loss in fibromyalgia patients: premature aging of the brain?. *J Neurosci*, 2007, vol. 27, 4004-4007
- **METZ, A. E.** ; **YAU, H. J.** ; **CENTENO, M. V.** ; **APKARIAN, A. V.** ; **MARTINA, M.** Morphological and functional reorganization of rat medial prefrontal cortex in neuropathic pain.. *Proc Natl Acad Sci U S A*, 2009, vol. 106, 2423-2428
- **SEMINOWICZ, D. A. et al.** MRI structural brain changes associated with sensory and emotional function in a rat model of long-term neuropathic pain.. *Neuroimage*, 2009, vol. 47, 1007-1014
- **JI, G. et al.** Cognitive impairment in pain through amygdala-driven prefrontal cortical deactivation.. *J Neurosci*, 2010, vol. 30, 5451-5464
- **ZHANG, Z. et al.** Role of Prelimbic GABAergic Circuits in Sensory and Emotional Aspects of Neuropathic Pain.. *Cell Rep*, 2015, vol. 12, 752-759
- **KELLY, C. J.** ; **HUANG, M.** ; **MELTZER, H.** ; **MARTINA, M.** Reduced Glutamatergic Currents and Dendritic Branching of Layer 5 Pyramidal Cells Contribute to Medial Prefrontal Cortex Deactivation in a Rat Model of Neuropathic Pain.. *Front Cell Neurosci*, 2016, vol. 10, 133
- **CHERIYAN, J.** ; **SHEETS, P. L.** Altered Excitability and Local Connectivity of mPFC-PAG Neurons in a Mouse Model of Neuropathic Pain.. *J Neurosci*, 2018, vol. 38, 4829-4839
- **GAURIAU, C.** ; **BERNARD, J. F.** Pain pathways and parabrachial circuits in the rat.. *Exp Physiol*, 2002, vol. 87, 251-258
- **GONCALVES, L.** ; **DICKENSON, A. H.** Asymmetric time-dependent activation of right central amygdala neurones in rats with peripheral neuropathy and pregabalin modulation.. *Eur J Neurosci*, 2012, vol. 36, 3204-3213
- **NEUGEBAUER, V.** ; **LI, W.** ; **BIRD, G. C.** ; **HAN, J. S.** The amygdala and persistent pain.. *Neuroscientist*, 2004, vol. 10, 221-234
- **AMORIM, I. S.** ; **LACH, G.** ; **GKOGKAS, C. G.** The Role of the Eukaryotic Translation Initiation Factor 4E (eIF4E) in Neuropsychiatric Disorders.. *Front Genet*, 2018, vol. 9, 561
- **AGUILAR-VALLES, A. et al.** Translational control of depression-like behavior via phosphorylation of eukaryotic translation initiation factor 4E.. *Nat Commun*, 2018, vol. 9, 2459
- **ABRAHAMSEN, B. et al.** The cell and molecular basis of mechanical, cold, and inflammatory pain.. *Science*, 2008, vol. 321, 702-705
- **KNOWLTON, W. M. et al.** A sensory-labeled line for cold: TRPM8-expressing sensory neurons define the cellular basis for cold, cold pain, and cooling-mediated analgesia.. *J Neurosci*, 2013, vol. 33, 2837-2848
- **PRICE, T. J.** ; **PRESCOTT, S. A.** Inhibitory regulation of the pain gate and how its failure causes pathological pain.. *Pain*, 2015, vol. 156, 789-792
- **LETERRIER, C.** The Axon Initial Segment: An Updated Viewpoint.. *J Neurosci*, 2018, vol. 38, 2135-2145
- **KUBA, H.** ; **YAMADA, R.** ; **ISHIGURO, G.** ; **ADACHI, R.** Redistribution of Kv1 and Kv7 enhances neuronal excitability during structural axon initial segment plasticity.. *Nat Commun*, 2015, vol. 6, 8815
- **MITRIC, M. et al.** Layer- and subregion-specific electrophysiological and morphological changes of the medial prefrontal cortex in a mouse model of neuropathic pain.. *Sci Rep*, 2019
- **SEMINOWICZ, D. A. et al.** Cognitive-behavioral therapy increases prefrontal cortex gray matter in patients with chronic pain.. *J Pain*, 2013, vol. 14, 1573-1584
- **RODRIGUEZ-RAECKE, R.** ; **NIEMEIER, A.** ; **IHLE, K.** ; **RUETHER, W.** ; **MAY, A.** Brain gray matter decrease in chronic pain is the consequence and not the cause of pain.. *J Neurosci*, 2009, vol. 29, 13746-13750
- **RODRIGUEZ-RAECKE, R.** ; **NIEMEIER, A.** ; **IHLE, K.** ; **RUETHER, W.** ; **MAY, A.** Structural brain changes in chronic pain reflect probably neither damage nor atrophy.. *PLoS One*, 2013, vol. 8, e54475
- **SEMINOWICZ, D. A. et al.** Effective treatment of chronic low back pain in humans reverses abnormal brain anatomy and function.. *J Neurosci*, 2011, vol. 31, 7540-7550
- **AZIZ-DONNELLY A** ; **HARRISON TB**. Update of HIV-Associated Sensory Neuropathies.. *Curr Treat Options Neurol*, 2017, vol. 19, 36
- **BALACHANDRAN S** ; **BARBER GN**. PKR in innate immunity, cancer, and viral oncolysis.. *Methods Mol Biol*, 2007, vol. 383, 277-301
- **BARAL P** ; **MILLS K** ; **PINHO-RIBEIRO FA** ; **CHIU IM**. Pain and Itch: Beneficial or Harmful to Antimicrobial Defense?. *Cell Host Microbe*, 2016, vol. 19, 755-759
- **BARRAGAN-IGLESIAS P** ; **KUHN J** ; **VIDAL-CANTU GC** ; **SALINAS-ABARCA AB** ; **GRANADOS-SOTO V** ; **DUSSOR GO** ; **CAMPBELL ZT** ; **PRICE TJ**. Activation of the integrated stress response in nociceptors drives methylglyoxal-induced pain.. *Pain*, 2019, vol. 160, 160-171
- **BARRAT FJ** ; **CROW MK** ; **IVASHKIV LB**. Interferon target-gene expression and epigenomic signatures in health and disease.. *Nat Immunol*, 2019, vol. 20, 1574-1583

- **BLAKE KJ** ; **BARAL P** ; **VOISIN T** ; **LUBKIN A** ; **PINHO-RIBEIRO FA** ; **ADAMS KL** ; **ROBERSON DP** ; **MA YC** ; **OTTO M** ; **WOOLF CJ**. Staphylococcus aureus produces pain through pore-forming toxins and neuronal TRPV1 that is silenced by QX-314.. *Nat Commun*, 2018, vol. 9, 37
- **BRIZZI KT** ; **LYONS JL**. Peripheral nervous system manifestations of infectious diseases.. *Neurohospitalist*, 2014, vol. 4, 230-240
- **BUTLER A** ; **HOFFMAN P** ; **SMIBERT P** ; **PAPALEXI E** ; **SATIJA R**. Integrating single-cell transcriptomic data across different conditions, technologies, and species.. *Nat Biotechnol*, 2018, vol. 36, 411-420
- **CAPURON L** ; **GUMNICK JF** ; **MUSSELMAN DL** ; **LAWSON DH** ; **REEMSNYDER A** ; **NEMEROFF CB** ; **MILLER AH**. Neurobehavioral effects of interferon-alpha in cancer patients: phenomenology and paroxetine responsiveness of symptom dimensions.. *Neuropsychopharmacology*, 2002, vol. 26, 643-652
- **CHENG L** ; **YU H** ; **LI G** ; **LI F** ; **MA J** ; **LI J** ; **CHI L** ; **ZHANG L** ; **SU L**. Type I interferons suppress viral replication but contribute to T cell depletion and dysfunction during chronic HIV-1 infection.. *JCI Insight*, 2017, 2
- **CHIU IM** ; **PINHO-RIBEIRO FA** ; **WOOLF CJ**. Pain and infection: pathogen detection by nociceptors.. *Pain*, 2016, vol. 157, 1192-1193
- **CHIU IM** ; **HEESTERS BA** ; **GHASEMLOU N** ; **VON HEHN CA** ; **ZHAO F** ; **TRAN J** ; **WAINGER B** ; **STROMINGER A** ; **MURALIDHARAN S** ; **HORSWILL AR**. Bacteria activate sensory neurons that modulate pain and inflammation.. *Nature*, 2013, vol. 501, 52-57
- **DE LA PENA JBI** ; **SONG JJ** ; **CAMPBELL ZT**. RNA control in pain: Blame it on the messenger. *Wiley Interdiscip Rev RNA*, 2019, vol. 10, e1546
- **DE WEERD NA** ; **NGUYEN T**. The interferons and their receptors--distribution and regulation.. *Immunol Cell Biol*, 2012, vol. 90, 483-491
- **FITZGIBBON M** ; **KERR DM** ; **HENRY RJ** ; **FINN DP** ; **ROCHE M**. Endocannabinoid modulation of inflammatory hyperalgesia in the IFN-alpha mouse model of depression.. *Brain Behav Immun*, 2019, vol. 82, 372-381
- **FORSTER S**. Interferon signatures in immune disorders and disease.. *Immunol Cell Biol*, 2012, vol. 90, 520-527
- **FOSTER SL** ; **SEEHUS CR** ; **WOOLF CJ** ; **TALBOT S**. Sense and Immunity: Context-Dependent Neuro-Immune Interplay.. *Front Immunol*, 2017, vol. 8, 1463
- **GAELINGS L et al.** Regulation of kynurenine biosynthesis during influenza virus infection.. *FEBS J*, 2017, vol. 284, 222-236
- **GERLACH N** ; **SCHIMMER S** ; **WEISS S** ; **KALINKE U** ; **DITTMER U**. Effects of type I interferons on Friend retrovirus infection.. *Journal of virology*, 2006, vol. 80, 3438-3444
- **HADLEY GR** ; **GAYLE JA** ; **RIPOLL J** ; **JONES MR** ; **ARGOFF CE** ; **KAYE RJ** ; **KAYE AD**. Post-herpetic Neuralgia: a Review.. *Curr Pain Headache Rep*, 2016, vol. 20, 17
- **HARGREAVES K** ; **DUBNER R** ; **BROWN F** ; **FLORES C** ; **JORIS J**. A new and sensitive method for measuring thermal nociception in cutaneous hyperalgesia.. *Pain*, 1988, vol. 32, 77-88
- **HILKENS CM** ; **SCHLAAK JF** ; **KERR IM**. Differential responses to IFN-alpha subtypes in human T cells and dendritic cells.. *J Immunol*, 2003, vol. 171, 5255-5263
- **HJORTSBERG L** ; **LINDVALL C** ; **CORCORAN M** ; **ARULAMPALAM V** ; **CHAN D** ; **THYRELL L** ; **NORDENSKJOLD M** ; **GRANDER D** ; **POKROVSKAJA K**. Phosphoinositide 3-kinase regulates a subset of interferon-alpha-stimulated genes.. *Exp Cell Res*, 2007, vol. 313, 404-414
- **HUANG L** ; **OU R** ; **RABELO DE SOUZA G** ; **CUNHA TM** ; **LEMOS H** ; **MOHAMED E** ; **LI L** ; **PACHOLCZYK G** ; **RANDALL J** ; **MUNN DH**. Virus Infections Incite Pain Hypersensitivity by Inducing Indoleamine 2,3 Dioxygenase.. *PLoS Pathog*, 2016, vol. 12, e1005615
- **INCEOGLU B** ; **BETTAIEB A** ; **TRINDADE DA SILVA CA** ; **LEE KS** ; **HAJ FG** ; **HAMMOCK BD**. Endoplasmic reticulum stress in the peripheral nervous system is a significant driver of neuropathic pain.. *Proc Natl Acad Sci U S A*, 2015, vol. 112, 9082-9087
- **IVASHKIV LB** ; **DONLIN LT**. Regulation of type I interferon responses.. *Nat Rev Immunol*, 2014, vol. 14, 36-49
- **JOSHI S** ; **KAUR S** ; **REDIG AJ** ; **GOLDSBOROUGH K** ; **DAVID K** ; **UEDA T** ; **WATANABE-FUKUNAGA R** ; **BAKER DP** ; **FISH EN** ; **FUKUNAGA R**. Type I interferon (IFN)-dependent activation of Mnk1 and its role in the generation of growth inhibitory responses.. *Proc Natl Acad Sci U S A*, 2009, vol. 106, 12097-12102
- **KAWAI T** ; **AKIRA S**. Toll-like receptor and RIG-I-like receptor signaling.. *Ann N Y Acad Sci*, 2008, vol. 1143, 1-20
- **KHOUTORSKY A** ; **SORGE RE** ; **PRAGER-KHOUTORSKY M** ; **PAWLOWSKI SA** ; **LONGO G** ; **JAFARNEJAD SM** ; **TAHMASEBI S** ; **MARTIN LJ** ; **PITCHER MH** ; **GKOGKAS CG**. eIF2alpha phosphorylation controls thermal nociception.. *Proc Natl Acad Sci U S A*, 2016, vol. 113, 11949-11954
- **LAI NY et al.** Gut-Innervating Nociceptor Neurons Regulate Peyer's Patch Microfold Cells and SFB Levels to Mediate Salmonella Host Defense.. *Cell*, 2019

- **LARNER AC** ; **CHAUDHURI A** ; **DARNELL JE, JR.** Transcriptional induction by interferon. New protein(s) determine the extent and length of the induction.. *J Biol Chem*, 1986, vol. 261, 453-459
- **LEVY DE** ; **DARNELL JE, JR.** Stats: transcriptional control and biological impact.. *Nat Rev Mol Cell Biol*, 2002, vol. 3, 651-662
- **LI CL** ; **LI KC** ; **WU D** ; **CHEN Y** ; **LUO H** ; **ZHAO JR** ; **WANG SS** ; **SUN MM** ; **LU YJ** ; **ZHONG YQ**. Somatosensory neuron types identified by high-coverage single-cell RNA-sequencing and functional heterogeneity.. *Cell research*, 2016, vol. 26, 83-102
- **LIU CC** ; **GAO YJ** ; **LUO H** ; **BERTA T** ; **XU ZZ** ; **JI RR** ; **TAN PH**. Interferon alpha inhibits spinal cord synaptic and nociceptive transmission via neuronal-glial interactions.. *Sci Rep*, 2016, vol. 6, 34356
- **LIU S** ; **KARAGANIS S** ; **MO RF** ; **LI XX** ; **WEN RX** ; **SONG XJ**. IFNbeta treatment inhibits nerve injury-induced mechanical allodynia and MAPK signaling by activating ISG15 in mouse spinal cord.. *J Pain*, 2019
- **LIU T** ; **GAO YJ** ; **JI RR**. Emerging role of Toll-like receptors in the control of pain and itch.. *Neurosci Bull*, 2012, vol. 28, 131-144
- **MALCANGIO M**. Role of the immune system in neuropathic pain.. *Scand J Pain*, 2019
- **MEGAT S** ; **RAY PR** ; **MOY JK** ; **LOU TF** ; **BARRAGAN-IGLESIAS P** ; **LI Y** ; **PRADHAN G** ; **WANGHZOU A** ; **AHMAD A** ; **BURTON MD**. Nociceptor Translational Profiling Reveals the Ragulator-Rag GTPase Complex as a Critical Generator of Neuropathic Pain.. *J Neurosci*, 2019, vol. 39, 393-411
- **MUNIR M** ; **BERG M**. The multiple faces of proteinkinase R in antiviral defense.. *Virulence*, 2013, vol. 4, 85-89
- **MURRAY C** ; **GRIFFIN EW** ; **O'LOUGHLIN E** ; **LYONS A** ; **SHERWIN E** ; **AHMED S** ; **STEVENSON NJ** ; **HARKIN A** ; **CUNNINGHAM C**. Interdependent and independent roles of type I interferons and IL-6 in innate immune, neuroinflammatory and sickness behaviour responses to systemic poly I:C.. *Brain Behav Immun*, 2015, vol. 48, 274-286
- **NOGUEIRA JB** ; **SENA LC** ; **QUINTANS JDE S** ; **ALMEIDA JR** ; **FRANCA AV** ; **JUNIOR LJ**. Side effects of the therapy with peginterferon and ribavirin in chronic hepatitis C: a small audit.. *J Pharm Pract*, 2012, vol. 25, 85-88
- **OBARA I** ; **HUNT SP**. Axonal protein synthesis and the regulation of primary afferent function.. *Dev Neurobiol*, 2014, vol. 74, 269-278
- **PINDEL A** ; **SADLER A**. The role of protein kinase R in the interferon response.. *J Interferon Cytokine Res*, 2011, vol. 31, 59-70
- **PINHO-RIBEIRO FA** ; **VERRI WA, JR.** ; **CHIU IM**. Nociceptor Sensory Neuron-Immune Interactions in Pain and Inflammation.. *Trends Immunol*, 2017, vol. 38, 5-19
- **PLATANIAS LC**. Mechanisms of type-I- and type-II-interferon-mediated signalling.. *Nat Rev Immunol*, 2005, vol. 5, 375-386
- **RODRIGUEZ Y** ; **VATTI N** ; **RAMIREZ-SANTANA C** ; **CHANG C** ; **MANCERA-PAEZ O** ; **GERSHWIN ME** ; **ANAYA JM**. Chronic inflammatory demyelinating polyneuropathy as an autoimmune disease.. *J Autoimmun*, 2019, vol. 102, 8-37
- **SALEIRO D** ; **MEHROTRA S** ; **KROCZYNSKA B** ; **BEAUCHAMP EM** ; **LISOWSKI P** ; **MAJCHRZAK-KITA B** ; **BHAGAT TD** ; **STEIN BL** ; **MCMAHON B** ; **ALTMAN JK**. Central role of ULK1 in type I interferon signaling.. *Cell*, 2015, vol. 11, 605-617
- **SCHREIBER G**. The molecular basis for differential type I interferon signaling.. *J Biol Chem*, 2017, vol. 292, 7285-7294
- **SEN GC** ; **SARKAR SN**. The interferon-stimulated genes: targets of direct signaling by interferons, double-stranded RNA, and viruses.. *Curr Top Microbiol Immunol*, 2007, vol. 316, 233-250
- **SHAKOOR A** ; **SHAFQAT F** ; **MEHMUD T** ; **AKRAM M** ; **RIAZ S** ; **IQBAL Z** ; **KHAN AA**. Frequency of depression and somatic symptoms in patients on interferon alpha/ribavirin for chronic hepatitis C.. *J Ayub Med Coll Abbottabad*, 2010, vol. 22, 6-9
- **SHIBAMIYA A** ; **HERSEMEYER K** ; **SCHMIDT WOLL T** ; **SEDDING D** ; **DANIEL JM** ; **BAUER S** ; **KOYAMA T** ; **PREISSNER KT** ; **KANSE SM**. A key role for Toll-like receptor-3 in disrupting the hemostasis balance on endothelial cells.. *Blood*, 2009, vol. 113, 714-722
- **SHIERS S** ; **MWIRIGI J** ; **PRADHAN G** ; **KUME M** ; **BLACK B** ; **BARRAGAN-IGLESIAS P** ; **MOY JK** ; **DUSSOR G** ; **PANCRAZIO JJ** ; **KROENER S**. Reversal of peripheral nerve injury-induced neuropathic pain and cognitive dysfunction via genetic and tomivosertib targeting of MNK.. *Neuropsychopharmacology*, 2019
- **STARK GR** ; **DARNELL JE, JR.** The JAK-STAT pathway at twenty.. *Immunity*, 2012, vol. 36, 503-514
- **THYRELL L** ; **HJORTSBERG L** ; **ARULAMPALAM V** ; **PANARETAKIS T** ; **UHLES S** ; **DAGNELL M** ; **ZHIVOTOVSKY B** ; **LEIBIGER I** ; **GRANDER D** ; **POKROVSKAJA K**. Interferon alpha-induced apoptosis in tumor cells is mediated through the phosphoinositide 3-kinase/mammalian target of rapamycin signaling pathway.. *J Biol Chem*, 2004, vol. 279, 24152-24162
- **VAN DER MAATEN L** ; **HINTON G**. Visualizing Data using t-SNE.. *Journal of Machine Learning Research*, 2008, vol. 9, 2579-2605

- **WALSH D** ; **MATHEWS MB** ; **MOHR I**. Tinkering with translation: protein synthesis in virus-infected cells.. *Cold Spring Harb Perspect Biol*, 2013, vol. 5, a012351
- **WASKIEWICZ AJ** ; **FLYNN A** ; **PROUD CG** ; **COOPER JA**. Mitogen-activated protein kinases activate the serine/threonine kinases Mnk1 and Mnk2.. *EMBO J*, 1997, vol. 16, 1909-1920
- **WASKIEWICZ AJ** ; **JOHNSON JC** ; **PENN B** ; **MAHALINGAM M** ; **KIMBALL SR** ; **COOPER JA**. Phosphorylation of the cap-binding protein eukaryotic translation initiation factor 4E by protein kinase Mnk1 in vivo.. *Mol Cell Biol*, 1999, vol. 19, 1871-1880
- **YAMAMOTO M** ; **SATO S** ; **HEMMI H** ; **HOSHINO K** ; **KAISHO T** ; **SANJO H** ; **TAKEUCHI O** ; **SUGIYAMA M** ; **OKABE M** ; **TAKEDA K**. Role of adaptor TRIF in the MyD88-independent toll-like receptor signaling pathway.. *Science*, 2003, vol. 301, 640-643